# EUROPEAN PATENT APPLICATION

(11) **EP 2 239 675 A1**
(43) Date of publication of application: **13.10.2010**
(21) Application number: 09157517.5
(22) Date of filing: 07.04.2009
(51) Int. Cl.: G06F 19/00

(54) **Method for in vitro diagnosing a complex disease**

(71) Applicant: BIOCRATES Life Sciences AG, 6020 Innsbruck (AT)
(72) Inventor: Deigner, Hans-Peter, 68623 Lampertheim (DE); Kohl, Matthias, 95326 Kulmbach (DE); Keller, Matthias, 45219 Essen (DE); Koal, Therese, 6020 Innsbruck (AT); Weinberger, Klaus, 6020 Innsbruck (AT)
(74) Representative: Winter, Brandl, Fürniss, Hübner, Röss, Kaiser, Polte - Partnerschaft

(57) **Abstract**

The present invention relates to a method and kit for in vitro diagnosing a complex disease such as cancer, in particular, acute myeloid leukemia (AML), colon cancer, kidney cancer, prostate cancer; transient ischemic attack (TIA), ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis, Alzheimer and Parkinson's disease; in a biological sample. For the diagnosis, use is made of measuring at least two different species of biomolecules and classifying the results by means of suitable classifier algorithms and other statistical procedures. With the present invention, a significant improvement of the reliability of, e.g. expression profiles alone, are achieved. In other words, in a defined collective, an up to 100% accurate positive diagnosis could be achieved, which renders the method of the present invention superior over the prior art.

SEQUENCE LISTING

<110>  BIOCRATES Life Sciences AG, Innrain 66/III, 6020 Innsbruck, AT

<120>  Method for in vitro Diagnosing a Complex Disease

<130>  BI6908

<160>  908

<170>  PatentIn version 3.3

<210>  1
<211>  40
<212>  DNA
<213>  Homo sapiens

<400>  1
tgctcatctg tgcacttctg ttcaacctat cacactgagt                              40

<210>  2
<211>  40
<212>  DNA
<213>  Homo sapiens

<400>  2
aaaccgtttt tcattattgc tcctgacccc ctctcatggg                              40

<210>  3
<211>  40
<212>  DNA
<213>  Homo sapiens

<400>  3
tgcacagggg accttaacca gatcattagt ttatatgcct                              40

<210>  4
<211>  40
<212>  DNA
<213>  Homo sapiens

<400>  4
cacacactcc agaacagatg gtatccagat gccttatggg                              40

<210>  5
<211>  40
<212>  DNA
<213>  Homo sapiens

<400>  5
gcgaaccatt tctaatgttc tgatttttca gagccagcca                              40

<210>  6
<211>  40
<212>  DNA
<213>  Homo sapiens

<400>  6
tgtgggatcc gtctcagtta ctttatagcc atacctggta                              40

<210>  7
<211>  40

<212>  DNA
<213>  Homo sapiens

<400>  7
agctgaatgg tgatggtgtg aagtataggt taaattgggt                              40


<210>  8
<211>  40
<212>  DNA
<213>  Homo sapiens

<400>  8
gtgcattgct gttgcattgc acgtgtgtga ggcgggtgca                              40


<210>  9
<211>  40
<212>  DNA
<213>  Homo sapiens

<400>  9
aaagctgtag ggcctccagg ttctcaagct gtgagtggaa                              40


<210>  10
<211>  40
<212>  DNA
<213>  Homo sapiens

<400>  10
tggttgacat atggctgcta atgccctcct ttctagtggg                              40


<210>  11
<211>  40
<212>  DNA
<213>  Homo sapiens

<400>  11
gtgtgcgtaa cggctggtgt gtttctctag ctgagctaat                              40


<210>  12
<211>  40
<212>  DNA
<213>  Homo sapiens

<400>  12
acctgctatg ccaacatatt gccatctttc ctgtctgaca                              40


<210>  13
<211>  40
<212>  DNA
<213>  Homo sapiens

<400>  13
acagtgagtg cgagtattat ttcttgccag cgggtggaag                              40


<210>  14
<211>  40
<212>  DNA
<213>  Homo sapiens

<400>  14
acactgctcg ctctatgtta attttagctc ttcccctgga                              40

<210>  15
<211>  73
<212>  RNA
<213>  Homo sapiens

<400>  15
cagggugugu gacugguuga ccagaggggc augcacugug uucacccugu gggccaccua      60

gucaccaacc cuc                                                          73


<210>  16
<211>  94
<212>  RNA
<213>  Homo sapiens

<400>  16
uguuuugagc gggggucaag agcaauaacg aaaaauguuu gucauaaacc guuuuucauu      60

auugcuccug accuccucuc auuugcuaua uuca                                  94


<210>  17
<211>  85
<212>  RNA
<213>  Homo sapiens

<400>  17
aggccucucu cuccguguuc acagcggacc uugauuuaaa uguccauaca auuaaggcac      60

gcggugaaug ccaagaaugg ggcug                                            85


<210>  18
<211>  95
<212>  RNA
<213>  Homo sapiens

<400>  18
uuguaccugg ugugauuaua aagcaaugag acugauuguc auaugucguu ugugggaucc      60

gucucaguua cuuuauagcc auaccuggua ucuua                                 95


<210>  19
<211>  99
<212>  RNA
<213>  Homo sapiens

<400>  19
cccuggcaug gugugguggg gcagcuggug uugugaauca ggccguugcc aaucagagaa      60

cggcuacuuc acaacaccag ggccacacca cacuacagg                             99


<210>  20
<211>  96
<212>  RNA
<213>  Homo sapiens

<400>  20
gcgggcggcc ccgcggugca uugcuguugc auugcacgug ugugaggcgg gugcagugcc      60

ucggcagugc agcccggagc cggccccugg caccac                                96

<210>  21
<211>  94
<212>  RNA
<213>  Homo sapiens

<400>  21
cggggcggcc gcucucccug uccuccagga gcucacgugu gccugccugu gagcgccucg      60

acgacagagc cggcgccugc cccagugucu gcgc                                  94


<210>  22
<211>  52
<212>  RNA
<213>  Homo sapiens

<400>  22
gcagcaagga aggcaggggu ccuaaggugu guccuccugc ccuccuugcu gu              52


<210>  23
<211>  110
<212>  RNA
<213>  Homo sapiens

<400>  23
ccuggccucc ugcagugcca cgcuccgugu auuugacaag cugaguugga cacuccaugu      60

gguagagugu caguuuguca aauaccccaa gugcggcaca ugcuuaccag                110


<210>  24
<211>  71
<212>  DNA
<213>  Homo sapiens

<400>  24
ggagaggagg caagaugcug gcauagcugu ugaacuggga accugcuaug ccaacauauu      60

gccaucuuuc c                                                           71


<210>  25
<211>  97
<212>  RNA
<213>  Homo sapiens

<400>  25
ccuagaaugu uauuaggucg gugcaaaagu aauugcgagu uuuaccauua cuuucaaugg      60

caaaacuggc aauuacuuuu gcaccaacgu aauacuu                               97


<210>  26
<211>  80
<212>  RNA
<213>  Homo sapiens

<400>  26
cucuaggaug ugcucauugc augggcugug uauaguauua uucaauaccc agagcaugca      60

gugugaacau aauagagauu                                                  80


<210>  27
<211>  22
<212>  DNA
<213>  Homo sapiens

<400>  27
atacatactt ctttacattc ca                                                22

<210>  28
<211>  22
<212>  DNA
<213>  Homo sapiens

<400>  28
acacaaattc ggttctacag gg                                                22

<210>  29
<211>  21
<212>  DNA
<213>  Homo sapiens

<400>  29
gccaatattt ctgtgctgct a                                                 21

<210>  30
<211>  22
<212>  DNA
<213>  Homo sapiens

<400>  30
acagctggtt gaaggggacc aa                                                22

<210>  31
<211>  22
<212>  DNA
<213>  Homo sapiens

<400>  31
cacataggaa tgaaaagcca ta                                                22

<210>  32
<211>  22
<212>  DNA
<213>  Homo sapiens

<400>  32
tgtgagttct accattgcca aa                                                22

<210>  33
<211>  20
<212>  DNA
<213>  Homo sapiens

<400>  33
tccagtcaag gatgtttaca                                                   20

<210>  34
<211>  21
<212>  DNA
<213>  Homo sapiens

<400>  34
cacaagatcg gatctacggg t                                                 21

<210>  35
<211>  85
<212>  RNA
<213>  Homo sapiens

<400>  35
accuacucag aguacauacu ucuuuaugua cccauaugaa cauacaaugc uauggaaugu      60

aaagaaguau guauuuuugg uaggc                                            85


<210>  36
<211>  110
<212>  RNA
<213>  Homo sapiens

<400>  36
ccagagguug uaacguuguc uauauauacc cuguagaacc gaauuugugu gguauccgua      60

uagucacaga uucgauucua ggggaauaua uggucgaugc aaaaacuuca                110


<210>  37
<211>  87
<212>  RNA
<213>  Homo sapiens

<400>  37
agcuucccug gcucuagcag cacagaaaua uuggcacagg gaagcgaguc ugccaauauu      60

ggcugugcug cuccaggcag gguggug                                          87


<210>  38
<211>  88
<212>  RNA
<213>  Homo sapiens

<400>  38
acaaugcuuu gcuagagcug guaaaaugga accaaaucgc cucuucaaug gauuuggucc      60

ccuucaacca gcuguagcua ugcauuga                                         88


<210>  39
<211>  97
<212>  RNA
<213>  Homo sapiens

<400>  39
cacucugcug uggccuaugg cuuuucauuc cuaugugauu gcugucccaa acucauguag      60

ggcuaaaagc caugggcuac agugaggggc gagcucc                               97


<210>  40
<211>  110
<212>  RNA
<213>  Homo sapiens

<400>  40
gagcugcuug ccuccccccg uuuuuggcaa ugguagaacu cacacuggug agguaacagg      60

auccgguggu ucuagacuug ccaacuaugg ggcgaggacu cagccggcac                110


<210>  41
<211>  92

<212>  RNA
<213>  Homo sapiens

<400>  41
gggcagucuu ugcuacugua aacauccuug acuggaagcu guaagguguu cagaggagcu      60

uucagucgga uguuuacagc ggcaggcugc ca                                    92


<210>  42
<211>  81
<212>  RNA
<213>  Homo sapiens

<400>  42
cccauuggca uaaacccgua gauccgaucu uguggugaag uggaccgcac aagcucgcuu      60

cuaugggucu gugucagugu g                                                81


<210>  43
<211>  25
<212>  DNA
<213>  Homo sapiens

<400>  43
aagatcattg ctcctcctga gcgca                                            25


<210>  44
<211>  25
<212>  DNA
<213>  Homo sapiens

<400>  44
cctcctgagc gcaagtactc cgtgt                                            25


<210>  45
<211>  25
<212>  DNA
<213>  Homo sapiens

<400>  45
tccgtgtgga tcggcggctc catcc                                            25


<210>  46
<211>  25
<212>  DNA
<213>  Homo sapiens

<400>  46
cagatgtgga tcagcaagca ggagt                                            25


<210>  47
<211>  25
<212>  DNA
<213>  Homo sapiens

<400>  47
gtccaccgca aatgcttcta ggcgg                                            25


<210>  48
<211>  25
<212>  DNA

<213>  Homo sapiens

<400>  48
accacggccg agcgggaaat cgtgc                                          25


<210>  49
<211>  25
<212>  DNA
<213>  Homo sapiens

<400>  49
ctgtgctacg tcgccctgga cttcg                                          25


<210>  50
<211>  25
<212>  DNA
<213>  Homo sapiens

<400>  50
gagcaagaga tggccacggc tgctt                                          25


<210>  51
<211>  25
<212>  DNA
<213>  Homo sapiens

<400>  51
tcctccctgg agaagagcta cgagc                                          25


<210>  52
<211>  25
<212>  DNA
<213>  Homo sapiens

<400>  52
ctgcctgacg gccaggtcat cacca                                          25


<210>  53
<211>  25
<212>  DNA
<213>  Homo sapiens

<400>  53
caggtcatca ccattggcaa tgagc                                          25


<210>  54
<211>  25
<212>  DNA
<213>  Homo sapiens

<400>  54
cggttccgct gccctgaggc actct                                          25


<210>  55
<211>  25
<212>  DNA
<213>  Homo sapiens

<400>  55
cctgaggcac tcttccagcc ttcct                                          25

<210>  56
<211>  25
<212>  DNA
<213>  Homo sapiens

<400>  56
gagtcctgtg gcatccacga aacta                                          25


<210>  57
<211>  25
<212>  DNA
<213>  Homo sapiens

<400>  57
atccacgaaa ctaccttcaa ctcca                                          25


<210>  58
<211>  25
<212>  DNA
<213>  Homo sapiens

<400>  58
aactccatca tgaagtgtga cgtgg                                          25


<210>  59
<211>  25
<212>  DNA
<213>  Homo sapiens

<400>  59
gacatccgca aagacctgta cgcca                                          25


<210>  60
<211>  25
<212>  DNA
<213>  Homo sapiens

<400>  60
aacacagtgc tgtctggcgg cacca                                          25


<210>  61
<211>  25
<212>  DNA
<213>  Homo sapiens

<400>  61
accatgtacc ctggcattgc cgaca                                          25


<210>  62
<211>  25
<212>  DNA
<213>  Homo sapiens

<400>  62
cagaaggaga tcactgccct ggcac                                          25


<210>  63
<211>  25
<212>  DNA
<213>  Homo sapiens

<400>  63
agattcgggc aagtccacca ctact                                          25


<210>  64
<211>  25
<212>  DNA
<213>  Homo sapiens

<400>  64
ttcgggcaag tccaccacta ctggc                                          25


<210>  65
<211>  25
<212>  DNA
<213>  Homo sapiens

<400>  65
caccactact ggccatctga tctat                                          25


<210>  66
<211>  25
<212>  DNA
<213>  Homo sapiens

<400>  66
ccatctgatc tataaatgcg gtggc                                          25


<210>  67
<211>  25
<212>  DNA
<213>  Homo sapiens

<400>  67
tctgatctat aaatgcggtg gcatc                                          25


<210>  68
<211>  25
<212>  DNA
<213>  Homo sapiens

<400>  68
tgcctgggtc ttggataaac tgaaa                                          25


<210>  69
<211>  25
<212>  DNA
<213>  Homo sapiens

<400>  69
tgaaagctga gcgtgaacgt ggtat                                          25


<210>  70
<211>  25
<212>  DNA
<213>  Homo sapiens

<400>  70
cgtgaacgtg gtatcaccat tgata                                          25

<210>  71
<211>  25
<212>  DNA
<213>  Homo sapiens

<400>  71
gaacgtggta tcaccattga tatct                                          25


<210>  72
<211>  25
<212>  DNA
<213>  Homo sapiens

<400>  72
gtggtatcac cattgatatc tcctt                                          25


<210>  73
<211>  25
<212>  DNA
<213>  Homo sapiens

<400>  73
tatcaccatt gatatctcct tgtgg                                          25


<210>  74
<211>  25
<212>  DNA
<213>  Homo sapiens

<400>  74
ccattgatat ctccttgtgg aaatt                                          25


<210>  75
<211>  25
<212>  DNA
<213>  Homo sapiens

<400>  75
gtactatgtg actatcattg atgcc                                          25


<210>  76
<211>  25
<212>  DNA
<213>  Homo sapiens

<400>  76
ctatgtgact atcattgatg cccca                                          25


<210>  77
<211>  25
<212>  DNA
<213>  Homo sapiens

<400>  77
ctcatatcaa cattgtcgtc attgg                                          25


<210>  78
<211>  25
<212>  DNA
<213>  Homo sapiens

<400>  78
tatcaacatt gtcgtcattg gacac                                              25


<210>  79
<211>  25
<212>  DNA
<213>  Homo sapiens

<400>  79
cattgtcgtc attggacacg tagat                                              25


<210>  80
<211>  25
<212>  DNA
<213>  Homo sapiens

<400>  80
tgtcgtcatt ggacacgtag attcg                                              25


<210>  81
<211>  25
<212>  DNA
<213>  Homo sapiens

<400>  81
cgtcattgga cacgtagatt cgggc                                              25


<210>  82
<211>  25
<212>  DNA
<213>  Homo sapiens

<400>  82
gggtcagaag gattcctatg tgggc                                              25


<210>  83
<211>  25
<212>  DNA
<213>  Homo sapiens

<400>  83
gaaggattcc tatgtgggcg acgag                                              25


<210>  84
<211>  25
<212>  DNA
<213>  Homo sapiens

<400>  84
ccccatcgag cacggcatcg tcacc                                              25


<210>  85
<211>  25
<212>  DNA
<213>  Homo sapiens

<400>  85
cgtcaccaac tgggacgaca tggag                                              25


<210>  86

<211>  25
<212>  DNA
<213>  Homo sapiens

<400>  86
caccttctac aatgagctgc gtgtg                                          25


<210>  87
<211>  25
<212>  DNA
<213>  Homo sapiens

<400>  87
tcccgaggag caccccgtgc tgctg                                          25


<210>  88
<211>  25
<212>  DNA
<213>  Homo sapiens

<400>  88
ggccaaccgc gagaagatga cccag                                          25


<210>  89
<211>  25
<212>  DNA
<213>  Homo sapiens

<400>  89
ccagatcatg tttgagacct tcaac                                          25


<210>  90
<211>  25
<212>  DNA
<213>  Homo sapiens

<400>  90
cccagccatg tacgttgcta tccag                                          25


<210>  91
<211>  25
<212>  DNA
<213>  Homo sapiens

<400>  91
cgttgctatc caggctgtgc tatcc                                          25


<210>  92
<211>  25
<212>  DNA
<213>  Homo sampiens

<400>  92
ggctgtgcta tccctgtacg cctct                                          25


<210>  93
<211>  25
<212>  DNA
<213>  Homo sapiens

<400>  93

cgcctctggc cgtaccactg gcatc 25

<210> 94
<211> 25
<212> DNA
<213> Homo sapiens

<400> 94
taccactggc atcgtgatgg actcc 25

<210> 95
<211> 25
<212> DNA
<213> Homo sapiens

<400> 95
cggtgacggg gtcacccaca ctgtg 25

<210> 96
<211> 25
<212> DNA
<213> Homo sapiens

<400> 96
ccacactgtg cccatctacg agggg 25

<210> 97
<211> 25
<212> DNA
<213> Homo sapiens

<400> 97
gcccatctac gaggggtatg ccctc 25

<210> 98
<211> 25
<212> DNA
<213> Homo sapiens

<400> 98
tgccatcctg cgtctggacc tggct 25

<210> 99
<211> 25
<212> DNA
<213> Homo sapiens

<400> 99
tgatatcgcc gcgctcgtcg tcgac 25

<210> 100
<211> 25
<212> DNA
<213> Homo sapiens

<400> 100
cgtcgtcgac aacggctccg gcatg 25

<210> 101
<211> 25

<212>  DNA
<213>  Homo sapiens

<400>  101
cggctccggc atgtgcaagg ccggc                                          25


<210>  102
<211>  25
<212>  DNA
<213>  Homo sapiens

<400>  102
accctcctaa tagtcatact agtag                                          25


<210>  103
<211>  25
<212>  DNA
<213>  Homo sapiens

<400>  103
ctaatagtca tactagtagt catac                                          25


<210>  104
<211>  25
<212>  DNA
<213>  Homo sapiens

<400>  104
gtcatactag tagtcatact ccctg                                          25


<210>  105
<211>  25
<212>  DNA
<213>  Homo sapiens

<400>  105
ctagtagtca tactccctgg tgtag                                          25


<210>  106
<211>  25
<212>  DNA
<213>  Homo sapiens

<400>  106
atgcagccag ccatcaaata gtgaa                                          25


<210>  107
<211>  25
<212>  DNA
<213>  Homo sapiens

<400>  107
tagtgaatgg tctctctttg gctgg                                          25


<210>  108
<211>  25
<212>  DNA
<213>  Homo sapiens

<400>  108
taacccatga aggataaaag cccca                                          25

<210>  109
<211>  25
<212>  DNA
<213>  Homo sapiens

<400>  109
atagcactaa tgctttaaga tttgg                                              25


<210>  110
<211>  25
<212>  DNA
<213>  Homo sapiens

<400>  110
ctttaagatt tggtcacact ctcac                                              25


<210>  111
<211>  25
<212>  DNA
<213>  Homo sapiens

<400>  111
gatttggtca cactctcacc taggt                                              25


<210>  112
<211>  25
<212>  DNA
<213>  Homo sapiens

<400>  112
cattgagcca gtggtgctaa atgct                                              25


<210>  113
<211>  25
<212>  DNA
<213>  Homo sapiens

<400>  113
ggtgctaaat gctacatact ccaac                                              25


<210>  114
<211>  25
<212>  DNA
<213>  Homo sapiens

<400>  114
tacatactcc aactgaaatg ttaag                                              25


<210>  115
<211>  25
<212>  DNA
<213>  Homo sapiens

<400>  115
ctccaactga aatgttaagg aagaa                                              25


<210>  116
<211>  25
<212>  DNA

<213>  Homo sapiens

<400>  116
aacacaggag attccagtct acttg                                          25


<210>  117
<211>  25
<212>  DNA
<213>  Homo sapiens

<400>  117
gcataataca gaagtcccct ctact                                          25


<210>  118
<211>  25
<212>  DNA
<213>  Homo sapiens

<400>  118
gtaacctgaa ctaatctgat gttaa                                          25


<210>  119
<211>  25
<212>  DNA
<213>  Homo sapiens

<400>  119
aatctgatgt taaccaatgt attta                                          25


<210>  120
<211>  25
<212>  DNA
<213>  Homo sapiens

<400>  120
ctgtttcctt gttccaattt gacaa                                          25


<210>  121
<211>  25
<212>  DNA
<213>  Homo sapiens

<400>  121
gctatcactg tacttgtaga gtggt                                          25


<210>  122
<211>  25
<212>  DNA
<213>  Homo sapiens

<400>  122
gcgcctggtc accagggctg ctttt                                          25


<210>  123
<211>  25
<212>  DNA
<213>  Homo sapiens

<400>  123
ggtcaccagg gctgctttta actct                                          25

<210>  124
<211>  25
<212>  DNA
<213>  Homo sapiens

<400>  124
tgcttttaac tctggtaaag tggat                                              25


<210>  125
<211>  25
<212>  DNA
<213>  Homo sapiens

<400>  125
ggatattgtt gccatcaatg acccc                                              25


<210>  126
<211>  25
<212>  DNA
<213>  Homo sapiens

<400>  126
catcaatgac cccttcattg acctc                                              25


<210>  127
<211>  25
<212>  DNA
<213>  Homo sapiens

<400>  127
cttcattgac ctcaactaca tggtt                                              25


<210>  128
<211>  25
<212>  DNA
<213>  Homo sapiens

<400>  128
caactacatg gtttacatgt tccaa                                              25


<210>  129
<211>  25
<212>  DNA
<213>  Homo sapiens

<400>  129
ggtttacatg ttccaatatg attcc                                              25


<210>  130
<211>  25
<212>  DNA
<213>  Homo sapiens

<400>  130
ccaatatgat tccacccatg gcaaa                                              25


<210>  131
<211>  25
<212>  DNA
<213>  Homo sapiens

<400>  131
tgattccacc catggcaaat tccat                                              25


<210>  132
<211>  25
<212>  DNA
<213>  Homo sapiens

<400>  132
attccatggc accgtcaagg ctgag                                              25


<210>  133
<211>  25
<212>  DNA
<213>  Homo sapiens

<400>  133
tggcaccgtc aaggctgaga acggg                                              25


<210>  134
<211>  25
<212>  DNA
<213>  Homo sapiens

<400>  134
catcaatgga aatcccatca ccatc                                              25


<210>  135
<211>  25
<212>  DNA
<213>  Homo sapiens

<400>  135
tcccatcacc atcttccagg agcga                                              25


<210>  136
<211>  25
<212>  DNA
<213>  Homo sapiens

<400>  136
cttccaggag cgagatccct ccaaa                                              25


<210>  137
<211>  25
<212>  DNA
<213>  Homo sapiens

<400>  137
gcgagatccc tccaaaatca agtgg                                              25


<210>  138
<211>  25
<212>  DNA
<213>  Homo sapiens

<400>  138
cgatgctggc gctgagtacg tcgtg                                              25

<210>  139
<211>  25
<212>  DNA
<213>  Homo sapiens

<400>  139
cgtggagtcc actggcgtct tcacc                                          25


<210>  140
<211>  25
<212>  DNA
<213>  Homo sapiens

<400>  140
cttcaccacc atggagaagg ctggg                                          25


<210>  141
<211>  25
<212>  DNA
<213>  Homo sapiens

<400>  141
cggatttggt cgtattgggc gcctg                                          25


<210>  142
<211>  25
<212>  DNA
<213>  Homo sapiens

<400>  142
tcctcctgag cgcaagtact ccgtg                                          25


<210>  143
<211>  25
<212>  DNA
<213>  Homo sapiens

<400>  143
tgagcgcaag tactccgtgt ggatc                                          25


<210>  144
<211>  25
<212>  DNA
<213>  Homo sapiens

<400>  144
cttccagcag atgtggatca gcaag                                          25


<210>  145
<211>  25
<212>  DNA
<213>  Homo sapiens

<400>  145
gtggatcagc aagcaggagt atgac                                          25


<210>  146
<211>  25
<212>  DNA
<213>  Homo sapiens

<400>  146
ccgcaaatgc ttctaggcgg actat                                              25


<210>  147
<211>  25
<212>  DNA
<213>  Homo sapiens

<400>  147
atgcttctag gcggactatg actta                                              25


<210>  148
<211>  25
<212>  DNA
<213>  Homo sapiens

<400>  148
taacttgcgc agaaaacaag atgag                                              25


<210>  149
<211>  25
<212>  DNA
<213>  Homo sapiens

<400>  149
cagcagtcgg ttggagcgag catcc                                              25


<210>  150
<211>  25
<212>  DNA
<213>  Homo sapiens

<400>  150
caatgtggcc gaggactttg attgc                                              25


<210>  151
<211>  25
<212>  DNA
<213>  Homo sapiens

<400>  151
ggccgaggac tttgattgca cattg                                              25


<210>  152
<211>  25
<212>  DNA
<213>  Homo sapiens

<400>  152
tgacgtggac atccgcaaag acctg                                              25


<210>  153
<211>  25
<212>  DNA
<213>  Homo sapiens

<400>  153
gtacgccaac acagtgctgt ctggc                                              25


<210>  154

<211>  25
<212>  DNA
<213>  Homo sapiens

<400>  154
caacacagtg ctgtctggcg gcacc                                              25


<210>  155
<211>  25
<212>  DNA
<213>  Homo sapiens

<400>  155
gtctggcggc accaccatgt accct                                              25


<210>  156
<211>  25
<212>  DNA
<213>  Homo sapiens

<400>  156
caccatgtac cctggcattg ccgac                                              25


<210>  157
<211>  25
<212>  DNA
<213>  Homo sapiens

<400>  157
gtaccctggc attgccgaca ggatg                                              25


<210>  158
<211>  25
<212>  DNA
<213>  Homo sapiens

<400>  158
tgccgacagg atgcagaagg agatc                                              25


<210>  159
<211>  25
<212>  DNA
<213>  Homo sapiens

<400>  159
ggagatcact gccctggcac ccagc                                              25


<210>  160
<211>  25
<212>  DNA
<213>  Homo sapiens

<400>  160
cctggcaccc agcacaatga agatc                                              25


<210>  161
<211>  25
<212>  DNA
<213>  Homo sapiens

<400>  161

acccagcaca atgaagatca agatc 25

<210> 162
<211> 25
<212> DNA
<213> Homo sapiens

<400> 162
tgaagcacta caggaggaat gcacc 25

<210> 163
<211> 25
<212> DNA
<213> Homo sapiens

<400> 163
agctctccgc caatttctct cagat 25

<210> 164
<211> 25
<212> DNA
<213> Homo sapiens

<400> 164
aatgtacatg ggccgcacca taatg 25

<210> 165
<211> 25
<212> DNA
<213> Homo sapiens

<400> 165
catgggccgc accataatga gatgt 25

<210> 166
<211> 25
<212> DNA
<213> Homo sapiens

<400> 166
ccgcaccata atgagatgtg agcct 25

<210> 167
<211> 25
<212> DNA
<213> Homo sapiens

<400> 167
tggctgttaa cccactgcat gcaga 25

<210> 168
<211> 25
<212> DNA
<213> Homo sapiens

<400> 168
ttaacccact gcatgcagaa acttg 25

<210> 169
<211> 25

<212>  DNA
<213>  Homo sapiens

<400>  169
cactgcatgc agaaacttgg atgtc                                          25


<210>  170
<211>  25
<212>  DNA
<213>  Homo sapiens

<400>  170
tggaattgac tgcctatgcc aagtc                                          25


<210>  171
<211>  25
<212>  DNA
<213>  Homo sapiens

<400>  171
tgactgccta tgccaagtcc ctgga                                          25


<210>  172
<211>  25
<212>  DNA
<213>  Homo sapiens

<400>  172
ctcataaaac atgaatcaag caatc                                          25


<210>  173
<211>  25
<212>  DNA
<213>  Homo sapiens

<400>  173
gaatcaagca atccagcctc atggg                                          25


<210>  174
<211>  25
<212>  DNA
<213>  Homo sapiens

<400>  174
ttgtaaagcc cttgcacagc tggag                                          25


<210>  175
<211>  25
<212>  DNA
<213>  Homo sapiens

<400>  175
tgcacagctg gagaaatggc atcat                                          25


<210>  176
<211>  25
<212>  DNA
<213>  Homo sapiens

<400>  176
gcatcattat aagctatgag ttgaa                                          25

<210>  177
<211>  25
<212>  DNA
<213>  Homo sapiens

<400>  177
aatgttctgt caaatgtgtc tcaca                                          25


<210>  178
<211>  25
<212>  DNA
<213>  Homo sapiens

<400>  178
aatgtgtctc acatctacac gtggc                                          25


<210>  179
<211>  25
<212>  DNA
<213>  Homo sapiens

<400>  179
tctcacatct acacgtggct tggag                                          25


<210>  180
<211>  25
<212>  DNA
<213>  Homo sapiens

<400>  180
ttccctattg tgacagagcc atggt                                          25


<210>  181
<211>  25
<212>  DNA
<213>  Homo sapiens

<400>  181
attgtgacag agccatggtg tgttt                                          25


<210>  182
<211>  25
<212>  DNA
<213>  Homo sapiens

<400>  182
ttctccctgc actcatgaaa cccca                                          25


<210>  183
<211>  25
<212>  DNA
<213>  Homo sapiens

<400>  183
tctccctgca ctcatgaaac cccaa                                          25


<210>  184
<211>  25
<212>  DNA

<213>  Homo sapiens

<400>  184
gcactcatga aaccccaata aatat                                          25


<210>  185
<211>  25
<212>  DNA
<213>  Homo sapiens

<400>  185
cactcatgaa accccaataa atatc                                          25


<210>  186
<211>  25
<212>  DNA
<213>  Homo sapiens

<400>  186
actcatgaaa ccccaataaa tatcc                                          25


<210>  187
<211>  25
<212>  DNA
<213>  Homo sapiens

<400>  187
ctcatgaaac cccaataaat atcct                                          25


<210>  188
<211>  25
<212>  DNA
<213>  Homo sapiens

<400>  188
tcatgaaacc ccaataaata tcctc                                          25


<210>  189
<211>  25
<212>  DNA
<213>  Homo sapiens

<400>  189
catgaaaccc caataaatat cctca                                          25


<210>  190
<211>  25
<212>  DNA
<213>  Homo sapiens

<400>  190
atgaaacccc aataaatatc ctcat                                          25


<210>  191
<211>  25
<212>  DNA
<213>  Homo sapiens

<400>  191
aaaccccaat aaatatcctc attga                                          25

<210>  192
<211>  25
<212>  DNA
<213>  Homo sapiens

<400>  192
aaccccaata aatatcctca ttgac                                          25


<210>  193
<211>  25
<212>  DNA
<213>  Homo sapiens

<400>  193
ggctgtccta gcagttgtgg tcatc                                          25


<210>  194
<211>  25
<212>  DNA
<213>  Homo sapiens

<400>  194
ctgtcctagc agttgtggtc atcgg                                          25


<210>  195
<211>  25
<212>  DNA
<213>  Homo sapiens

<400>  195
tgtcctagca gttgtggtca tcgga                                          25


<210>  196
<211>  25
<212>  DNA
<213>  Homo sapiens

<400>  196
gtcctagcag ttgtggtcat cggag                                          25


<210>  197
<211>  25
<212>  DNA
<213>  Homo sapiens

<400>  197
tcctagcagt tgtggtcatc ggagc                                          25


<210>  198
<211>  25
<212>  DNA
<213>  Homo sapiens

<400>  198
ctagcagttg tggtcatcgg agctg                                          25


<210>  199
<211>  25
<212>  DNA
<213>  Homo sapiens

<400>  199
tagcagttgt ggtcatcgga gctgt                                          25


<210>  200
<211>  25
<212>  DNA
<213>  Homo sapiens

<400>  200
tttattggca tggagtccgc tggaa                                          25


<210>  201
<211>  25
<212>  DNA
<213>  Homo sapiens

<400>  201
attggcatgg agtccgctgg aattc                                          25


<210>  202
<211>  25
<212>  DNA
<213>  Homo sapiens

<400>  202
tccgctggaa ttcatgagac aacct                                          25


<210>  203
<211>  25
<212>  DNA
<213>  Homo sapiens

<400>  203
ggaattcatg agacaaccta caatt                                          25


<210>  204
<211>  25
<212>  DNA
<213>  Homo sapiens

<400>  204
attcatgaga caacctacaa ttcca                                          25


<210>  205
<211>  25
<212>  DNA
<213>  Homo sapiens

<400>  205
cacaggaagt gcttctaaag tcaga                                          25


<210>  206
<211>  25
<212>  DNA
<213>  Homo sapiens

<400>  206
aagtgcttct aaagtcagaa caggt                                          25

<210>  207
<211>  25
<212>  DNA
<213>  Homo sapiens

<400>  207
tgcttctaaa gtcagaacag gttct                                          25


<210>  208
<211>  25
<212>  DNA
<213>  Homo sapiens

<400>  208
agtcagaaca ggttctccaa ggatc                                          25


<210>  209
<211>  25
<212>  DNA
<213>  Homo sapiens

<400>  209
aacaggttct ccaaggatcc cctcg                                          25


<210>  210
<211>  25
<212>  DNA
<213>  Homo sapiens

<400>  210
ttctccaagg atcccctcga gacta                                          25


<210>  211
<211>  25
<212>  DNA
<213>  Homo sapiens

<400>  211
tccaaggatc ccctcgagac tactc                                          25


<210>  212
<211>  25
<212>  DNA
<213>  Homo sapiens

<400>  212
gatcccctcg agactactct gttac                                          25


<210>  213
<211>  25
<212>  DNA
<213>  Homo sapiens

<400>  213
ctcgagacta ctctgttacc agtca                                          25


<210>  214
<211>  25
<212>  DNA
<213>  Homo sapiens

<400>  214
gagactactc tgttaccagt catga                                              25


<210>  215
<211>  25
<212>  DNA
<213>  Homo sapiens

<400>  215
actactctgt taccagtcat gaaac                                              25


<210>  216
<211>  25
<212>  DNA
<213>  Homo sapiens

<400>  216
actctgttac cagtcatgaa acatt                                              25


<210>  217
<211>  25
<212>  DNA
<213>  Homo sapiens

<400>  217
ctgttaccag tcatgaaaca ttaaa                                              25


<210>  218
<211>  25
<212>  DNA
<213>  Homo sapiens

<400>  218
ttaccagtca tgaaacatta aaacc                                              25


<210>  219
<211>  25
<212>  DNA
<213>  Homo sapiens

<400>  219
atgaaacatt aaaacctaca agcct                                              25


<210>  220
<211>  25
<212>  DNA
<213>  Homo sapiens

<400>  220
ggtttgcctg aggctgtaac tgaga                                              25


<210>  221
<211>  25
<212>  DNA
<213>  Homo sapiens

<400>  221
cctgaggctg taactgagag aaaga                                              25


<210>  222

<211>  25
<212>  DNA
<213>  Homo sapiens

<400>  222
attctggggc tgtcttatga aaata                                              25


<210>  223
<211>  25
<212>  DNA
<213>  Homo sapiens

<400>  223
atagacattc tcacataagc ccagt                                              25


<210>  224
<211>  25
<212>  DNA
<213>  Homo sapiens

<400>  224
acataagccc agttcatcac cattt                                              25


<210>  225
<211>  25
<212>  DNA
<213>  Homo sapiens

<400>  225
tcacattagg ctgttggttc aaact                                              25


<210>  226
<211>  25
<212>  DNA
<213>  Homo sapiens

<400>  226
gagcacggac tgtcagttct ctggg                                              25


<210>  227
<211>  25
<212>  DNA
<213>  Homo sapiens

<400>  227
ggactgtcag ttctctggga agtgg                                              25


<210>  228
<211>  25
<212>  DNA
<213>  Homo sapiens

<400>  228
gaagtggtca gcgcatcctg caggg                                              25


<210>  229
<211>  25
<212>  DNA
<213>  Homo sapiens

<400>  229

gtcagcgcat cctgcagggc ttctc                                          25

<210>  230
<211>  25
<212>  DNA
<213>  Homo sapiens

<400>  230
tttggagaac cagggctctt ctcag                                          25

<210>  231
<211>  25
<212>  DNA
<213>  Homo sapiens

<400>  231
gaaccagggc tcttctcagg ggctc                                          25

<210>  232
<211>  25
<212>  DNA
<213>  Homo sapiens

<400>  232
ttctcagggg ctctagggac tgcca                                          25

<210>  233
<211>  25
<212>  DNA
<213>  Homo sapiens

<400>  233
ctagggactg ccaggctgtt tcagc                                          25

<210>  234
<211>  25
<212>  DNA
<213>  Homo sapiens

<400>  234
tttcagccag gaaggccaaa atcaa                                          25

<210>  235
<211>  25
<212>  DNA
<213>  Homo sapiens

<400>  235
gggatggtcg gatctcacag gctga                                          25

<210>  236
<211>  25
<212>  DNA
<213>  Homo sapiens

<400>  236
gtcggatctc acaggctgag aactc                                          25

<210>  237
<211>  25

<212>  DNA
<213>  Homo sapiens

<400>  237
tctcacaggc tgagaactcg ttcac                                              25


<210>  238
<211>  25
<212>  DNA
<213>  Homo sapiens

<400>  238
cctccaagca tttcatgaaa aagct                                              25


<210>  239
<211>  25
<212>  DNA
<213>  Homo sapiens

<400>  239
agcatttcat gaaaaagctg cttct                                              25


<210>  240
<211>  25
<212>  DNA
<213>  Homo sapiens

<400>  240
caggatctgg gcccagtccc catgt                                              25


<210>  241
<211>  25
<212>  DNA
<213>  Homo sapiens

<400>  241
ggcccagtcc ccatgtgaga gcagc                                              25


<210>  242
<211>  25
<212>  DNA
<213>  Homo sapiens

<400>  242
cccatgtgag agcagcagag gcggt                                              25


<210>  243
<211>  25
<212>  DNA
<213>  Homo sapiens

<400>  243
agagcagcag aggcggtctt caaca                                              25


<210>  244
<211>  25
<212>  DNA
<213>  Homo sapiens

<400>  244
acacagctac agctttcttg ctccc                                              25

<210>  245
<211>  25
<212>  DNA
<213>  Homo sapiens

<400>  245
caagacaaac caagtcggaa cagca                                          25


<210>  246
<211>  25
<212>  DNA
<213>  Homo sapiens

<400>  246
caagtcggaa cagcagataa caatg                                          25


<210>  247
<211>  25
<212>  DNA
<213>  Homo sapiens

<400>  247
tgcccaatct ccatctgtca acagg                                          25


<210>  248
<211>  25
<212>  DNA
<213>  Homo sapiens

<400>  248
tgaggtccca ggaagtggcc aaaag                                          25


<210>  249
<211>  25
<212>  DNA
<213>  Homo sapiens

<400>  249
agctagacag atccccgttc ctgac                                          25


<210>  250
<211>  25
<212>  DNA
<213>  Homo sapiens

<400>  250
gacatcacag cagcctccaa cacaa                                          25


<210>  251
<211>  25
<212>  DNA
<213>  Homo sapiens

<400>  251
caacacaagg ctccaagacc taggc                                          25


<210>  252
<211>  25
<212>  DNA

<213>  Homo sapiens

<400>  252
aagacctagg ctcatggacg agatg                                          25


<210>  253
<211>  25
<212>  DNA
<213>  Homo sapiens

<400>  253
ccagacccca ggctggacat gctga                                          25


<210>  254
<211>  25
<212>  DNA
<213>  Homo sapiens

<400>  254
cctttggcct tggcttttct agcct                                          25


<210>  255
<211>  25
<212>  DNA
<213>  Homo sapiens

<400>  255
ttggcttttc tagcctattt acctg                                          25


<210>  256
<211>  25
<212>  DNA
<213>  Homo sapiens

<400>  256
agcctattta cctgcaggct gagcc                                          25


<210>  257
<211>  25
<212>  DNA
<213>  Homo sapiens

<400>  257
gctcagccaa gcttgttatc agctt                                          25


<210>  258
<211>  25
<212>  DNA
<213>  Homo sapiens

<400>  258
aagcttgtta tcagctttca gggcc                                          25


<210>  259
<211>  25
<212>  DNA
<213>  Homo sapiens

<400>  259
atcagctttc agggccatgg ttcac                                          25

<210>  260
<211>  25
<212>  DNA
<213>  Homo sapiens

<400>  260
tccctgcact catgaaaccc caata                                          25


<210>  261
<211>  25
<212>  DNA
<213>  Homo sapiens

<400>  261
ccctgcactc atgaaacccc aataa                                          25


<210>  262
<211>  25
<212>  DNA
<213>  Homo sapiens

<400>  262
cctgcactca tgaaacccca ataaa                                          25


<210>  263
<211>  25
<212>  DNA
<213>  Homo sapiens

<400>  263
ctgcactcat gaaaccccaa taaat                                          25


<210>  264
<211>  25
<212>  DNA
<213>  Homo sapiens

<400>  264
tgcactcatg aaaccccaat aaata                                          25


<210>  265
<211>  1793
<212>  DNA
<213>  Homo sapiens

<400>  265
   cgcgtccgcc ccgcgagcac agagcctcgc ctttgccgat ccgccgcccg tccacacccg      60
   ccgccagctc accatggatg atgatatcgc cgcgctcgtc gtcgacaacg gctccggcat     120
   gtgcaaggcc ggcttcgcgg gcgacgatgc cccccgggcc gtcttcccct ccatcgtggg     180
   gcgccccagg caccagggcg tgatggtggg catgggtcag aaggattcct atgtgggcga     240
   cgaggcccag agcaagagag gcatcctcac cctgaagtac cccatcgagc acggcatcgt     300
   caccaactgg gacgacatgg agaaaatctg gcaccacacc ttctacaatg agctgcgtgt     360
   ggctcccgag gagcaccccg tgctgctgac cgaggccccc ctgaacccca aggccaaccg     420
   cgagaagatg acccagatca tgtttgagac cttcaacacc ccagccatgt acgttgctat     480
   ccaggctgtg ctatccctgt acgcctctgg ccgtaccact ggcatcgtga tggactccgg 540
   tgacggggtc acccacactg tgcccatcta cgaggggtat gccctccccc atgccatcct 600
   gcgtctggac ctggctggcc gggacctgac tgactacctc atgaagatcc tcaccgagcg 660
   cggctacagc ttcaccacca cggccgagcg ggaaatcgtg cgtgacatta aggagaagct 720
   gtgctacgtc gccctggact tcgagcaaga gatggccacg gctgcttcca gctcctccct 780
   ggagaagagc tacgagctgc ctgacggcca ggtcatcacc attggcaatg agcggttccg 840
   ctgccctgag gcactcttcc agccttcctt cctgggcatg gagtcctgtg gcatccacga 900
   aactaccttc aactccatca tgaagtgtga cgtggacatc cgcaaagacc tgtacgccaa 960
   cacagtgctg tctggcggca ccaccatgta ccctggcatt gccgacagga tgcagaagga 1020
   gatcactgcc ctggcaccca gcacaatgaa gatcaagatc attgctcctc ctgagcgcaa 1080
   gtactccgtg tggatcggcg gctccatcct ggcctcgctg tccaccttcc agcagatgtg 1140
   gatcagcaag caggagtatg acgagtccgg cccctccatc gtccaccgca aatgcttcta 1200
   ggcggactat gacttagttg cgttacaccc tttcttgaca aaacctaact tgcgcagaaa 1260
   acaagatgag attggcatgg ctttatttgt tttttttgtt ttgttttggt tttttttttt 1320
   tttttggctt gactcaggat ttaaaaactg gaacggtgaa ggtgacagca gtcggttgga 1380
   gcgagcatcc cccaaagttc acaatgtggc cgaggacttt gattgcacat tgttgttttt 1440
   ttaatagtca ttccaaatat gagatgcatt gttacaggaa gtcccttgcc atcctaaaag 1500
   ccaccccact tctctctaag gagaatggcc cagtcctctc ccaagtccac acaggggagg 1560
   tgatagcatt gctttcgtgt aaattatgta atgcaaaatt tttttaatct tcgccttaat 1620
   acttttttat tttgttttat tttgaatgat gagccttcgt gccccccctt cccccttttt 1680
   gtcccccaac ttgagatgta tgaaggcttt tggtctccct gggagtgggt ggaggcagcc 1740
   agggcttacc tgtacactga cttgagacca gttgaataaa agtgcacacc tta 1793

<210> 266
<211> 3528
<212> DNA
<213> Homo sapiens

<400> 266
ctttttcgca acgggtttgc cgccagaaca caggtgtcgt gaaaactacc cctaaaagcc 60

aaaatgggaa aggaaaagac tcatatcaac attgtcgtca ttggacacgt agattcgggc 120

aagtccacca ctactggcca tctgatctat aaatgcggtg gcatcgacaa aagaaccatt 180

gaaaaatttg agaaggaggc tgctgagatg ggaaagggct ccttcaagta tgcctgggtc 240

ttggataaac tgaaagctga gcgtgaacgt ggtatcacca ttgatatctc cttgtggaaa 300

tttgagacca gcaagtacta tgtgactatc attgatgccc caggacacag agactttatc 360

aaaaacatga ttacagggac atctcaggct gactgtgctg tcctgattgt tgctgctggt 420

gttggtgaat ttgaagctgg tatctccaag aatgggcaga cccgagagca tgcccttctg 480

gcttacacac tgggtgtgaa acaactaatt gtcggtgtta acaaaatgga ttccactgag 540

ccaccctaca gccagaagag atatgaggaa attgttaagg aagtcagcac ttacattaag 600
aaaattggct acaaccccga cacagtagca tttgtgccaa tttctggttg gaatggtgac 660
aacatgctgg agccaagtgc taacatgcct tggttcaagg gatggaaagt cacccgtaag 720
gatggcaatg ccagtggaac cacgctgctt gaggctctgg actgcatcct accaccaact 780
cgtccaactg acaagccctt gcgcctgcct ctccaggatg tctacaaaat tggtggtatt 840
ggtactgttc ctgttggccg agtggagact ggtgttctca aacccggtat ggtggtcacc 900
tttgctccag tcaacgttac aacggaagta aaatctgtcg aaatgcacca tgaagctttg 960
agtgaagctc ttcctgggga caatgtgggc ttcaatgtca agaatgtgtc tgtcaaggat 1020
gttcgtcgtg gcaacgttgc tggtgacagc aaaaatgacc caccaatgga agcagctggc 1080
ttcactgctc aggtgattat cctgaaccat ccaggccaaa taagcgccgg ctatgcccct 1140
gtattggatt gccacacggc tcacattgca tgcaagtttg ctgagctgaa ggaaaagatt 1200
gatcgccgtt ctggtaaaaa gctggaagat ggccctaaat tcttgaagtc tggtgatgct 1260
gccattgttg atatggttcc tggcaagccc atgtgtgttg agagcttctc agactatcca 1320
cctttgggtc gctttgctgt tcgtgatatg agacagacag ttgcggtggg tgtcatcaaa 1380
gcagtggaca agaaggctgc tggagctggc aaggtcacca agtctgccca gaaagctcag 1440
aaggctaaat gaatattatc cctaatacct gccaccccac tcttaatcag tggtggaaga 1500
acggtctcag aactgtttgt ttcaattggc catttaagtt tagtagtaaa agactggtta 1560
atgataacaa tgcatcgtaa aaccttcaga aggaaaggag aatgttttgt ggaccacttt 1620
ggttttcttt tttgcgtgtg gcagttttaa gttattagtt tttaaaatca gtacttttta 1680
atggaaacaa cttgaccaaa aatttgtcac agaattttga gacccattaa aaaagttaaa 1740
tgagaaacct gtgtgttcct ttggtcaaca ccgagacatt taggtgaaag acatctaatt 1800
ctggttttac gaatctggaa acttcttgaa aatgtaattc ttgagttaac acttctgggt 1860
ggagaatagg gttgttttcc ccccacataa ttggaagggg aaggaatatc atttaaagct 1920
atgggagggt tgctttgatt acaacactgg agagaaatgc agcatgttgc tgattgcctg 1980
tcactaaaac aggccaaaaa ctgagtcctt gtgttgcata gaaagcttca tgttgctaaa 2040
ccaatgttaa gtgaatcttt ggaaacaaaa tgtttccaaa ttactgggat gtgcatgttg 2100
aaacgtgggt taaaatgact gggcagtgaa agttgactat ttgccatgac ataagaaata 2160
agtgtagtgg ctagtgtaca ccctatgagt ggaagggtcc attttgaagt cagtggagta 2220
agctttatgc cagtttgatg gtttcacaag ttctattgag tgctattcag aataggaaca 2280
aggttctaat agaaaaagat ggcaatttga agtagctata aaattagact aatctacatt 2340
gcttttctcc tgcagagtct aatacctttt atgctttgat aattagcagt ttgtctactt 2400
ggtcactagg aatgaaacta catggtaata ggcttaacag gtgtaatagc ccacttactc 2460
ctgaatcttt aagcatttgt gcatttgaaa aatgcttttc gcgatcttcc tgctgggatt 2520
acaggcatga gccactgtgc ctgacctccc atatgtaaaa gtgtctaaag gttttttttt 2580

ggttataaaa ggaaaatttt tgcttaagtt tgaaggatag gtaaaattaa aggacatgct 2640
ttctgtttgt gtgatggttt ttaaaaattt tttttaagat ggagttcttg ttgcccaggc 2700
tagaatgcaa tggcaaaatc tcactgcaat ctcctcctcc tgggttcaag caattctcct 2760
acttcagcct cccaagtagc tgggattaca ggcatgtgct aatttggtgt ttttaataga 2820
gatgaggttt ttccatgttg gtcaggctgg tctcaaactc ctgaccttag gtgatcgcct 2880
cggcctccta aagtgctgga attacaggca tgagccacca tgcctggcca ggacatgtgt 2940
tcttaaggac atgctaagca ggagttaaag cagcccaaga gataaggcct cttaaagtga 3000
ctggcaatgt gtattgctca agattcaaag gtacttgaat tggccataga caagtctgta 3060
atgaagtgtt atcgttttcc ctcatctgag tctgaattag ataaaatgcc ttcccatcag 3120
ccagtgctct gaggtatcaa gtctaaattg aactagagat ttttgtcctt agtttctttg 3180
ctatctaatg tttacacaag taaatagtct aagatttgct ggatgacaga aaaaacaggt 3240
aaggccttta atagatggcc aatagatgcc ctgataatga aagttgacac ctgtaagatt 3300
taccagtaga gaattcttga catgcaagga agcaagattt aactgaaaaa ttgttcccac 3360
tggaagcagg aatgagtcag tttacttgca tatactgaga ttgagattaa cttcctgtga 3420
aacccagtgt cttagacaac tgtggcttga gcaccacctg ctggtattca ttacaaactt 3480
gctcactaca ataaatgaat tttaagcttt aaaaaaaaaa aaaaaaaa 3528

<210> 267
<211> 2527
<212> DNA
<213> Homo sapiens

<400> 267
ctcaagctcc tctacaaaga ggtggacaga gaagacagca gagaccatgg gacccccctc 60
agcccctccc tgcagattgc atgtcccctg gaaggaggtc ctgctcacag cctcacttct 120
aaccttctgg aacccaccca ccactgccaa gctcactatt gaatccacgc cattcaatgt 180
cgcagagggg aaggaggttc ttctactcgc ccacaacctg ccccagaatc gtattggtta 240
cagctggtac aaaggcgaaa gagtggatgg caacagtcta attgtaggat atgtaatagg 300
aactcaacaa gctaccccag ggcccgcata cagtggtcga gagacaatat accccaatgc 360
atccctgctg atccagaacg tcacccagaa tgacacagga ttctataccc tacaagtcat 420
aaagtcagat cttgtgaatg aagaagcaac cggacagttc catgtatacc cggagctgcc 480
caagccctcc atctccagca acaactccaa ccccgtggag gacaaggatg ctgtggcctt 540
cacctgtgaa cctgaggttc agaacacaac ctacctgtgg tgggtaaatg gtcagagcct 600
cccggtcagt cccaggctgc agctgtccaa tggcaacatg accctcactc tactcagcgt 660
caaaaggaac gatgcaggat cctatgaatg tgaaatacag aacccagcga gtgccaaccg 720
cagtgaccca gtcaccctga atgtcctcta tggcccagat ggccccacca tttccccctc 780
aaaggccaat taccgtccag gggaaaatct gaacctctcc tgccacgcag cctctaaccc 840

acctgcacag tactcttggt ttatcaatgg gacgttccag caatccacac aagagctctt 900

tatccccaac atcactgtga ataatagcgg atcctatatg tgccaagccc ataactcagc 960

cactggcctc aataggacca cagtcacgat gatcacagtc tctggaagtg ctcctgtcct 1020

ctcagctgtg gccaccgtcg gcatcacgat tggagtgctg gccagggtcg ctctgatata 1080

gcagccctgg tgtattttcg atatttcagg aagactggca gattggacca gaccctgaat 1140

tcttctagct cctccaatcc cattttatcc atggaaccac taaaaacaag gtctgctctg 1200

ctcctgaagc cctatatgct ggagatggac aactcaatga aaatttaaag ggaaaaccct 1260

caggcctgag gtgtgtgcca ctcagagact tcacctaact agagacaggc aaactgcaaa 1320

ccatggtgag aaattgacga cttcacacta tggacagctt ttcccaagat gtcaaaacaa 1380

gactcctcat catgataagg ctcttacccc cttttaattt gtccttgctt atgcctgcct 1440

ctttcgcttg gcaggatgat gctgtcatta gtattcacaa gaagtagctt cagagggtaa 1500

cttaacagag tatcagattc tatcttgtca atcccaacgt tttacataaa ataagagatc 1560

ctttagtgca cccagtgact gacattagca gcatctttaa cacagccgtg tgttcaaatg 1620

tacagtggtc cttttcagag ttggacttct agactcacct gttctcactc cctgttttaa 1680

tttcaaccca gccatgcaat gccaaataat agaattgctc cctaccagct gaacagggag 1740

gagtctgtgc agtttctgac acttgttgtt gaacatggct aaatacaatg ggtatcgctg 1800

agactaagtt gtagaaatta acaaatgtgc tgctggtaaa atggctacac tcatctgact 1860

cattctttat tctattttag ttggtttgta tcttgcctaa ggtgcgtagt ccaactcttg 1920

gtattaccct cctaatagtc atactagtag tcatactccc tggtgtagtg tattctctaa 1980

aagctttaaa tgtctgcatg cagccagcca tcaaatagtg aatggtctct ctttggctgg 2040

aattacaaaa ctcagagaaa tgtgtcatca ggagaacatc ataacccatg aaggataaaa 2100

gccccaaatg gtggtaactg ataatagcac taatgcttaa gatttggtca cactctctca 2160

cctaggtgag cgcattgagc cagtggtgct aaatgctaca tactccaact gaaatgttaa 2220

ggaagaagat agatccaatt aaaaaaaatt aaaaccaatt taaaaaaaaa aagaacacag 2280

gagattccag tctacttgag ttagcataat acagaagtcc cctctacttt aacttttaca 2340

aaaaagtaac ctgaactaat ctgatgttaa ccaatgtatt tatttctgtg gttctgtttc 2400

cttgttccaa tttgacaaaa cccactgttc ttgtattgta ttgccagggg ggagctatca 2460

ctgtacttgt agagtggtgc tgctttaatt cataaatcac aaataaaagc caattagctc 2520

tataact 2527

<210> 268
<211> 1310
<212> DNA
<213> Homo sapiens

<400> 268
aaattgagcc cgcagcctcc cgcttcgctc tctgctcctc ctgttcgaca gtcagccgca 60

tcttcttttg cgtcgccagc cgagccacat cgctcagaca ccatggggaa ggtgaaggtc 120

ggagtcaacg gatttggtcg tattgggcgc ctggtcacca gggctgcttt taactctggt 180

aaagtggata ttgttgccat caatgacccc ttcattgacc tcaactacat ggtttacatg 240

ttccaatatg attccaccca tggcaaattc catggcaccg tcaaggctga gaacgggaag 300

cttgtcatca atggaaatcc catcaccatc ttccaggagc gagatccctc caaaatcaag 360

tggggcgatg ctggcgctga gtacgtcgtg gagtccactg gcgtcttcac caccatggag 420

aaggctgggg ctcatttgca ggggggagcc aaaagggtca tcatctctgc cccctctgct 480

gatgccccca tgttcgtcat gggtgtgaac catgagaagt atgacaacag cctcaagatc 540

atcagcaatg cctcctgcac caccaactgc ttagcacccc tggccaaggt catccatgac 600

aactttggta tcgtggaagg actcatgacc acagtccatg ccatcactgc cacccagaag 660

actgtggatg gcccctccgg gaaactgtgg cgtgatggcc gcggggctct ccagaacatc 720

atccctgcct ctactggcgc tgccaaggct gtgggcaagg tcatccctga gctgaacggg 780

aagctcactg gcatggcctt ccgtgtcccc actgccaacg tgtcagtggt ggacctgacc 840

tgccgtctag aaaaacctgc caaatatgat gacatcaaga aggtggtgaa gcaggcgtcg 900

gagggccccc tcaagggcat cctgggctac actgagcacc aggtggtctc ctctgacttc 960

aacagcgaca cccactcctc cacctttgac gctggggctg gcattgccct caacgaccac 1020

tttgtcaagc tcatttcctg gtatgacaac gaatttggct acagcaacag ggtggtggac 1080

ctcatggccc acatggcctc caaggagtaa gacccctgga ccaccagccc cagcaagagc 1140

acaagaggaa gagagagacc ctcactgctg gggagtccct gccacactca gtcccccacc 1200

acactgaatc tcccctcctc acagttgcca tgtagacccc ttgaagaggg gaggggccta 1260

gggagccgca ccttgtcatg taccatcaat aaagtaccct gtgctcaacc 1310

<210> 269
<211> 2691
<212> DNA
<213> Homo sapiens

<400> 269
gcttgcccgt cggtcgctag ctcgctcggt gcgcgtcgtc ccgctccatg gcgctcttcg 60

tgcggctgct ggctctcgcc ctggctctgg ccctgggccc cgccgcgacc ctggcgggtc 120

ccgccaagtc gccctaccag ctggtgctgc agcacagcag gctccggggc cgccagcacg 180

gccccaacgt gtgtgctgtg cagaaggtta ttggcactaa taggaagtac ttcaccaact 240

gcaagcagtg gtaccaaagg aaaatctgtg gcaaatcaac agtcatcagc tacgagtgct 300

gtcctggata tgaaaaggtc cctggggaga agggctgtcc agcagcccta ccactctcaa 360

acctttacga gaccctggga gtcgttggat ccaccaccac tcagctgtac acggaccgca 420

cggagaagct gaggcctgag atggaggggc ccggcagctt caccatcttc gcccctagca 480

acgaggcctg ggcctccttg ccagctgaag tgctggactc cctggtcagc aatgtcaaca 540

ttgagctgct caatgccctc cgctaccata tggtgggcag gcgagtcctg actgatgagc 600

tgaaacacgg catgaccctc acctctatgt accagaattc caacatccag atccaccact 660
atcctaatgg gattgtaact gtgaactgtg cccggctcct gaaagccgac caccatgcaa 720
ccaacggggt ggtgcacctc atcgataagg tcatctccac catcaccaac aacatccagc 780
agatcattga gatcgaggac acctttgaga cccttcgggc tgctgtggct gcatcagggc 840
tcaacacgat gcttgaaggt aacggccagt acacgctttt ggccccgacc aatgaggcct 900
tcgagaagat ccctagtgag actttgaacc gtatcctggg cgacccagaa gccctgagag 960
acctgctgaa caaccacatc ttgaagtcag ctatgtgtgc tgaagccatc gttgcggggc 1020
tgtctgtaga gaccctggag ggcacgacac tggaggtggg ctgcagcggg gacatgctca 1080
ctatcaacgg gaaggcgatc atctccaata aagacatcct agccaccaac ggggtgatcc 1140
actacattga tgagctactc atcccagact cagccaagac actatttgaa ttggctgcag 1200
agtctgatgt gtccacagcc attgaccttt tcagacaagc cggcctcggc aatcatctct 1260
ctggaagtga gcggttgacc ctcctggctc ccctgaattc tgtattcaaa gatggaaccc 1320
ctccaattga tgcccataca aggaatttgc ttcggaacca cataattaaa gaccagctgg 1380
cctctaagta tctgtaccat ggacagaccc tggaaactct gggcggcaaa aaactgagag 1440
tttttgttta tcgtaatagc ctctgcattg agaacagctg catcgcggcc cacgacaaga 1500
gggggaggta cgggaccctg ttcacgatgg accgggtgct gaccccccca atggggactg 1560
tcatggatgt cctgaaggga gacaatcgct ttagcatgct ggtagctgcc atccagtctg 1620
caggactgac ggagaccctc aaccgggaag gagtctacac agtctttgct cccacaaatg 1680
aagccttccg agccctgcca ccaagagaac ggagcagact cttgggagat gccaaggaac 1740
ttgccaacat cctgaaatac cacattggtg atgaaatcct ggttagcgga ggcatcgggg 1800
ccctggtgcg gctaaagtct ctccaaggtg acaagctgga agtcagcttg aaaaacaatg 1860
tggtgagtgt caacaaggag cctgttgccg agcctgacat catggccaca aatggcgtgg 1920
tccatgtcat caccaatgtt ctgcagcctc cagccaacag acctcaggaa agaggggatg 1980
aacttgcaga ctctgcgctt gagatcttca aacaagcatc agcgttttcc agggcttccc 2040
agaggtctgt gcgactagcc cctgtctatc aaaagttatt agagaggatg aagcattagc 2100
ttgaagcact acaggaggaa tgcaccacgg cagctctccg ccaatttctc tcagatttcc 2160
acagagactg tttgaatgtt ttcaaaacca agtatcacac tttaatgtac atgggccgca 2220
ccataatgag atgtgagcct tgtgcatgtg ggggaggagg gagagagatg tactttttaa 2280
atcatgttcc ccctaaacat ggctgttaac ccactgcatg cagaaacttg gatgtcactg 2340
cctgacattc acttccagag aggacctatc ccaaatgtgg aattgactgc ctatgccaag 2400
tccctggaaa aggagcttca gtattgtggg gctcataaaa catgaatcaa gcaatccagc 2460
ctcatgggaa gtcctggcac agtttttgta aagcccttgc acagctggag aaatggcatc 2520
attataagct atgagttgaa atgttctgtc aaatgtgtct cacatctaca cgtggcttgg 2580
aggcttttat ggggccctgt ccaggtagaa aagaaatggt atgtagagct tagatttccc 2640

tattgtgaca gagccatggt gtgtttgtaa taataaaacc aaagaaacat a          2691

<210>  270
<211>  914
<212>  DNA
<213>  Homo sapiens

<400>  270
   tgtggcagat ttcagaggcc cttaaaatga ggccaagtga ggtggacagg tccgagccag      60
   ctgaggactc ctcagccaca cggcacagct gcctgagggg atgtgtcact cagggagttg     120
   ctgggaccta ctgggcccag cgttgccatc agcaccaaca gcttcagaga gggggacaca     180
   tgccggggtg actccaaggc tgtgggcggc acctgcctca gatagagaac aggcacagag     240
   acactactgg gggacactac tgggacactg gccacccccc taccctgtgc ctagatcaca     300
   gcctacacac tgcagccctg tgcccctcac acccagcagg ttcctgctcc agcgcggctc     360
   ctggactggc cccgggtgct ggccccgggg gtttcaatcc aagcataatt cagtgaagca     420
   tgtgtttggc agcgggaccc agctcaccgt tttaggtcag cccaaggcca ccccctcggt     480
   cactctgttc ctgccgtcct ctgaggagct ccaagccaac aaggccacac tggtgtgtct     540
   catgaatgac ttctatctgg gaatcttgac ggtgacctgg aaggcagatg gtacccccat     600
   cacccagggc gtggagatga ccacgccctc caaacagagc aacagcaagt acatggccag     660
   cagctacctg agcctgacgc ccgagcagtg gaggtcccgc agaagctaca gctgccaggt     720
   catgcatgaa gggagcactg cagagaagac ggtggcccct gcagaatgtt cataggttcc     780
   cagcccccac cccacccaca ggggcctgga gctgcaggat cccaggggag gcgtctctct     840
   ctgcatccca agccatccag cccttctccc tgtacccagt aaaccctcag taaatatcct     900
   ctttgtcaac caga                                                       914

<210>  271
<211>  1533
<212>  DNA
<213>  Homo sapiens

<400>  271
atgctggtca tggcgccccg aaccgtcctc ctgctgctct cggcggccct ggccctgacc      60

gagacctggg ccggctccca ctccatgagg tatttctaca cctccgtgtc ccggcccggc     120

cgcggggagc cccgcttcat ctcagtgggc tacgtggacg acacccagtt cgtgaggttc     180

gacagcgacg ccgcgagtcc gagagaggag ccgcgggcgc cgtggataga gcaggagggg     240

ccggagtatt gggaccggaa cacacagatc tacaaggccc aggcacagac tgaccgagag     300

agcctgcgga acctgcgcgg ctactacaac cagagcgagg ccgggtctca caccctccag     360

agcatgtacg gctgcgacgt ggggccggac gggcgcctcc tccgcgggca tgaccagtac     420

gcctacgacg gcaaggatta catcgccctg aacgaggacc tgcgctcctg gaccgccgcg     480

gacacggcgg ctcagatcac ccagcgcaag tgggaggcgg cccgtgaggc ggagcagcgg     540

agagcctacc tggagggcga gtgcgtggag tggctccgca gatacctgga gaacgggaag     600

gacaagctgg agcgcgctga ccccccaaag acacacgtga cccaccaccc catctctgac 660

catgaggcca ccctgaggtg ctgggccctg ggtttctacc ctgcggagat cacactgacc 720

tggcagcggg atggcgagga ccaaactcag gacactgagc ttgtggagac cagaccagca 780

ggagatagaa ccttccagaa gtgggcagct gtggtggtgc cttctggaga agagcagaga 840

tacacatgcc atgtacagca tgaggggctg ccgaagcccc tcaccctgag atgggagccg 900

tcttcccagt ccaccgtccc catcgtgggc attgttgctg gcctggctgt cctagcagtt 960

gtggtcatcg gagctgtggt cgctgctgtg atgtgtagga ggaagagttc aggtggaaaa 1020

ggagggagct actctcaggc tgcgtgcagc gacagtgccc agggctctga tgtgtctctc 1080

acagcttgaa aagcctgaga cagctgtctt gtgagggact gagatgcagg atttcttcac 1140

gcctcccctt tgtgacttca agagcctctg gcatctcttt ctgcaaaggc acctgaatgt 1200

gtctgcgtcc ctgttagcat aatgtgagga ggtggagaga cagcccaccc ttgtgtccac 1260

tgtgacccct gttcccatgc tgacctgtgt ttcctcccca gtcatctttc ttgttccaga 1320

gaggtggggc tggatgtctc catctctgtc tcaactttac gtgcactgag ctgcaacttc 1380

ttacttccct actgaaaata agaatctgaa tataaatttg ttttctcaaa tatttgctat 1440

gagaggttga tggattaatt aaataagtca attcctggaa tttgaaagag caaataaaga 1500

cctgagaacc ttccagaaaa aaaaaaaaaa aaa 1533

<210> 272
<211> 1345
<212> DNA
<213> Homo sapiens

<400> 272
gcctctgggg ttttatattg ctctggtatt catgccaaag acacaccagc cctcagtcac 60

tgggagaaga acctctcata ccctcggtgc tccagtcccc agctcactca gccacacaca 120

ccatgtgtga agaggagacc accgcgctcg tgtgtgacaa tggctctggc ctgtgcaagg 180

caggcttcgc aggagatgat gccccccggg ctgtcttccc ctccattgtg ggccgccctc 240

gccaccaggg tgtgatggtg ggaatgggcc agaaagacag ctatgtgggg gatgaggctc 300

agagcaagcg agggatccta actctcaaat accccattga acacggcatc atcaccaact 360

gggatgacat ggagaagatc tggcaccact ccttctacaa tgagctgcgt gtagcacctg 420

aagagcaccc caccctgctc acagaggctc ccctaaatcc caaggccaac agggaaaaga 480

tgacccagat catgtttgaa accttcaatg tccctgccat gtacgtcgcc attcaagctg 540

tgctctccct ctatgcctct ggccgcacga caggcatcgt cctggattca ggtgatggcg 600

tcacccacaa tgtccccatc tatgaaggct atgccctgcc ccatgccatc atgcgcctgg 660

acttggctgg ccgtgacctc acggactacc tcatgaagat cctcacagag agaggctatt 720

cctttgtgac cacagctgag agagaaattg tgcgagacat caaggagaag ctgtgctatg 780

tggccctgga ttttgagaat gagatggcca cagcagcttc ctcttcctcc ctggagaaga 840

gctatgagct gccagatggg caggttatca ccattggcaa tgagcgcttc cgctgccctg 900

agaccctctt ccagccttcc tttattggca tggagtccgc tggaattcat gagacaacct 960

acaattccat catgaagtgt gacattgaca tccgtaagga cttatatgcc aacaatgtcc 1020

tctctggggg caccaccatg taccctggca ttgctgacag gatgcagaag gagatcacag 1080

ccctggcccc cagcaccatg aagatcaaga ttattgctcc cccagagcgg aagtactcag 1140

tctggatcgg gggctctatc ctggcctctc tctccacctt ccagcagatg tggatcagca 1200

agcctgagta tgatgaggca gggccctcca ttgtccacag gaagtgcttc taaagtcaga 1260

acaggttctc caaggatccc ctcgagacta ctctgttacc agtcatgaaa cattaaaacc 1320

tacaagcctt aaaaaaaaaa aaaaa 1345

<210> 273
<211> 8815
<212> DNA
<213> Homo sapiens

<400> 273
gcccgcgccg gctgtgctgc acagggggag gagagggaac cccaggcgcg agcgggaaga 60

ggggacctgc agccacaact tctctggtcc tctgcatccc ttctgtccct ccacccgtcc 120

ccttccccac cctctggccc ccaccttctt ggaggcgaca acccccggga ggcattagaa 180

gggatttttc ccgcaggttg cgaagggaag caaacttggt ggcaacttgc ctcccggtgc 240

gggcgtctct cccccaccgt ctcaacatgc ttaggggtcc ggggcccggg ctgctgctgc 300

tggccgtcca gtgcctgggg acagcggtgc cctccacggg agcctcgaag agcaagaggc 360

aggctcagca aatggttcag ccccagtccc cggtggctgt cagtcaaagc aagcccggtt 420

gttatgacaa tggaaaacac tatcagataa atcaacagtg ggagcggacc tacctaggca 480

atgcgttggt ttgtacttgt tatggaggaa gccgaggttt taactgcgag agtaaacctg 540

aagctgaaga gacttgcttt gacaagtaca ctgggaacac ttaccgagtg ggtgacactt 600

atgagcgtcc taaagactcc atgatctggg actgtacctg catcggggct gggcgaggga 660

gaataagctg taccatcgca aaccgctgcc atgaaggggg tcagtcctac aagattggtg 720

acacctggag gagaccacat gagactggtg gttacatgtt agagtgtgtg tgtcttggta 780

atggaaaagg agaatggacc tgcaagccca tagctgagaa gtgttttgat catgctgctg 840

ggacttccta tgtggtcgga gaaacgtggg agaagcccta ccaaggctgg atgatggtag 900

attgtacttg cctgggagaa ggcagcggac gcatcacttg cacttctaga aatagatgca 960

acgatcagga cacaaggaca tcctatagaa ttggagacac ctggagcaag aaggataatc 1020

gaggaaacct gctccagtgc atctgcacag gcaacggccg aggagagtgg aagtgtgaga 1080

ggcacacctc tgtgcagacc acatcgagcg gatctggccc cttcaccgat gttcgtgcag 1140

ctgtttacca accgcagcct cacccccagc ctcctcccta tggccactgt gtcacagaca 1200

gtggtgtggt ctactctgtg gggatgcagt ggctgaagac acaaggaaat aagcaaatgc 1260

tttgcacgtg cctgggcaac ggagtcagct gccaagagac agctgtaacc cagacttacg 1320

gtggcaactc aaatggagag ccatgtgtct taccattcac ctacaatggc aggacgttct 1380
actcctgcac cacagaaggg cgacaggacg gacatctttg gtgcagcaca acttcgaatt 1440
atgagcagga ccagaaatac tctttctgca cagaccacac tgttttggtt cagactcgag 1500
gaggaaattc caatggtgcc ttgtgccact tccccttcct atacaacaac cacaattaca 1560
ctgattgcac ttctgagggc agaagagaca acatgaagtg gtgtgggacc acacagaact 1620
atgatgccga ccagaagttt gggttctgcc ccatggctgc ccacgaggaa atctgcacaa 1680
ccaatgaagg ggtcatgtac cgcattggag atcagtggga taagcagcat gacatgggtc 1740
acatgatgag gtgcacgtgt gttgggaatg gtcgtgggga atggacatgc attgcctact 1800
cgcagcttcg agatcagtgc attgttgatg acatcactta caatgtgaac gacacattcc 1860
acaagcgtca tgaagagggg cacatgctga actgtacatg cttcggtcag ggtcggggca 1920
ggtggaagtg tgatcccgtc gaccaatgcc aggattcaga gactgggacg ttttatcaaa 1980
ttggagattc atgggagaag tatgtgcatg gtgtcagata ccagtgctac tgctatggcc 2040
gtggcattgg ggagtggcat tgccaacctt tacagaccta tccaagctca agtggtcctg 2100
tcgaagtatt tatcactgag actccgagtc agcccaactc ccaccccatc cagtggaatg 2160
caccacagcc atctcacatt tccaagtaca ttctcaggtg gagacctaaa aattctgtag 2220
gccgttggaa ggaagctacc ataccaggcc acttaaactc ctacaccatc aaaggcctga 2280
agcctggtgt ggtatacgag ggccagctca tcagcatcca gcagtacggc caccaagaag 2340
tgactcgctt tgacttcacc accaccagca ccagcacacc tgtgaccagc aacaccgtga 2400
caggagagac gactcccttt tctcctcttg tggccacttc tgaatctgtg accgaaatca 2460
cagccagtag ctttgtggtc tcctgggtct cagcttccga caccgtgtcg ggattccggg 2520
tggaatatga gctgagtgag gagggagatg agccacagta cctggatctt ccaagcacag 2580
ccacttctgt gaacatccct gacctgcttc ctggccgaaa atacattgta aatgtctatc 2640
agatatctga ggatggggag cagagtttga tcctgtctac ttcacaaaca acagcgcctg 2700
atgcccctcc tgacccgact gtggaccaag ttgatgacac ctcaattgtt gttcgctgga 2760
gcagacccca ggctcccatc acagggtaca gaatagtcta ttcgccatca gtagaaggta 2820
gcagcacaga actcaacctt cctgaaactg caaactccgt caccctcagt gacttgcaac 2880
ctggtgttca gtataacatc actatctatg ctgtggaaga aaatcaagaa agtacacctg 2940
ttgtcattca acaagaaacc actggcaccc cacgctcaga tacagtgccc tctcccaggg 3000
acctgcagtt tgtggaagtg acagacgtga aggtcaccat catgtggaca ccgcctgaga 3060
gtgcagtgac cggctaccgt gtggatgtga tccccgtcaa cctgcctggc gagcacgggc 3120
agaggctgcc catcagcagg aacacctttg cagaagtcac cgggctgtcc cctggggtca 3180
cctattactt caaagtcttt gcagtgagcc atgggaggga gagcaagcct ctgactgctc 3240
aacagacaac caaactggat gctcccacta acctccagtt tgtcaatgaa actgattcta 3300
ctgtcctggt gagatggact ccacctcggg cccagataac aggataccga ctgaccgtgg 3360

gccttacccg aagaggacag cccaggcagt acaatgtggg tccctctgtc tccaagtacc 3420
cactgaggaa tctgcagcct gcatctgagt acaccgtatc cctcgtggcc ataaagggca 3480
accaagagag ccccaaagcc actggagtct ttaccacact gcagcctggg agctctattc 3540
caccttacaa caccgaggtg actgagacca ccattgtgat cacatggacg cctgctccaa 3600
gaattggttt taagctgggt gtacgaccaa gccagggagg agaggcacca cgagaagtga 3660
cttcagactc aggaagcatc gttgtgtccg gcttgactcc aggagtagaa tacgtctaca 3720
ccatccaagt cctgagagat ggacaggaaa gagatgcgcc aattgtaaac aaagtggtga 3780
caccattgtc tccaccaaca aacttgcatc tggaggcaaa ccctgacact ggagtgctca 3840
cagtctcctg ggagaggagc accaccccag acattactgg ttatagaatt accacaaccc 3900
ctacaaacgg ccagcaggga aattctttgg aagaagtggt ccatgctgat cagagctcct 3960
gcacttttga taacctgagt cccggcctgg agtacaatgt cagtgtttac actgtcaagg 4020
atgacaagga aagtgtccct atctctgata ccatcatccc agaggtgccc caactcactg 4080
acctaagctt tgttgatata accgattcaa gcatcggcct gaggtggacc ccgctaaact 4140
cttccaccat tattgggtac cgcatcacag tagttgcggc aggagaaggt atccctattt 4200
ttgaagattt tgtggactcc tcagtaggat actacacagt cacagggctg gagccgggca 4260
ttgactatga tatcagcgtt atcactctca ttaatggcgg cgagagtgcc cctactacac 4320
tgacacaaca aacggctgtt cctcctccca ctgacctgcg attcaccaac attggtccag 4380
acaccatgcg tgtcacctgg gctccacccc catccattga tttaaccaac ttcctggtgc 4440
gttactcacc tgtgaaaaat gaggaagatg ttgcagagtt gtcaatttct ccttcagaca 4500
atgcagtggt cttaacaaat ctcctgcctg gtacagaata tgtagtgagt gtctccagtg 4560
tctacgaaca acatgagagc acacctctta gaggaagaca gaaaacaggt cttgattccc 4620
caactggcat tgacttttct gatattactg ccaactcttt tactgtgcac tggattgctc 4680
ctcgagccac catcactggc tacaggatcc gccatcatcc cgagcacttc agtgggagac 4740
ctcgagaaga tcgggtgccc cactctcgga attccatcac cctcaccaac ctcactccag 4800
gcacagagta tgtggtcagc atcgttgctc ttaatggcag agaggaaagt cccttattga 4860
ttggccaaca atcaacagtt tctgatgttc cgagggacct ggaagttgtt gctgcgaccc 4920
ccaccagcct actgatcagc tgggatgctc ctgctgtcac agtgagatat tacaggatca 4980
cttacggaga gacaggagga aatagccctg tccaggagtt cactgtgcct gggagcaagt 5040
ctacagctac catcagcggc cttaaacctg gagttgatta taccatcact gtgtatgctg 5100
tcactggccg tggagacagc cccgcaagca gcaagccaat ttccattaat taccgaacag 5160
aaattgacaa accatcccag atgcaagtga ccgatgttca ggacaacagc attagtgtca 5220
agtggctgcc ttcaagttcc cctgttactg gttacagagt aaccaccact cccaaaaatg 5280
gaccaggacc aacaaaaact aaaactgcag gtccagatca aacagaaatg actattgaag 5340
gcttgcagcc cacagtggag tatgtggtta gtgtctatgc tcagaatcca agcggagaga 5400

gtcagcctct ggttcagact gcagtaacca acattgatcg ccctaaagga ctggcattca 5460
ctgatgtgga tgtcgattcc atcaaaattg cttgggaaag cccacagggg caagtttcca 5520
ggtacagggt gacctactcg agccctgagg atggaatcca tgagctattc cctgcacctg 5580
atggtgaaga agacactgca gagctgcaag gcctcagacc gggttctgag tacacagtca 5640
gtgtggttgc cttgcacgat gatatggaga gccagcccct gattggaacc cagtccacag 5700
ctattcctgc accaactgac ctgaagttca ctcaggtcac acccacaagc ctgagcgccc 5760
agtggacacc acccaatgtt cagctcactg gatatcgagt gcgggtgacc cccaaggaga 5820
agaccggacc aatgaaagaa atcaaccttg ctcctgacag ctcatccgtg gttgtatcag 5880
gacttatggt ggccaccaaa tatgaagtga gtgtctatgc tcttaaggac actttgacaa 5940
gcagaccagc tcagggagtt gtcaccactc tggagaatgt cagcccacca agaagggctc 6000
gtgtgacaga tgctactgag accaccatca ccattagctg gagaaccaag actgagacga 6060
tcactggctt ccaagttgat gccgttccag ccaatggcca gactccaatc cagagaacca 6120
tcaagccaga tgtcagaagc tacaccatca caggtttaca accaggcact gactacaaga 6180
tctacctgta caccttgaat gacaatgctc ggagctcccc tgtggtcatc gacgcctcca 6240
ctgccattga tgcaccatcc aacctgcgtt tcctggccac cacacccaat tccttgctgg 6300
tatcatggca gccgccacgt gccaggatta ccggctacat catcaagtat gagaagcctg 6360
ggtctcctcc cagagaagtg gtccctcggc cccgccctgg tgtcacagag gctactatta 6420
ctggcctgga accgggaacc gaatatacaa tttatgtcat tgccctgaag aataatcaga 6480
agagcgagcc cctgattgga aggaaaaaga cagacgagct tccccaactg gtaacccttc 6540
cacaccccaa tcttcatgga ccagagatct tggatgttcc ttccacagtt caaaagaccc 6600
ctttcgtcac ccaccctggg tatgacactg gaaatggtat tcagcttcct ggcacttctg 6660
gtcagcaacc cagtgttggg caacaaatga tctttgagga acatggtttt aggcggacca 6720
caccgcccac aacggccacc cccataaggc ataggccaag accatacccg ccgaatgtag 6780
gtgaggaaat ccaaattggt cacatcccca gggaagatgt agactatcac ctgtacccac 6840
acggtccggg actcaatcca aatgcctcta caggacaaga agctctctct cagacaacca 6900
tctcatgggc cccattccag gacacttctg agtacatcat ttcatgtcat cctgttggca 6960
ctgatgaaga acccttacag ttcagggttc ctggaacttc taccagtgcc actctgacag 7020
gcctcaccag aggtgccacc tacaacatca tagtggaggc actgaaagac cagcagaggc 7080
ataaggttcg ggaagaggtt gttaccgtgg gcaactctgt caacgaaggc ttgaaccaac 7140
ctacggatga ctcgtgcttt gacccctaca cagtttccca ttatgccgtt ggagatgagt 7200
gggaacgaat gtctgaatca ggctttaaac tgttgtgcca gtgcttaggc tttggaagtg 7260
gtcatttcag atgtgattca tctagatggt gccatgacaa tggtgtgaac tacaagattg 7320
gagagaagtg ggaccgtcag ggagaaaatg gccagatgat gagctgcaca tgtcttggga 7380
acggaaaagg agaattcaag tgtgaccctc atgaggcaac gtgttatgat gatgggaaga 7440

cataccacgt aggagaacag tggcagaagg aatatctcgg tgccatttgc tcctgcacat 7500

gctttggagg ccagcggggc tggcgctgtg acaactgccg cagacctggg ggtgaaccca 7560

gtcccgaagg cactactggc cagtcctaca accagtattc tcagagatac catcagagaa 7620

caaacactaa tgttaattgc ccaattgagt gcttcatgcc tttagatgta caggctgaca 7680

gagaagattc ccgagagtaa atcatctttc caatccagag gaacaagcat gtctctctgc 7740

caagatccat ctaaactgga gtgatgttag cagacccagc ttagagttct tctttctttc 7800

ttaagccctt tgctctggag gaagttctcc agcttcagct caactcacag cttctccaag 7860

catcaccctg ggagtttcct gagggttttc tcataaatga gggctgcaca ttgcctgttc 7920

tgcttcgaag tattcaatac cgctcagtat tttaaatgaa gtgattctaa gatttggttt 7980

gggatcaata ggaaagcata tgcagccaac caagatgcaa atgttttgaa atgatatgac 8040

caaaatttta agtaggaaag tcacccaaac acttctgctt tcacttaagt gtctggcccg 8100

caatactgta ggaacaagca tgatcttgtt actgtgatat tttaaatatc cacagtactc 8160

actttttcca aatgatccta gtaattgcct agaaatatct ttctcttacc tgttatttat 8220

caatttttcc cagtattttt atacggaaaa aattgtattg aaaacactta gtatgcagtt 8280

gataagagga atttggtata attatggtgg gtgattattt tttatactgt atgtgccaaa 8340

gctttactac tgtggaaaga caactgtttt aataaaagat ttacattcca caacttgaag 8400

ttcatctatt tgatataaga caccttcggg ggaaataatt cctgtgaata ttctttttca 8460

attcagcaaa catttgaaaa tctatgatgt gcaagtctaa ttgttgattt cagtacaaga 8520

ttttctaaat cagttgctac aaaaactgat tggtttttgt cacttcatct cttcactaat 8580

ggagatagct ttacactttc tgctttaata gatttaagtg gaccccaata tttattaaaa 8640

ttgctagttt accgttcaga agtataatag aaataatctt tagttgctct tttctaacca 8700

ttgtaattct tcccttcttc cctccacctt tccttcattg aataaacctc tgttcaaaga 8760

gattgcctgc aagggaaata aaaatgacta agatattaaa aaaaaaaaaa aaaaa 8815

<210> 274
<211> 3178
<212> DNA
<213> Homo sapiens

<400> 274
gttgcctgtc tctaaacccc tccacattcc cgcggtcctt cagactgccc ggagagcgcg 60

ctctgcctgc cgcctgcctg cctgccactg agggttccca gcaccatgag ggcctggatc 120

ttctttctcc tttgcctggc cgggagggcc ttggcagccc ctcagcaaga agccctgcct 180

gatgagacag aggtggtgga agaaactgtg gcagaggtga ctgaggtatc tgtgggagct 240

aatcctgtcc aggtggaagt aggagaattt gatgatggtg cagaggaaac cgaagaggag 300

gtggtggcgg aaaatccctg ccagaaccac cactgcaaac acggcaaggt gtgcgagctg 360

gatgagaaca acacccccat gtgcgtgtgc caggacccca ccagctgccc agcccccatt 420

ggcgagtttg agaaggtgtg cagcaatgac aacaagacct tcgactcttc ctgccacttc 480

tttgccacaa agtgcaccct ggagggcacc aagaagggcc acaagctcca cctggactac 540
atcgggcctt gcaaatacat ccccccttgc ctggactctg agctgaccga attccccctg 600
cgcatgcggg actggctcaa gaacgtcctg gtcaccctgt atgagaggga tgaggacaac 660
aaccttctga ctgagaagca gaagctgcgg gtgaagaaga tccatgagaa tgagaagcgc 720
ctggaggcag gagaccaccc cgtggagctg ctggcccggg acttcgagaa gaactataac 780
atgtacatct tccctgtaca ctggcagttc ggccagctgg accagcaccc cattgacggg 840
tacctctccc acaccgagct ggctccactg cgtgctcccc tcatccccat ggagcattgc 900
accacccgct ttttcgagac ctgtgacctg gacaatgaca agtacatcgc cctggatgag 960
tgggccggct gcttcggcat caagcagaag gatatcgaca aggatcttgt gatctaaatc 1020
cactccttcc acagtaccgg attctctctt taaccctccc cttcgtgttt cccccaatgt 1080
ttaaaatgtt tggatggttt gttgttctgc ctggagacaa ggtgctaaca tagatttaag 1140
tgaatacatt aacggtgcta aaaatgaaaa ttctaaccca agacatgaca ttcttagctg 1200
taacttaact attaaggcct tttccacacg cattaatagt cccatttttc tcttgccatt 1260
tgtagctttg cccattgtct tattggcaca tgggtggaca cggatctgct gggctctgcc 1320
ttaaacacac attgcagctt caacttttct ctttagtgtt ctgtttgaaa ctaatactta 1380
ccgagtcaga ctttgtgttc atttcatttc agggtcttgg ctgcctgtgg gcttccccag 1440
gtggcctgga ggtgggcaaa gggaagtaac agacacacga tgttgtcaag gatggttttg 1500
ggactagagg ctcagtggtg ggagagatcc ctgcagaacc caccaaccag aacgtggttt 1560
gcctgaggct gtaactgaga gaaagattct ggggctgtgt tatgaaaata tagacattct 1620
cacataagcc cagttcatca ccatttcctc ctttaccttt cagtgcagtt tcttttcaca 1680
ttaggctgtt ggttcaaact tttgggagca cggactgtca gttctctggg aagtggtcag 1740
cgcatcctgc agggcttctc ctcctctgtc ttttggagaa ccagggctct tctcaggggc 1800
tctagggact gccaggctgt ttcagccagg aaggccaaaa tcaagagtga gatgtagaaa 1860
gttgtaaaat agaaaaagtg gagttggtga atcggttgtt ctttcctcac atttggatga 1920
ttgtcataag gtttttagca tgttcctcct tttcttcacc ctcccctttt ttcttctatt 1980
aatcaagaga aacttcaaag ttaatgggat ggtcggatct cacaggctga gaactcgttc 2040
acctccaagc atttcatgaa aaagctgctt cttattaatc atacaaactc tcaccatgat 2100
gtgaagagtt tcacaaatcc ttcaaaataa aaagtaatga cttagaaact gccttcctgg 2160
gtgatttgca tgtgtcttag tcttagtcac cttattatcc tgacacaaaa acacatgagc 2220
atacatgtct acacatgact acacaaatgc aaacctttgc aaacacatta tgcttttgca 2280
cacacacacc tgtacacaca caccggcatg tttatacaca gggagtgtat ggttcctgta 2340
agcactaagt tagctgtttt catttaatga cctgtggttt aacccttttg atcactacca 2400
ccattatcag caccagactg agcagctata tccttttatt aatcatggtc attcattcat 2460
tcattcattc acaaaatatt tatgatgtat ttactctgca ccaggtccca tgccaagcac 2520

tggggacaca gttatggcaa agtagacaaa gcatttgttc atttggagct tagagtccag 2580

gaggaataca ttagataatg acacaatcaa atataaattg caagatgtca caggtgtgat 2640

gaagggagag taggagagac catgagtatg tgtaacagga ggacacagca ttattctagt 2700

gctgtactgt tccgtacggc agccactacc cacatgtaac tttttaagat ttaaatttaa 2760

attagttaac attcaaaacg cagctcccca atcacactag caacatttca agtgcttgag 2820

agccatgcat gattagtggt taccctattg aataggtcag aagtagaatc ttttcatcat 2880

cacagaaagt tctattggac agtgctcttc tagatcatca taagactaca gagcactttt 2940

caaagctcat gcatgttcat catgttagtg tcgtattttg agctggggtt ttgagactcc 3000

ccttagagat agagaaacag acccaagaaa tgtgctcaat tgcaatgggc cacataccta 3060

gatctccaga tgtcatttcc cctctcttat tttaagttat gttaagatta ctaaaacaat 3120

aaaagctcct aaaaaatcaa aaaaaaaaaa aaaaaaaaaa aaaaaaaaaa aaaaaaaa 3178

<210> 275
<211> 1519
<212> DNA
<213> Homo sapiens

<400> 275
cagggtccca gatgcacagg aggagaagca ggagctgtcg ggaagatcag aagccagtca 60

tggatgacca gcgcgacctt atctccaaca atgagcaact gcccatgctg ggccggcgcc 120

ctggggcccc ggagagcaag tgcagccgcg gagccctgta cacaggcttt tccatcctgg 180

tgactctgct cctcgctggc caggccacca ccgcctactt cctgtaccag cagcagggcc 240

ggctggacaa actgacagtc acctcccaga acctgcagct ggagaacctg cgcatgaagc 300

ttcccaagcc tcccaagcct gtgagcaaga tgcgcatggc caccccgctg ctgatgcagg 360

cgctgcccat gggagccctg ccccaggggc ccatgcagaa tgccaccaag tatggcaaca 420

tgacagagga ccatgtgatg cacctgctcc agaatgctga ccccctgaag gtgtacccgc 480

cactgaaggg gagcttcccg gagaacctga gacaccttaa gaacaccatg gagaccatag 540

actggaaggt ctttgagagc tggatgcacc attggctcct gtttgaaatg agcaggcact 600

ccttggagca aaagcccact gacgctccac cgaaagtact gaccaagtgc caggaagagg 660

tcagccacat ccctgctgtc cacccgggtt cattcaggcc caagtgcgac gagaacggca 720

actatctgcc actccagtgc tatgggagca tcggctactg ctggtgtgtc ttccccaacg 780

gcacggaggt ccccaacacc agaagccgcg ggcaccataa ctgcagtgag tcactggaac 840

tggaggaccc gtcttctggg ctgggtgtga ccaagcagga tctgggccca gtccccatgt 900

gagagcagca gaggcggtct tcaacatcct gccagcccca cacagctaca gctttcttgc 960

tcccttcagc ccccagcccc tcccccatct cccaccctgt acctcatccc atgagaccct 1020

ggtgcctggc tctttcgtca cccttggaca agacaaacca agtcggaaca gcagataaca 1080

atgcagcaag gccctgctgc ccaatctcca tctgtcaaca ggggcgtgag gtcccaggaa 1140

gtggccaaaa gctagacaga tccccgttcc tgacatcaca gcagcctcca acacaaggct 1200

ccaagaccta ggctcatgga cgagatggga aggcacaggg agaagggata accctacacc 1260

cagaccccag gctggacatg ctgactgtcc tctcccctcc agcctttggc cttggctttt 1320

ctagcctatt tacctgcagg ctgagccact ctcttccctt tccccagcat cactccccaa 1380

ggaagagcca atgttttcca cccataatcc tttctgccga cccctagttc cctctgctca 1440

gccaagcttg ttatcagctt tcagggccat ggttcacatt agaataaaag gtagtaatta 1500

gaaaaaaaaa aaaaaaaaa 1519

<210> 276
<211> 901
<212> DNA
<213> Homo sapiens

<400> 276
ggccacatgg actggggtgc aatgggacag ctgctgccag cgagagggac cagggcacca 60

ctctctaggg agcccacact gcaagtcagg ccacaaggac ctctgaccct gagggccgat 120

gaggccaggg acaggccagg ggggccttga ggcccctggt gagccaggcc ccaacctcag 180

gcagcgctgg cccctgctgc tgctgggtct ggccgtggta acccatggcc tgctgcgccc 240

aacagctgca tcgcagagca gggccctggg ccctggagcc cctggaggaa gcagccggtc 300

cagcctgagg agccggtggg gcaggttcct gctccagcgc ggctcctgga ctggccccag 360

gtgctggccc cgggggtttc aatccaagca taactcagtg acgcatgtgt ttggcagcgg 420

gacccagctc accgttttaa gtcagcccaa ggccaccccc tcggtcactc tgttcccgcc 480

gtcctctgag gagctccaag ccaacaaggc tacactggtg tgtctcatga atgactttta 540

tccgggaatc ttgacggtga cctggaaggc agatggtacc cccatcaccc agggcgtgga 600

gatgaccacg ccctccaaac agagcaacaa caagtacgcg gccagcagct acctgagcct 660

gacgcccgag cagtggaggt cccgcagaag ctacagctgc caggtcatgc acgaagggag 720

caccgtggag aagacggtgg cccctgcaga atgttcatag gttcccagcc ccgaccccac 780

ccaaaggggc ctggagctgc aggatcccag gggaagggtc tctctctgca tcccaagcca 840

tccagccctt ctccctgtac ccagtaaacc ctaaataaat accctctttg tcaaccagaa 900

a 901

<210> 277
<211> 23
<212> DNA
<213> Homo sapiens

<400> 277
ctgactgact gactgactga ctg 23

<210> 278
<211> 21
<212> DNA
<213> Homo sapiens

<400>  278
ttgtacgttt acatggaggt c                                                21

<210>  279
<211>  22
<212>  DNA
<213>  Homo sapiens

<400>  279
aaccacacaa cctactacct ca                                               22

<210>  280
<211>  20
<212>  DNA
<213>  Homo sapiens

<400>  280
tgtattcctc gcctgtccag                                                  20

<210>  281
<211>  22
<212>  DNA
<213>  Homo sapiens

<400>  281
tcgccctctc aacccagctt tt                                               22

<210>  282
<211>  22
<212>  DNA
<213>  Homo sapiens

<400>  282
aactatacaa cctactacct ca                                               22

<210>  283
<211>  22
<212>  DNA
<213>  Homo sapiens

<400>  283
aaccatacaa cctactacct ca                                               22

<210>  284
<211>  21
<212>  DNA
<213>  Homo sapiens

<400>  284
gctgagtgta ggatgtttac a                                                21

<210>  285
<211>  22
<212>  DNA
<213>  Homo sapiens

<400>  285
acacaaattc ggttctacag gg                                               22

<210>  286

<211>  20
<212>  DNA
<213>  Homo sapiens

<400>  286
cattgaggct cgctgagagt                                                    20


<210>  287
<211>  21
<212>  DNA
<213>  Homo sapiens

<400>  287
tgagctcctg gaggacaggg a                                                  21


<210>  288
<211>  20
<212>  DNA
<213>  Homo sapiens

<400>  288
cttgtaccag ttatctgcaa                                                    20


<210>  289
<211>  68
<212>  RNA
<213>  Homo sapiens

<400>  289
ccucuacuuu aacauggagg cacuugcugu gacaugacaa aaauaagugc uuccauguuu      60

gagugugg                                                                 68


<210>  290
<211>  83
<212>  RNA
<213>  Homo sapiens

<400>  290
cggggugagg uaguagguug ugugguuuca gggcagugau guugccccuc ggaagauaac      60

uauacaaccu acugccuucc cug                                                83


<210>  291
<211>  83
<212>  RNA
<213>  Homo sapiens

<400>  291
gugaguggga gccccagugu gugguugggg ccauggcggg ugggcagccc agccucugag      60

ccuuccucgu cugucugccc cag                                                83


<210>  292
<211>  82
<212>  RNA
<213>  Homo sapiens

<400>  292
   gcuucgcucc ccuccgccuu cucuucccgg uucuucccgg agucgggaaa agcugguugg      60
   agagggcgaa aaaggaugag gu                                                 82

<210>  293
<211>  80
<212>  RNA
<213>  Homo sapiens

<400>  293
ugggaugagg uaguagguug uauaguuuua gggucacacc caccacuggg agauaacuau      60

acaaucuacu gucuuuccua                                                  80


<210>  294
<211>  84
<212>  RNA
<213>  Homo sapiens

<400>  294
gcauccgggu ugagguagua gguuguaugg uuuagaguua cacccuggga guuaacugua      60

caaccuucua gcuuuccuug gagc                                             84


<210>  295
<211>  88
<212>  RNA
<213>  Homo sapiens

<400>  295
accaaguuuc aguucaugua aacauccuac acucagcugu aauacaugga uuggcuggga      60

gguggauguu uacuucagcu gacuugga                                         88


<210>  296
<211>  110
<212>  RNA
<213>  Homo sapiens

<400>  296
ccagagguug uaacguuguc uauauauacc cuguagaacc gaauuugugu gguauccgua      60

uagucacaga uucgauucua ggggaauaua uggucgaugc aaaaacuuca                110


<210>  297
<211>  84
<212>  RNA
<213>  Homo sapiens

<400>  297
cuccccaugg cccugucucc caacccuugu accagugcug ggcucagacc cugguacagg      60

ccugggggac agggaccugg ggac                                             84


<210>  298
<211>  25
<212>  DNA
<213>  Homo sapiens

<400>  298
ggaaagcgcc gagatgacgg gcttt                                            25


<210>  299
<211>  25

<212>  DNA
<213>  Homo sapiens

<400>  299
gatgacgggc tttctgctgc cgccc                                          25


<210>  300
<211>  25
<212>  DNA
<213>  Homo sapiens

<400>  300
cccaagtagc tttgtggctt cgtgt                                          25


<210>  301
<211>  25
<212>  DNA
<213>  Homo sapiens

<400>  301
tagctttgtg gcttcgtgtc caacc                                          25


<210>  302
<211>  25
<212>  DNA
<213>  Homo sapiens

<400>  302
tgtggcttcg tgtccaaccc tcttg                                          25


<210>  303
<211>  25
<212>  DNA
<213>  Homo sapiens

<400>  303
cgcctgtgtg cctggagcca gtccc                                          25


<210>  304
<211>  25
<212>  DNA
<213>  Homo sapiens

<400>  304
gctcgcgttt cctcctgtag tgctc                                          25


<210>  305
<211>  25
<212>  DNA
<213>  Homo sapiens

<400>  305
gtttcctcct gtagtgctca caggt                                          25


<210>  306
<211>  25
<212>  DNA
<213>  Homo sapiens

<400>  306
agtgctcaca ggtcccagca ccgat                                          25

<210>  307
<211>  25
<212>  DNA
<213>  Homo sapiens

<400>  307
tcccagcacc gatggcattc ccttt                                          25


<210>  308
<211>  25
<212>  DNA
<213>  Homo sapiens

<400>  308
tccctttgcc ctgagtctgc agcgg                                          25


<210>  309
<211>  25
<212>  DNA
<213>  Homo sapiens

<400>  309
tgccctgagt ctgcagcggg tccct                                          25


<210>  310
<211>  25
<212>  DNA
<213>  Homo sapiens

<400>  310
tcaggtagcc tctcttcccc ttggg                                          25


<210>  311
<211>  25
<212>  DNA
<213>  Homo sapiens

<400>  311
acccgcggta accagcgtga gctcg                                          25


<210>  312
<211>  25
<212>  DNA
<213>  Homo sapiens

<400>  312
gcccgccaga agaatatgaa aaagc                                          25


<210>  313
<211>  25
<212>  DNA
<213>  Homo sapiens

<400>  313
gactcggtta agggaaagcg ccgag                                          25


<210>  314
<211>  25
<212>  DNA

<213>  Homo sapiens

<400>  314
ttatgaatgt ccaaatctgt gtttc                                              25


<210>  315
<211>  25
<212>  DNA
<213>  Homo sapiens

<400>  315
atgaatgtcc aaatctgtgt ttccc                                              25


<210>  316
<211>  25
<212>  DNA
<213>  Homo sapiens

<400>  316
gaatgtccaa atctgtgttt ccccc                                              25


<210>  317
<211>  25
<212>  DNA
<213>  Homo sapiens

<400>  317
atgtccaaat ctgtgtttcc ccctg                                              25


<210>  318
<211>  25
<212>  DNA
<213>  Homo sapiens

<400>  318
ctcccagact gtgtggccag ttgaa                                              25


<210>  319
<211>  25
<212>  DNA
<213>  Homo sapiens

<400>  319
agactgtgtg gccagttgaa agtgt                                              25


<210>  320
<211>  25
<212>  DNA
<213>  Homo sapiens

<400>  320
actgtgtggc cagttgaaag tgtct                                              25


<210>  321
<211>  25
<212>  DNA
<213>  Homo sapiens

<400>  321
tggccagttg aaagtgtctg gtttg                                              25

<210>  322
<211>  25
<212>  DNA
<213>  Homo sapiens

<400>  322
ttgaaagtgt ctggtttgtg ttcat                                          25


<210>  323
<211>  25
<212>  DNA
<213>  Homo sapiens

<400>  323
agtgtctggt ttgtgttcat ctctc                                          25


<210>  324
<211>  25
<212>  DNA
<213>  Homo sapiens

<400>  324
tgtctggttt gtgttcatct ctccc                                          25


<210>  325
<211>  25
<212>  DNA
<213>  Homo sapiens

<400>  325
gtgttcatct ctccctcatt tctgg                                          25


<210>  326
<211>  25
<212>  DNA
<213>  Homo sapiens

<400>  326
tgcatccacg cctcttttgg acatt                                          25


<210>  327
<211>  25
<212>  DNA
<213>  Homo sapiens

<400>  327
catccacgcc tcttttggac attaa                                          25


<210>  328
<211>  25
<212>  DNA
<213>  Homo sapiens

<400>  328
tccacgcctc ttttggacat taaag                                          25


<210>  329
<211>  25
<212>  DNA
<213>  Homo sapiens

<400>  329
ggtggccttc ttgcaggtcc ccgta                                              25


<210>  330
<211>  25
<212>  DNA
<213>  Homo sapiens

<400>  330
tggccttctt gcaggtcccc gtagc                                              25


<210>  331
<211>  25
<212>  DNA
<213>  Homo sapiens

<400>  331
ggccttcttg caggtccccg tagca                                              25


<210>  332
<211>  25
<212>  DNA
<213>  Homo sapiens

<400>  332
gccttcttgc aggtccccgt agcac                                              25


<210>  333
<211>  25
<212>  DNA
<213>  Homo sapiens

<400>  333
tcttgcaggt ccccgtagca ccctg                                              25


<210>  334
<211>  25
<212>  DNA
<213>  Homo sapiens

<400>  334
tgcaggtccc cgtagcaccc tgagc                                              25


<210>  335
<211>  25
<212>  DNA
<213>  Homo sapiens

<400>  335
aggtccccgt agcaccctga gcctg                                              25


<210>  336
<211>  25
<212>  DNA
<213>  Homo sapiens

<400>  336
ggtccccgta gcaccctgag cctgt                                              25

<210>  337
<211>  25
<212>  DNA
<213>  Homo sapiens

<400>  337
ccgtagcacc ctgagcctgt acctt                                          25


<210>  338
<211>  25
<212>  DNA
<213>  Homo sapiens

<400>  338
tagcaccctg agcctgtacc ttggg                                          25


<210>  339
<211>  25
<212>  DNA
<213>  Homo sapiens

<400>  339
caccctgagc ctgtaccttg ggtgg                                          25


<210>  340
<211>  25
<212>  DNA
<213>  Homo sapiens

<400>  340
accctgagcc tgtaccttgg gtggc                                          25


<210>  341
<211>  25
<212>  DNA
<213>  Homo sapiens

<400>  341
ccctgagcct gtaccttggg tggca                                          25


<210>  342
<211>  25
<212>  DNA
<213>  Homo sapiens

<400>  342
gagcctgtac cttgggtggc acttg                                          25


<210>  343
<211>  25
<212>  DNA
<213>  Homo sapiens

<400>  343
gcctgtacct tgggtggcac ttgtt                                          25


<210>  344
<211>  25
<212>  DNA
<213>  Homo sapiens

<400>  344
tgctgcctct ggggacatgc ggagt                                              25


<210>  345
<211>  25
<212>  DNA
<213>  Homo sapiens

<400>  345
ggggaagcct tcctctcaat ttgtt                                              25


<210>  346
<211>  25
<212>  DNA
<213>  Homo sapiens

<400>  346
gggaagcctt cctctcaatt tgttg                                              25


<210>  347
<211>  25
<212>  DNA
<213>  Homo sapiens

<400>  347
ggaagccttc ctctcaattt gttgt                                              25


<210>  348
<211>  25
<212>  DNA
<213>  Homo sapiens

<400>  348
gaagccttcc tctcaatttg ttgtc                                              25


<210>  349
<211>  25
<212>  DNA
<213>  Homo sapiens

<400>  349
aagccttcct ctcaatttgt tgtca                                              25


<210>  350
<211>  25
<212>  DNA
<213>  Homo sapiens

<400>  350
agccttcctc tcaatttgtt gtcag                                              25


<210>  351
<211>  25
<212>  DNA
<213>  Homo sapiens

<400>  351
ccttcctctc aatttgttgt cagtg                                              25


<210>  352

<211>  25
<212>  DNA
<213>  Homo sapiens

<400>  352
cttcctctca atttgttgtc agtga                                              25


<210>  353
<211>  25
<212>  DNA
<213>  Homo sapiens

<400>  353
ttcctctcaa tttgttgtca gtgaa                                              25


<210>  354
<211>  25
<212>  DNA
<213>  Homo sapiens

<400>  354
tcctctcaat ttgttgtcag tgaaa                                              25


<210>  355
<211>  25
<212>  DNA
<213>  Homo sapiens

<400>  355
cctctcaatt tgttgtcagt gaaat                                              25


<210>  356
<211>  25
<212>  DNA
<213>  Homo sapiens

<400>  356
ctctcaattt gttgtcagtg aaatt                                              25


<210>  357
<211>  25
<212>  DNA
<213>  Homo sapiens

<400>  357
aattccaata aatgggattt gctct                                              25


<210>  358
<211>  25
<212>  DNA
<213>  Homo sapiens

<400>  358
tgagggtgca cgtcttccct cctgt                                              25


<210>  359
<211>  25
<212>  DNA
<213>  Homo sapiens

<400>  359

tggagtgctg cctctgggga catgc                                          25

<210>  360
<211>  25
<212>  DNA
<213>  Homo sapiens

<400>  360
ggttaatccg caagccccag ccccg                                          25

<210>  361
<211>  25
<212>  DNA
<213>  Homo sapiens

<400>  361
ttaatccgca agccccagcc ccgag                                          25

<210>  362
<211>  25
<212>  DNA
<213>  Homo sapiens

<400>  362
ggcgtccccc agagcctgag aaagc                                          25

<210>  363
<211>  25
<212>  DNA
<213>  Homo sapiens

<400>  363
ccccagagcc tgagaaagcg cctcc                                          25

<210>  364
<211>  25
<212>  DNA
<213>  Homo sapiens

<400>  364
ccagagcctg agaaagcgcc tcccg                                          25

<210>  365
<211>  25
<212>  DNA
<213>  Homo sapiens

<400>  365
gagcctgaga aagcgcctcc cgctg                                          25

<210>  366
<211>  25
<212>  DNA
<213>  Homo sapiens

<400>  366
gcctgagaaa gcgcctcccg ctgcc                                          25

<210>  367
<211>  25

<212>  DNA
<213>  Homo sapiens

<400>  367
ctgagaaagc gcctcccgct gcccc                                              25


<210>  368
<211>  25
<212>  DNA
<213>  Homo sapiens

<400>  368
tgccccgacg cggccctcgg ccctg                                              25


<210>  369
<211>  25
<212>  DNA
<213>  Homo sapiens

<400>  369
ctcggccctg gagctgaagg tggag                                              25


<210>  370
<211>  25
<212>  DNA
<213>  Homo sapiens

<400>  370
cggccctgga gctgaaggtg gagga                                              25


<210>  371
<211>  25
<212>  DNA
<213>  Homo sapiens

<400>  371
gccctggagc tgaaggtgga ggagc                                              25


<210>  372
<211>  25
<212>  DNA
<213>  Homo sapiens

<400>  372
cctggagctg aaggtggagg agctg                                              25


<210>  373
<211>  25
<212>  DNA
<213>  Homo sapiens

<400>  373
gctgaaggtg gaggagctgg aggag                                              25


<210>  374
<211>  25
<212>  DNA
<213>  Homo sapiens

<400>  374
aaggtggagg agctggagga gaagg                                              25

<210>  375
<211>  25
<212>  DNA
<213>  Homo sapiens

<400>  375
gactgcttga aaccaggagt ttgag                                              25


<210>  376
<211>  25
<212>  DNA
<213>  Homo sapiens

<400>  376
gcttgaaacc aggagtttga gacca                                              25


<210>  377
<211>  25
<212>  DNA
<213>  Homo sapiens

<400>  377
aaccaggagt ttgagaccag cctga                                              25


<210>  378
<211>  25
<212>  DNA
<213>  Homo sapiens

<400>  378
ttgagaccag cctgagcaac aaagc                                              25


<210>  379
<211>  25
<212>  DNA
<213>  Homo sapiens

<400>  379
agaccagcct gagcaacaaa gcaag                                              25


<210>  380
<211>  25
<212>  DNA
<213>  Homo sapiens

<400>  380
gagcaacaaa gcaagacccc atctc                                              25


<210>  381
<211>  25
<212>  DNA
<213>  Homo sapiens

<400>  381
caacaaagca agaccccatc tctat                                              25


<210>  382
<211>  25
<212>  DNA

<213>  Homo sapiens

<400>  382
aagcaagacc ccatctctat aaaaa                                              25


<210>  383
<211>  25
<212>  DNA
<213>  Homo sapiens

<400>  383
aagacagggt cttgctcatg ttgta                                              25


<210>  384
<211>  25
<212>  DNA
<213>  Homo sapiens

<400>  384
attagttggg catggtggca catgc                                              25


<210>  385
<211>  25
<212>  DNA
<213>  Homo sapiens

<400>  385
agttgggcat ggtggcacat gcctg                                              25


<210>  386
<211>  25
<212>  DNA
<213>  Homo sapiens

<400>  386
atcatctgag cctcaggagg ttgag                                              25


<210>  387
<211>  25
<212>  DNA
<213>  Homo sapiens

<400>  387
atctgagcct caggaggttg aggct                                              25


<210>  388
<211>  25
<212>  DNA
<213>  Homo sapiens

<400>  388
tgaggctgca gtgagctgtg actgc                                              25


<210>  389
<211>  25
<212>  DNA
<213>  Homo sapiens

<400>  389
cttgctcatg ttgtacattc atcat                                              25

<210>  390
<211>  25
<212>  DNA
<213>  Homo sapiens

<400>  390
aagaggctgg gtgcagtggc tcaca                                          25


<210>  391
<211>  25
<212>  DNA
<213>  Homo sapiens

<400>  391
tcatttcctg gtatgacaac gaatt                                          25


<210>  392
<211>  25
<212>  DNA
<213>  Homo sapiens

<400>  392
acaacgaatt tggctacagc aacag                                          25


<210>  393
<211>  25
<212>  DNA
<213>  Homo sapiens

<400>  393
gggtggtgga cctcatggcc cacat                                          25


<210>  394
<211>  25
<212>  DNA
<213>  Homo sapiens

<400>  394
tcatggccca catggcctcc aagga                                          25


<210>  395
<211>  25
<212>  DNA
<213>  Homo sapiens

<400>  395
acatggcctc caaggagtaa gaccc                                          25


<210>  396
<211>  25
<212>  DNA
<213>  Homo sapiens

<400>  396
aggagtaaga cccctggacc accag                                          25


<210>  397
<211>  25
<212>  DNA
<213>  Homo sapiens

<400>  397
gccccagcaa gagcacaaga ggaag                                           25


<210>  398
<211>  25
<212>  DNA
<213>  Homo sapiens

<400>  398
gagagagacc ctcactgctg gggag                                           25


<210>  399
<211>  25
<212>  DNA
<213>  Homo sapiens

<400>  399
cctcactgct ggggagtccc tgcca                                           25


<210>  400
<211>  25
<212>  DNA
<213>  Homo sapiens

<400>  400
cctcctcaca gttgccatgt agacc                                           25


<210>  401
<211>  25
<212>  DNA
<213>  Homo sapiens

<400>  401
agttgccatg tagacccctt gaaga                                           25


<210>  402
<211>  25
<212>  DNA
<213>  Homo sapiens

<400>  402
catgtagacc ccttgaagag gggag                                           25


<210>  403
<211>  25
<212>  DNA
<213>  Homo sapiens

<400>  403
tagggagccg caccttgtca tgtac                                           25


<210>  404
<211>  25
<212>  DNA
<213>  Homo sapiens

<400>  404
gccgcacctt gtcatgtacc atcaa                                           25

<210>  405
<211>  25
<212>  DNA
<213>  Homo sapiens

<400>  405
tgtcatgtac catcaataaa gtacc                                          25


<210>  406
<211>  25
<212>  DNA
<213>  Homo sapiens

<400>  406
cctctgactt caacagcgac accca                                          25


<210>  407
<211>  25
<212>  DNA
<213>  Homo sapiens

<400>  407
gggctggcat tgccctcaac gacca                                          25


<210>  408
<211>  25
<212>  DNA
<213>  Homo sapiens

<400>  408
ccctcaacga ccactttgtc aagct                                          25


<210>  409
<211>  25
<212>  DNA
<213>  Homo sapiens

<400>  409
accactttgt caagctcatt tcctg                                          25


<210>  410
<211>  25
<212>  DNA
<213>  Homo sapiens

<400>  410
ttgtcaagct catttcctgg tatga                                          25


<210>  411
<211>  25
<212>  DNA
<213>  Homo sapiens

<400>  411
gaattctggt accgtcagca tccac                                          25


<210>  412
<211>  25
<212>  DNA
<213>  Homo sapiens

<400>  412
gagagagacc tcatctttca tgctt                                          25


<210>  413
<211>  25
<212>  DNA
<213>  Homo sapiens

<400>  413
tgactctcct gggggcacct cctat                                          25


<210>  414
<211>  25
<212>  DNA
<213>  Homo sapiens

<400>  414
actctcctgg gggcacctcc tatga                                          25


<210>  415
<211>  25
<212>  DNA
<213>  Homo sapiens

<400>  415
tcctgggggc acctcctatg agaga                                          25


<210>  416
<211>  25
<212>  DNA
<213>  Homo sapiens

<400>  416
cctgggggca cctcctatga gagat                                          25


<210>  417
<211>  25
<212>  DNA
<213>  Homo sapiens

<400>  417
ctgggggcac ctcctatgag agata                                          25


<210>  418
<211>  25
<212>  DNA
<213>  Homo sapiens

<400>  418
tgggggcacc tcctatgaga gatac                                          25


<210>  419
<211>  25
<212>  DNA
<213>  Homo sapiens

<400>  419
gggggcacct cctatgagag atacg                                          25


<210>  420

<211>  25
<212>  DNA
<213>  Homo sapiens

<400>  420
ggggcacctc ctatgagaga tacga                                              25


<210>  421
<211>  25
<212>  DNA
<213>  Homo sapiens

<400>  421
gggcacctcc tatgagagat acgat                                              25


<210>  422
<211>  25
<212>  DNA
<213>  Homo sapiens

<400>  422
ggcacctcct atgagagata cgatt                                              25


<210>  423
<211>  25
<212>  DNA
<213>  Homo sapiens

<400>  423
gcacctccta tgagagatac gattg                                              25


<210>  424
<211>  25
<212>  DNA
<213>  Homo sapiens

<400>  424
cacctcctat gagagatacg attgc                                              25


<210>  425
<211>  25
<212>  DNA
<213>  Homo sapiens

<400>  425
acctcctatg agagatacga ttgct                                              25


<210>  426
<211>  25
<212>  DNA
<213>  Homo sapiens

<400>  426
cctcctatga gagatacgat tgcta                                              25


<210>  427
<211>  25
<212>  DNA
<213>  Homo sapiens

<400>  427

ctcctattcc ggactcagac ctctg                                              25


<210>  428
<211>  25
<212>  DNA
<213>  Homo sapiens

<400>  428
tcctattccg gactcagacc tctga                                              25


<210>  429
<211>  25
<212>  DNA
<213>  Homo sapiens

<400>  429
cctattccgg actcagacct ctgac                                              25


<210>  430
<211>  25
<212>  DNA
<213>  Homo sapiens

<400>  430
ctattccgga ctcagacctc tgacc                                              25


<210>  431
<211>  25
<212>  DNA
<213>  Homo sapiens

<400>  431
attccggact cagacctctg accct                                              25


<210>  432
<211>  25
<212>  DNA
<213>  Homo sapiens

<400>  432
ttccggactc agacctctga ccctg                                              25


<210>  433
<211>  25
<212>  DNA
<213>  Homo sapiens

<400>  433
cggactcaga cctctgaccc tgcaa                                              25


<210>  434
<211>  25
<212>  DNA
<213>  Homo sapiens

<400>  434
ggactcagac ctctgaccct gcaat                                              25


<210>  435
<211>  25

<212>  DNA
<213>  Homo sapiens

<400>  435
actcagacct ctgaccctgc aatgc                                          25


<210>  436
<211>  25
<212>  DNA
<213>  Homo sapiens

<400>  436
cagacctctg accctgcaat gctgc                                          25


<210>  437
<211>  25
<212>  DNA
<213>  Homo sapiens

<400>  437
acctctgacc ctgcaatgct gccta                                          25


<210>  438
<211>  25
<212>  DNA
<213>  Homo sapiens

<400>  438
tctgaccctg caatgctgcc tacca                                          25


<210>  439
<211>  25
<212>  DNA
<213>  Homo sapiens

<400>  439
ctgaccctgc aatgctgcct accat                                          25


<210>  440
<211>  25
<212>  DNA
<213>  Homo sapiens

<400>  440
tgaccctgca atgctgccta ccatg                                          25


<210>  441
<211>  25
<212>  DNA
<213>  Homo sapiens

<400>  441
accctgcaat gctgcctacc atgat                                          25


<210>  442
<211>  25
<212>  DNA
<213>  Homo sapiens

<400>  442
cctgcaatgc tgcctaccat gattg                                          25

<210>  443
<211>  25
<212>  DNA
<213>  Homo sapiens

<400>  443
aggcacgtac caccatgccc agata                                          25


<210>  444
<211>  25
<212>  DNA
<213>  Homo sapiens

<400>  444
ttttttgaga caaagtcctc actct                                          25


<210>  445
<211>  25
<212>  DNA
<213>  Homo sapiens

<400>  445
ggggtttcac catgttggct aggat                                          25


<210>  446
<211>  25
<212>  DNA
<213>  Homo sapiens

<400>  446
ccatgttggc taggatggtc tccat                                          25


<210>  447
<211>  25
<212>  DNA
<213>  Homo sapiens

<400>  447
gttggctagg atggtctcca tcgcc                                          25


<210>  448
<211>  25
<212>  DNA
<213>  Homo sapiens

<400>  448
ctaggatggt ctccatcgcc tgacc                                          25


<210>  449
<211>  25
<212>  DNA
<213>  Homo sapiens

<400>  449
tgagacaaag tcctcactct gtcac                                          25


<210>  450
<211>  25
<212>  DNA

<213>  Homo sapiens

<400>  450
cttggcctcc caaagtgctg ggatt                                              25


<210>  451
<211>  25
<212>  DNA
<213>  Homo sapiens

<400>  451
cctcccaaag tgctgggatt acagg                                              25


<210>  452
<211>  25
<212>  DNA
<213>  Homo sapiens

<400>  452
ggattacagg catgagccac cacag                                              25


<210>  453
<211>  25
<212>  DNA
<213>  Homo sapiens

<400>  453
caaagtcctc actctgtcac caagt                                              25


<210>  454
<211>  25
<212>  DNA
<213>  Homo sapiens

<400>  454
gcatgagcca ccacagctgg ccgta                                              25


<210>  455
<211>  25
<212>  DNA
<213>  Homo sapiens

<400>  455
gagccaccac agctggccgt aaata                                              25


<210>  456
<211>  25
<212>  DNA
<213>  Homo sapiens

<400>  456
gtgcagtggc agcaatctca gctca                                              25


<210>  457
<211>  25
<212>  DNA
<213>  Homo sapiens

<400>  457
gtggcagcaa tctcagctca ctgca                                              25

<210>  458
<211>  25
<212>  DNA
<213>  Homo sapiens

<400>  458
agcaatctca gctcactgca aacct                                          25


<210>  459
<211>  25
<212>  DNA
<213>  Homo sapiens

<400>  459
caccgcgcct ggccctaaat agatt                                          25


<210>  460
<211>  25
<212>  DNA
<213>  Homo sapiens

<400>  460
gggattcatc atgttgacca ggctg                                          25


<210>  461
<211>  25
<212>  DNA
<213>  Homo sapiens

<400>  461
ttcatcatgt tgaccaggct ggcct                                          25


<210>  462
<211>  25
<212>  DNA
<213>  Homo sapiens

<400>  462
tgtttgtctt tctgataggt tgaaa                                          25


<210>  463
<211>  25
<212>  DNA
<213>  Homo sapiens

<400>  463
tgtctttctg ataggttgaa aattg                                          25


<210>  464
<211>  25
<212>  DNA
<213>  Homo sapiens

<400>  464
gttgaccagg ctggcctcaa actcc                                          25


<210>  465
<211>  25
<212>  DNA
<213>  Homo sapiens

<400>  465
accaggctgg cctcaaactc ctgac                                          25


<210>  466
<211>  25
<212>  DNA
<213>  Homo sapiens

<400>  466
aggctggcct caaactcctg acttc                                          25


<210>  467
<211>  25
<212>  DNA
<213>  Homo sapiens

<400>  467
tggcctcaaa ctcctgactt caagc                                          25


<210>  468
<211>  25
<212>  DNA
<213>  Homo sapiens

<400>  468
ctcaaactcc tgacttcaag cgatc                                          25


<210>  469
<211>  25
<212>  DNA
<213>  Homo sapiens

<400>  469
aaactcctga cttcaagcga tctcc                                          25


<210>  470
<211>  25
<212>  DNA
<213>  Homo sapiens

<400>  470
ttggcctccc aaagtgctgg gattg                                          25


<210>  471
<211>  25
<212>  DNA
<213>  Homo sapiens

<400>  471
cctcccaaag tgctgggatt gcagg                                          25


<210>  472
<211>  25
<212>  DNA
<213>  Homo sapiens

<400>  472
gctgggattg caggtgtgag ccacc                                          25

<210>  473
<211>  25
<212>  DNA
<213>  Homo sapiens

<400>  473
attgcaggtg tgagccaccg cgcct                                          25


<210>  474
<211>  25
<212>  DNA
<213>  Homo sapiens

<400>  474
tcttgacaaa acctaacttg cgcag                                          25


<210>  475
<211>  25
<212>  DNA
<213>  Homo sapiens

<400>  475
atgagattgg catggcttta tttgt                                          25


<210>  476
<211>  25
<212>  DNA
<213>  Homo sapiens

<400>  476
gcagtcggtt ggagcgagca tcccc                                          25


<210>  477
<211>  25
<212>  DNA
<213>  Homo sapiens

<400>  477
ccaaagttca caatgtggcc gagga                                          25


<210>  478
<211>  25
<212>  DNA
<213>  Homo sapiens

<400>  478
aagttcacaa tgtggccgag gactt                                          25


<210>  479
<211>  25
<212>  DNA
<213>  Homo sapiens

<400>  479
atgtggccga ggactttgat tgcac                                          25


<210>  480
<211>  25
<212>  DNA
<213>  Homo sapiens

<400>  480
ccgaggactt tgattgcaca ttgtt                                              25


<210>  481
<211>  25
<212>  DNA
<213>  Homo sapiens

<400>  481
tttaatagtc attccaaata tgaga                                              25


<210>  482
<211>  25
<212>  DNA
<213>  Homo sapiens

<400>  482
agtcattcca aatatgagat gcatt                                              25


<210>  483
<211>  25
<212>  DNA
<213>  Homo sapiens

<400>  483
tgttacagga agtcccttgc catcc                                              25


<210>  484
<211>  25
<212>  DNA
<213>  Homo sapiens

<400>  484
tacaggaagt cccttgccat cctaa                                              25


<210>  485
<211>  25
<212>  DNA
<213>  Homo sapiens

<400>  485
tcccttgcca tcctaaaagc caccc                                              25


<210>  486
<211>  25
<212>  DNA
<213>  Homo sapiens

<400>  486
cttctctcta aggagaatgg cccag                                              25


<210>  487
<211>  25
<212>  DNA
<213>  Homo sapiens

<400>  487
gaggtgatag cattgctttc gtgta                                              25


<210>  488

<211>  25
<212>  DNA
<213>  Homo sapiens

<400>  488
tattttgaat gatgagcctt cgtgc                                              25


<210>  489
<211>  25
<212>  DNA
<213>  Homo sapiens

<400>  489
tttgaatgat gagccttcgt gcccc                                              25


<210>  490
<211>  25
<212>  DNA
<213>  Homo sapiens

<400>  490
gtatgaaggc ttttggtctc cctgg                                              25


<210>  491
<211>  25
<212>  DNA
<213>  Homo sapiens

<400>  491
ggtggaggca gccagggctt acctg                                              25


<210>  492
<211>  25
<212>  DNA
<213>  Homo sapiens

<400>  492
cagggcttac ctgtacactg acttg                                              25


<210>  493
<211>  25
<212>  DNA
<213>  Homo sapiens

<400>  493
ttacctgtac actgacttga gacca                                              25


<210>  494
<211>  25
<212>  DNA
<213>  Homo sapiens

<400>  494
gcctggccaa catggtgaaa ccccg                                              25


<210>  495
<211>  25
<212>  DNA
<213>  Homo sapiens

<400>  495

gcgcgcgcct gtaatcccag ctact                                              25


<210>  496
<211>  25
<212>  DNA
<213>  Homo sapiens

<400>  496
gcgcgcctgt aatcccagct actcg                                              25


<210>  497
<211>  25
<212>  DNA
<213>  Homo sapiens

<400>  497
cgcgcctgta atcccagcta ctcgg                                              25


<210>  498
<211>  25
<212>  DNA
<213>  Homo sapiens

<400>  498
gcgcctgtaa tcccagctac tcggg                                              25


<210>  499
<211>  25
<212>  DNA
<213>  Homo sapiens

<400>  499
cgcctgtaat cccagctact cggga                                              25


<210>  500
<211>  25
<212>  DNA
<213>  Homo sapiens

<400>  500
gcctgtaatc ccagctactc gggag                                              25


<210>  501
<211>  25
<212>  DNA
<213>  Homo sapiens

<400>  501
cctgtaatcc cagctactcg ggagg                                              25


<210>  502
<211>  25
<212>  DNA
<213>  Homo sapiens

<400>  502
ctgtaatccc agctactcgg gaggc                                              25


<210>  503
<211>  25

<212>  DNA
<213>  Homo sapiens

<400>  503
tgtaatccca gctactcggg aggct                                          25


<210>  504
<211>  25
<212>  DNA
<213>  Homo sapiens

<400>  504
gtaatcccag ctactcggga ggctg                                          25


<210>  505
<211>  25
<212>  DNA
<213>  Homo sapiens

<400>  505
taatcccagc tactcgggag gctga                                          25


<210>  506
<211>  25
<212>  DNA
<213>  Homo sapiens

<400>  506
aatcccagct actcgggagg ctgag                                          25


<210>  507
<211>  25
<212>  DNA
<213>  Homo sapiens

<400>  507
atcccagcta ctcgggaggc tgagg                                          25


<210>  508
<211>  25
<212>  DNA
<213>  Homo sapiens

<400>  508
tcccagctac tcgggaggct gaggc                                          25


<210>  509
<211>  25
<212>  DNA
<213>  Homo sapiens

<400>  509
cccagctact cgggaggctg aggca                                          25


<210>  510
<211>  25
<212>  DNA
<213>  Homo sapiens

<400>  510
ccagctactc gggaggctga ggcag                                          25

<210>  511
<211>  25
<212>  DNA
<213>  Homo sapiens

<400>  511
tggtggctca cgcctgtaat cccag                                              25


<210>  512
<211>  25
<212>  DNA
<213>  Homo sapiens

<400>  512
gagccgagat cgcgccactg cactc                                              25


<210>  513
<211>  25
<212>  DNA
<213>  Homo sapiens

<400>  513
gtggctcacg cctgtaatcc cagca                                              25


<210>  514
<211>  25
<212>  DNA
<213>  Homo sapiens

<400>  514
cactgcactc cagcctgggc gacag                                              25


<210>  515
<211>  25
<212>  DNA
<213>  Homo sapiens

<400>  515
actgcactcc agcctgggcg acaga                                              25


<210>  516
<211>  25
<212>  DNA
<213>  Homo sapiens

<400>  516
ctgcactcca gcctgggcga cagag                                              25


<210>  517
<211>  25
<212>  DNA
<213>  Homo sapiens

<400>  517
tgcactccag cctgggcgac agagc                                              25


<210>  518
<211>  25
<212>  DNA

<213>  Homo sapiens

<400>  518
gcactccagc ctgggcgaca gagcg                                              25


<210>  519
<211>  25
<212>  DNA
<213>  Homo sapiens

<400>  519
cactccagcc tgggcgacag agcga                                              25


<210>  520
<211>  25
<212>  DNA
<213>  Homo sapiens

<400>  520
tggctcacgc ctgtaatccc agcac                                              25


<210>  521
<211>  25
<212>  DNA
<213>  Homo sapiens

<400>  521
actccagcct gggcgacaga gcgag                                              25


<210>  522
<211>  25
<212>  DNA
<213>  Homo sapiens

<400>  522
ctccagcctg ggcgacagag cgaga                                              25


<210>  523
<211>  25
<212>  DNA
<213>  Homo sapiens

<400>  523
tccagcctgg gcgacagagc gagac                                              25


<210>  524
<211>  25
<212>  DNA
<213>  Homo sapiens

<400>  524
ccagcctggg cgacagagcg agact                                              25


<210>  525
<211>  25
<212>  DNA
<213>  Homo sapiens

<400>  525
cagcctgggc gacagagcga gactc                                              25

<210>  526
<211>  25
<212>  DNA
<213>  Homo sapiens

<400>  526
agcctgggcg acagagcgag actcc                                          25


<210>  527
<211>  25
<212>  DNA
<213>  Homo sapiens

<400>  527
ggctcacgcc tgtaatccca gcact                                          25


<210>  528
<211>  25
<212>  DNA
<213>  Homo sapiens

<400>  528
gctcacgcct gtaatcccag cactt                                          25


<210>  529
<211>  25
<212>  DNA
<213>  Homo sapiens

<400>  529
ctcacgcctg taatcccagc acttt                                          25


<210>  530
<211>  25
<212>  DNA
<213>  Homo sapiens

<400>  530
tcacgcctgt aatcccagca ctttg                                          25


<210>  531
<211>  25
<212>  DNA
<213>  Homo sapiens

<400>  531
cacgcctgta atcccagcac tttgg                                          25


<210>  532
<211>  25
<212>  DNA
<213>  Homo sapiens

<400>  532
acgcctgtaa tcccagcact ttggg                                          25


<210>  533
<211>  25
<212>  DNA
<213>  Homo sapiens

<400>  533
cgcctgtaat cccagcactt tggga                                          25


<210>  534
<211>  25
<212>  DNA
<213>  Homo sapiens

<400>  534
gcctgtaatc ccagcacttt gggag                                          25


<210>  535
<211>  25
<212>  DNA
<213>  Homo sapiens

<400>  535
cctgtaatcc cagcactttg ggagg                                          25


<210>  536
<211>  25
<212>  DNA
<213>  Homo sapiens

<400>  536
ctgtaatccc agcactttgg gaggc                                          25


<210>  537
<211>  25
<212>  DNA
<213>  Homo sapiens

<400>  537
tgtaatccca gcactttggg aggcc                                          25


<210>  538
<211>  25
<212>  DNA
<213>  Homo sapiens

<400>  538
gtaatcccag cactttggga ggccg                                          25


<210>  539
<211>  25
<212>  DNA
<213>  Homo sapiens

<400>  539
taatcccagc actttgggag gccga                                          25


<210>  540
<211>  25
<212>  DNA
<213>  Homo sapiens

<400>  540
aatcccagca ctttgggagg ccgag                                          25

<210>  541
<211>  25
<212>  DNA
<213>  Homo sapiens

<400>  541
atcccagcac tttgggaggc cgagg                                          25


<210>  542
<211>  25
<212>  DNA
<213>  Homo sapiens

<400>  542
tcccagcact ttgggaggcc gaggt                                          25


<210>  543
<211>  25
<212>  DNA
<213>  Homo sapiens

<400>  543
cccagcactt tgggaggccg aggtg                                          25


<210>  544
<211>  25
<212>  DNA
<213>  Homo sapiens

<400>  544
gtggatcacc tgaggtcagg agttc                                          25


<210>  545
<211>  25
<212>  DNA
<213>  Homo sapiens

<400>  545
ggatcacctg aggtcaggag ttcaa                                          25


<210>  546
<211>  25
<212>  DNA
<213>  Homo sapiens

<400>  546
gatcacctga ggtcaggagt tcaag                                          25


<210>  547
<211>  25
<212>  DNA
<213>  Homo sapiens

<400>  547
atcacctgag gtcaggagtt caaga                                          25


<210>  548
<211>  25
<212>  DNA
<213>  Homo sapiens

<400>  548
tcacctgagg tcaggagttc aagac                                              25


<210>  549
<211>  25
<212>  DNA
<213>  Homo sapiens

<400>  549
aggagttcaa gaccagcctg gccaa                                              25


<210>  550
<211>  25
<212>  DNA
<213>  Homo sapiens

<400>  550
ggagttcaag accagcctgg ccaac                                              25


<210>  551
<211>  25
<212>  DNA
<213>  Homo sapiens

<400>  551
gagttcaaga ccagcctggc caaca                                              25


<210>  552
<211>  25
<212>  DNA
<213>  Homo sapiens

<400>  552
agttcaagac cagcctggcc aacat                                              25


<210>  553
<211>  25
<212>  DNA
<213>  Homo sapiens

<400>  553
gttcaagacc agcctggcca acatg                                              25


<210>  554
<211>  25
<212>  DNA
<213>  Homo sapiens

<400>  554
ttcaagacca gcctggccaa catgg                                              25


<210>  555
<211>  25
<212>  DNA
<213>  Homo sapiens

<400>  555
tcaagaccag cctggccaac atggt                                              25


<210>  556

<211>  25
<212>  DNA
<213>  Homo sapiens

<400>  556
caagaccagc ctggccaaca tggtg                                          25


<210>  557
<211>  25
<212>  DNA
<213>  Homo sapiens

<400>  557
aagaccagcc tggccaacat ggtga                                          25


<210>  558
<211>  25
<212>  DNA
<213>  Homo sapiens

<400>  558
agaccagcct ggccaacatg gtgaa                                          25


<210>  559
<211>  25
<212>  DNA
<213>  Homo sapiens

<400>  559
gaccagcctg gccaacatgg tgaaa                                          25


<210>  560
<211>  25
<212>  DNA
<213>  Homo sapiens

<400>  560
accagcctgg ccaacatggt gaaac                                          25


<210>  561
<211>  25
<212>  DNA
<213>  Homo sapiens

<400>  561
ccagcctggc caacatggtg aaacc                                          25


<210>  562
<211>  25
<212>  DNA
<213>  Homo sapiens

<400>  562
cagcctggcc aacatggtga aaccc                                          25


<210>  563
<211>  25
<212>  DNA
<213>  Homo sapiens

<400>  563

tagaattctg tgcagatgtc ctgac                                          25

<210>  564
<211>  25
<212>  DNA
<213>  Homo sapiens

<400>  564
aattctgtgc agatgtcctg acttg                                          25

<210>  565
<211>  25
<212>  DNA
<213>  Homo sapiens

<400>  565
tgacttggca attttgtgtc cctgc                                          25

<210>  566
<211>  25
<212>  DNA
<213>  Homo sapiens

<400>  566
ggcaattttg tgtccctgcc tcact                                          25

<210>  567
<211>  25
<212>  DNA
<213>  Homo sapiens

<400>  567
gtcctagtgt tgttctgcct cctgt                                          25

<210>  568
<211>  25
<212>  DNA
<213>  Homo sapiens

<400>  568
ttgttctgcc tcctgtcctc tcttg                                          25

<210>  569
<211>  25
<212>  DNA
<213>  Homo sapiens

<400>  569
ctgtcctctc ttgctctctt gtcag                                          25

<210>  570
<211>  25
<212>  DNA
<213>  Homo sapiens

<400>  570
gctctcttgt cagtctctgg cttcc                                          25

<210>  571
<211>  25

<212>  DNA
<213>  Homo sapiens

<400>  571
gtctctggct tcctcggccc cattt                                          25


<210>  572
<211>  25
<212>  DNA
<213>  Homo sapiens

<400>  572
ggccccattt cacttcactg agtcc                                          25


<210>  573
<211>  25
<212>  DNA
<213>  Homo sapiens

<400>  573
cccatttcac ttcactgagt cctga                                          25


<210>  574
<211>  25
<212>  DNA
<213>  Homo sapiens

<400>  574
tcacttcact gagtcctgac accca                                          25


<210>  575
<211>  25
<212>  DNA
<213>  Homo sapiens

<400>  575
aagggtctgt tctgctcagc tccat                                          25


<210>  576
<211>  25
<212>  DNA
<213>  Homo sapiens

<400>  576
tgctcagctc catgtccccc atttt                                          25


<210>  577
<211>  25
<212>  DNA
<213>  Homo sapiens

<400>  577
tttacagcat cctgcactcc agcct                                          25


<210>  578
<211>  25
<212>  DNA
<213>  Homo sapiens

<400>  578
tcctccacaa taaaactggg gactg                                          25

<210>  579
<211>  25
<212>  DNA
<213>  Homo sapiens

<400>  579
gctgaggctc ccttgcctga ctgtg                                             25


<210>  580
<211>  25
<212>  DNA
<213>  Homo sapiens

<400>  580
gaggctccct tgcctgactg tgact                                             25


<210>  581
<211>  25
<212>  DNA
<213>  Homo sapiens

<400>  581
ggctcccttg cctgactgtg acttg                                             25


<210>  582
<211>  25
<212>  DNA
<213>  Homo sapiens

<400>  582
gctcccttgc ctgactgtga cttgt                                             25


<210>  583
<211>  25
<212>  DNA
<213>  Homo sapiens

<400>  583
ctcccttgcc tgactgtgac ttgtg                                             25


<210>  584
<211>  25
<212>  DNA
<213>  Homo sapiens

<400>  584
tcccttgcct gactgtgact tgtgc                                             25


<210>  585
<211>  25
<212>  DNA
<213>  Homo sapiens

<400>  585
cccttgcctg actgtgactt gtgcc                                             25


<210>  586
<211>  25
<212>  DNA

<213>  Homo sapiens

<400>  586
ccttgcctga ctgtgacttg tgcct                                          25


<210>  587
<211>  25
<212>  DNA
<213>  Homo sapiens

<400>  587
cttgcctgac tgtgacttgt gcctc                                          25


<210>  588
<211>  25
<212>  DNA
<213>  Homo sapiens

<400>  588
ctgactgtga cttgtgcctc tctcc                                          25


<210>  589
<211>  25
<212>  DNA
<213>  Homo sapiens

<400>  589
tgactgtgac ttgtgcctct ctcct                                          25


<210>  590
<211>  25
<212>  DNA
<213>  Homo sapiens

<400>  590
gactgtgact tgtgcctctc tcctg                                          25


<210>  591
<211>  25
<212>  DNA
<213>  Homo sapiens

<400>  591
ctgtgacttg tgcctctctc ctgcc                                          25


<210>  592
<211>  25
<212>  DNA
<213>  Homo sapiens

<400>  592
ggtgggcagg tgacccaagg aacct                                          25


<210>  593
<211>  25
<212>  DNA
<213>  Homo sapiens

<400>  593
caggtgaccc aaggaacctt tctgg                                          25

<210>  594
<211>  25
<212>  DNA
<213>  Homo sapiens

<400>  594
tgaaggtact gaacgccacc tcact                                            25


<210>  595
<211>  25
<212>  DNA
<213>  Homo sapiens

<400>  595
aggtactgaa cgccacctca ctgta                                            25


<210>  596
<211>  25
<212>  DNA
<213>  Homo sapiens

<400>  596
gtactgaacg ccacctcact gtaag                                            25


<210>  597
<211>  25
<212>  DNA
<213>  Homo sapiens

<400>  597
tgaacgccac ctcactgtaa gacgg                                            25


<210>  598
<211>  25
<212>  DNA
<213>  Homo sapiens

<400>  598
aacgccacct cactgtaaga cggta                                            25


<210>  599
<211>  25
<212>  DNA
<213>  Homo sapiens

<400>  599
acgccacctc actgtaagac ggtag                                            25


<210>  600
<211>  25
<212>  DNA
<213>  Homo sapiens

<400>  600
gccacctcac tgtaagacgg tagat                                            25


<210>  601
<211>  25
<212>  DNA
<213>  Homo sapiens

<400>  601
ccacctcact gtaagacggt agatt                                          25


<210>  602
<211>  25
<212>  DNA
<213>  Homo sapiens

<400>  602
acctcactgt aagacggtag atttt                                          25


<210>  603
<211>  25
<212>  DNA
<213>  Homo sapiens

<400>  603
cctcactgta agacggtaga ttttg                                          25


<210>  604
<211>  25
<212>  DNA
<213>  Homo sapiens

<400>  604
tcactgtaag acggtagatt ttgta                                          25


<210>  605
<211>  25
<212>  DNA
<213>  Homo sapiens

<400>  605
gacagggctg ccttctgggt gatga                                          25


<210>  606
<211>  25
<212>  DNA
<213>  Homo sapiens

<400>  606
acagggctgc cttctgggtg atgag                                          25


<210>  607
<211>  25
<212>  DNA
<213>  Homo sapiens

<400>  607
agggctgcct tctgggtgat gagaa                                          25


<210>  608
<211>  25
<212>  DNA
<213>  Homo sapiens

<400>  608
aatcagatgg gatggctgca cggcg                                          25

<210>  609
<211>  25
<212>  DNA
<213>  Homo sapiens

<400>  609
ctgcacggcg tggtgaaggt actga                                          25


<210>  610
<211>  25
<212>  DNA
<213>  Homo sapiens

<400>  610
ctgcagttca tgtcccccgc caggc                                          25


<210>  611
<211>  25
<212>  DNA
<213>  Homo sapiens

<400>  611
ccccgccagg cctcgaggct caggg                                          25


<210>  612
<211>  25
<212>  DNA
<213>  Homo sapiens

<400>  612
cgccaggcct cgaggctcag ggtgg                                          25


<210>  613
<211>  25
<212>  DNA
<213>  Homo sapiens

<400>  613
gcctcgaggc tcagggtggg agagg                                          25


<210>  614
<211>  25
<212>  DNA
<213>  Homo sapiens

<400>  614
gaggctcagg gtgggagagg gcccc                                          25


<210>  615
<211>  25
<212>  DNA
<213>  Homo sapiens

<400>  615
gctcagggtg ggagagggcc ccggg                                          25


<210>  616
<211>  25
<212>  DNA
<213>  Homo sapiens

<400>  616
ccccgggctg ccctgtcact cctct                                          25


<210>  617
<211>  25
<212>  DNA
<213>  Homo sapiens

<400>  617
cgggctgccc tgtcactcct ctaac                                          25


<210>  618
<211>  25
<212>  DNA
<213>  Homo sapiens

<400>  618
gctgccctgt cactcctcta acact                                          25


<210>  619
<211>  25
<212>  DNA
<213>  Homo sapiens

<400>  619
cctgtcactc ctctaacact tccct                                          25


<210>  620
<211>  25
<212>  DNA
<213>  Homo sapiens

<400>  620
tcactcctct aacacttccc tcccg                                          25


<210>  621
<211>  25
<212>  DNA
<213>  Homo sapiens

<400>  621
ctcctctaac acttccctcc cgtgt                                          25


<210>  622
<211>  25
<212>  DNA
<213>  Homo sapiens

<400>  622
ccccaacatg ccctgtaata aaatt                                          25


<210>  623
<211>  25
<212>  DNA
<213>  Homo sapiens

<400>  623
caacatgccc tgtaataaaa ttaga                                          25


<210>  624

<211>  25
<212>  DNA
<213>  Homo sapiens

<400>  624
catgccctgt aataaaatta gagaa                                          25


<210>  625
<211>  25
<212>  DNA
<213>  Homo sapiens

<400>  625
tagaatgacc cttgggaaca gtgaa                                          25


<210>  626
<211>  25
<212>  DNA
<213>  Homo sapiens

<400>  626
gacccttggg aacagtgaac gtaga                                          25


<210>  627
<211>  25
<212>  DNA
<213>  Homo sapiens

<400>  627
tttagcagag tttgtgacca aagtc                                          25


<210>  628
<211>  25
<212>  DNA
<213>  Homo sapiens

<400>  628
gctctggctg ccttctgcat ttatt                                          25


<210>  629
<211>  25
<212>  DNA
<213>  Homo sapiens

<400>  629
gctgccttct gcatttattt gcctt                                          25


<210>  630
<211>  25
<212>  DNA
<213>  Homo sapiens

<400>  630
gccttggcct gttgtcttcc cctat                                          25


<210>  631
<211>  25
<212>  DNA
<213>  Homo sapiens

<400>  631

gcctgttgtc ttcccctatt ttctg                                                25

<210>  632
<211>  25
<212>  DNA
<213>  Homo sapiens

<400>  632
tgtcttcccc tattttctgt cccag                                                25

<210>  633
<211>  25
<212>  DNA
<213>  Homo sapiens

<400>  633
ctattttctg tcccagctca tccgt                                                25

<210>  634
<211>  25
<212>  DNA
<213>  Homo sapiens

<400>  634
ttttctgtcc cagctcatcc gtgtc                                                25

<210>  635
<211>  25
<212>  DNA
<213>  Homo sapiens

<400>  635
tctgtcccag ctcatccgtg tctct                                                25

<210>  636
<211>  25
<212>  DNA
<213>  Homo sapiens

<400>  636
gtcccagctc atccgtgtct ctgaa                                                25

<210>  637
<211>  25
<212>  DNA
<213>  Homo sapiens

<400>  637
ccagctcatc cgtgtctctg aagaa                                                25

<210>  638
<211>  25
<212>  DNA
<213>  Homo sapiens

<400>  638
gctcatccgt gtctctgaag aacaa                                                25

<210>  639
<211>  25

<212>  DNA
<213>  Homo sapiens

<400>  639
ccgtgtctct gaagaacaaa tatgc                                              25


<210>  640
<211>  25
<212>  DNA
<213>  Homo sapiens

<400>  640
ttgccaccct gagcactgcc cggat                                              25


<210>  641
<211>  25
<212>  DNA
<213>  Homo sapiens

<400>  641
ggatcccgtg caccctggga cccag                                              25


<210>  642
<211>  25
<212>  DNA
<213>  Homo sapiens

<400>  642
tcccgtgcac cctgggaccc agaag                                              25


<210>  643
<211>  25
<212>  DNA
<213>  Homo sapiens

<400>  643
cgtgcaccct gggacccaga agtgc                                              25


<210>  644
<211>  25
<212>  DNA
<213>  Homo sapiens

<400>  644
ccgccagcac gtccagagca actta                                              25


<210>  645
<211>  25
<212>  DNA
<213>  Homo sapiens

<400>  645
gccagcacgt ccagagcaac ttacc                                              25


<210>  646
<211>  25
<212>  DNA
<213>  Homo sapiens

<400>  646
agcacgtcca gagcaactta ccccg                                              25

<210>  647
<211>  25
<212>  DNA
<213>  Homo sapiens

<400>  647
gcacgtccag agcaacttac cccgg                                          25


<210>  648
<211>  25
<212>  DNA
<213>  Homo sapiens

<400>  648
ccgtgccgcc gaccacgatg tgggc                                          25


<210>  649
<211>  25
<212>  DNA
<213>  Homo sapiens

<400>  649
cgtgccgccg accacgatgt gggct                                          25


<210>  650
<211>  25
<212>  DNA
<213>  Homo sapiens

<400>  650
tgccgccgac cacgatgtgg gctct                                          25


<210>  651
<211>  25
<212>  DNA
<213>  Homo sapiens

<400>  651
cgccgaccac gatgtgggct ctgag                                          25


<210>  652
<211>  25
<212>  DNA
<213>  Homo sapiens

<400>  652
gaccacgatg tgggctctga gctgc                                          25


<210>  653
<211>  25
<212>  DNA
<213>  Homo sapiens

<400>  653
cacgatgtgg gctctgagct gcccc                                          25


<210>  654
<211>  25
<212>  DNA

<213>  Homo sapiens

<400>  654
tgtgaaacgc ctagagaccc cggcg                                              25


<210>  655
<211>  25
<212>  DNA
<213>  Homo sapiens

<400>  655
tcacagcccc gttcagctgg tggct                                              25


<210>  656
<211>  25
<212>  DNA
<213>  Homo sapiens

<400>  656
ccccgttcag ctggtggctt ttaga                                              25


<210>  657
<211>  25
<212>  DNA
<213>  Homo sapiens

<400>  657
ttttagaggc ttccagagtg tgctt                                              25


<210>  658
<211>  25
<212>  DNA
<213>  Homo sapiens

<400>  658
ccagagtgtg cttggcccct ttacc                                              25


<210>  659
<211>  25
<212>  DNA
<213>  Homo sapiens

<400>  659
tggccccttt acctctatgc cattg                                              25


<210>  660
<211>  25
<212>  DNA
<213>  Homo sapiens

<400>  660
ctctatgcca ttgggcccag gggga                                              25


<210>  661
<211>  25
<212>  DNA
<213>  Homo sapiens

<400>  661
cctttctgtg tcttgcttgc cccgt                                              25

<210>  662
<211>  25
<212>  DNA
<213>  Homo sapiens

<400>  662
tgtgtcttgc ttgccccgtg tctcc                                          25


<210>  663
<211>  25
<212>  DNA
<213>  Homo sapiens

<400>  663
ttgcttgccc cgtgtctccc agtga                                          25


<210>  664
<211>  25
<212>  DNA
<213>  Homo sapiens

<400>  664
gccccgtgtc tcccagtgag tggcc                                          25


<210>  665
<211>  25
<212>  DNA
<213>  Homo sapiens

<400>  665
tgtctcccag tgagtggccg ccctg                                          25


<210>  666
<211>  25
<212>  DNA
<213>  Homo sapiens

<400>  666
cggacaagtc gcagcctcag gggga                                          25


<210>  667
<211>  25
<212>  DNA
<213>  Homo sapiens

<400>  667
agtcgcagcc tcagggggac ctccc                                          25


<210>  668
<211>  25
<212>  DNA
<213>  Homo sapiens

<400>  668
ctggcactgc atctttctgg gcctg                                          25


<210>  669
<211>  25
<212>  DNA
<213>  Homo sapiens

<400>  669
ctttctgggc ctggctctgc tgcct                                          25


<210>  670
<211>  25
<212>  DNA
<213>  Homo sapiens

<400>  670
cagagttata agccccaaac aggtc                                          25


<210>  671
<211>  25
<212>  DNA
<213>  Homo sapiens

<400>  671
agagttataa gccccaaaca ggtca                                          25


<210>  672
<211>  25
<212>  DNA
<213>  Homo sapiens

<400>  672
gagttataag ccccaaacag gtcat                                          25


<210>  673
<211>  25
<212>  DNA
<213>  Homo sapiens

<400>  673
agttataagc cccaaacagg tcatg                                          25


<210>  674
<211>  25
<212>  DNA
<213>  Homo sapiens

<400>  674
gttataagcc ccaaacaggt catgc                                          25


<210>  675
<211>  25
<212>  DNA
<213>  Homo sapiens

<400>  675
ttataagccc caaacaggtc atgct                                          25


<210>  676
<211>  25
<212>  DNA
<213>  Homo sapiens

<400>  676
tataagcccc aaacaggtca tgctc                                          25

<210>  677
<211>  25
<212>  DNA
<213>  Homo sapiens

<400>  677
ataagcccca aacaggtcat gctcc                                              25


<210>  678
<211>  25
<212>  DNA
<213>  Homo sapiens

<400>  678
taagccccaa acaggtcatg ctcca                                              25


<210>  679
<211>  25
<212>  DNA
<213>  Homo sapiens

<400>  679
aagccccaaa caggtcatgc tccaa                                              25


<210>  680
<211>  25
<212>  DNA
<213>  Homo sapiens

<400>  680
agccccaaac aggtcatgct ccaat                                              25


<210>  681
<211>  25
<212>  DNA
<213>  Homo sapiens

<400>  681
gccccaaaca ggtcatgctc caata                                              25


<210>  682
<211>  25
<212>  DNA
<213>  Homo sapiens

<400>  682
ccccaaacag gtcatgctcc aataa                                              25


<210>  683
<211>  25
<212>  DNA
<213>  Homo sapiens

<400>  683
cccaaacagg tcatgctcca ataaa                                              25


<210>  684
<211>  25
<212>  DNA
<213>  Homo sapiens

<400>  684
ccaaacaggt catgctccaa taaaa                                          25


<210>  685
<211>  25
<212>  DNA
<213>  Homo sapiens

<400>  685
cttgcaacct ccgggaccat cttct                                          25


<210>  686
<211>  25
<212>  DNA
<213>  Homo sapiens

<400>  686
gcaacctccg ggaccatctt ctcgg                                          25


<210>  687
<211>  25
<212>  DNA
<213>  Homo sapiens

<400>  687
gcttctggga cctgccagca ccgtt                                          25


<210>  688
<211>  25
<212>  DNA
<213>  Homo sapiens

<400>  688
gggacctgcc agcaccgttt ttgtg                                          25


<210>  689
<211>  25
<212>  DNA
<213>  Homo sapiens

<400>  689
tgccagcacc gtttttgtgg ttagc                                          25


<210>  690
<211>  25
<212>  DNA
<213>  Homo sapiens

<400>  690
cagcaccgtt tttgtggtta gctcc                                          25


<210>  691
<211>  25
<212>  DNA
<213>  Homo sapiens

<400>  691
ttgccaacca accatgagct cccag                                          25


<210>  692

<211>  25
<212>  DNA
<213>  Homo sapiens

<400>  692
gccaaccaac catgagctcc cagat                                          25


<210>  693
<211>  25
<212>  DNA
<213>  Homo sapiens

<400>  693
aaccaaccat gagctcccag attcg                                          25


<210>  694
<211>  25
<212>  DNA
<213>  Homo sapiens

<400>  694
ccatgagctc ccagattcgt cagaa                                          25


<210>  695
<211>  25
<212>  DNA
<213>  Homo sapiens

<400>  695
tgagctccca gattcgtcag aatta                                          25


<210>  696
<211>  25
<212>  DNA
<213>  Homo sapiens

<400>  696
gctcccagat tcgtcagaat tattc                                          25


<210>  697
<211>  25
<212>  DNA
<213>  Homo sapiens

<400>  697
cccagattcg tcagaattat tccac                                          25


<210>  698
<211>  25
<212>  DNA
<213>  Homo sapiens

<400>  698
gattcgtcag aattattcca ccgac                                          25


<210>  699
<211>  25
<212>  DNA
<213>  Homo sapiens

<400>  699

tcgtcagaat tattccaccg acgtg                                              25

<210>  700
<211>  25
<212>  DNA
<213>  Homo sapiens

<400>  700
tcagaattat tccaccgacg tggag                                              25

<210>  701
<211>  25
<212>  DNA
<213>  Homo sapiens

<400>  701
actggcatgg ccttccgtgt cccca                                              25

<210>  702
<211>  25
<212>  DNA
<213>  Homo sapiens

<400>  702
ccactgccaa cgtgtcagtg gtgga                                              25

<210>  703
<211>  25
<212>  DNA
<213>  Homo sapiens

<400>  703
actgccaacg tgtcagtggt ggacc                                              25

<210>  704
<211>  25
<212>  DNA
<213>  Homo sapiens

<400>  704
tgccaacgtg tcagtggtgg acctg                                              25

<210>  705
<211>  25
<212>  DNA
<213>  Homo sapiens

<400>  705
ccaacgtgtc agtggtggac ctgac                                              25

<210>  706
<211>  25
<212>  DNA
<213>  Homo sapiens

<400>  706
cgtgtcagtg gtggacctga cctgc                                              25

<210>  707
<211>  25

<212>  DNA
<213>  Homo sapiens

<400>  707
gtcagtggtg gacctgacct gccgt                                              25


<210>  708
<211>  25
<212>  DNA
<213>  Homo sapiens

<400>  708
cagtggtgga cctgacctgc cgtct                                              25


<210>  709
<211>  25
<212>  DNA
<213>  Homo sapiens

<400>  709
gtggtggacc tgacctgccg tctag                                              25


<210>  710
<211>  25
<212>  DNA
<213>  Homo sapiens

<400>  710
ggtggacctg acctgccgtc tagaa                                              25


<210>  711
<211>  25
<212>  DNA
<213>  Homo sapiens

<400>  711
gacctgacct gccgtctaga aaaac                                              25


<210>  712
<211>  25
<212>  DNA
<213>  Homo sapiens

<400>  712
ctgacctgcc gtctagaaaa acctg                                              25


<210>  713
<211>  25
<212>  DNA
<213>  Homo sapiens

<400>  713
gacctgccgt ctagaaaaac ctgcc                                              25


<210>  714
<211>  25
<212>  DNA
<213>  Homo sapiens

<400>  714
tgccgtctag aaaaacctgc caaat                                              25

<210> 715
<211> 25
<212> DNA
<213> Homo sapiens

<400> 715
ccgtctagaa aaacctgcca aatat 25


<210> 716
<211> 25
<212> DNA
<213> Homo sapiens

<400> 716
gtctagaaaa acctgccaaa tatga 25


<210> 717
<211> 2611
<212> DNA
<213> Homo sapiens

<400> 717
   aggaacgact gtgctacgtt gccagaaggg gcgggacctg caacgtccga cagaacgagg 60
   ggacgtaacg gaggcaggtt ggagccgctg ccgtcgccat gacccgcggt aaccagcgtg 120
   agctcgcccg ccagaagaat atgaaaaagc agagcgactc ggttaaggga aagcgccgag 180
   atgacgggct ttctgctgcc gcccgcaagc agagggactc ggagatcatg cagcagaagc 240
   agaaaaaggc aaacgagaag aaggaggaac ccaagtagct ttgtggcttc gtgtccaacc 300
   ctcttgccct tcgcctgtgt gcctggagcc agtcccacca cgctcgcgtt tcctcctgta 360
   gtgctcacag gtcccagcac cgatggcatt ccctttgccc tgagtctgca gcgggtccct 420
   tttgtgcttc cttcccctca ggtagcctct ctccccctgg gccactcccg ggggtgaggg 480
   ggttacccct tcccagtgtt ttttattcct gtggggctca ccccaaagta ttaaaagtag 540
   ctttgtaatt ccttgagcgc ctggtttgac tggggacttg gggggatggg gttggaagaa 600
   tgactgccct ttcccaccaa aaaagggaga actctttaga ttcagattgt gggtatgtag 660
   acttaataag tgaaacatca cagaagaagc ctttattata caatgacaac caaacaagta 720
   ctccggatat gcagtagagg aatcctctaa gaaccataga gacttctttt ctgtgatttt 780
   tgttccccac ccttgaacac catctctagg atggagttgg cctaagagtg aatgctgcaa 840
   gatctgtgtt tatgcctctt ttcctcattc ttcctcagtt tgttcgtctg cttgaaagtt 900
   ggccaaaaaa tcctgctgct caccgacttc ccgtggtcag ctgctgtcaa gcgttcactt 960
   tctcttctgt cattcctcat ggaatgaggg tggttttgtc ttcccgcttc ccttgacctc 1020
   aaaatcagga ttaaaacctg gggtagcctc tgtgctcctt tcttctatgc cctggtttgt 1080
   tctgtggttc tgggcttctt atatccgtgt gcccagggct gaactcctta ttttcctttc 1140
   tccaagggca gagccgagtc ttcagtccct gttggtcttt ccccaccccc acttccagcc 1200
   caagagccag gaaagggctg gtgccacact gtctgctggg atcagcggtg gttctttgag 1260
   ctgctgattt gggtgttagg ctcttgagct gggatgcaga tgtaacagta gctccagtga 1320
   gtcagacact ctgcccagca cattagactg tgtttgacca cttcttccag ttcatagtat 1380
   tgacttcagc ccaaacggag ataactccct gtgtgtcctt gaggtattga gctgggctgg 1440
   acagctcccc ttgagccaac tctaggagta caatgtcagg ggaaccccag tttgtgaaaa 1500
   ggacttagac tggaggatat ttgttatctg gggatatgat gcggtggcgg cggcgcctca 1560
   agataagggg ctggggtttc tgggtggggg gccaacagag tggtgccagt aacagcccca 1620
   gatagaggag tacgcaggcc cagcatgagg caaccttgac ccagaaggtg gcccagctac 1680
   ccttgatgaa ggtcttttcc agttctgctc cctcatagct gtgtaaccaa aggctctggt 1740
   tagagaatat gaagggcctt agcttttaga cctgttctac ctcctcacca aatataatgg 1800
   cagacccatg tgtgtctgga atggccttga attgctcttt ccttaaaata gctagctctt 1860
   caggagagta tctaaggccc actccatctt acctgaacca gttggtaagg gtaaccatga 1920
   catagagtga ggcaaggaag aagacgaagt ggaaggcaga atagttgtag gaaagatgct 1980
   ggacttggac tggaggagct ggaggggttt cttggtcagc tggcctcgca gccccacccc 2040
   tttgccctgg agagaggaaa tggctgctgg gagcagagct gctgaaacac ctcttcccct 2100
   ctcccccaac tacctttgtt aaggctcttg agggttctta tggcactcca cagagatcta 2160
   ccacttctta tggttcctca cttggcactc acctttgtct gcctccactg tttcagggca 2220
   gcagaaacac agtgagggct tctgcaaaac agaacgcagg ttttggaatg gtcttaaaag 2280
   atgtgagggt gttaatctag gaaacttccc ccgtgaaaag attggtctag tattaaaaag 2340
   tggaggcaca cctgggttca aattctagct ccagcatata agtggctgtg cagactttgg 2400
   taagatgttt aatcttttgt gcctcgattt ctccatttgt aaaatggagc aaatacctac 2460
   ctcacagggt tgttgtgagg gttaaattaa atgagattat gtaaaagtat ctagcacagt 2520
   tgcctagcac attgtgggta ctcaataaaa ggtaacagca gctataatct gagcattctg 2580
   ggtagaggtt ggtaaaaaaa aaaaaaaaaa a 2611

<210> 718
<211> 3432
<212> DNA
<213> Homo sapiens

<400> 718
actacttctg cgcctgcgcg accgtgattc cccgctcgcg actccccacc ccccagggct 60

ccctaaagag ggccacgagc tgcgaaaggg cgggaaaggc agttggagaa gaggtaagcg 120

gttactcact ccatggctgc agcaaggaga ggcggcggcg gcctcggctg aagaaagaag 180

gtgggagcgg agagcgcagg cgtgccgagg tggatgtccg tcttttctct gttgcagaaa 240

cccaccttgt cccatccaca tcaggacatc ccagctggag ttcaaccttc atcccttctg 300

tggcagttag gagactgaat caaggtccag agaaggtgga ggaatcctga tactgagcga 360

aatcttccca aggctgcaga caccgacgga tttgctttgg gagccagagt agctgccgcc 420

accagagtcc ggagccatga gcgggtttaa ttttggaggc actggggccc ctacaggcgg 480

gttcacgttt ggcactgcaa agacggcaac aaccacacct gctacagggt tttctttctc 540
cacctctggc actggagggt ttaattttgg ggctcccttc caaccagcca caagtacccc 600
ttccaccggc ctgttctcac ttgccaccca gactccggcc acacagacga caggcttcac 660
ttttggaaca gcgactcttg cttcgggggg aactggattt tctttgggga tcggtgcttc 720
aaagctcaac ttgagcaaca cagctgccac cccagccatg gcaaacccca gcggctttgg 780
gctgggcagc agcaacctca ctaatgccat atcgagcacc gtcacctcca gccagggcac 840
agcacccacc ggctttgtgt ttggcccctc caccacctct gtggctccag ctaccacatc 900
tggaggcttc tcattcactg gtggaagcac ggcccaaccc tccggtttca acattggctc 960
agcagggaat tcagcccagc ccacggcacc tgccacgttg cccttcactc cggccacgcc 1020
agcagccacc acagcaggtg ccacacagcc agctgctccc acacccacag ccaccatcac 1080
cagtactggg cccagcctct ttgcgtcaat agcaactgct ccaacctcat ctgccaccac 1140
tggactctcc ctctgtaccc ctgtgaccac agcgggcgcc cccactgctg ggacacaggg 1200
cttcagctta aaggcacctg gagcagcttc cggcacctcc acaacaacat ccaccgctgc 1260
caccgccacc gccaccacca ccagcagcag cagcaccacc ggctttgcct tgaatttaaa 1320
accactggcg ccagccggga tccccagcaa tacagcagct gccgtgaccg ctccacctgg 1380
ccctggcgca gctgcagggg cggctgccag ctccgccatg acctacgcgc agctggagag 1440
cctgatcaac aaatggagcc tggagctaga ggaccaggag cggcacttcc tccagcaggc 1500
cacccaggtc aacgcctggg accgcacgct gatcgagaat ggagaaaaga tcaccagcct 1560
gcaccgcgag gtggagaagg tgaagctgga ccagaagagg ctggaccagg agctcgactt 1620
catcctgtcc cagcagaagg agctggaaga cctgctgagc ccactggagg agttggtcaa 1680
ggagcagagc gggaccatct acctgcagca cgcggatgag gagcgtgaga aaacctacaa 1740
gctggctgag aacatcgacg cacagctcaa gcgcatggcc caggatctca aggacatcat 1800
cgagcacctg aacacgtccg gggcccccgc cgacaccagt gacccactgc agcagatctg 1860
caagatcctc aatgcgcaca tggactcact gcagtggatc gaccagaact cggccctgct 1920
gcagaggaag gtggaggagg tgaccaaggt gtgcgagggc cggcgcaagg agcaggagcg 1980
cagcttccgg atcacctttg actgagcgac agcagccctg gggcccgcag gtccctaggg 2040
agttcatgag gggaatgcgc cctgttgtct gtagtttggg gttgtggcaa gatacttgtt 2100
tgtttctttc tttctttcac atgactgccc ttgacatgat cgctgtgtgc tttgcgtttt 2160
tccatttagg agggtattct gggccttctg cccaggcagc agcctcatgg gtgtggcttc 2220
tgtggctttc atttgagtat ctttggcccc ttttcaccta ctgcgaccac ccacctcatc 2280
ctggctcagc ctggtgatgg agaagtgctg atggtcttgg tcccagccag ggtcgtgggg 2340
gcagccactc tctccaaagc atagtcatag gtgtcatgaa aaaataccaa atgtaagaga 2400
acctccaagt cagggcgcag tggctcaccc ctgtaatctc agcactttgg gtggccaagg 2460
cgggcagatg acttgaggtc aggagttcga gaccagcctg gccaacatgg tgaaaccccg 2520

tctctactaa aaatacaaaa attagtcagg tgtggtggac gcctgtgatc tcaatctcag 2580
ctactcggga ggctgaggca ggagaatcac ttgaacccag gaggtgttgc agtgaaccaa 2640
gatcacacca ctgcactcca gcctaggcaa cagagactct gtctcaaaaa aaaaaaaaaa 2700
aaaaaagaaa ctcccaggag acagcagcct agttttcgag tgtgagcttg tgcttgtgaa 2760
agctaaccat gctaaccacc aaggcaaagc agcacagtgt gaatagaaca gagcgggatc 2820
aagaatttca cagaagacag gtcagctgag gggcctgcac acacagggtg ttgaggaacc 2880
acagatgggc gccgagaggc ctgccttttg cctggcccag gctcaccccc accttgggcc 2940
tcacctcctc caggaagcct tcccagctac ccgaagctca ggtggccttc ttgcaggtcc 3000
ccgtagcacc ctgagcctgt accttgggtg gcacttgtta tgctatcctg tgctagccgt 3060
ttgtgcctcg tctcgctgtt agattgtgag ttcccatggg cagagaccca ctgtcgttcc 3120
ccgtgtgtcc ccagcccggt ccctgtcaca tttgttaaat gaaagaacaa tgaagcccag 3180
tgtaacgtca gtccacagaa atagccacag cttccagtgg tggccgtaga cttggctcgg 3240
aacttagtgg caccagagta actctagtca gttacagtaa aatccactgt gtgtggaagg 3300
cagaagctag cggttgtatc ccaagcatct tttgtatttg tctttatact ttgctgaatt 3360
ctctgaaata cctattactg tatgttgctt ttctaaataa atgtattgtg aaaccaaaaa 3420
aaaaaaaaaa aa 3432

<210> 719
<211> 1986
<212> DNA
<213> Homo sapiens

<400> 719
aaggccccgc tgcgtcttcc gagccgcagg cgcaggccca gctgagcggc cgccgagcgg 60
gtgcgggtgc gggcgcatcg gccatcaccg cgcggccgcg cagcggacac cgtgcgtacc 120
ggcctgcggc gcccggccac cggtgagtcc ccggcccgag cccaggagcg cctctgaccc 180
gctgcgccgc gcggcctgcc gcccccgccc ccgcccccac gcggatcttg cgcatccgag 240
cgtggccgcc tcgggggcgg accgcggaac ccgaggccat gtcccatgaa aagagttttt 300
tggtgtctgg ggacaactat cctcccccca accctggata tccggggggg ccccagccac 360
ccatgccccc ctatgctcag cctccctacc ctgggccccc ttacccacag ccccctttcc 420
agccctcccc ctacggtcag ccagggtacc cccatggccc cagcccctac ccccaagggg 480
gctacccaca gggtccctac ccccaagggg gctacccaca gggcccctac ccacaagagg 540
gctacccaca gggcccctac ccccaagggg gctaccccca ggggccatat ccccagagcc 600
ccttcccccc caacccctat ggacagccac aggtcttccc aggacaagac cctgactcac 660
cccagcatgg aaactaccag gaggagggtc ccccatccta ctatgacaac caggacttcc 720
ctgccaccaa ctgggatgac aagagcatcc gacaggcctt catccgcaag gtgttcctag 780
tgctgacctt gcagctgtcg gtgaccctgt ccacggtgtc tgtgttcact tttgttgcgg 840

aggtgaaggg ctttgtccgg gagaatgtct ggacctacta tgtctcctat gctgtcttct 900

tcatctctct catcgtcctc agctgttgtg gggacttccg gcgaaagcac ccctggaacc 960

ttgttgcact gtcggtcctg accgccagcc tgtcgtacat ggtggggatg atcgccagct 1020

tctacaacac cgaggcagtc atcatggccg tgggcatcac cacagccgtc tgcttcaccg 1080

tcgtcatctt ctccatgcag acccgctacg acttcacctc atgcatgggc gtgctcctgg 1140

tgagcatggt ggtgctcttc atcttcgcca ttctctgcat cttcatccgg aaccgcatcc 1200

tggagatcgt gtacgcctca ctgggcgctc tgctcttcac ctgcttcctc gcagtggaca 1260

cccagctgct gctggggaac aagcagctgt ccctgagccc agaagagtat gtgtttgctg 1320

cgctgaacct gtacacagac atcatcaaca tcttcctgta catcctcacc atcattggcc 1380

gcgccaagga gtagccgagc tccagctcgc tgtgcccgct caggtggcac ggctggcctg 1440

gaccctgccc ctggcacggc agtgccagct gtacttcccc tctctcttgt ccccaggcac 1500

agcctaggga aaaggatgcc tctctccaac cctcctgtat gtacactgca gatacttcca 1560

tttggacccg ctgtggccac agcatggccc ctttagtcct cccgcccccg ccaaggggca 1620

ccaaggccac gtttccgtgc cacctcctgt ctactcattg ttgcatgagc cctgtctgcc 1680

agcccacccc agggactggg ggcagcacca ggtcccgggg agagggattg agccaagagg 1740

tgagggtgca cgtcttccct cctgtcccag ctccccagcc tggcgtagag cacccctccc 1800

ctccccccca cccccctgga gtgctgccct ctggggacat gcggagtggg ggtcttatcc 1860

ctgtgctgag ccctgagggc agagaggatg gcatgtttca ggggaggggg aagccttcct 1920

ctcaatttgt tgtcagtgaa attccaataa atgggatttg ctctctgcaa aaaaaaaaaa 1980

aaaaaa 1986

<210> 720
<211> 3973
<212> DNA
<213> Homo sapiens

<400> 720
cggacggggc cgccccgatg ggacgccgcg ctccggcccc tgcgcgccgc tgagccgagc 60

gccccccgct gccgagaccc ccgccgccac cgccagccgc tgccccctcg cccccgcccg 120

ggccgggagc ctcgtccccg tcccccggaa agctggattt ccgaggctgg aggcgcctgg 180

ccggctgggt ggggaccacc atgggcaacg cggccggcag cgccgagcag cccgcgggcc 240

ccgccgcgcc gccccccaag cagcccgcgc ctcccaagca gccgatgccc gcggccggag 300

agctggagga gaggttcaac cgcgccctga actgcatgaa cttgccccca gacaaggtcc 360

agctgctgag ccagtatgac aacgagaaga agtgggagct catctgtgat caggagcggt 420

ttcaagtcaa gaatcccccc gcagcctaca tccagaagct gaagagctat gtggatactg 480

gtggggtcag ccgaaaggta gcagctgatt ggatgtccaa cctggggttt aagaggcgag 540

ttcaggagtc cacgcaggtg ctacgggagc tggagacctc cctgaggacc aaccacattg 600

ggtgggtgca ggagttcctc aatgaagaga accgtggcct ggatgtgctg ctcgagtacc 660

tggcctttgc ccagtgctct gtcacgtatg acatggagag cacagacaac ggggcttcca 720
actcagagaa aaacaagccc ctggagcagt ctgtggaaga cctcagcaag ggtccaccct 780
cctccgtgcc caaaagccgc cacctgacca tcaagctgac cccagcccac agcaggaagg 840
ccctgcggaa ttcccgcatc gtcagccaga aggacgacgt ccacgtctgt attatgtgcc 900
tacgcgccat catgaactac cagtctggct tcagccttgt catgaaccac ccagcctgtg 960
tcaatgagat tgctctgagc ctcaacaaca agaaccccag aaccaaggct ctggtgctgg 1020
agctgctggc ggccgtgtgc ttggtgcggg gaggacatga catcatcctt gcagcctttg 1080
acaacttcaa ggaggtgtgt ggggagcagc accgctttga aaagctgatg gaatatttcc 1140
ggaatgagga cagcaacatc gacttcatgg tggcctgcat gcagttcatc aacattgtgg 1200
tacattcggt ggagaacatg aacttccgtg tcttcctgca atatgagttc acccacttgg 1260
gcctggacct gtacttggag aggcttcggc tcaccgagag tgacaagctg caggtgcaga 1320
tccaggcgta cctggacaat atttttgatg tgggggcgct gctggaggac acagagacca 1380
agaacgctgt gctggagcac atggaggaac tgcaggagca agtggcgctg ctgacagagc 1440
ggcttcggga cgcggagaac gaatccatgg ccaagattgc agaactggaa aaacagctaa 1500
gccaggcgcg caaggagttg gagaccctgc gggagcgctt cagcgaatcg accgccatgg 1560
gcgcctccag gcgtccccca gagcctgaga aagcgcctcc cgctgccccg acgcggccct 1620
cggccctgga gctgaaggtg gaggagctgg aggagaaggg gttaatccgt attctgcggg 1680
ggccggggga tgctgtctcc atcgagatcc tccccgtcgc tgtggcaact ccgagcggcg 1740
gtgatgctcc gactccgggg gtgccgaccg gctcccccag cccagatctc gcacctgcag 1800
cagagccggc tcccggagca gcgccaccgc cgccgccccc actgcccggc ctcccctccc 1860
cgcaggaagc cccgccctct gcgcccccac aggccccgcc tctccctggc agcccggagc 1920
ccccgcctgc gccgccgctg cccggagacc tgccgccccc acccccgcca ccgccaccac 1980
ctccgggcac tgacgggccg gtgcctccgc cgccgccgcc gccgccgccg cctcccggag 2040
gtcctcctga tgccctagga agacgcgact cagaattggg cccaggagtg aaggccaaga 2100
agcccatcca gactaagttc cgaatgccac tcttgaactg ggtggcactg aaacccagcc 2160
agatcaccgg cactgtcttc acagagctca atgatgagaa ggtgctgcag gagctagaca 2220
tgagtgattt tgaggaacag ttcaagacca agtcccaagg ccccagcctg gacctcagcg 2280
ctctcaagag taaggcagcc cagaaggccc ccagcaaggc gacactcatt gaggccaacc 2340
gggccaagaa cttggccatc accctgcgga agggcaacct gggggccgag cgcatctgcc 2400
aagccattga ggcgtacgac ctgcaggctc tgggcctgga cttcctggag ctgctgatgc 2460
gcttcctgcc cacagagtat gagcgcagcc tcatcacccg ctttgagcgg gagcagcggc 2520
caatggagga gctgtcagag gaggaccgct tcatgctatg cttcagccgc atcccgcgcc 2580
tgccggagcg catgaccaca ctcaccttcc tgggcaactt cccggacaca gcccagctgc 2640
tcatgccgca actgaatgcc atcattgcag cctcaatgtc catcaagtcc tctgacaaac 2700

tccgccagat cctggagatt gtcctggcct ttggcaacta catgaacagt agcaagcgtg 2760
gggcagccta tggcttccgg ctccagagcc tggatgcgct gttggagatg aagtcgactg 2820
atcgcaagca gacgctgctg cactacctgg tgaaggtcat tgctgagaag tacccgcaac 2880
tcacaggctt ccacagcgac ctgcacttcc tggacaaggc gggctcagtg tccctggaca 2940
gtgtcctggc ggacgtgcgc tccctgcagc gaggcctaga gttgacacag agagagtttg 3000
tgcggcagga tgactgcatg gtgctcaagg agttcctgag ggccaactcg cccaccatgg 3060
acaagctgct ggcagacagc aagacggctc aggaggcctt tgagtctgtg gtggagtact 3120
tcggagagaa ccccaagacc acatccccag gcctgttctt ctccctcttt agccgcttca 3180
ttaaggccta caagaaagct gagcaggagg tggaacagtg gaaaaaagaa gccgctgccc 3240
aggaggcagg cgctgatacc ccgggcaaag gggagccccc agcacccaag tcaccgccaa 3300
aggcccggcg gccacagatg gacctcatct ctgagctgaa acggaggcag cagaaggagc 3360
cactcattta tgagagcgac cgtgatgggg ccattgaaga catcatcaca gtgatcaaga 3420
cggtgccctt cacggcccgc accggcaagc ggacatcccg gctcctctgt gaggccagcc 3480
tgggagaaga gatgcccctc tagcccctca gatctgcgga accagcccta catccgcgca 3540
gacacaggcc gccgcagtgc ccgtcggcgt cccccgggcc ccccactgca ggtcacctcc 3600
gacctctcgc tgtagccgct atttctgcag gtggattctg caggggtgtg gggccgtgga 3660
caggctgagg ctcaaggaag gtggtcctca gctcggctgg ccgggcagcc cctcctccgc 3720
tgtggcccgc ctcaaacggg ctggtgcatc ctcctcttgg ccacagaggg cagcatcgcc 3780
cgccccttcc cccaaatgct gcttgcagca cccaccctaa agccccctcc aaatagccat 3840
acttagcctc agcaggagcc tggcctgtaa cttataaagt gcacctcgcc cccgcaagcc 3900
ccagccccga ggaccgtcca tggaccttat ttttatatga gattaataaa gatgtttgca 3960
aaaaaaaaaa aaa 3973

<210> 721
<211> 5532
<212> DNA
<213> Homo sapiens

<400> 721
ggcccaccgc cgcccaggca aggccgccct gccttgggcg cagcgctgcc atggctgggg 60
gccgtggggc ccccgggcgc ggccgggacg agcctccgga gagctacccg caacgacagg 120
accacgagct acaggccctg gaggccatct acggcgcgga cttccaagac ctgcggccgg 180
acgcttgcgg accggtcaaa gagccccctg aaatcaattt agttttgtac cctcaaggcc 240
taactggtga agaagtatat gtaaaagtgg atttgagggt taaatgccca cctacctatc 300
cagatgtagt tcctgaaata gagttaaaaa atgccaaagg tctatcaaat gaaagtgtca 360
atttgttaaa atctcgccta gaagaactgg ccaagaaaca ctgtggggag gtgatgatct 420
ttgaactggc ttaccacgtg cagtcatttc tcagcgagca taacaagccc cctcccaagt 480

cttttcatga agaaatgctg gaaaggcggg ctcaggagga gcagcagagg ctgttggagg 540
ccaagcggaa agaagagcag gagcaacgtg aaatcctgca tgagattcag agaaggaaag 600
aagagataaa agaagagaaa aaaaggaaag aaatggctaa gcaggaacgt ttggaaattg 660
ctagtttgtc aaaccaagat catacctcta agaaggaccc aggaggacac agaacggctg 720
ccattctaca tggaggctct cctgactttg taggaaatgg taaacatcgg gcaaactcct 780
caggaaggtc taggcgagaa cgtcagtatt ctgtatgtaa tagtgaagat tctcctggct 840
cttgtgaaat tctgtatttc aatatgggga gtcctgatca gctcatggtg cacaaaggga 900
aatgtattgg cagtgatgaa caacttggaa aattagtcta caatgctttg gaaacagcca 960
ctggtggctt tgtcttgttg tatgagtggg tccttcagtg gcagaaaaaa atgggtccat 1020
tccttaccag tcaagaaaaa gagaagattg ataagtgcaa aaagcagatt caaggaacag 1080
aaacagaatt caactcactg gtaaaattga gccatccaaa tgtagtacgc taccttgcaa 1140
tgaatctcaa agagcaagac gactccatcg tggtggacat tttagtggag cacattagtg 1200
gggtctctct tgctgcacac ctgagccact caggccccat ccctgtgcat cagcttcgca 1260
ggtacacagc tcagctcctg tcaggccttg attatctgca cagcaattct gtggtgcata 1320
aggtcctgag tgcatctaat gtcttggtgg atgcagaagg caccgtcaag attacggact 1380
atagcatttc taagcgcctc gcagacattt gcaaggagga tgtgtttgag caaacccgag 1440
ttcgttttag tgacaatgct ctgccttata aaacggggaa gaaaggagat gtttggcgtc 1500
ttggccttct gctgctgtcc ctcagccaag gacaggaatg tggagagtac cctgtgacca 1560
tccctagtga cttaccagct gactttcaag attttctaaa gaaatgtgtg tgcttggatg 1620
acaaggaaag atggagtccc cagcagttgt tgaaacacag ctttataaat ccccagccaa 1680
aaatgcctct agtggaacaa agtcctgaag attctgaagg acaagattat gttgagactg 1740
ttattcctag caaccggcta cccagtgctg ccttctttag tgagacacag agacagtttt 1800
cccgatactt cattgagttt gaagaattac aacttcttgg taaaggagct tttggagctg 1860
tcatcaaggt gcagaacaag ttggacggct gctgctacgc agtgaagcgc atccccatca 1920
acccggccag ccggcagttc cgcaggatca agggcgaagt gacactgctg tcacggctgc 1980
accatgagaa cattgtgcgc tactacaacg cctggatcga gcggcacgag cggccggcgg 2040
gaccggggac gccgcccccg gactccgggc ccctggccaa ggatgaccga gctgcacgcg 2100
ggcagccggc gagcgacaca gacggcctgg acagcgtaga ggccgccgcg ccgccaccca 2160
tcctcagcag ctcggtggag tggagcactt cgggcgagcg ctcggccagt gcccgtttcc 2220
ccgccaccgg cccgggctcc agcgatgacg aggacgacga cgaggacgag cacggtggcg 2280
tcttctccca gtccttcctg cctgcttcag attctgaaag tgatattatc tttgacaatg 2340
aagatgagaa cagtaaaagt cagaatcagg atgaagattg caatgaaaag aatggctgcc 2400
atgaaagtga gccatcagtg acgactgagg ctgtgcacta cctatacatc cagatggagt 2460
actgtgagaa gagcacttta cgagacacca ttgaccaggg actgtatcga gacaccgtca 2520

gactctggag gctttttcga gagattctgg atggattagc ttatatccat gagaaaggaa 2580
tgattcaccg ggatttgaag cctgtcaaca tttttttgga ttctgatgac catgtgaaaa 2640
taggtgattt tggtttggcg acagaccatc tagccttttc tgctgacagc aaacaagacg 2700
atcagacagg agacttgatt aagtcagacc cttcaggtca cttaactggg atggttggca 2760
ctgctctcta tgtaagccca gaggtccaag gaagcaccaa atctgcatac aaccagaaag 2820
tggatctctt cagcctggga attatcttct ttgagatgtc ctatcacccc atggtcacgg 2880
cttcagaaag gatctttgtt ctcaaccaac tcagagatcc cacttcgcct aagtttccag 2940
aagactttga cgatggagag catgcaaagc agaaatcagt catctcctgg ctgttgaacc 3000
acgatccagc aaaacggccc acagccacag aactgctcaa gagtgagctg ctgcccccac 3060
cccagatgga ggagtcagag ctgcatgaag tgctgcacca cacgctgacc aacgtggatg 3120
ggaaggccta ccgcaccatg atggcccaga tcttctcgca gcgcatctcc cctgccatcg 3180
attacaccta tgacagcgac atactgaagg gcaacttctc aatccgtaca gccaagatgc 3240
agcagcatgt gtgtgaaacc atcatccgca tctttaaaag acatggagct gttcagttgt 3300
gtactccact actgcttccc cgaaacagac aaatatatga gcacaacgaa gctgccctat 3360
tcatggacca cagcgggatg ctggtgatgc ttccttttga cctgcggatc ccttttgcaa 3420
gatatgtggc aagaaataat atattgaatt taaaacgata ctgcatagaa cgtgtgttca 3480
ggccgcgcaa gttagatcga tttcatccca aagaacttct ggagtgtgca tttgatattg 3540
tcacttctac caccaacagc tttctgccca ctgctgaaat tatctacact atctatgaaa 3600
tcatccaaga gtttccagca cttcaggaaa gaaattacag tatttatttg aaccatacca 3660
tgttattgaa agcaatactc ttacactgtg ggatcccaga agataaactc agtcaagtct 3720
acattattct gtatgatgct gtgacagaga agctgacgag gagagaagtg gaagctaaat 3780
tttgtaatct gtctttgtct tctaatagtc tgtgtcgact ctacaagttt attgaacaga 3840
agggagattt gcaagatctt atgccaacaa taaattcatt aataaaacag aaaacaggta 3900
ttgcacagtt ggtgaagtat ggcttaaaag acctagagga ggttgttgga ctgttgaaga 3960
aactcggcat caagttacag gtcttgatca atttgggctt ggtttacaag gtgcagcagc 4020
acaatggaat catcttccag tttgtggctt tcatcaaacg aaggcaaagg gctgtacctg 4080
aaatcctcgc agctggaggc agatatgacc tgctgattcc ccagtttaga gggccacaag 4140
ctctggggcc agttcccact gccattgggg tcagcatagc tatagacaag atatctgctg 4200
ctgtcctcaa catggaggaa tctgttacaa taagctcttg tgacctcctg gttgtaagtg 4260
ttggccagat gtctatgtcc agggccatca acctaaccca gaaactctgg acagcaggca 4320
tcacagcaga aatcatgtac gactggtcac agtcccaaga ggaattacaa gagtactgca 4380
gacatcatga aatcacctat gtggcccttg tctcggataa agaaggaagc catgtcaagg 4440
ttaagtcttt cgagaaggaa aggcagacag agaagcgtgt gctggagact gaacttgtgg 4500
accatgtact gcagaaactg aggactaaag tcactgatga aaggaatggc agagaagctt 4560

ccgataatct tgcagtgcaa aatctgaagg ggtcattttc taatgcttca ggtttgtttg 4620

aaatccatgg agcaacagtg gttcccattg tgagtgtgct agccccggag aagctgtcag 4680

ccagcactag gaggcgctat gaaactcagg tacaaactcg acttcagacc tcccttgcca 4740

acttacatca gaaaagcagt gaaattgaaa ttctggctgt ggatctaccc aaagaaacaa 4800

tattacagtt tttatcatta gagtgggatg ctgatgaaca ggcatttaac acaactgtga 4860

agcagctgct gtcacgcctg ccaaagcaaa gatacctcaa attagtctgt gatgaaattt 4920

ataacatcaa agtagaaaaa aaggtgtctg tgctatttct gtacagctat agagatgact 4980

actacagaat cttattttaa ccctaaagaa ctgtcgttaa cctcattcaa acagacagag 5040

gcttatactg gaataatgga atgttgtaca ttcatcataa tttaaaatta aattctaaga 5100

agaggctggg tgcagtggct cacaccttta atcccagcac tttgggaagc caaggcagga 5160

agactgcttg aaaccaggag tttgagacca gcctgagcaa caaagcaaga ccccatctct 5220

ataaaaacta aaaaaattag ttgggcatgg tggcacatgc ctgtagtccc agctactcca 5280

gaggctgaga tggatcatct gagcctcagg aggttgaggc tgcagtgagc tgtgactgcg 5340

ccactgcact ccagtctggg acaacagagc aagaccctgt cttaaaaaaa aaaagaaaaa 5400

aaaaattttt ttctaagaag ctgtcctaca aagttgagct ttgttagttt ttcatgtgta 5460

atatattata aatttatctt ttgggatata ataaatgctt tcatatacct gcaaaaaaaa 5520

aaaaaaaaaa aa 5532

<210> 722
<211> 736
<212> DNA
<213> Homo sapiens

<400> 722
cccttccggc tggccccgct cagtcacccg cagcaggcgt gcagtttccc ggctctccgc 60

gcggccgggg aaggtcagcg ccgtaatggc gttcttggcg tcgggaccct acctgaccca 120

tcagcaaaag gtgttgcggc tttataagcg ggcgctacgc cacctcgagt cgtggtgcgt 180

ccagagagac aaataccgat actttgcttg tttgatgaga gcccggtttg aagaacataa 240

gaatgaaaag gatatggcga aggccaccca gctgctgaag gaggccgagg aagaattctg 300

gtaccgtcag catccacagc catacatctt ccctgactct cctgggggca cctcctatga 360

gagatacgat tgctacaagg tcccagaatg gtgcttagat gactggcatc cttctgagaa 420

ggcaatgtat cctgattact ttgccaagag agaacagtgg aagaaactgc ggagggaaag 480

ctgggaacga gaggttaagc agctgcagga ggaaacgcca cctggtggtc ctttaactga 540

agctttgccc cctgcccgaa aggaaggtga tttgccccca ctgtggtggt atattgtgac 600

cagaccccgg gagcggccca tgtagaaaga gagagacctc atctttcatg cttgcaagtg 660

aaatatgtta cagaacatgc acttgcccta ataaaaaatc agtgaaatgg tctctggtaa 720

aaaaaaaaaa aaaaaa 736

<210>  723
<211>  1968
<212>  DNA
<213>  Homo sapiens

<400>  723
gaggaggagg aggagatgac tggggagcgg gagctcgaga atactgccca gttactctag      60

cgcgccaggc cgaaccgcag cttcttggct taggtacttc tactcacagc ggccgattcc     120

gaggccaact ccagcaatgg cttttgcaaa tctgcggaaa gtgctcatca gtgacagcct     180

ggacccttgc tgccggaaga tcttgcaaga tggagggctg caggtggtgg aaaagcagaa     240

ccttagcaaa gaggagctga tagcggagct gcaggactgt gaaggcctta ttgttcgctc     300

tgccaccaag gtgaccgctg atgtcatcaa cgcagctgag aaactccagg tggtgggcag     360

ggctggcaca ggtgtggaca atgtggatct ggaggccgca acaaggaagg gcatcttggt     420

tatgaacacc cccaatggga acagcctcag tgccgcagaa ctcacttgtg gaatgatcat     480

gtgcctggcc aggcagattc cccaggcgac ggcttcgatg aaggacggca aatgggagcg     540

gaagaagttc atgggaacag agctgaatgg aaagaccctg ggaattcttg gcctgggcag     600

gattgggaga gaggtagcta cccggatgca gtcctttggg atgaagacta tagggtatga     660

ccccatcatt tccccagagg tctcggcctc ctttggtgtt cagcagctgc ccctggagga     720

gatctggcct ctctgtgatt tcatcactgt gcacactcct ctcctgccct ccacgacagg     780

cttgctgaat gacaacacct ttgcccagtg caagaagggg gtgcgtgtgg tgaactgtgc     840

ccgtggaggg atcgtggacg aaggcgccct gctccgggcc ctgcagtctg gccagtgtgc     900

cggggctgca ctggacgtgt ttacggaaga gccgccacgg gaccgggcct tggtggacca     960

tgagaatgtc atcagctgtc cccacctggg tgccagcacc aaggaggctc agagccgctg    1020

tggggaggaa attgctgttc agttcgtgga catggtgaag gggaaatctc tcacgggggt    1080

tgtgaatgcc caggccctta ccagtgcctt ctctccacac accaagcctt ggattggtct    1140

ggcagaagct ctggggacac tgatgcgagc ctgggctggg tcccccaaag ggaccatcca    1200

ggtgataaca cagggaacat ccctgaagaa tgctgggaac tgcctaagcc ccgcagtcat    1260

tgtcggcctc ctgaaagagg cttccaagca ggcggatgtg aacttggtga acgctaagct    1320

gctggtgaaa gaggctggcc tcaatgtcac cacctcccac agccctgctg caccagggga    1380

gcaaggcttc ggggaatgcc tcctggccgt ggccctggca ggcgcccctt accaggctgt    1440

gggcttggtc caaggcacta cacctgtact gcaggggctc aatggagctg tcttcaggcc    1500

agaagtgcct ctccgcaggg acctgcccct gctcctattc cggactcaga cctctgaccc    1560

tgcaatgctg cctaccatga ttggcctcct ggcagaggca ggcgtgcggc tgctgtccta    1620

ccagacttca ctggtgtcag atggggagac ctggcacgtc atgggcatct cctccttgct    1680

gcccagcctg gaagcgtgga agcagcatgt gactgaagcc ttccagttcc acttctaacc    1740

ttggagctca ctggtccctg cctctggggc ttttctgaag aaacccaccc actgtgatca    1800

atagggagag aaaatccaca ttcttgggct gaacgcgggc ctctgacact gcttacactg    1860

cactctgacc ctgtagtaca gcaataaccg tctaataaag agcctacccc caaaaaaaaa 1920

aaaaaaaaaa aaaaaaaaaa aaaaaaaaaa aaaaaaaaaa aaaaaaaa 1968

<210> 724
<211> 3524
<212> DNA
<213> Homo sapiens

<400> 724
gagtcgcggg ccttttgagg gaggaggcag agcgcgccgg gccggtggca tcttccttac 60

tttgtccatc ctccggactc gcgatcttcc ttccggagcc atgtcagaag gagtggactt 120

gattgatata tatgctgacg aggagttcaa ccaggaccca gagttcaaca atacagatca 180

gattgacctg tatgatgatg tgctgacagc cacctcacag ccctcagatg acagaagcag 240

cagcactgaa ccacctcctc ctgttcgcca ggagccatct cccaagccca acaacaagac 300

ccctgcaatt ctgtatacct acagtggcct gcgtaataga cgagctgccg tttatgtggg 360

cagcttctcc tggtggacca cagaccagca gctgatccag gttattcgct ctataggagt 420

ctatgatgtg gtggagttga aatttgcaga gaatcgagca aatggccagt ccaaagggta 480

tgctgaggtg gtggtagcct ctgaaaactc tgtccacaaa ttgttggaac tcctaccagg 540

gaaagttctt aatggagaaa aagtggacgt gaggccggcc acccggcaga acctgtcaca 600

gtttgaggca caggctcgga aacgtgagtg tgtccgagtc ccaagagggg gaatacctcc 660

acgggcccat tcccgagatt ctagtgattc tgctgatgga cgggccacac cctctgagaa 720

ccttgtaccc tcatctgctc gtgtggataa gccccccagt gtgctgccct acttcaatcg 780

tcctccttcg gcccttcccc tgatgggtct gcccccacca ccaattccac ccccaccacc 840

tctctcctca agctttgggg tccctcctcc tcctcctggt atccactacc agcatctcat 900

gcccccacct cctcgattac ctcctcatct tgctgtacct ccccctgggg ccatcccacc 960

tgcccttcac ctcaatccag ccttcttccc cccaccaaac gctacagtgg ggcctccacc 1020

agatacttac atgaaggcct ctgcccccta taaccaccat ggcagccgag attcgggccc 1080

tccaccctct acagtgagtg aagccgaatt tgaagatatc atgaagcgaa acagagcaat 1140

ttccagcagt gccatttcca aagcagtatc tggagccagt gcaggggatt acagtgacgc 1200

aattgagacg ctgctcacag ccattgcggt tatcaaacag tcccgggttg ccaatgatga 1260

gcgttgccgt gtcctcatct cctctcttaa ggactgtctt catggcattg aagccaagtc 1320

ctacagtgtg ggtgccagtg ggagctcttc caggaaaaga catcgctccc gggaaaggtc 1380

acctagccgg tcccgggaga gcagcaggag gcaccgggat ctgcttcata atgaagatcg 1440

gcatgatgat tatttccaag aaaggaaccg ggagcatgag agacaccggg atagagaacg 1500

ggaccggcac cactgagaaa ggagtctggt tggaagcaaa tgttttttta atggacttgc 1560

atctcctcac cttgatcagg actaaaggac ggaggccgcc ccaccccctt ccctttcctc 1620

caaaccccta actccctcca gacacccagg gaataccctc tgccccacag gattgaagac 1680

tgcttggcag tcctcccaat cccacacctc ctgtttgcca ggggaaagaa cctaaagact 1740

tcgtgtgatt gggaggggtg gcagacagga agaaaacatg tccaggcccc tggtctccat 1800
agagaatggt gctttgtcca agaaaacgta tgagtttctg attctccggg agccgttcaa 1860
tggtgaggtt gatgggaaga cttccttccc aaagaaaata gatcctccat gcaggatcta 1920
ggagagtgac tgggtgtgcc aaaatatgcc cagggtcctg ccctcagcac tagatttaat 1980
ggggccaaga gggtccaaac cccttgctaa cataccactt ctttgtttaa ctcctttacc 2040
tttccagccc tttgaggagg gaccatgaga acagaaatta ccttatgaaa agctacttct 2100
gttcctgctt tccctctcac gtattgacgg tttatttctt tgacctccca gagggctgaa 2160
ctctttcaac tctgcgctgc ccagccttct cagtggactt gcccctccta agcagagaag 2220
gcctatgagg ttgcttgctg ctgggaagcc tggcagagcc aattaccacc ctctgctgct 2280
tagtgcttgg gtacctcttg caataaccag ctcttagttg ttccctttcc ctggggcttt 2340
tccatttaac acatggagcc cttcccccag aaggctactt tcttgtttta gaggaaggta 2400
ctgcccattg ggagatgggg acattgggac ctcagcaatg aagaaccctt gtgaagtaac 2460
caggaggaat ggggaaagaa gcaagttggg caggatatgg cctacttcca taggcttttc 2520
ttttttcagg tttgatgtaa gcatgggctt acatccccca ggtacatact tttacttatt 2580
gtgggataac ctggcactag taggcaggta aagtcacaaa tttggtgtct tttcaccttt 2640
tgactgttga cttaatagct cctctcactc tgcctggaga tacttcctgc ctcagatgag 2700
gagccagaag aaacagagcc cgacttgaat gaactcagct cagagttcta aggaccagca 2760
ttctgggggc cattttctct acaggcaaat ggaattgctt ttccataaca tccaaattgt 2820
aatgtggttg ctgctgaagg aggaggcagc agcgaggtcc tgcggtaccc atggggtgat 2880
gctacttctg catgcatcta cagggcatct gacacctaac atgagacgtg gcatgtgaga 2940
tgagacttgg catgtgagac atagggtcac tagagaccct tctgggtcag aggagagaga 3000
ctgaattgga ctaaacccgt cctctgttcc cagcacgttt ctcatatagc cctcagtcac 3060
tgagggagtc ccccgcagga ttggagaggc acattccctt gggacagagg ctacaggttg 3120
gagctttttt tcccctgtcc cccaacccca tccccacctc cacttcagaa catggcaccc 3180
cacccaactg gccaagtgtt aagtgatgtg cttattgaga gcaactccgg gtgtctttta 3240
aaatgtagag aaaaggtgac agtttaagga aaaatatata tagaatacca gaaatgccgt 3300
ttacccggag aatttttttc tccccatttg ttttgttttt actcaatgac accattttta 3360
gttttatttc ctgatagcaa aaggaaaaaa aacacccatc cctcaaaaag gccaaggtcc 3420
cgtccccctg ttgtcggtga tttgtttgtc tttctgatag gttgaaaatt gtgtaataaa 3480
cttgatgacg ctgtcaaaaa aaaaaaaaaa aaaaaaaaaa aaaa 3524

<210> 725
<211> 1128
<212> DNA
<213> Homo sapiens

<400> 725

gcttctcgtt gtgccccgcc cgcaagcgcc ctcctccggg ccttcgtgac agccaggtcg 60

tgcgcgggtc atcctgggat tggtagttcg ctttctctca tttagccagt ttctttctct 120

accggggact ccgtgtcccg gcatccaccg cggcacctga cccttggcgc ttgcgtgttg 180

ccctcttccc caccctccct aatttccact ccccccaccc cacttcgcct gccgcggtcg 240

ggtccgcggc ctgcgctgta gcggtcgccg ccgttccctg gaagtagcaa cttccctacc 300

ccaccccagt cctggtcccc gtccagccgc tgacgtgaag atgagcagct cagaggaggt 360

gtcctggatt tcctggttct gtgggctccg tggcaatgaa ttcttctgtg aagtggatga 420

agactacatc caggacaaat ttaatcttac tggactcaat gagcaggtcc ctcactaccg 480

acaagctcta gacatgatct tggacctgga gcctgatgaa gaactggaag acaaccccaa 540

ccagagtgac ctgattgagc aggcagccga gatgctttat ggattgatcc acgcccgcta 600

catccttacc aaccgtggca tcgcccagat gttggaaaag taccagcaag gagactttgg 660

ttactgtcct cgtgtgtact gtgagaacca gccaatgctt cccattggcc tttcagacat 720

cccaggtgaa gccatggtga agctctactg ccccaagtgc atggatgtgt acacacccaa 780

gtcatcaaga caccatcaca cggatggcgc ctacttcggc actggtttcc ctcacatgct 840

cttcatggtg catcccgagt accggcccaa gagacctgcc aaccagtttg tgcccaggct 900

ctacggtttc aagatccatc cgatggccta ccagctgcag ctccaagccg ccagcaactt 960

caagagccca gtcaagacga ttcgctgatt ccctccccca cctgtcctgc agtctttgac 1020

ttttcctttc ttttttgcca ccctttcagg aaccctgtat ggtttttagt ttaaattaaa 1080

ggagtcgtta ttgtggtggg aatatgaaat aaagtagaag aaaaggcc 1128

<210> 726
<211> 886
<212> DNA
<213> Homo sapiens

<400> 726
   ggggccgcgc gtgctgcagc cgccgctgct gctgctcctg ctggcgctgc tgctggcggc 60
   gctgccgtgc ggtgccgaag aggcctcgcc gctgcgcccc gcgcaggtca cgttgtcgcc 120
   gccgccggcc gtgacgaacg ggagccagcc gggcgcgcca cacaacagca cgcacacgcg 180
   tccgccgggg gcgtcgggct cggcgctgac gcgctccttc tacgtgatcc tgggcttctg 240
   cggcctgacc gcgctctact tcctgatccg ggcgtttagg ttgaagaagc ctcagcggag 300
   gcgatacggc ctcctcgcca acactgagga ccccacggag atggcctcgc tggacagcga 360
   cgaggagacg gtctttgagt cccggaatct gagatgatgc tgagccaggg aggcggccct 420
   tccagcagcc atgagggaag gacaggagat ggggcccacc ccagtgccca gcaaccccct 480
   gctccaccgc tcattcccct gctggccccg gggctggtct cacccagtgc caacccgaga 540
   gctccttttg gaacctgcac agcccgccga cctgttgcca cctgcaccca ccgctggacc 600
   atgcagcctc gcctcctgga tgctgtccca gcctggccga gggtcccagg tgaagactgg 660
   agggacccca acagccaccg cccaggacgc tgaggctccc ttgcctgact gtgacttgtg 720
   cctctctcct gcccccgtgg ggacatggca gcccagagcc aaggctgggt gggcaggtga 780
   cccaaggaac ctttctggga acaccttctc gccgggctgg gaacaataaa tgcagccatg 840
   tctctgcaaa aaaaaaaaaa aaaaaaaaaa aaaaaaaaaa aaaaaa 886

<210> 727
<211> 2284
<212> DNA
<213> Homo sapiens

<400> 727
tggggcggac gcggcggacg tgggtgaggg cgcggccgta agagagcggg acgcggggtg 60

cccggcgcgt ggtgggggtc cccggcgcct gcccccacgg cacccaagaa ggcctggcca 120

gggtaccctc cgcggagccc gggggtgggg ggcgcgggcc cggcgccgcg atgggcccgg 180

gacccccagc ggccggagcg gcgccgtccc cgcggccgct gtccctggtg gcgcggctga 240

gctacgccgt gggccacttc ctcaacgacc tgtgcgcgtc catgtggttc acctacctgc 300

tgctctacct gcactcggtg cgcgcctaca gctcccgcgg cgcggggctg ctgctgctgc 360

tgggccaggt ggccgacggg ctgtgcacac cgctcgtggg ctacgaggcc gaccgcgccg 420

ccagctgctg cgcccgctac ggcccgcgca aggcctggca cctggtcggc accgtctgcg 480

tcctgctgtc cttccccttc atcttcagcc cctgcctggg ctgtggggcg gccacgcccg 540

agtgggctgc cctcctctac tacggcccgt tcatcgtgat cttccagttt ggctgggcct 600

ccacacagat ctcccacctc agcctcatcc cggagctcgt caccaacgac catgagaagg 660

tggagctcac ggcactcagg tatgcgttca ccgtggtggc caacatcacc gtctacggcg 720

ccgcctggct cctgctgcac ctgcagggct cgtcgcgggt ggagcccacc caagacatca 780

gcatcagcga ccagctgggg ggccaggacg tgcccgtgtt ccggaacctg tccctgctgg 840

tggtgggtgt cggcgccgtg ttctcactgc tattccacct gggcacccgg gagaggcgcc 900

ggccgcatgc ggaggagcca ggcgagcaca cccccctgtt ggcccctgcc acggcccagc 960

ccctgctgct ctggaagcac tggctccggg agccggcttt ctaccaggtg ggcatactgt 1020

acatgaccac caggctcatc gtgaacctgt cccagaccta catggccatg tacctcacct 1080

actcgctcca cctgcccaag aagttcatcg cgaccattcc cctggtgatg tacctcagcg 1140

gcttcttgtc ctccttcctc atgaagccca tcaacaagtg cattgggagg aacatgacct 1200

acttctcagg cctcctggtg atcctggcct ttgccgcctg ggtggcgctg gcggagggac 1260

tgggtgtggc cgtgtacgca gcggctgtgc tgctgggtgc tggctgtgcc accatcctcg 1320

tcacctcgct ggccatgacg gccgacctca tcggtcccca cacgaacagc ggagcgttcg 1380

tgtacggctc catgagcttc ttggataagg tggccaatgg gctggcagtc atggccatcc 1440

agagcctgca cccttgcccc tcagagctct gctgcagggc ctgcgtgagc ttttaccact 1500

gggcgatggt ggctgtgacg ggcggcgtgg gcgtggccgc tgccctgtgt ctctgtagcc 1560

tcctgctgtg gccgacccgc ctgcgacgct cttttcttgc ctggagaaga gggaggggag 1620

aggacaaggg ccctggctac tcctggattc ctacagtcct tgtccagcct ccaagaccca 1680

caagtccctt cctctgggaa gcccccctgg cctggaggtg caccaggaag aagtggtctg 1740

gggctggcac taagccatgg cccagggaag actgggggac ccactaggcc aggatgagac 1800

ctgcacgcag tggctcacag cagcacgatt tgtgacagcc cgaggcggag aacaccgaac 1860

acccagtgaa ggtgagggga tcagcacggc gccgccaccg tgctggaacg agactcagcc 1920

acaaggaggt gcgaagctct gacccaggcc acagtgcgga tgcaccttga ggatgtcacg 1980

ctcagtgaga gacaccagac acagaagggt acgctgtgat cccacttcta tgaaatgtcc 2040

aggacagacc aatccacaga atcagggaga ggattcgtgg gtgccgggac tggggagggg 2100

gacctggggg tgactaggtg acataatggg gacagggctg ccttctgggt gatgagaatg 2160

ttctggaatc agatgggatg gctgcacggc gtggtgaagg tactgaacgc cacctcactg 2220

taagacggta gattttgtat tttaccacaa taaacaaaac aaaacaaaac caaaccaaac 2280

ccaa 2284

<210> 728
<211> 4357
<212> DNA
<213> Homo sapiens

<400> 728
ccccgccccg ctctttcgct tcccgggccg ccggcagccg ccgccagccg cagccatggg 60

ccgggcccgg ccgggccaac gcgggccgcc cagccccggc cccgccgcgc agcctcccgc 120

gccaccgcgc cgccgcgccc gttccctggc gctgctcgga gccctgctgg ccgccgccgc 180

tgccgccgcc gtccgggtct gcgcccgcca cgccgaggcc caggcggccg cgcggcagga 240

actggcgctg aagaccctgg ggacagatgg cctttttctc ttttcctcct tggacactga 300

cggggatatg tacatcagcc ctgaggagtt caaacccatt gctgagaagc taacagggtc 360

ttgttctgtc acccagactg gagtgcagtg gtgcagtcac agctcactgc agcctcaact 420

tccctggctc aattgatcct cctgcctcag cctcctgagg tcaactcccg cggccagctg 480

cgaggaggag gagttgcccc ctgaccctag cgaggagacg ctcaccatag aagcccgatt 540

ccagcctctg ctcccggaga ccatgaccaa gagcaaagat ggcttcctag gggtctcccg 600

cctcgccctg tccggcctcc gaaactggac agccgccgcc tcaccaagtg cagtgtttgc 660

cacccgccac ttccagccct tccttccccc gccaggccag gagctgggtg agccctggtg 720

gatcatcccc agtgagctga gcatgttcac tggctacctg tccaacaacc gcttctatcc 780

accgccgccc aagggcaagg aggtcatcat ccaccggctc ctgagcatgt tccaccctcg 840

gccctttgtg aagacccgct ttgcccctca gggagctgtg gcctgcctga ctgccatcag 900

cgacttctac tacactgtga tgttccggat ccatgccgag ttccagctca gtgagccgcc 960

cgacttcccc ttttggttct cccctgctca gttcaccggc cacatcatcc tctccaaaga 1020

cgccacccac gtccgcgact tccggctctt cgtgcccaac cacaggtctc tgaatgtgga 1080

catggagtgg ctttacgggg ccagtgaaag cagcaacatg gaggtggaca tcggctacat 1140

accccagatg gagctggagg ccacgggccc ctctgtgccc tccgtgatcc tggatgagga 1200
tggcagcatg atcgacagcc acctgccttc aggggagccc ctgcagtttg tgtttgagga 1260
gatcaagtgg cagcaggagc tgagctggga ggaggctgcc cggcgcctgg aggtggccat 1320
gtaccccttc aagaaggtct cctacttgcc gttcactgag gccttcgacc gagccaaggc 1380
tgagaacaag ctggtgcact caatcctgct gtgggggccc ctggatgacc agtcctgctg 1440
aggttcaggg cggactctcc gggagactgt cctggaaagt tcgcccatcc tcaccctgct 1500
caacgagagc ttcatcagca cctggtccct ggtgaaggag ctggaggaac tgcagaacaa 1560
ccaggagaac tcgtcccacc agaagctggc tggcctgcac ctggagaagt acagcttccc 1620
cgtggagatg atgatctgcc tgcccaatgg caccgtggtc catcacatca atgccaacta 1680
cttcttggac atcacctccg tgaagcccga ggaaatcgag agcaatctct tcagcttctc 1740
atccaccttt gaagacccgt ccacggccac ctacatgcag ttcctgaagg agggactccg 1800
gcgtggcctg cccctcctcc agccctagag tgcctggacg ggatctgatg cacaggcccc 1860
cacgcctcag agccagagtg gtcctcagcc catttcagac tgcagatgcc gcccactccc 1920
accccactcc taggctgcct tggagggtac aagatccact gagggtggcc accacagcct 1980
tggctccatg gtggcgggta gacaagggat gcctgggctg actgggcaga ggaacctcta 2040
gctctgactg tcactcggct ctccctaccc atttggctct ggaagctgct tggccccccc 2100
agatcagggc ctgggtgaac tccctggacc tttcctagcc agccgcacag tctaggccct 2160
tgtggggtga agaatggagg gaggagcagg ctaggaagac ggggccacca ccctctcctt 2220
gctttcagcc cttcccacag gaaacatcaa gaagccccag ccaggagggg ccaggctgcc 2280
aaggcggctc ccctgtttat ctagagcctt cgttcctggc cataccccgg actgccctcc 2340
tgtgcctgat gtccccagct ggggtcagtc tcaacaggag ccagtcttct ggagcctctg 2400
ggcagaaccc tccatcagag tggaaatcag acgggacccc ctgcagcttc cctgaccacg 2460
ccactgacca gctatctggg gaagtttact gtgaaggggt ttctgccttt agcaatgggg 2520
ttcactaagg gggttcccga ggcccagggc caaggcactc ccaccgccta ccttagcaca 2580
gggtctctgc aggactgcgg gagccagcgc tcctgccgcc cctcttgccc ctcagacctt 2640
gcatccacag aagcacaacc cagccaaaca ccacagcctt ctccagagcc ggcactgtcc 2700
cggcaaccag gggtgcccca ggctagctct tctacctctg gggcaccacg gactcccctt 2760
ggccactctt gggactttgg tccacgtcct gagccactga ccacggccag tctctctttt 2820
tatatgtgca gaaaagtgtt tttacacaaa ctttctcatg gtttgtaggt atttttttat 2880
aaccccagtg ctgaggagaa aggaggggca gtggcttccc cggcagcagc cccatgatgg 2940
ctgaatccga aatcctcgat gggtccagct tgatgtcttt gcagctgcac ctatgggaag 3000
aagtagtcct ctcttccttc tcctcttcag ctttttaaaa acagtcctca gaggatccat 3060
gatccccagc actgtcccat cctccacaaa ggcccacagg catgcctgta ctctctttca 3120
ttaaggtctt gaagtcaggc tgccccctcc ccagccccca gttctctccc caccccctca 3180

ccccacccgg ggctcactca gcctggcaga ggaagaagga aggcagacat ctccgcagcc 3240
actcctgggc cttttatgtg ccgagttacc ccacttgcct tgggcgtgtc cactgagcct 3300
tccccagcca gtcttgttct caattttgtt ttgttttgtt ttgagacgga gtcttgctct 3360
gtcacccagg ctggagtgct atggctcgat cttggctcac tgcaacctcc acctcccagg 3420
ttcaagcaat tctcttgcct cagcctcccg agtagctggg attacaggtg catgccacca 3480
tggctggcta atttttgtat ttttagtaga gatggggttt caccatattg gtcaggctga 3540
tctggaactt ctgacctcag gtgatccacc tgcctcagcc tcccaaagtg ctgggattac 3600
aggcgtgagc aatcgtgccc agccttgttc ttaattttgt atcatccagt catcgctaat 3660
attacacgca ccttctcact taatcctcac gacaagcctg tgaggcagat gctcattgtt 3720
cccatcttga tgaaacttga gtctcaggga agtgaagtga cttgcccagg gtcactcagg 3780
tagagttgag attcaaaccc acatgtggct ccaaagtctg catctggatt tgggggtgtt 3840
ttttggcatg gcaccctcac ctctctccct gcctgttttc cccaaagtgg aaaggaaggc 3900
ctttcaaacc agagtgtctc actcccctct gacctccaga ccagatgggg catgagccag 3960
ccagctcagc caggctccct gtgtcctggg aggaagtgtc cccatccccc atgcccctta 4020
tggggaggga gggcgtctga tgctctctct ctgcctcccc ccccatcctg tcaggcacag 4080
gtgacggggg cagcccatgc gagcccttct cctgctgctc tgggagggcc agttccacat 4140
tgagccagcc tggtcccatg gaaaatgatg gcctgggctt tctgaggcct tatctgatgc 4200
ctctgcagtt catgtccccc accaggcctc gaggctcagg gtgggagagg gccccgggct 4260
gccctgtcac tcctctaaca cttccctccc ctgtccccaa catgccctgt aataaaatta 4320
gagaagacta acaaaaaaaa aaaaaaaaaa aaaaaaa 4357

<210> 729
<211> 2144
<212> DNA
<213> Homo sapiens

<400> 729
acagcagcgg cgcggagact gcggggcggg ccatggcggc gaacctgagc cggaacgggc 60
cagcgctgca agaggcctac gtgcgggtgg tcaccgagaa gtccccgacc gactgggctc 120
tctttaccta tgaaggcaac agcaatgaca tccgcgtggc tggcacaggg gagggtggcc 180
tggaggagat ggtggaggag ctcaacagcg ggaaggtgat gtacgccttc tgcagagtga 240
aggaccccaa ctctggactg cccaaatttg tcctcatcaa ctggacaggc gagggcgtga 300
acgatgtgcg gaagggagcc tgtgccagcc acgtcagcac catggccagc ttcctgaagg 360
gggcccatgt gaccatcaac gcacgggccg aggaggatgt ggagcctgag tgcatcatgg 420
agaaggtggc caaggcttca ggtgccaact acagctttca caaggagagt ggccgcttcc 480
aggacgtggg accccaggcc ccagtgggct ctgtgtacca gaagaccaat gccgtgtctg 540
agattaaaag ggttggtaaa gacagcttct gggccaaagc agagaaggag gaggagaacc 600

gtcggctgga ggaaaagcgg cgggccgagg aggcacagcg gcagctggag caggagcgcc 660

gggagcgtga gctgcgtgag gctgcacgcc gggagcagcg ctatcaggag cagggtggcg 720

aggccagccc ccagaggacg tgggagcagc agcaagaagt ggtttcaagg aaccgaaatg 780

agcaggagtc tgccgtgcac ccgagggaga ttttcaagca gaaggagagg gccatgtcca 840

ccacctccat ctccagtcct cagcctggca agctgaggag ccccttcctg cagaagcagc 900

tcacccaacc agagacccac tttggcagag agccagctgc tgccatctca aggcccaggg 960

cagatctccc tgctgaggag ccggcgccca gcactcctcc atgtctggtg caggcagaag 1020

aggaggctgt gtatgaggaa cctccagagc aggagacctt ctacgagcag cccccactgg 1080

tgcagcagca aggtgctggc tctgagcaca ttgaccacca cattcagggc caggggctca 1140

gtgggcaagg gctctgtgcc cgtgccctgt acgactacca ggcagccgac gacacagaga 1200

tctcctttga ccccgagaac ctcatcacgg gcatcgaggt gatcgacgaa ggctggtggc 1260

gtggctatgg gccggatggc cattttggca tgttccctgc caactacgtg gagctcattg 1320

agtgaggctg agggcacatc ttgcccttcc cctctcagac atggcttcct tattgctgga 1380

agaggaggcc tgggagttga cattcagcac tcttccagga ataggacccc cagtgaggat 1440

gaggcctcag ggctccctcc ggcttggcag actcagcctg tcaccccaaa tgcagcaatg 1500

gcctggtgat tcccacacat ccttcctgca tcccccgacc ctcccagaca gcttggctct 1560

tgcccctgac aggatactga gccaagccct gcctgtggcc aagccctgag tggccactgc 1620

caagctgcgg ggaagggtcc tgagcagggg catctgggag gctctggctg ccttctgcat 1680

ttatttgcct tttttctttt tctcttgctt ctaaggggtg gtggccacca ctgtttagaa 1740

tgacccttgg gaacagtgaa cgtagagaat tgtttttagc agagtttgtg accaaagtca 1800

gagtggatca tggtggtttg gcagcaggga atttgtcttg ttggagcctg ctctgtgctc 1860

cccactccat ttctctgtcc ctctgcctgg gctatgggaa gtggggatgc agatggccaa 1920

gctcccaccc tgggtattca aaaacggcag acacaacatg ttcctccacg cggctcactc 1980

gatgcctgca ggccccagtg tgtgcctcaa ctgattctga cttcaggaaa agtaacacag 2040

agtggccttg gcctgttgtc ttcccctatt ttctgtccca gctcatccgt gtctctgaag 2100

aacaaatatg cttttggacc acgaaaaaaa aaaaaaaaaa aaaa 2144

<210> 730
<211> 990
<212> DNA
<213> Homo sapiens

<400> 730
tccggagccc ggctcgctgg ggcagcatgg cggggtcgcc gctgctctgg gggccgcggg 60

ccgggggcgt cggccttttg gtgctgctgc tgctcggcct gtttcggccg ccccccgcgc 120

tctgcgcgcg gccggtaaag gagccccgcg gcctaagcgc agcgtctccg cccttggctg 180

agactggcgc tcctcgccgc ttccggcggt cagtgccccg aggtgaggcg gcgggggcgg 240

tgcaggagct ggcgcgggcg ctggcgcatc tgctggaggc cgaacgtcag gagcgggcgc 300

gggccgaggc gcaggaggct gaggatcagc aggcgcgcgt cctggcgcag ctgctgcgcg 360
tctggggcgc cccccgcaac tctgatccgg ctctgggcct ggacgacgac cccgacgcgc 420
ctgcagcgca gctcgctcgc gctctgctcc gcgcccgcct tgaccctgcc gccctcgcag 480
cccagcttgt ccccgcgccc gtccccgccg cggcgctccg accccggccc ccggtctacg 540
acgacggccc cgcgggcccg gatgctgagg aggcaggcga cgagacaccc gacgtggacc 600
ccgagctgtt gaggtacttg ctgggacgga ttcttgcggg aagcgcggac tccgaggggg 660
tggcagcccc gcgccgcctc cgccgtgccg ccgaccacga tgtgggctct gagctgcccc 720
ctgagggcgt gctgggggcg ctgctgcgtg tgaaacgcct agagaccccg gcgccccagg 780
tgcctgcacg ccgcctcttg ccaccctgag cactgcccgg atcccgtgca ccctgggacc 840
cagaagtgcc cccgccatcc cgccaccagg actgctcccc gccagcacgt ccagagcaac 900
ttaccccggc cagccagccc tctcacccga ggatccctac cccctggccc cacaataaac 960
atgatctgaa gcaaaaaaaa aaaaaaaaaa 990

<210> 731
<211> 8187
<212> DNA
<213> Homo sapiens

<400> 731
cccgagaagc ggcggggcgg cgggccggcg ggcggggcgc agagccaggc agcgcaggta 60
tagccaggct ggagaaaaga agctgccacc atggttgcac tttcactgaa gatcagcatt 120
gggaatgtgg tgaagacgat gcagtttgag ccgtctacca tggtgtacga cgcctgccgc 180
atcattcgtg agcggatccc agaggcccca gctggtcctc ccagcgactt tgggctcttt 240
ctgtcagatg atgaccccaa aaagggtata tggctggagg ctgggaaagc tttggactac 300
tacatgctcc gaaatgggga cactatggag tacaggaaga aacagagacc cctgaagatc 360
cgtatgctgg atggaactgt gaagacgatc atggtggatg actctaagac tgtcactgac 420
atgctcatga ccatctgtgc ccgcattggc atcaccaatc atgatgaata ttcattggtt 480
cgagagctga tggaagagaa aaaggaggaa ataacaggga ccttaagaaa ggacaagaca 540
ttgctgcgag atgaaaagaa gatggagaaa ctaaagcaga aattgcacac agatgatgag 600
ttgaactggc tggaccatgg tcggacactg agggagcagg gtgtagagga gcacgagacg 660
ctgctgctgc ggaggaagtt cttttactca gaccagaatg tggattcccg ggaccctgta 720
cagctgaacc tcctgtatgt gcaggcacga gatgacatcc tgaatggctc ccaccctgtc 780
tcctttgaca aggcctgtga gtttgctggc ttccaatgcc agatccagtt tgggccccac 840
aatgagcaga agcacaaggc tggcttcctt gacctgaagg acttcctgcc caaggagtat 900
gtgaagcaga agggagagcg taagatcttc caggcacaca agaattgtgg gcagatgagt 960
gagattgagg ccaaggtccg ctacgtgaag ctagcccgtt ctctcaagac ttacggtgtc 1020
tccttcttcc tggtgaagga aaaaatgaaa gggaagaaca agctagtgcc caggcttctg 1080

ggcatcacca aggagtgtgt gatgcgagtg gatgagaaga ccaaggaagt gatccaggag 1140
tggaacctca ccaacatcaa acgctgggct gcgtctccca aaagcttcac cctggatttt 1200
ggagattacc aagatggcta ttactcagta cagacaactg aaggggagca gattgcacag 1260
ctcattgccg gctacatcga tatcatcctg aagaagaaaa aaagcaagga tcactttggg 1320
ctggaaggag atgaggagtc tactatgctg gaggactcag tgtcccccaa aaagtcaaca 1380
gtcctgcagc agcaatacaa ccgggtgggg aaagtggagc atggctctgt ggccctgcct 1440
gccatcatgc gctctggagc ctctggtcct gagaatttcc aggtgggcag catgccccct 1500
gcccagcagc agattaccag cggccagatg caccgaggac acatgcctcc tctgacttca 1560
gcccagcagg cactcactgg aaccattaac tccagcatgc aggccgtgca ggctgcccag 1620
gccaccctgg atgactttga cactctgccg cctcttggcc aggatgctgc ctctaaggcc 1680
tggcgtaaaa acaagatgga tgaatcaaag catgagatcc actctcaggt agatgccatc 1740
acagctggta ctgcgtctgt ggtgaacctg acagcagggg accctgctga gacagactat 1800
accgcagtgg gctgtgcagt caccacaatc tcctccaacc tgacggagat gtcccgtggg 1860
gtgaagctgc tggctgcctt gctggaggac gaaggcggca gtggtcggcc cctgttgcag 1920
gcagcaaagg gccttgcggg agcagtgtca gaactgctgc gcagtgccca accagccagt 1980
gctgagcccc gtcagaacct gctgcaagca gctgggaacg tgggccaggc cagtggggag 2040
ctgttgcaac aaattgggga aagtgatact gacccccact tccaggatgc gctaatgcag 2100
ctcgccaaag ctgtggcaag tgctgcagct gccctggtcc tcaaggccaa gagtgtggcc 2160
cagcggacag aggactcggg acttcagacc caagttattg ctgcagcaac acagtgtgcc 2220
ctatccactt cccaactagt ggcctgtact aaggtggtgg cacctacaat cagctcacct 2280
gtctgccaag agcaactggt ggaggctgga cgactggtag ccaaagccgt ggagggctgt 2340
gtgtctgcct cccaggcagc tacagaggat gggcaactgt tgcgaggggt aggagcagca 2400
gccacagctg tcacccaggc cctaaatgag ctgctgcagc atgtgaaagc ccatgccaca 2460
ggggctgggc ctgctggccg ttatgaccag gctactgaca ccatcctaac cgtcactgag 2520
aacatcttta gctccatggg tgatgctggg gagatggtgg gacaggcccg catcctggcc 2580
caagccacat ctgacctggt caatgccatc aaggctgatg ctgaggggga aagtgatctg 2640
gagaactccc gcaagctctt aagtgctgcc aagatcctag ctgatgccac agccaagatg 2700
gtagaggctg ccaagggagc agctgcccac cctgacagtg aggagcagca gcagcggctg 2760
cgggaggcag ctgaggggct gcgcatggcc accaatgcag ctgcgcagaa tgccatcaag 2820
aaaaagctgg tgcagcgcct ggagcatgca gccaagcagg ctgcagcctc agccacacag 2880
accatcgctg cagctcagca cgcagcctct acccccaaag cctctgccgg cccccagccc 2940
ctgctggtgc agagctgcaa ggcagtggca gagcagattc cactgctggt gcagggcgtc 3000
cgaggaagcc aagcccagcc tgacagcccc agcgctcagc ttgccctcat tgctgccagc 3060
cagagcttcc tgcagccagg tgggaagatg gtggcagctg caaaggcctc agtgccaacg 3120

attcaggacc aggcttcagc catgcagctg agtcagtgtg ccaagaacct gggcaccgcg 3180
ctggctgaac tccggacggc tgcccagaag gctcaggaag catgtggacc tttggagatg 3240
gattctgcac tgagtgtggt acagaatcta gagaaagatc tacaggaagt gaaggcagca 3300
gctcgagatg gcaagcttaa acccttacct ggggagacaa tggagaagtg tacccaggac 3360
ctgggcaaca gcaccaaagc cgtgagctca gccatcgccc agctactggg agaggttgcc 3420
cagggcaatg agaattatgc aggtattgca gctcgggatg tggcaggtgg gctgcggtca 3480
ctggcccagg ccgctagggg agtcgctgca ctgacgtcag atcctgcagt gcaggccatt 3540
gtacttgata cggccagtga tgtgctggac aaggccagca gcctcattga ggaggcgaaa 3600
aaggcagctg gccatccagg ggaccctgag agccagcagc ggcttgccca ggtggctaaa 3660
gcagtgaccc aggctctgaa ccgctgtgtc agctgcctac ctggccagcg cgatgtggat 3720
aatgccctga gggcagttgg agatgccagc aagcgactcc tgagtgactc gcttcctcct 3780
agcactggga catttcaaga agctcagagc cggttgaatg aagctgctgc tgggctgaat 3840
caggcagcca cagaactggt gcaggcctct cggggaaccc ctcaggacct ggctcgagcc 3900
tcaggccgat ttggacagga cttcagcacc ttcctggaag ctggtgtgga gatggcaggc 3960
caggctccga gccaggagga ccgagcccaa gttgtgtcca acttgaaggg catctccatg 4020
tcttcaagca aacttcttct ggctgccaag gccctgtcca cggaccctgc tgcccctaac 4080
ctcaagagtc agctggctgc agctgccagg gcagtaactg acagcatcaa tcagctcatc 4140
actatgtgca cccagcaggc acccggccag aaggagtgtg ataacgccct gcgggaattg 4200
gagacggtcc gggaactcct ggagaaccca gtccagccca tcaatgacat gtcctacttt 4260
ggttgcctgg acagtgtaat ggagaactca aaggtgctgg gcgaggccat gactggcatc 4320
tcccaaaatg ccaagaacgg aaacctgcca gagtttggag atgccatttc cacagcctca 4380
aaggcacttt gtggcttcac cgaggcagct gcacaggctg catatctggt tggtgtctct 4440
gaccccaata gccaagctgg acagcaaggg ctagtggagc ccacacagtt tgcccgtgca 4500
aaccaggcaa ttcagatggc ctgccagagt ttgggagagc ctggctgtac ccaggcccag 4560
gtgctctctg cagccaccat tgtggctaaa cacacctctg cactgtgtaa cagctgtcgc 4620
ctggcttctg cccgtaccac caatcctact gccaagcgcc agtttgtaca gtcagccaag 4680
gaggtggcca acagcacagc taatcttgtc aagaccatca aggcgctaga tggggccttc 4740
acagaggaga accgtgccca gtgccgagca gcaacagccc ctctgctgga ggctgtggac 4800
aatctgagtg cctttgcgtc caaccctgag ttctccagca ttcctgccca gatcagccct 4860
gagggtcggg ctgccatgga gcccattgtg atctctgcca agacaatgtt agagagtgcc 4920
gggggactca tccagacagc ccgggccctc gcagtcaatc cccgggaccc cccgagctgg 4980
tcggtgctgg ccggccactc ccgtactgtc tcagactcca tcaagaagct aattacaagc 5040
atgagggaca aggctccagg gcagctggag tgtgaaacgg ccattgcagc tctgaacagt 5100
tgtctacggg acctagacca ggcttccctc gctgcagtca gccagcagct tgctccccgt 5160

gagggaatct ctcaagaggc cttgcacact cagatgctca ctgcagtcca agagatctcc 5220
catctcattg agccgctggc caatgctgcc cgggctgaag cctcccagct gggacacaag 5280
gtgtcccaga tggcgcagta ctttgagccg ctcaccctgg ctgcagtggg tgctgcctcc 5340
aagaccctga gccacccgca gcagatggca ctcctggacc agactaaaac attggcagag 5400
tctgccctgc agttgctata cactgccaag gaggctggtg gtaacccaaa gcaagcagct 5460
cacacccagg aagccctgga ggaggctgtg cagatgatga ccgaggccgt agaggacctg 5520
acaacaaccc tcaacgaggc agccagtgct gctggggtcg tgggtggcat ggtggactcc 5580
atcacccagg ccatcaacca gctagatgaa ggaccaatgg gtgaaccaga aggttccttc 5640
gtggattacc aaacaactat ggtgcggaca gccaaggcca ttgcagtgac cgttcaggag 5700
atggttacca agtcaaacac cagcccagag gagctgggcc ctcttgctaa ccagctgacc 5760
agtgactatg gccgtctggc ctcggaggcc aagcctgcag cggtggctgc tgaaaatgaa 5820
gagataggtt cccatatcaa acaccgggta caggagctgg gccatggctg tgccgctctg 5880
gtcaccaagg caggcgccct gcagtgcagc cccagtgatg cctacaccaa gaaggagctc 5940
atagagtgtg cccggagagt ctctgagaag gtctcccacg tcctggctgc gctccaggct 6000
gggaatcgtg gcacccaggc ctgcatcaca gcagccagcg ctgtgtctgg tatcattgct 6060
gacctcgaca ccaccatcat gttcgccact gctggcacgc tcaatcgtga gggtactgaa 6120
actttcgctg accaccggga gggcatcctg aagactgcga aggtgctggt ggaggacacc 6180
aaggtcctgg tgcaaaacgc agctgggagc caggagaagt tggcgcaggc tgcccagtcc 6240
tccgtggcga ccatcacccg cctcgctgat gtggtcaagc tgggtgcagc cagcctggga 6300
gctgaggacc ctgagaccca ggtggtacta atcaacgcag tgaaagatgt agccaaagcc 6360
ctgggagacc tcatcagtgc aacgaaggct gcagctggca aagttggaga tgaccctgct 6420
gtgtggcagc taaagaactc tgccaaggtg atggtgacca atgtgacatc attgcttaag 6480
acagtaaaag ccgtggaaga tgaggccacc aaaggcactc gggccctgga ggcaaccaca 6540
gaacacatac ggcaggagct ggcggttttc tgttccccag agccacctgc caagacctct 6600
accccagaag acttcatccg aatgaccaag ggtatcacca tggcaaccgc caaggccgtt 6660
gctgctggca attcctgtcg ccaggaagat gtcattgcca cagccaatct gagccgccgt 6720
gctattgcag atatgcttcg ggcttgcaag gaagcagctt accacccaga agtggcccct 6780
gatgtgcggc ttcgagccct gcactatggc cgggagtgtg ccaatggcta cctggaactg 6840
ctggaccatg tactgctgac cctgcagaag ccaagcccag aactgaagca gcagttgaca 6900
ggacattcaa agcgtgtggc tggttccgtc actgagctca tccaggctgc tgaagccatg 6960
aagggaacag aatgggtaga cccagaggac cccacagtca ttgctgagaa tgagctcctg 7020
ggagctgcag ccgccattga ggctgcagcc aaaaagctag agcagctgaa gccccgggcc 7080
aaacccaagg aggcagatga gtccttgaac tttgaggagc agatactaga agctgccaag 7140
tccattgcag cagccaccag tgcactggta aaggctgcgt cggctgccca gagagaacta 7200

gtggcccaag ggaaggtggg tgccattcca gccaatgcac tggacgatgg gcagtggtcc 7260

cagggcctca tttctgctgc ccggatggtg gctgcggcca ccaacaatct gtgtgaggca 7320

gccaatgcag ctgtacaagg ccatgccagc caggagaagc tcatctcatc agccaagcag 7380

gtagctgcct ccacagccca gctccttgtg gcctgcaagg tcaaggctga ccaggactcg 7440

gaggcaatga aacgacttca ggctgctggc aacgcagtga agcgagcctc agataatctg 7500

gtgaaagcag cacagaaggc tgcagccttt gaagagcagg agaatgagac agtggtggtg 7560

aaagagaaga tggttggcgg cattgcccag atcatcgcag cacaggaaga aatgcttcgg 7620

aaggaacgag agctggaaga ggcgcggaag aaactggccc agatccggca gcagcagtac 7680

aagtttctgc cttcagagct tcgagatgag cactaaagaa gcctcttcta tttaatgcag 7740

acccggccca gagactgtgc gtgccactac caaagccttc tgggctgtcg gggcccaacc 7800

tgcccaaccc cagcactccc caaagtgcct gccaaacccc agggcctggc cccgcccagt 7860

cccgcagtac atcccctgtc ccctccccaa ccccaagtgc cttcatgccc tagggccccc 7920

caagtgcctg cccctcccca gagtattaac gctccaagag tattattaac gctgctgtac 7980

ctcgatctga atctgccggg gccccagccc actccaccct gccagcagct tccagccagt 8040

ccccacagcc tcatcagctc tcttcaccgt tttttgatac tatcttcccc cacccccagc 8100

tacccatagg ggctgcagag ttataagccc caaacaggtc atgctccaat aaaaatgatt 8160

ctacctacaa aaaaaaaaaa aaaaaaa 8187

<210> 732
<211> 889
<212> DNA
<213> Homo sapiens

<400> 732
gcagttcggc ggtcccgcgg gtctgtctct tgcttcaaca gtgtttggac ggaacagatc 60

cggggactct cttccagcct ccgaccgccc tccgatttcc tctccgcttg caacctccgg 120

gaccatcttc tcggccatct cctgcttctg ggacctgcca gcaccgtttt tgtggttagc 180

tccttcttgc caaccaacca tgagctccca gattcgtcag aattattcca ccgacgtgga 240

ggcagccgtc aacagcctgg tcaatttgta cctgcaggcc tcctacacct acctctctct 300

gggcttctat ttcgaccgcg atgatgtggc tctggaaggc gtgagccact tcttccgcga 360

attggccgag gagaagcgcg agggctacga gcgtctcctg aagatgcaaa accagcgtgg 420

cggccgcgct ctcttccagg acatcaagaa gccagctgaa gatgagtggg gtaaaacccc 480

agacgccatg aaagctgcca tggccctgga gaaaaagctg aaccaggccc ttttggatct 540

tcatgccctg ggttctgccc gcacggaccc ccatctctgt gacttcctgg agactcactt 600

cctagatgag gaagtgaagc ttatcaagaa gatgggtgac cacctgacca acctccacag 660

gctgggtggc ccggaggctg ggctgggcga gtatctcttc gaaaggctca ctctcaagca 720

cgactaagag ccttctgagc ccagcgactt ctgaagggcc ccttgcaaag taatagggct 780

tctgcctaag cctctccctc cagccaatag gcagctttct taactatcct aacaagcctt 840

ggaccaaatg gaaataaagc tttttgatgc aaaaaaaaaa aaaaaaaaa 889

<210> 733
<211> 22
<212> DNA
<213> Homo sapiens

<400> 733
agctgctttt gggattccgt tg 22

<210> 734
<211> 21
<212> DNA
<213> Homo sapiens

<400> 734
ctaccatagg gtaaaaccac t 21

<210> 735
<211> 22
<212> DNA
<213> Homo sapiens

<400> 735
ccacacactt ccttacattc ca 22

<210> 736
<211> 92
<212> RNA
<213> Homo sapiens

<400> 736
cggcuggaca gcgggcaacg gaaucccaaa agcagcuguu gucuccagag cauuccagcu 60

gcgcuuggau uucguccccu gcucuccugc cu 92

<210> 737
<211> 100
<212> RNA
<213> Homo sapiens

<400> 737
ugugucucuc ucuguguccu gccagugguu uuacccuaug guagguuacg ucaugcuguu 60

cuaccacagg guagaaccac ggacaggaua ccggggcacc 100

<210> 738
<211> 86
<212> RNA
<213> Homo sapiens

<400> 738
ugcuucccga ggccacaugc uucuuuauau ccccauaugg auuacuuugc uauggaaugu 60

aaggaagugu gugguuucgg caagug 86

<210> 739
<211> 25
<212> DNA
<213> Homo sapiens

<400>  739
gaatgaactc gagttgactg gaatg                                              25


<210>  740
<211>  25
<212>  DNA
<213>  Homo sapiens

<400>  740
aatgaactcg agttgactgg aatgg                                              25


<210>  741
<211>  25
<212>  DNA
<213>  Homo sapiens

<400>  741
atgaactcga gttgactgga atgga                                              25


<210>  742
<211>  25
<212>  DNA
<213>  Homo sapiens

<400>  742
tgaactcgag ttgactggaa tggaa                                              25


<210>  743
<211>  25
<212>  DNA
<213>  Homo sapiens

<400>  743
gaactcgagt tgactggaat ggaat                                              25


<210>  744
<211>  25
<212>  DNA
<213>  Homo sapiens

<400>  744
aactcgagtt gactggaatg gaatg                                              25


<210>  745
<211>  25
<212>  DNA
<213>  Homo sapiens

<400>  745
gaatggaatc aactcgagtg gaatg                                              25


<210>  746
<211>  25
<212>  DNA
<213>  Homo sapiens

<400>  746
aatggaatca actcgagtgg aatgg                                              25

<210>  747
<211>  25
<212>  DNA
<213>  Homo sapiens

<400>  747
atggaatcaa ctcgagtgga atgga                                          25


<210>  748
<211>  25
<212>  DNA
<213>  Homo sapiens

<400>  748
tggaatcaac tcgagtggaa tggaa                                          25


<210>  749
<211>  25
<212>  DNA
<213>  Homo sapiens

<400>  749
ggaatcaact cgagtggaat ggaat                                          25


<210>  750
<211>  25
<212>  DNA
<213>  Homo sapiens

<400>  750
gaatcaactc gagtggaatg gaatg                                          25


<210>  751
<211>  25
<212>  DNA
<213>  Homo sapiens

<400>  751
aatcaactcg agtggaatgg aatgg                                          25


<210>  752
<211>  25
<212>  DNA
<213>  Homo sapiens

<400>  752
atcaactcga gtggaatgga atgga                                          25


<210>  753
<211>  25
<212>  DNA
<213>  Homo sapiens

<400>  753
caactcgagt ggaatggaat ggaat                                          25


<210>  754
<211>  25
<212>  DNA
<213>  Homo sapiens

<400>  754
aatgcagtac aatgcaatag aatgg                                          25

<210>  755
<211>  25
<212>  DNA
<213>  Homo sapiens

<400>  755
taagcaagag ccatggcatg gtgaa                                          25

<210>  756
<211>  25
<212>  DNA
<213>  Homo sapiens

<400>  756
gcaagagcca tggcatggtg aaaat                                          25

<210>  757
<211>  25
<212>  DNA
<213>  Homo sapiens

<400>  757
agagtctggc caatctacaa ataga                                          25

<210>  758
<211>  25
<212>  DNA
<213>  Homo sapiens

<400>  758
tggccaatct acaaatagag aacaa                                          25

<210>  759
<211>  25
<212>  DNA
<213>  Homo sapiens

<400>  759
aaacggcaga caccaacatg gatct                                          25

<210>  760
<211>  25
<212>  DNA
<213>  Homo sapiens

<400>  760
cggcagacac caacatggat ctcat                                          25

<210>  761
<211>  25
<212>  DNA
<213>  Homo sapiens

<400>  761
acatggatct catgggggat tggat                                          25

<210>  762

<211>  25
<212>  DNA
<213>  Homo sapiens

<400>  762
atctcatggg ggattggata ttgta                                              25


<210>  763
<211>  25
<212>  DNA
<213>  Homo sapiens

<400>  763
agatgacagt gatcgtcatt tggca                                              25


<210>  764
<211>  25
<212>  DNA
<213>  Homo sapiens

<400>  764
tgacagtgat cgtcatttgg cacaa                                              25


<210>  765
<211>  25
<212>  DNA
<213>  Homo sapiens

<400>  765
tgatcgtcat ttggcacaac atctt                                              25


<210>  766
<211>  25
<212>  DNA
<213>  Homo sapiens

<400>  766
ggcacaacat cttaacaacg accga                                              25


<210>  767
<211>  25
<212>  DNA
<213>  Homo sapiens

<400>  767
cccattattt acataaacct accat                                              25


<210>  768
<211>  25
<212>  DNA
<213>  Homo sapiens

<400>  768
aacctaccat tcggtaacca tgtga                                              25


<210>  769
<211>  25
<212>  DNA
<213>  Homo sapiens

<400>  769

tcagttgacc tcagtgaatt ctgtg                                          25

<210>  770
<211>  25
<212>  DNA
<213>  Homo sapiens

<400>  770
gagatgcaga ctcccgtgta gtttc                                          25

<210>  771
<211>  25
<212>  DNA
<213>  Homo sapiens

<400>  771
actcccgtgt agtttcagat tcttg                                          25

<210>  772
<211>  25
<212>  DNA
<213>  Homo sapiens

<400>  772
aattaggctt tcctaacctg aagcg                                          25

<210>  773
<211>  25
<212>  DNA
<213>  Homo sapiens

<400>  773
taggctttcc taacctgaag cgcct                                          25

<210>  774
<211>  25
<212>  DNA
<213>  Homo sapiens

<400>  774
gctttcctaa cctgaagcgc cttca                                          25

<210>  775
<211>  25
<212>  DNA
<213>  Homo sapiens

<400>  775
ccagtgtgag acccgaacca tgctg                                          25

<210>  776
<211>  25
<212>  DNA
<213>  Homo sapiens

<400>  776
tgagacccga accatgctgc tgcag                                          25

<210>  777
<211>  25

<212>  DNA
<213>  Homo sapiens

<400>  777
cctcggctcc tacagctacc ggagt                                          25


<210>  778
<211>  25
<212>  DNA
<213>  Homo sapiens

<400>  778
cagctaccgg agtccccact ggggc                                          25


<210>  779
<211>  25
<212>  DNA
<213>  Homo sapiens

<400>  779
ctggggcagc acctactccg tgtca                                          25


<210>  780
<211>  25
<212>  DNA
<213>  Homo sapiens

<400>  780
ctactccgtg tcagtggtgg agacc                                          25


<210>  781
<211>  25
<212>  DNA
<213>  Homo sapiens

<400>  781
cgtgtcagtg gtggagaccg actac                                          25


<210>  782
<211>  25
<212>  DNA
<213>  Homo sapiens

<400>  782
cgaccagtac gcgctgctgt acagc                                          25


<210>  783
<211>  25
<212>  DNA
<213>  Homo sapiens

<400>  783
gtacagccag ggcagcaagg gccct                                          25


<210>  784
<211>  25
<212>  DNA
<213>  Homo sapiens

<400>  784
tggcgaggac ttccgcatgg ccacc                                          25

<210>  785
<211>  25
<212>  DNA
<213>  Homo sapiens

<400>  785
caaggcccag ggcttcacag aggat                                          25


<210>  786
<211>  25
<212>  DNA
<213>  Homo sapiens

<400>  786
ccagggcttc acagaggata ccatt                                          25


<210>  787
<211>  25
<212>  DNA
<213>  Homo sapiens

<400>  787
ccaaaccgat aagtgcatga cggaa                                          25


<210>  788
<211>  25
<212>  DNA
<213>  Homo sapiens

<400>  788
gtgcatgacg gaacaatagg actcc                                          25


<210>  789
<211>  25
<212>  DNA
<213>  Homo sapiens

<400>  789
acaataggac tccccagggc tgaag                                          25


<210>  790
<211>  25
<212>  DNA
<213>  Homo sapiens

<400>  790
ggactcccca gggctgaagc tggga                                          25


<210>  791
<211>  25
<212>  DNA
<213>  Homo sapiens

<400>  791
gctgggatcc cggccagcca ggtga                                          25


<210>  792
<211>  25
<212>  DNA

<213>  Homo sapiens

<400>  792
tggatgtctc tgctctgttc cttcc                                            25


<210>  793
<211>  25
<212>  DNA
<213>  Homo sapiens

<400>  793
actcgggctt catcctgcac aataa                                            25


<210>  794
<211>  25
<212>  DNA
<213>  Homo sapiens

<400>  794
acaataaact ccggaagcaa gtcag                                            25


<210>  795
<211>  25
<212>  DNA
<213>  Homo sapiens

<400>  795
ttgcgctgct gtgcctcgat ggcaa                                            25


<210>  796
<211>  25
<212>  DNA
<213>  Homo sapiens

<400>  796
gcctcgatgg caaacggaag cctgt                                            25


<210>  797
<211>  25
<212>  DNA
<213>  Homo sapiens

<400>  797
aacggaagcc tgtgactgag gctag                                            25


<210>  798
<211>  25
<212>  DNA
<213>  Homo sapiens

<400>  798
agcctgtgac tgaggctaga agctg                                            25


<210>  799
<211>  25
<212>  DNA
<213>  Homo sapiens

<400>  799
aggctagaag ctgccatctt gccat                                            25

<210>  800
<211>  25
<212>  DNA
<213>  Homo sapiens

<400>  800
gaagctgcca tcttgccatg gcccc                                              25


<210>  801
<211>  25
<212>  DNA
<213>  Homo sapiens

<400>  801
cgaatcatgc cgtggtgtct cggat                                              25


<210>  802
<211>  25
<212>  DNA
<213>  Homo sapiens

<400>  802
atgccgtggt gtctcggatg gataa                                              25


<210>  803
<211>  25
<212>  DNA
<213>  Homo sapiens

<400>  803
aacgcctgaa acaggtgctg ctcca                                              25


<210>  804
<211>  25
<212>  DNA
<213>  Homo sapiens

<400>  804
aggtgctgct ccaccaacag gctaa                                              25


<210>  805
<211>  25
<212>  DNA
<213>  Homo sapiens

<400>  805
acaagttttg cttattccag tctga                                              25


<210>  806
<211>  25
<212>  DNA
<213>  Homo sapiens

<400>  806
ttctgttcaa tgacaacact gagtg                                              25


<210>  807
<211>  25
<212>  DNA
<213>  Homo sapiens

<400>  807
acaacactga gtgtctggcc agact                                              25


<210>  808
<211>  25
<212>  DNA
<213>  Homo sapiens

<400>  808
gtctggccag actccatggc aaaac                                              25


<210>  809
<211>  25
<212>  DNA
<213>  Homo sapiens

<400>  809
ccagactcca tggcaaaaca acata                                              25


<210>  810
<211>  25
<212>  DNA
<213>  Homo sapiens

<400>  810
atgtcgcagg cattactaat ctgaa                                              25


<210>  811
<211>  25
<212>  DNA
<213>  Homo sapiens

<400>  811
gctccccaag aaagcctcag ccatt                                              25


<210>  812
<211>  25
<212>  DNA
<213>  Homo sapiens

<400>  812
caagaaagcc tcagccattc actgc                                              25


<210>  813
<211>  25
<212>  DNA
<213>  Homo sapiens

<400>  813
gggattgccc atccatctgc ttaca                                              25


<210>  814
<211>  25
<212>  DNA
<213>  Homo sapiens

<400>  814
ctgctgtcgt cttagcaaga agtaa                                              25

<210>  815
<211>  25
<212>  DNA
<213>  Homo sapiens

<400>  815
aggaaatggc tcgtcacctt cgtga                                              25


<210>  816
<211>  25
<212>  DNA
<213>  Homo sapiens

<400>  816
ttcctccctg aacctgaggg aaact                                              25


<210>  817
<211>  25
<212>  DNA
<213>  Homo sapiens

<400>  817
aaactaatct ggattcactc cctct                                              25


<210>  818
<211>  25
<212>  DNA
<213>  Homo sapiens

<400>  818
aatctggatt cactccctct ggttg                                              25


<210>  819
<211>  25
<212>  DNA
<213>  Homo sapiens

<400>  819
ggattcactc cctctggttg atacc                                              25


<210>  820
<211>  25
<212>  DNA
<213>  Homo sapiens

<400>  820
ctccctctgg ttgataccca ctcaa                                              25


<210>  821
<211>  25
<212>  DNA
<213>  Homo sapiens

<400>  821
ctggttgata cccactcaaa aagga                                              25


<210>  822
<211>  25
<212>  DNA
<213>  Homo sapiens

<400>  822
tatcaacgaa acttctcagc atcac                                          25


<210>  823
<211>  25
<212>  DNA
<213>  Homo sapiens

<400>  823
acgaaacttc tcagcatcac gatga                                          25


<210>  824
<211>  25
<212>  DNA
<213>  Homo sapiens

<400>  824
cagcatcacg atgaccttga ataaa                                          25


<210>  825
<211>  25
<212>  DNA
<213>  Homo sapiens

<400>  825
taaagaaaca gctttcaagt gcctt                                          25


<210>  826
<211>  25
<212>  DNA
<213>  Homo sapiens

<400>  826
gtgcctttct gcagtttttc aggag                                          25


<210>  827
<211>  25
<212>  DNA
<213>  Homo sapiens

<400>  827
tttctgcagt ttttcaggag cgcaa                                          25


<210>  828
<211>  25
<212>  DNA
<213>  Homo sapiens

<400>  828
taagctctag ttcttaacaa ccgac                                          25


<210>  829
<211>  25
<212>  DNA
<213>  Homo sapiens

<400>  829
tctagttctt aacaaccgac actcc                                          25


<210>  830

<211>  25
<212>  DNA
<213>  Homo sapiens

<400>  830
tcttaacaac cgacactcct acaag                                              25


<210>  831
<211>  25
<212>  DNA
<213>  Homo sapiens

<400>  831
cgacactcct acaagattta gaaaa                                              25


<210>  832
<211>  25
<212>  DNA
<213>  Homo sapiens

<400>  832
tacaacataa tctagtttac agaaa                                              25


<210>  833
<211>  25
<212>  DNA
<213>  Homo sapiens

<400>  833
gctatccagc attcaggttt actca                                              25


<210>  834
<211>  25
<212>  DNA
<213>  Homo sapiens

<400>  834
atcctgaagc tgacagcatt cgggc                                              25


<210>  835
<211>  25
<212>  DNA
<213>  Homo sapiens

<400>  835
cctgaagctg acagcattcg ggccg                                              25


<210>  836
<211>  25
<212>  DNA
<213>  Homo sapiens

<400>  836
aagctgacag cattcgggcc gagat                                              25


<210>  837
<211>  25
<212>  DNA
<213>  Homo sapiens

<400>  837

gctgacagca ttcgggccga gatgt                                          25


<210>  838
<211>  25
<212>  DNA
<213>  Homo sapiens

<400>  838
tgacagcatt cgggccgaga tgtct                                          25


<210>  839
<211>  25
<212>  DNA
<213>  Homo sapiens

<400>  839
cattcgggcc gagatgtctc gctcc                                          25


<210>  840
<211>  25
<212>  DNA
<213>  Homo sapiens

<400>  840
ggccgagatg tctcgctccg tggcc                                          25


<210>  841
<211>  25
<212>  DNA
<213>  Homo sapiens

<400>  841
ggaggtttga agatgccgca ggatc                                          25


<210>  842
<211>  25
<212>  DNA
<213>  Homo sapiens

<400>  842
gagatgtctc gctccgtggc cttag                                          25


<210>  843
<211>  25
<212>  DNA
<213>  Homo sapiens

<400>  843
gatgtctcgc tccgtggcct tagct                                          25


<210>  844
<211>  25
<212>  DNA
<213>  Homo sapiens

<400>  844
tgtctcgctc cgtggcctta gctgt                                          25


<210>  845
<211>  25

<212>  DNA
<213>  Homo sapiens

<400>  845
cgtggcctta gctgtgctcg cgcta                                              25


<210>  846
<211>  25
<212>  DNA
<213>  Homo sapiens

<400>  846
cttagctgtg ctcgcgctac tctct                                              25


<210>  847
<211>  25
<212>  DNA
<213>  Homo sapiens

<400>  847
tagctgtgct cgcgctactc tctct                                              25


<210>  848
<211>  25
<212>  DNA
<213>  Homo sapiens

<400>  848
gctgtgctcg cgctactctc tcttt                                              25


<210>  849
<211>  25
<212>  DNA
<213>  Homo sapiens

<400>  849
tgtgctcgcg ctactctctc tttct                                              25


<210>  850
<211>  25
<212>  DNA
<213>  Homo sapiens

<400>  850
gcctggaggc tatccagcat tcagg                                              25


<210>  851
<211>  25
<212>  DNA
<213>  Homo sapiens

<400>  851
ctggaggcta tccagcattc aggtt                                              25


<210>  852
<211>  25
<212>  DNA
<213>  Homo sapiens

<400>  852
ggaggctatc cagcattcag gttta                                              25

<210>  853
<211>  25
<212>  DNA
<213>  Homo sapiens

<400>  853
gcctggtact caagcccgcg gggac                                          25


<210>  854
<211>  25
<212>  DNA
<213>  Homo sapiens

<400>  854
ctggtactca agcccgcggg gacat                                          25


<210>  855
<211>  25
<212>  DNA
<213>  Homo sapiens

<400>  855
tggtactcaa gcccgcgggg acatt                                          25


<210>  856
<211>  25
<212>  DNA
<213>  Homo sapiens

<400>  856
ggtactcaag cccgcgggga cattg                                          25


<210>  857
<211>  25
<212>  DNA
<213>  Homo sapiens

<400>  857
gtactcaagc ccgcggggac attgg                                          25


<210>  858
<211>  25
<212>  DNA
<213>  Homo sapiens

<400>  858
actcaagccc gcggggacat tggga                                          25


<210>  859
<211>  25
<212>  DNA
<213>  Homo sapiens

<400>  859
ctcaagcccg cggggacatt gggaa                                          25


<210>  860
<211>  25
<212>  DNA

<213>  Homo sapiens

<400>  860
tcaagcccgc ggggacattg ggaag                                          25


<210>  861
<211>  25
<212>  DNA
<213>  Homo sapiens

<400>  861
cctctctgca ccgtactgtg gaaaa                                          25


<210>  862
<211>  25
<212>  DNA
<213>  Homo sapiens

<400>  862
ctctctgcac cgtactgtgg aaaag                                          25


<210>  863
<211>  25
<212>  DNA
<213>  Homo sapiens

<400>  863
tctctgcacc gtactgtgga aaaga                                          25


<210>  864
<211>  25
<212>  DNA
<213>  Homo sapiens

<400>  864
ctctgcaccg tactgtggaa aagaa                                          25


<210>  865
<211>  25
<212>  DNA
<213>  Homo sapiens

<400>  865
gaaacacgca cttagtctct aaaga                                          25


<210>  866
<211>  25
<212>  DNA
<213>  Homo sapiens

<400>  866
aacacgcact tagtctctaa agagt                                          25


<210>  867
<211>  25
<212>  DNA
<213>  Homo sapiens

<400>  867
acacgcactt agtctctaaa gagtt                                          25

<210>  868
<211>  25
<212>  DNA
<213>  Homo sapiens

<400>  868
acgcacttag tctctaaaga gttta                                          25


<210>  869
<211>  25
<212>  DNA
<213>  Homo sapiens

<400>  869
cgcacttagt ctctaaagag tttat                                          25


<210>  870
<211>  25
<212>  DNA
<213>  Homo sapiens

<400>  870
gcacttagtc tctaaagagt ttatt                                          25


<210>  871
<211>  25
<212>  DNA
<213>  Homo sapiens

<400>  871
cacttagtct ctaaagagtt tattt                                          25


<210>  872
<211>  25
<212>  DNA
<213>  Homo sapiens

<400>  872
taagacgtgt ttgtgtttgt gtgtg                                          25


<210>  873
<211>  25
<212>  DNA
<213>  Homo sapiens

<400>  873
tgagaagatc ccaacaacct ttgag                                          25


<210>  874
<211>  25
<212>  DNA
<213>  Homo sapiens

<400>  874
gatcccaaca acctttgaga atgga                                          25


<210>  875
<211>  25
<212>  DNA
<213>  Homo sapiens

<400>  875
ctttgagaat ggacgctgca tccag                                          25


<210>  876
<211>  25
<212>  DNA
<213>  Homo sapiens

<400>  876
acgctgcatc caggccaact actca                                          25


<210>  877
<211>  25
<212>  DNA
<213>  Homo sapiens

<400>  877
catccaggcc aactactcac taatg                                          25


<210>  878
<211>  25
<212>  DNA
<213>  Homo sapiens

<400>  878
ttcctggttt atgccatcgg caccg                                          25


<210>  879
<211>  25
<212>  DNA
<213>  Homo sapiens

<400>  879
gtttatgcca tcggcaccgt actgg                                          25


<210>  880
<211>  25
<212>  DNA
<213>  Homo sapiens

<400>  880
gatcctggcc accgactatg agaac                                          25


<210>  881
<211>  25
<212>  DNA
<213>  Homo sapiens

<400>  881
ggccaccgac tatgagaact atgcc                                          25


<210>  882
<211>  25
<212>  DNA
<213>  Homo sapiens

<400>  882
tgagaactat gccctcgtgt attcc                                          25

<210>  883
<211>  25
<212>  DNA
<213>  Homo sapiens

<400>  883
cctcgtgtat tcctgtacct gcatc                                              25


<210>  884
<211>  25
<212>  DNA
<213>  Homo sapiens

<400>  884
gtattcctgt acctgcatca tccaa                                              25


<210>  885
<211>  25
<212>  DNA
<213>  Homo sapiens

<400>  885
ctgtacctgc atcatccaac ttttt                                              25


<210>  886
<211>  25
<212>  DNA
<213>  Homo sapiens

<400>  886
ctgcatcatc caactttttc acgtg                                              25


<210>  887
<211>  25
<212>  DNA
<213>  Homo sapiens

<400>  887
tgcttggatc ttggcaagaa accct                                              25


<210>  888
<211>  25
<212>  DNA
<213>  Homo sapiens

<400>  888
cacagaccag gtgaactgcc ccaag                                              25


<210>  889
<211>  25
<212>  DNA
<213>  Homo sapiens

<400>  889
ccaggtgaac tgccccaagc tctcg                                              25


<210>  890
<211>  25
<212>  DNA
<213>  Homo sapiens

<400>  890
aggttctaca gggaggctgc accca                                          25


<210>  891
<211>  25
<212>  DNA
<213>  Homo sapiens

<400>  891
actccatgtt acttctgctt cgctt                                          25


<210>  892
<211>  25
<212>  DNA
<213>  Homo sapiens

<400>  892
cctgttacct tgctagctgc aaaat                                          25


<210>  893
<211>  1649
<212>  DNA
<213>  Homo sapiens

<400>  893
agacaaggtt ttccaagcaa gatgaagccc aacatcatct ttgtactttc cctgctcctc      60

atcttggaga agcaagcagc tgtgatggga caaaaaggtg gatcaaaagg ccgattacca     120

agtgaatttt cccaatttcc acacggacaa aagggccagc actattctgg acaaaaaggc     180

aagcaacaaa ctgaatccaa aggcagtttt tctattcaat acacatatca tgtagatgcc     240

aatgatcatg accagtcccg aaaaagtcag caatatgatt tgaatgccct acataagacg     300

acaaaatcac aacgacatct aggtggaagt caacaactgc tccataataa acaagaaggc     360

agagaccatg ataaatcaaa aggtcatttt cacagggtag ttatacacca taaaggaggc     420

aaagctcatc gtgggacaca aaatccttct caagatcagg ggaatagccc atctggaaag     480

ggaatatcca gtcaatattc aaacacagaa gaaaggctgt gggttcatgg actaagtaaa     540

gaacaaactt ccgtctctgg tgcacaaaaa ggtagaaaac aaggcggatc ccaaagcagt     600

tatgttctcc aaactgaaga gctagtagct aacaaacaac aacgtgagac taaaaattct     660

catcaaaata aagggcatta ccaaaatgtg gttgaagtga gagaggaaca ttcaagtaaa     720

gtacaaacct cactctgtcc tgcgcaccaa gacaaactcc aacatggatc caaagacatt     780

ttttctaccc aagatgagct cctagtatat aacaagaatc aacaccagac aaaaaatctc     840

aatcaagatc aacagcatgg ccgaaaggca aataaaatat cataccaatc ttcaagtaca     900

gaagaaagac gactccacta tggagaaaat ggtgtgcaga aagatgtatc ccaaagcagt     960

atttatagcc aaactgaaga gaaagcacag ggcaagtctc aaaaacagat aacaattccc    1020

agtcaagagc aagagcatag ccaaaaggca aataaaatat cataccaatc ttcaagtacg    1080

gaagaaagac gactccacta tggagaaaat ggtgtgcaga aagatgtatc ccaacgcagt    1140

atttatagcc aaactgaaaa gctagtagca ggcaagtctc aaatccaggc accaaatcct    1200

aagcaagagc catggcatgg tgaaaatgca aaaggagagt ctggccaatc tacaaataga 1260

gaacaagacc tactcagtca tgaacaaaaa ggcagacacc aacatggatc tcatggggga 1320

ttggatattg taattataga gcaggaagat gacagtgatc gtcatttggc acaacatctt 1380

aacaacgacc gaaacccatt atttacataa acctaccatt cggtaaccat gtgaaaggat 1440

ggaccaatat caaggtgtca gttgacctca gtgaattctg tgatgtttct gagatgcaga 1500

ctcccgtgta gtttcagatt cttggtccat ggatgacacc acctgcccat gcttccttga 1560

attaggcttt cctaacctga agcgccttca aacttccaat aaagagatca ttttctgctt 1620

caaaaaaaaa aaaaaaaaaa aaaaaaaaa 1649

<210> 894
<211> 837
<212> DNA
<213> Homo sapiens

<400> 894
gctcctcctg cacacctccc tcgctctccc acaccactgg caccaggccc cggacacccg 60

ctctgctgca ggagaatggc tactcatcac acgctgtgga tgggactggc cctgctgggg 120

gtgctgggcg acctgcaggc agcaccggag gcccaggtct ccgtgcagcc caacttccag 180

caggacaagt tcctggggcg ctggttcagc gcgggcctcg cctccaactc gagctggctc 240

cgggagaaga aggcggcgtt gtccatgtgc aagtctgtgg tggcccctgc cacggatggt 300

ggcctcaacc tgacctccac cttcctcagg aaaaaccagt gtgagacccg aaccatgctg 360

ctgcagcccg cggggtccct cggctcctac agctaccgga gtccccactg gggcagcacc 420

tactccgtgt cagtggtgga gaccgactac gaccagtacg cgctgctgta cagccagggc 480

agcaagggcc ctggcgagga cttccgcatg gccaccctct acagccgaac ccagaccccc 540

agggctgagt taaaggagaa attcaccgcc ttctgcaagg cccagggctt cacagaggat 600

accattgtct tcctgcccca aaccgataag tgcatgacgg aacaatagga ctccccaggg 660

ctgaagctgg gatcccggcc agccaggtga cccccacgct ctggatgtct ctgctctgtt 720

ccttccccga gcccctgccc cggctccccg ccaaagcaac cctgcccact caggcttcat 780

cctgcacaat aaactccgga agcaagtcag taaaaaaaaa aaaaaaaaaa aaaaaaa 837

<210> 895
<211> 2390
<212> DNA
<213> Homo sapiens

<400> 895
agagccttcg tttgccaagt cgcctccaga ccgcagacat gaaacttgtc ttcctcgtcc 60

tgctgttcct cggggccctc ggactgtgtc tggctggccg taggaggagt gttcagtggt 120

gcgccgtatc ccaacccgag gccacaaaat gcttccaatg gcaaaggaat atgagaaaag 180

tgcgtggccc tcctgtcagc tgcataaaga gagactcccc catccagtgt atccaggcca 240

ttgcggaaaa cagggccgat gctgtgaccc ttgatggtgg tttcatatac gaggcaggcc 300

tggcccccta caaactgcga cctgtagcgg cggaagtcta cgggaccgaa agacagccac 360
gaactcacta ttatgccgtg gctgtggtga agaagggcgg cagctttcag ctgaacgaac 420
tgcaaggtct gaagtcctgc cacacaggcc ttcgcaggac cgctggatgg aatgtcccta 480
tagggacact tcgtccattc ttgaattgga cgggtccacc tgagcccatt gaggcagctg 540
tggccaggtt cttctcagcc agctgtgttc ccggtgcaga taaaggacag ttccccaacc 600
tgtgtcgcct gtgtgcgggg acaggggaaa acaaatgtgc cttctcctcc caggaaccgt 660
acttcagcta ctctggtgcc ttcaagtgtc tgagagacgg ggctggagac gtggctttta 720
tcagagagag cacagtgttt gaggacctgt cagacgaggc tgaaagggac gagtatgagt 780
tactctgccc agacaacact cggaagccag tggacaagtt caaagactgc catctggccc 840
gggtcccttc tcatgccgtt gtggcacgaa gtgtgaatgg caaggaggat gccatctgga 900
atcttctccg ccaggcacag gaaaagtttg gaaaggacaa gtcaccgaaa ttccagctct 960
ttggctcccc tagtgggcag aaagatctgc tgttcaagga ctctgccatt gggttttcga 1020
gggtgccccc gaggatagat tctgggctgt accttggctc cggctacttc actgccatcc 1080
agaacttgag gaaaagtgag gaggaagtgg ctgcccggcg tgcgcgggtc gtgtggtgtg 1140
cggtgggcga gcaggagctg cgcaagtgta accagtggag tggcttgagc gaaggcagcg 1200
tgacctgctc ctcggcctcc accacagagg actgcatcgc cctggtgctg aaaggagaag 1260
ctgatgccat gagtttggat ggaggatatg tgtacactgc aggcaaatgt ggtttggtgc 1320
ctgtcctggc agagaactac aaatcccaac aaagcagtga ccctgatcct aactgtgtgg 1380
atagacctgt ggaaggatat cttgctgtgg cggtggttag gagatcagac actagcctta 1440
cctggaactc tgtgaaaggc aagaagtcct gccacaccgc cgtggacagg actgcaggct 1500
ggaatatccc catgggcctg ctcttcaacc agacgggctc ctgcaaattt gatgaatatt 1560
tcagtcaaag ctgtgcccct gggtctgacc cgagatctaa tctctgtgct ctgtgtattg 1620
gcgacgagca gggtgagaat aagtgcgtgc ccaacagcaa cgagagatac tacggctaca 1680
ctggggcttt ccggtgcctg gctgagaatg ctggagacgt tgcatttgtg aaagatgtca 1740
ctgtcttgca gaacactgat ggaaataaca atgaggcatg ggctaaggat ttgaagctgg 1800
cagactttgc gctgctgtgc ctcgatggca aacggaagcc tgtgactgag gctagaagct 1860
gccatcttgc catggccccg aatcatgccg tggtgtctcg gatggataag gtggaacgcc 1920
tgaaacaggt gttgctccac caacaggcta aatttgggag aaatggatct gactgcccgg 1980
acaagttttg cttattccag tctgaaacca aaaaccttct gttcaatgac aacactgagt 2040
gtctggccag actccatggc aaaacaacat atgaaaaata tttgggacca cagtatgtcg 2100
caggcattac taatctgaaa aagtgctcaa cctcccccct cctggaagcc tgtgaattcc 2160
tcaggaagta aaaccgaaga agatggccca gctccccaag aaagcctcag ccattcactg 2220
cccccagctc ttctccccag gtgtgttggg gccttggcct cccctgctga aggtggggat 2280
tgcccatcca tctgcttaca attccctgct gtcgtcttag caagaagtaa aatgagaaat 2340

tttgttgata ttctctcctt aaaaaaaaaa aaaaaaaaaa aaaaaaaaaa 2390

<210> 896
<211> 1847
<212> DNA
<213> Homo sapiens

<400> 896
   gtccccgcgc cagagacgca gccgcgctcc caccacccac acccaccgcg ccctcgttcg 60
   cctcttctcc gggagccagt ccgcgccacc gccgccgccc aggccatcgc caccctccgc 120
   agccatgtcc accaggtccg tgtcctcgtc ctcctaccgc aggatgttcg gcggcccggg 180
   caccgcgagc cggccgagct ccagccggag ctacgtgact acgtccaccc gcacctacag 240
   cctgggcagc gcgctgcgcc ccagcaccag ccgcagcctc tacgcctcgt ccccgggcgg 300
   cgtgtatgcc acgcgctcct ctgccgtgcg cctgcggagc agcgtgcccg gggtgcggct 360
   cctgcaggac tcggtggact tctcgctggc cgacgccatc aacaccgagt tcaagaacac 420
   ccgcaccaac gagaaggtgg agctgcagga gctgaatgac cgcttcgcca actacatcga 480
   caaggtgcgc ttcctggagc agcagaataa gatcctgctg gccgagctcg agcagctcaa 540
   gggccaaggc aagtcgcgcc tgggggacct ctacgaggag gagatgcggg agctgcgccg 600
   gcaggtggac cagctaacca acgacaaagc ccgcgtcgag gtggagcgcg acaacctggc 660
   cgaggacatc atgcgcctcc gggagaaatt gcaggaggag atgcttcaga gagaggaagc 720
   cgaaaacacc ctgcaatctt tcagacagga tgttgacaat gcgtctctgg cacgtcttga 780
   ccttgaacgc aaagtggaat ctttgcaaga agagattgcc tttttgaaga aactccacga 840
   agaggaaatc caggagctgc aggctcagat tcaggaacag catgtccaaa tcgatgtgga 900
   tgtttccaag cctgacctca cggctgccct gcgtgacgta cgtcagcaat atgaaagtgt 960
   ggctgccaag aacctgcagg aggcagaaga atggtacaaa tccaagtttg ctgacctctc 1020
   tgaggctgcc aaccggaaca atgacgccct gcgccaggca aagcaggagt ccactgagta 1080
   ccggagacag gtgcagtccc tcacctgtga agtggatgcc cttaaaggaa ccaatgagtc 1140
   cctggaacgc cagatgcgtg aaatggaaga gaactttgcc gttgaagctg ctaactacca 1200
   agacactatt ggccgcctgc aggatgagat tcagaatatg aaggaggaaa tggctcgtca 1260
   ccttcgtgaa taccaagacc tgctcaatgt taagatggcc cttgacattg agattgccac 1320
   ctacaggaag ctgctggaag gcgaggagag caggatttct ctgcctcttc caaacttttc 1380
   ctccctgaac ctgagggaaa ctaatctgga ttcactccct ctggttgata cccactcaaa 1440
   aaggacactt ctgattaaga cggttgaaac tagagatgga caggttatca acgaaacttc 1500
   tcagcatcac gatgaccttg aataaaaatt gcacacactc agtgcagcaa tatattacca 1560
   gcaagaataa aaaagaaatc catatcttaa agaaacagct ttcaagtgcc tttctgcagt 1620
   ttttcaggag cgcaagatag atttggaata ggaataagct ctagttctta acaaccgaca 1680
   ctcctacaag atttagaaaa aagtttacaa cataatctag tttacagaaa aatcttgtgc 1740
   tagaatactt tttaaaaggt attttgaata ccattaaaac tgcttttttt tttccagcaa 1800
   gtatccaacc aacttggttc tgcttcaata aatctttgga aaaactc 1847

<210> 897
<211> 626
<212> DNA
<213> Homo sapiens

<400> 897
acatttgctt ctgacacaac tgtgttcact agcaacctca aacagacacc atggtgcatc 60

tgactcctga ggagaagtct gccgttactg ccctgtgggg caaggtgaac gtggatgaag 120

ttggtggtga ggccctgggc aggctgctgg tggtctaccc ttggacccag aggttctttg 180

agtcctttgg ggatctgtcc actcctgatg ctgttatggg caaccctaag gtgaaggctc 240

atggcaagaa agtgctcggt gcctttagtg atggcctggc tcacctggac aacctcaagg 300

gcacctttgc cacactgagt gagctgcact gtgacaagct gcacgtggat cctgagaact 360

tcaggctcct gggcaacgtg ctggtctgtg tgctggccca tcactttggc aaagaattca 420

ccccaccagt gcaggctgcc tatcagaaag tggtggctgg tgtggctaat gccctggccc 480

acaagtatca ctaagctcgc tttcttgctg tccaatttct attaaaggtt cctttgttcc 540

ctaagtccaa ctactaaact gggggatatt atgaagggcc ttgagcatct ggattctgcc 600

taataaaaaa catttatttt cattgc 626

<210> 898
<211> 987
<212> DNA
<213> Homo sapiens

<400> 898
aatataagtg gaggcgtcgc gctggcgggc attcctgaag ctgacagcat tcgggccgag 60

atgtctcgct ccgtggcctt agctgtgctc gcgctactct ctctttctgg cctggaggct 120

atccagcgta ctccaaagat tcaggtttac tcacgtcatc cagcagagaa tggaaagtca 180

aatttcctga attgctatgt gtctgggttt catccatccg acattgaagt tgacttactg 240

aagaatggag agagaattga aaaagtggag cattcagact tgtctttcag caaggactgg 300

tctttctatc tcttgtacta cactgaattc acccccactg aaaaagatga gtatgcctgc 360

cgtgtgaacc atgtgacttt gtcacagccc aagatagtta agtgggatcg agacatgtaa 420

gcagcatcat ggaggtttga agatgccgca tttggattgg atgaattcca aattctgctt 480

gcttgctttt taatattgat atgcttatac acttacactt tatgcacaaa atgtagggtt 540

ataataatgt taacatggac atgatcttct ttataattct actttgagtg ctgtctccat 600

gtttgatgta tctgagcagg ttgctccaca ggtagctcta ggagggctgg caacttagag 660

gtggggagca gagaattctc ttatccaaca tcaacatctt ggtcagattt gaactcttca 720

atctcttgca ctcaaagctt gttaagatag ttaagcgtgc ataagttaac ttccaattta 780

catactctgc ttagaatttg ggggaaaatt tagaaatata attgacagga ttattggaaa 840

tttgttataa tgaatgaaac attttgtcat ataagattca tatttacttc ttatacattt 900

gataaagtaa ggcatggttg tggttaatct ggtttatttt tgttccacaa gttaaataaa 960

tcataaaact tgatgtgtta tctctta 987

<210> 899
<211> 1832
<212> DNA
<213> Homo sapiens

<400> 899
gagcggccag gccagcctcg gagccagcag ggagctggga gctgggggaa acgacgccag 60

gaaagctatc gcgccagaga gggcgacggg ggctcgggaa gcctgacagg gcttttgcgc 120

acagctgccg gctggctgct acccgcccgc gccagccccc gagaacgcgc gaccaggcac 180

ccagtccggt caccgcagcg gagagctcgc cgctcgctgc agcgaggccc ggagcggccc 240

cgcagggacc ctccccagac cgcctgggcc gcccggatgt gcactaaaat ggaacagccc 300

ttctaccacg acgactcata cacagctacg ggatacggcc gggcccctgg tggcctctct 360

ctacacgact acaaactcct gaaaccgagc ctggcggtca acctggccga cccctaccgg 420

agtctcaaag cgcctggggc tcgcggaccc ggcccagagg gcggcggtgg cggcagctac 480

ttttctggtc agggctcgga caccggcgcg tctctcaagc tcgcctcttc ggagctggaa 540

cgcctgattg tccccaacag caacggcgtg atcacgacga cgcctacacc cccgggacag 600

tacttttacc cccgcggggg tggcagcggt ggaggtgcag ggggcgcagg gggcggcgtc 660

accgaggagc aggagggctt cgccgacggc tttgtcaaag ccctggacga tctgcacaag 720

atgaaccacg tgacaccccc caacgtgtcc ctgggcgcta ccggggggcc cccggctggg 780

cccgggggcg tctacgccgg cccggagcca cctcccgttt acaccaacct cagcagctac 840

tccccagcct ctgcgtcctc gggaggcgcc ggggctgccg tcgggaccgg gagctcgtac 900

ccgacgacca ccatcagcta cctcccacac gcgccgccct tcgccggtgg ccacccggcg 960

cagctgggct tgggccgcgg cgcctccacc ttcaaggagg aaccgcagac cgtgccggag 1020

gcgcgcagcc gggacgccac gccgccggtg tcccccatca acatggaaga ccaagagcgc 1080

atcaaagtgg agcgcaagcg gctgcggaac cggctggcgg ccaccaagtg ccggaagcgg 1140

aagctggagc gcatcgcgcg cctggaggac aaggtgaaga cgctcaaggc cgagaacgcg 1200

gggctgtcga gtaccgccgg cctcctccgg gagcaggtgg cccagctcaa acagaaggtc 1260

atgacccacg tcagcaacgg ctgtcagctg ctgcttgggg tcaagggaca cgccttctga 1320

acgtcccctg cccctttacg gacaccccct cgcttggacg gctgggcaca cgcctcccac 1380

tggggtccag ggagcaggcg gtgggcaccc accctgggac ctaggggcgc cgcaaaccac 1440

actggactcc ggccctccta ccctgcgccc agtccttcca cctcgacgtt tacaagcccc 1500

cccttccact tttttttgta tgtttttttt ctgctggaaa cagactcgat tcatattgaa 1560

tataatatat ttgtgtattt aacagggagg ggaagagggg gcgatcgcgg cggagctggc 1620

cccgccgcct ggtactcaag cccgcgggga cattgggaag gggacccccg ccccctgccc 1680

tcccctctct gcaccgtact gtggaaaaga aacacgcact tagtctctaa agagtttatt 1740

ttaagacgtg tttgtgtttg tgtgtgtttg ttctttttat tgaatctatt taagtaaaaa 1800

aaaaattggt tctttaaaaa aaaaaaaaaa aa 1832

<210> 900
<211> 1061
<212> DNA
<213> Homo sapiens

<400> 900
tgtgaaggaa atcgggggag gaggatggac acaacatccc atctttgtgt ttcgatacag 60

actaagcttt taggccaacc ctcctgactg gatgggggcg gcgggcgtgg catgcatgaa 120

aagtaaacat cagagacctg aagaagctta taaaatagct tgggagaggc cagtcaccaa 180

gacaggcatc tcaaatcggc tgattctgca tctggaaact gccttcatct tgaaagaaaa 240

gctccaggtc ccttctccag ccacccagcc ccaagatggt gatgctgctg ctgctgcttt 300

ccgcactggc tggcctcttc ggtgcggcag agggacaagc atttcatctt gggaagtgcc 360

ccaatcctcc ggtgcaggag aattttgacg tgaataagta tctcggaaga tggtacgaaa 420

ttgagaagat cccaacaacc tttgagaatg gacgctgcat ccaggccaac tactcactaa 480

tggaaaacgg aaagatcaaa gtgttaaacc aggagttgag agctgatgga actgtgaatc 540

aaatcgaagg tgaagccacc ccagttaacc tcacagagcc tgccaagctg gaagttaagt 600

tttcctggtt tatgccatcg gcaccgtact ggatcctggc caccgactat gagaactatg 660

ccctcgtgta ttcctgtacc tgcatcatcc aactttttca cgtggatttt gcttggatct 720

tggcaagaaa ccctaatctc cctccagaaa cagtggactc tctaaaaaat atcctgactt 780

ctaataacat tgatgtcaag aaaatgacgg tcacagacca ggtgaactgc cccaagctct 840

cgtaaccagg ttctacaggg aggctgcacc cactccatgt tacttctgct tcgctttccc 900

ctaccccacc ccccccccat aaagacaaac caatcaacca cgacaaagga agttgacctg 960

aacatgtaac catgccctac cctgttacct tgctagctgc aaaataaact tgttgctgac 1020

ctgctgtgct cgcagtagat tccaaaaaaa aaaaaaaaaa a 1061

<210> 901
<211> 20
<212> DNA
<213> Homo sapiens

<400> 901
agtgacggta agccagtcag 20

<210> 902
<211> 20
<212> DNA
<213> Homo sapiens

<400> 902
tccactttaa tttcgggtca 20

<210>  903
<211>  20
<212>  DNA
<213>  Homo sapiens

<400>  903
gaaagggaaa gggtcaaaaa                                                    20


<210>  904
<211>  20
<212>  DNA
<213>  Homo sapiens

<400>  904
cacatctgca agtacgttcg                                                    20


<210>  905
<211>  20
<212>  DNA
<213>  Homo sapiens

<400>  905
aagagctatg agctgcctga                                                    20


<210>  906
<211>  20
<212>  DNA
<213>  Homo sapiens

<400>  906
tacggatgtc aacgtcacac                                                    20


<210>  907
<211>  3542
<212>  DNA
<213>  Homo sapiens

<400>  907
gcactttcac tctccgtcag ccgcattgcc cgctcggcgt ccggcccccg acccgcgctc      60

gtccgcccgc ccgcccgccc gcccgcgcca tgaacgccaa ggtcgtggtc gtgctggtcc     120

tcgtgctgac cgcgctctgc ctcagcgacg ggaagcccgt cagcctgagc tacagatgcc     180

catgccgatt cttcgaaagc catgttgcca gagccaacgt caagcatctc aaaattctca     240

acactccaaa ctgtgccctt cagattgtag cccggctgaa gaacaacaac agacaagtgt     300

gcattgaccc gaagctaaag tggattcagg agtacctgga gaaagcttta aacaagaggt     360

tcaagatgtg agagggtcag acgcctgagg aacccttaca gtaggagccc agctctgaaa     420

ccagtgttag ggaagggcct gccacagcct cccctgccag ggcagggccc caggcattgc     480

caagggcttt gttttgcaca ctttgccata ttttcaccat ttgattatgt agcaaaatac     540

atgacattta tttttcattt agtttgatta ttcagtgtca ctggcgacac gtagcagctt     600

agactaaggc cattattgta cttgccttat tagagtgtct ttccacggag ccactcctct     660

gactcagggc tcctgggttt tgtattctct gagctgtgca ggtggggaga ctgggctgag     720

ggagcctggc cccatggtca gccctagggt ggagagccac caagagggac gcctgggggt     780

gccaggacca gtcaacctgg gcaaagccta gtgaaggctt ctctctgtgg gatgggatgg 840
tggagggcca catgggaggc tcaccccctt ctccatccac atgggagccg ggtctgcctc 900
ttctgggagg gcagcagggc taccctgagc tgaggcagca gtgtgaggcc agggcagagt 960
gagacccagc cctcatcccg agcacctcca catcctccac gttctgctca tcattctctg 1020
tctcatccat catcatgtgt gtccacgact gtctccatgg ccccgcaaaa ggactctcag 1080
gaccaaagct ttcatgtaaa ctgtgcacca agcaggaaat gaaaatgtct tgtgttacct 1140
gaaaacactg tgcacatctg tgtcttgttt ggaatattgt ccattgtcca atcctatgtt 1200
tttgttcaaa gccagcgtcc tcctctgtga ccaatgtctt gatgcatgca ctgttccccc 1260
tgtgcagccg ctgagcgagg agatgctcct tgggcccttt gagtgcagtc ctgatcagag 1320
ccgtggtcct ttggggtgaa ctaccttggt tcccccactg atcacaaaaa catggtgggt 1380
ccatgggcag agcccaaggg aattcggtgt gcaccagggt tgaccccaga ggattgctgc 1440
cccatcagtg ctccctcaca tgtcagtacc ttcaaactag ggccaagccc agcactgctt 1500
gaggaaaaca agcattcaca acttgttttt ggtttttaaa acccagtcca caaaataacc 1560
aatcctggac atgaagattc tttcccaatt cacatctaac ctcatcttct tcaccatttg 1620
gcaatgccat catctcctgc cttcctcctg ggccctctct gctctgcgtg tcacctgtgc 1680
ttcgggccct tcccacagga catttctcta agagaacaat gtgctatgtg aagagtaagt 1740
caacctgcct gacatttgga gtgttcccct tccactgagg gcagtcgata gagctgtatt 1800
aagccactta aaatgttcac ttttgacaaa ggcaagcact tgtgggtttt tgttttgttt 1860
ttcattcagt cttacgaata cttttgccct ttgattaaag actccagtta aaaaaaattt 1920
taatgaagaa agtggaaaac aaggaagtca aagcaaggaa actatgtaac atgtaggaag 1980
taggaagtaa attatagtga tgtaatcttg aattgtaact gttcttgaat ttaataatct 2040
gtagggtaat tagtaacatg tgttaagtat tttcataagt atttcaaatt ggagcttcat 2100
ggcagaaggc aaacccatca acaaaaattg tcccttaaac aaaaattaaa atcctcaatc 2160
cagctatgtt atattgaaaa aatagagcct gagggatctt tactagttat aaagatacag 2220
aactctttca aaaccttttg aaattaacct ctcactatac cagtataatt gagttttcag 2280
tggggcagtc attatccagg taatccaaga tattttaaaa tctgtcacgt agaacttgga 2340
tgtacctgcc cccaatccat gaaccaagac cattgaattc ttggttgagg aaacaaacat 2400
gaccctaaat cttgactaca gtcaggaaag gaatcatttc tatttctcct ccatgggaga 2460
aaatagataa gagtagaaac tgcagggaaa attatttgca taacaattcc tctactaaca 2520
atcagctcct tcctggagac tgcccagcta aagcaatatg catttaaata cagtcttcca 2580
tttgcaaggg aaaagtctct tgtaatccga atctcttttt gctttcgaac tgctagtcaa 2640
gtgcgtccac gagctgttta ctagggatcc ctcatctgtc cctccgggac ctggtgctgc 2700
ctctacctga cactcccttg ggctccctgt aacctcttca gaggccctcg ctgccagctc 2760
tgtatcagga cccagaggaa ggggccagag gctcgttgac tggctgtgtg ttgggattga 2820

gtctgtgcca cgtgtttgtg ctgtggtgtg tccccctctg tccaggcact gagataccag 2880

cgaggaggct ccagagggca ctctgcttgt tattagagat tacctcctga gaaaaaaggt 2940

tccgcttgga gcagaggggc tgaatagcag aaggttgcac ctcccccaac cttagatgtt 3000

ctaagtcttt ccattggatc tcattggacc cttccatggt gtgatcgtct gactggtgtt 3060

atcaccgtgg gctccctgac tgggagttga tcgcctttcc caggtgctac acccttttcc 3120

agctggatga gaatttgagt gctctgatcc ctctacagag cttccctgac tcattctgaa 3180

ggagccccat tcctgggaaa tattccctag aaacttccaa atcccctaag cagaccactg 3240

ataaaaccat gtagaaaatt tgttattttg caacctcgct ggactctcag tctctgagca 3300

gtgaatgatt cagtgttaaa tgtgatgaat actgtatttt gtattgtttc aattgcatct 3360

cccagataat gtgaaaatgg tccaggagaa ggccaattcc tatacgcagc gtgctttaaa 3420

aaataaataa gaaacaactc tttgagaaac aacaatttct actttgaagt cataccaatg 3480

aaaaaatgta tatgcactta taattttcct aataaagttc tgtactcaaa tgtagccacc 3540

aa 3542

<210> 908
<211> 3665
<212> DNA
<213> Homo sapiens

<400> 908
ggcttggggc agccgggtag ctcggaggtc gtggcgctgg gggctagcac cagcgctctg 60

tcgggaggcg cagcggttag gtggaccggt cagcggactc accggccagg gcgctcggtg 120

ctggaatttg atattcattg atccgggttt tatccctctt ctttttcttt aaacattttt 180

ttttaaaact gtattgtttc tcgttttaat ttatttttgc ttgccattcc ccacttgaat 240

cgggccgacg gcttggggag attgctctac ttccccaaat cactgtggat tttggaaacc 300

agcagaaaga ggaaagaggt agcaagagct ccagagagaa gtcgaggaag agagagacgg 360

ggtcagagag agcgcgcggg cgtgcgagca gcgaaagcga caggggcaaa gtgagtgacc 420

tgcttttggg ggtgaccgcc ggagcgcggc gtgagccctc ccccttggga tcccgcagct 480

gaccagtcgc gctgacggac agacagacag acaccgcccc cagccccagc taccacctcc 540

tccccggccg gcggcggaca gtggacgcgg cggcgagccg cgggcagggg ccggagcccg 600

cgcccggagg cggggtggag ggggtcgggg ctcgcggcgt cgcactgaaa cttttcgtcc 660

aacttctggg ctgttctcgc ttcggaggag ccgtggtccg cgcgggggaa gccgagccga 720

gcggagccgc gagaagtgct agctcgggcc gggaggagcc gcagccggag gagggggagg 780

aggaagaaga gaaggaagag gagagggggc cgcagtggcg actcggcgct cggaagccgg 840

gctcatggac gggtgaggcg gcggtgtgcg cagacagtgc tccagccgcg cgcgctcccc 900

aggccctggc ccgggcctcg ggccggggag gaagagtagc tcgccgaggc gccgaggaga 960

gcgggccgcc ccacagcccg agccggagag ggagcgcgag ccgcgccggc cccggtcggg 1020

cctccgaaac catgaacttt ctgctgtctt gggtgcattg gagccttgcc ttgctgctct 1080

acctccacca tgccaagtgg tcccaggctg cacccatggc agaaggagga gggcagaatc 1140
atcacgaagt ggtgaagttc atggatgtct atcagcgcag ctactgccat ccaatcgaga 1200
ccctggtgga catcttccag gagtaccctg atgagatcga gtacatcttc aagccatcct 1260
gtgtgcccct gatgcgatgc gggggctgct gcaatgacga gggcctggag tgtgtgccca 1320
ctgaggagtc caacatcacc atgcagatta tgcggatcaa acctcaccaa ggccagcaca 1380
taggagagat gagcttccta cagcacaaca aatgtgaatg cagaccaaag aaagatagag 1440
caagacaaga aaaaaaatca gttcgaggaa agggaaaggg gcaaaaacga aagcgcaaga 1500
aatcccggta taagtcctgg agcgtgtacg ttggtgcccg ctgctgtcta atgccctgga 1560
gcctccctgg cccccatccc tgtgggcctt gctcagagcg gagaaagcat ttgtttgtac 1620
aagatccgca gacgtgtaaa tgttcctgca aaaacacaga ctcgcgttgc aaggcgaggc 1680
agcttgagtt aaacgaacgt acttgcagat gtgacaagcc gaggcggtga gccgggcagg 1740
aggaaggagc ctccctcagg gtttcgggaa ccagatctct caccaggaaa gactgataca 1800
gaacgatcga tacagaaacc acgctgccgc caccacacca tcaccatcga cagaacagtc 1860
cttaatccag aaacctgaaa tgaaggaaga ggagactctg cgcagagcac tttgggtccg 1920
gagggcgaga ctccggcgga agcattcccg ggcgggtgac ccagcacggt ccctcttgga 1980
attggattcg ccattttatt tttcttgctg ctaaatcacc gagcccggaa gattagagag 2040
ttttatttct gggattcctg tagacacacc cacccacata catacattta tatatatata 2100
tattatatat atataaaaat aaatatctct attttatata tataaaatat atatattctt 2160
tttttaaatt aacagtgcta atgttattgg tgtcttcact ggatgtattt gactgctgtg 2220
gacttgagtt gggaggggaa tgttcccact cagatcctga cagggaagag gaggagatga 2280
gagactctgg catgatcttt tttttgtccc acttggtggg gccagggtcc tctcccctgc 2340
ccaggaatgt gcaaggccag ggcatggggg caaatatgac ccagttttgg gaacaccgac 2400
aaacccagcc ctggcgctga gcctctctac cccaggtcag acggacagaa agacagatca 2460
caggtacagg gatgaggaca ccggctctga ccaggagttt ggggagcttc aggacattgc 2520
tgtgctttgg ggattccctc cacatgctgc acgcgcatct cgcccccagg ggcactgcct 2580
ggaagattca ggagcctggg cggccttcgc ttactctcac ctgcttctga gttgcccagg 2640
agaccactgg cagatgtccc ggcgaagaga agagacacat tgttggaaga agcagcccat 2700
gacagctccc cttcctggga ctcgccctca tcctcttcct gctccccttc ctggggtgca 2760
gcctaaaagg acctatgtcc tcacaccatt gaaaccacta gttctgtccc cccaggagac 2820
ctggttgtgt gtgtgtgagt ggttgacctt cctccatccc ctggtccttc ccttcccttc 2880
ccgaggcaca gagagacagg gcaggatcca cgtgcccatt gtggaggcag agaaaagaga 2940
aagtgtttta tatacggtac ttatttaata tcccttttta attagaaatt aaaacagtta 3000
atttaattaa agagtagggt tttttttcag tattcttggt taatatttaa tttcaactat 3060
ttatgagatg tatcttttgc tctctcttgc tctcttattt gtaccggttt ttgtatataa 3120

aattcatgtt tccaatctct ctctccctga tcggtgacag tcactagctt atcttgaaca 3180

gatatttaat tttgctaaca ctcagctctg ccctccccga tcccctggct ccccagcaca 3240

cattcctttg aaataaggtt tcaatataca tctacatact atatatatat ttggcaactt 3300

gtatttgtgt gtatatatat atatatatgt ttatgtatat atgtgattct gataaaatag 3360

acattgctat tctgtttttt atatgtaaaa acaaaacaag aaaaaataga gaattctaca 3420

tactaaatct ctctcctttt ttaattttaa tatttgttat catttattta ttggtgctac 3480

tgtttatccg taataattgt ggggaaaaga tattaacatc acgtctttgt ctctagtgca 3540

gtttttcgag atattccgta gtacatattt atttttaaac aacgacaaag aaatacagat 3600

atatcttaaa aaaaaaaaag cattttgtat taaagaattt aattctgatc tcaaaaaaaa 3660

aaaaa 3665

## Description

The present invention relates to a method for in vitro diagnosing a complex disease or subtypes thereof in accordance with claim 1 and to a Kit for carrying out the method in accordance with claim 18.

In classical patient screening and diagnosis, the medical practitioner uses a number of diagnostic tools for diagnosing a patient suffering from a certain disease. Among these tools, measurement of a series of single routine parameters, e.g. in a blood sample, is a common diagnostic laboratory approach. These single parameters comprise for example enzyme activities and enzyme concentration and/or detection of metabolic indicators such as glucose and the like. As far as such diseases are concerned which easily and unambiguously can be correlated with one single parameter or a few number of parameters achieved by clinical chemistry, these parameters have proved to be indispensable tools in modern laboratory medicine and diagnosis. Under the provision that excellently validated cut-off values can be provided, such as in the case of diabetes, clinical chemical parameters such as blood glucose can be reliably used in diagnosis.

In particular, when investigating pathophysiological states underlying essentially a well known pathophysiological mechanism, from which the guiding parameter is resulting, such as a high glucose concentration in blood typically reflects an inherited defect of an insulin gene, such single parameters have proved to be reliable biomarkers for "its" diseases.

However, in pathophysiological conditions, such as cancer or demyelinating diseases such as multiple sclerosis which share a lack of an unambiguously assignable single parameter or marker, differential diagnosis from blood or tissue samples is currently difficult to impossible.

In cancer prevention, screening, diagnosis, treatment and aftertreatment, it is meanwhile clinical routine to use a series of so called "tumor markers" each being somewhat specific for a certain kind of cancer to diagnose and to monitor therapy of malign processes. Such currently used tumor markers are for example Alpha-1-fetoprotein, cancer antigen 125 (CA 125), cancer antigen 15-3, CA 50, CA 72-4, carbohydrate antigen 19-9, calcitonin, carcino embryonic antigen (CEA), cytokeratine fragment 21-1, mucin-like carcinoma-associated antigen, neuron specific enolase, nuclear matrix protein 22, alkaline phosphatase, prostate specific antigen (PSA), squamous cell carcinoma antigen, telomerase, thymidine kinase, Thyreoglobulin, and tissue polypeptid antigen.

Although, in the prior art already a number of the above tumor markers are meanwhile routinely used, it very often is difficult from a single measurement to achieve a reliable diagnosis. Just by way of example, the cut-off values of the CEA is 4,6 ng/ml for non-smokers, whereas 25% of smokers show normal values in the range of 3,5 to 10 ng/ml and 1% of smokers show normal values of more than 10 ng/ml. Thus, only values above 20 ng/ml have to be interpreted as being "highly suspicious for a malign process", which leaves a significant grey zone in which the physician cannot rely upon the CEA-values measured in a patient's sample.

EP 540 573 B1 discloses similar cut-off values' problems with respect to the prostate specific antigen (PSA) in which typically total PSA is measured for diagnosing or excluding prostate cancer in a patient, and if the values are in the grey zone, it is the current approach to measure in addition to total PSA also free PSA with a monoclonal antibody assay being specific for free PSA and calculate a ratio of both parameters in order to get a more accurate approach for diagnosing prostate cancer and to differentiate from benign prostate hyperplasia.

The above examples of CEA and PSA detection impressively demonstrate what is common with all single tumor markers, namely on one hand, the relatively poor specificity, and on the other hand, uncertain and unreliable cut-off values so that the achieved values are difficult to interpret.

Thus, as a general consequence; it is recommended to consider the use of tumor markers in screening as critical. It is not rarely that increased levels of tumor markers without further clinical correlation lead to unnerving of the patients and do not have any diagnostic value at all.

Furthermore, in aftertreatment of malign diseases, it has to be noticed that every tumor marker needs a "critical mass" of cancer cells first, until it responds positively in clinical test. In addition, not every recurrent tumor must involve an increase of tumor marker levels.

In summary, single tumor markers proved to be useful in clinical practice only mostly in context with other diagnostic tools such as endoscopy and biopsy, followed by histological examination, but are not reliable in routine cancer screening.

Vis-á-vie the prior art of single tumor markers, it was a great progress to use gene expression levels of a plurality of genes with the microarray technology.

WO 2004111197A2, e.g. discloses minimally invasive sample procurement method for obtaining airway epithelial cell RNA that can be analyzed by expression profiling, e.g., by array-based gene expression profiling. These methods can be used to identify patterns of gene expression that are diagnostic of lung disorders, such as cancer, to identify subjects at risk for developing lung disorders and to custom design an array, e.g., a microarray, for the diagnosis or prediction of lung disorders or susceptibility to lung disorders. Arrays and informative genes are also disclosed for this purpose.

Such multiple gene approaches are much more reliable then the above mentioned single parameters, however, are subject to complex mathematical and bioinformatics procedures. Nevertheless, these gene expression signatures are promising tools in cancer diagnosis, but sometimes also have uncertainty limits what leads due to their underlying statistics and being restricted to one kind nucleic acids also to sometimes unreliable results and validation problems.

Staring from the above mentioned prior art, it is the problem of the present invention to provide a use of biomarkers in diagnostics tools with the highest possible sensitivity and specificity for early diagnosis to identify diseased subjects, for use in patient pre-selection and stratification and for therapy control is a main goal in diagnostic development and still an urgent need in various complex diseases, in particular cancer.

The above problem is solved by a method in accordance with claim 1 and a kit in accordance with claim 18.

In particular, the present invention provides a method for in vitro diagnosing a complex disease or subtypes thereof, selected from the group consisting of:
cancer, in particular, acute myeloid leukemia (AML), colon cancer, kidney cancer, prostate cancer; ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis;
in at least one biological sample of at least one tissue of a mammalian subject comprising the steps of:
   a) selecting at least two different species of biomolecules, wherein said species of biomolecules are selected from the group consisting of: RNA and/or its DNA counterparts, microRNA and/or its DNA counterparts, peptides, proteins, and metabolites;
   b) measuring at least one parameter selected from the group consisting of presence (positive or negative), qualitative and/or quantitative molecular pattern and/or molecular signature, level, amount, concentration and expression level of a plurality of biomolecules of each species in said sample using at least two sets of different species of biomolecules and storing the obtained set of values as raw data in a database;
   c) mathematically preprocessing said raw data in order to reduce technical errors being inherent to the measuring procedures used in step b);
   d) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), self organizing maps (SOM), recursive partitioning and regression trees, K-nearest neighbor classifiers (K-NN), fuzzy classifiers, bagging, boosting, and naïve Bayes; and applying said selected classifier algorithm to said preprocessed data of step c);
   e) said classifier algorithms of step d) being trained on at least one training data set containing preprocessed data from subjects being divided into classes according to their pathophysiological, physiological, prognostic, or responder conditions, in order to select a classifier function to map said preprocessed data to said conditions;
   f) applying said trained classifier algorithms of step e) to a preprocessed data set of a subject with unknown pathophysiological, physiological, prognostic, or responder condition, and using the trained classifier algorithms to predict the class label of said data set in order to diagnose the condition of the subject.

Dependant claims 2 to 18 are preferred embodiments of the present invention.

The present invention provides a solution to the problem described above, and generally relates to the use of "omics" data comprising, but not limited to mRNA expression data, microRNA expression data, proteomics data, and metabolomics data, statistical learning respectively machine learning for identification of molecular signatures and biomarkers. It comprises the determination of the concentrations of the aforementioned biomolecules via known methods such as polymerase chain reaction (PCR), microarrays and other methods such as sequencing to determine RNA concentrations, protein identification and quantification by mass spectrometry (MS), in particular MS-technologies such as MALDI, ESI, atmospheric pressure pressure chemical ionization (APCI), and other methods, determination of metabolite concentrations by use of MS-technologies or alternative methods, subsequent feature selection and the combination of these features to classifiers including molecular data of at least two molecular levels (that is at least two different types of endogenous biomolecules, e.g. RNA concentrations plus metabolomics data respectively concentrations of metabolites or RNA concentrations plus concentrations of proteins or peptides etc.) and optimal composed marker sets are extracted by statistical methods and data classification methods.

The concentrations of the individual markers of the distinct molecular levels (RNA molecules, peptides/proteins, metabolites etc.) thus are measured and data processed to classifiers indicating diseased states etc. with superior sensitivities and specificities compared to procedures and biomarker confined to one type of biomolecules.

A method for the selection and combination of biomarkers and molecular signatures of biomolecules in particular utilizing one or several individual molecules of the biomolecule types mRNA, microRNA, proteins, or peptides, small endogenous compounds (metabolites) in combination (combining at least two of the aforementioned types of biomolecules), with the biomolecules obtained from body liquids or tissue, identified by use of statistical methods and classifiers derived from the data of these groups of molecules for use in diagnosis and early diagnosis, for patient stratification, therapy selection, therapy monitoring and theragnostics in complex diseases is described.

### BACKGROUND OF THE INVENTION - Prior ART

Systems biology approaches utilizing varying omics approaches such as genomics, proteomics and metabolomics are increasingly applied to research and diagnostics of complex diseases. These technologies may provide data and biological indicators, so-called (prognostic, predictive and pharmacodynamic) biomarkers with the potential to revolutionize clinical practice in diagnosis.

For early cancer detection single biomarkers are commonly used. However, the widely used cancer antigen 125 (CA125) for instance can only detect 50%-60% of patients with stage I ovarian cancer. Analogously, the single use of the prostate specific antigen (PSA) value for early stage prostate cancer identification is not specific enough to reduce the number of false positives [Petricoin EF 3rd, Ornstein DK, Paweletz CP, Ardekani A, Hackett PS, Hitt BA, Velassco A, Trucco C, Wiegand L, Wood K, Simone CB, Levine PJ, Linehan WM, Emmert-Buck MR, Steinberg SM, Kohn EC, Liotta LA, Serum proteomic patterns for detection of prostate cancer, J Natl Cancer Inst. 2002; 94(20): 176-8.] and it is evident that it is highly unlikely that a complex disease can be characterized or diagnosed and the effect of therapies assessed by use of single biomarkers.

Recent advances in diagnostic tools e.g. in cancer diagnostics typically comprise multicomponent tests utilizing several biomarkers of the same class of biomolecules such as several proteins, RNA or microRNA species and the analysis of high dimensional data gives a deeper insight into the abnormal signaling and networking which has a high potential to identify previously not discovered marker candidates. However, methods according to the present state of the art utilize single biomolecules or sets of a single type of biomolecules for biomarkers sets such as several RNA, microRNA or protein molecules. See Garzon R, Volinia S, Liu CG, Fernandez-Cymering C, Palumbo T, Pichiorri F, Fabbri M, Coombes K, Alder H, Nakamura T, Flomenberg N, Marcucci G, Calin GA, Kornblau SM, Kantarjian H, Bloomfield CD, Andreeff M, Croce CM, MicroRNA signatures associated with cytogenetics and prognosis in acute myeloid leukemia, Blood. 2008; 111(6):3183-9 and Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR., Multiclass cancer diagnosis using tumor gene expression signatures. Proc Natl Acad Sci USA. 2001;98(26):11149-54. For miRNA in Cancer see WO2008055158.

In addition, Oncotype DX is an example of a recent multicomponent RNA-based test, like a multigene activity assay, to predict recurrence oftamoxifen-treated, node-negative breast cancer is disclosed in Paik S, Shak S, Tang G, Kim C, Baker J, Cronin M, Baehner FL, Walker MG, Watson D, Park T, Hiller W, Fisher ER, Wickerham DL, Bryant J, Wolmark N, Engl J Med. 2004; 351 (27):2817-26.

Habel LA, Shak S, Jacobs MK, Capra A, Alexander C, Pho M, Baker J, Walker M, Watson D, Hackett J, Blick NT, Greenberg D, Fehrenbacher L, Langholz B, Quesenberry CP describe a population-based study of tumor gene expression and risk of breast cancer death among lymph node-negative patients in Breast Cancer Res. 2006; 8(3):R25.

Other recent examples include breast-cancer gene-expression signatures - marketed for clinical use as), MammaPrint (Agendia).

Furthermore, Glas AM, Floore A, Delahaye LJ, Witteveen AT, Pover RC, Bakx N, Lahti-Domenici JS, Bruinsma TJ, Warmoes MO, Bernards R, Wessels LF, Van't Veer LJ.Disclose a method for converting a breast cancer microarray signature into a high-throughput diagnostic test in BMC Genomics. 2006; 7:278.

Another known approach is disclosed as the so called H/I test (AviaraDx), developped by Nicholas C Turner and Alison L Jones BMJ. 2008 July 19; 337(7662): 164-169, which estimates the probability of the original breast cancer recurring after it has been resected.

Although these products and prototypes demonstrate significant progress for specific areas of diagnostics, there is still an urgent need for reliable and early diagnostics with high sensitivities and specificities in a number of complex diseases such as, but not limited to, cancer, in particular, acute myeloid leukemia (AML), colon cancer, kidney cancer, prostate cancer; ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis, Alzheimer and Parkinson disease. These diagnostic tools and biomarkers are also being used for the selection of responders among patients, for an assessment of disease recurrence, the selection of therapeutic options, efficacy, drug resistance and toxicity.

The invention provides the principle and the method for the generation of novel diagnostic tools to diagnose complex diseases with superior sensitivities and specificities to address these problems.

Data integration of various "omics" data, e.g. to identify possible alterations of protein concentrations from altered RNA transcripts is an issue familiar to systems biology and to persons skilled in the arts for years.

Despite of that, the statistical combination of biomarker sets from different types of biomolecules, independent of data integration and biochemical interpretation to combined diagnostic signatures (combining several types of biomolecules) on a statistical basis applying various classification methods as described here is not obvious, unknown to persons skilled in the art, and has not been described in the literature. It clearly is distinct to approaches utilizing an integrative multi-dimensional analysis and combining e.g. genomes, epigenomes and transcriptomes (see SIGMA2: A system for the integrative genomic multi-dimensional analysis of cancer genomes, epigenomes, and transcriptomes, Raj Chari et al. BMC Bioinformatics 2008, 9:422) which attempt to analyse biological relationships between different omics data by various means.

Essentially, the method according to the present invention combines statistically significant biomolecule parameters of at least two different types of biomolecules on a statistical basis, entirely irrespective of known or unknown biological relationship of any kind, links or apparent biological plausibility to afford a combined biomarker composed of several types of biomolecules. The patient cases underlying the invention demonstrate that a diagnostic method and disease state specific classifier composed of at least two of the aforementioned biomolecule types and those combined biomolecules of at least two types describing the respective state of cells, a tissue, an organ or an organisms best among a collective of measured molecules, is superior to a composition of molecules or markers and their delineated molecular signatures. It is further superior to classifiers of biomolecules of just one type of biomolecules and as demonstrated here yields higher sensitivities and specificities in diagnostic applications. In that, the present invention goes far beyond the current state of the art and provides a method for generating diagnostic molecular signatures affording higher sensitivities and specificities and decreased false discovery rates compared to methods available so far. The method can be applied for diagnosing various complex and completely unrelated complex diseases such as cancer and ischemia and is of general diagnostic use.

### DETAILED DESCRIPTION OF THE INVENTION

### Definitions

As used herein, the term "gene expression" refers to the process of converting genetic information encoded in a gene into ribonucleic acid, RNA (e.g., mRNA, rRNA, tRNA, or snRNA) through "transcription" of the gene (i.e., via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (i.e., RNA or protein), while "down-regulation" or "repression" refers to regulation that decrease production

Polynucleotide: A nucleic acid polymer, having more than 2 bases.

"Peptides" are short heteropolymers formed from the linking, in a defined order, of α-amino acids. The link between one amino acid residue and the next is known as an amide bond or a peptide bond.

Proteins are polypeptide molecules (or consist of multiple polypeptide subunits). The distinction is that peptides are short and polypeptides/proteins are long. There are several different conventions to determine these, all of which have caveats and nuances.

A "Complex disease" within the scope of the present invention is one belonging to the following group, but is not limited to this group: cancer, in particular, acute myeloid leukemia (AML), colon cancer, kidney cancer, prostate cancer; transient ischemic attack (TIA), ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis, Alzheimer and Parkinson's disease.

Metabolite: as used here, the term "metabolite" denotes endogenous organic compounds of a cell, an organism, a tissue or being present in body liquids and in extracts obtained from the aforementioned sources with a molecular weight typically below 1500 Dalton. Typical examples of metabolites are carbohydrates, lipids, phospholipids, sphingolipids and sphingophospholipids, amino acids, cholesterol, steroid hormones and oxidized sterols and other compounds such as collected in the Human Metabolite database (http;//www.hmdb.ca/) and other databases and literature. This includes any substance produced by metabolism or by a metabolic process and any substance involved in metabolism.

"Metabolomics" as understood within the scope of the present invention designates the comprehensive quantitative measurement of several (2-thousands) metabolites by, but not limited to, methods such as mass spectroscopy, coupling of liquid chromatography, gas chromatography and other separation methods chromatography with mass spectroscopy.

"Oligonucelotide arrays "or" oligonucleotide chips" or "gene chips": relates to a "microarray", also referred to as a "chip", "biochip", or "biological chip", is an array of regions having a suitable density of discrete regions, e. g., of at least 100/cm², and preferably at least about 1000/cm². The regions in a microarray have dimensions, e. g. diameters, preferably in the range of between about 10-250 µm, and are separated from other regions in the array by the same distance. Commonly used formats include products from Agilent, Affymetrix, Illumina as well as spotted fabricated arrays where oligonucleotides and cDNAs are deposited on solid surfaces by means of a dispenser or manually.

It is clear to a person skilled in the art that nucleic acids, proteins and peptides as well as metabolites can be quantified by a variety of methods including the above mentioned array systems as well as, but not limited to: quantitative sequencing, quantitative polymerase chain reaction and quantitative reverse transcription polymerase chain reaction (qPCR and .RT-PCR), immunoassays, protein arrays utilizing antibodies, mass spectrometry.

"microRNAs" (miRNAs) are small RNAs of 19 to 25 nucleotides that are negative regulators of gene expression. To determine whether miRNAs are associated with cytogenetic abnormalities and clinical features in acute myeloid leukemia (AML), the miRNA expression of CD34(+) cells and 122 untreated adult AML cases is evaluated using a microarray platform.

Under different species or types or classes of biomolecule in this context is understood: RNA, microRNA, proteins and peptides of various lengths as well as metabolites.

A biomarker in this context is a characteristic, comprising data of at least two biomolecules of at least two different types (RNA, microRNA, proteins and peptides, metabolites) that is measured and evaluated as an indicator of biologic processes, pathogenic processes, or responses to an therapeutic intervention. A combined biomarker as used here may be selected from at least two of the following types of biomolecules: sense and antisense nucleic acids, messenger RNA, small RNA i.e. siRNA and microRNA, polypeptides, proteins including antibodies, small endogenous molecules and metabolites.

Data classification is the categorization of data for its most effective and efficient use. Classifiers are typically deterministic functions that map a multi-dimensional vector of biological measurements to a binary (or n-ary) outcome variable that encodes the absence or existence of a clinically-relevant class, phenotype, distinct physiological state or distinct state of disease. To achieve this various classification methods such as, but not limited to, logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), self organizing maps (SOM), recursive partitioning and regression trees, K-nearest neighbor classifiers (K-NN), fuzzy classifiers, bagging, boosting, and naive Bayes and many more can be used.

The term "binding", "to bind", "binds", "bound" or any derivation thereof refers to any stable, rather than transient, chemical bond between two or more molecules, including, but not limited to, covalent bonding, ionic bonding, and hydrogen bonding. Thus, this term also encompasses hybridization between two nucleic acid molecules among other types of chemical bonding between two or more molecules.

### Description

In the method of the present invention, biomarker data and classifier obtained by combination of at least two different types of biomolecules out of two different species of biomolecules, wherein said species of biomolecules are selected from the group consisting of: RNA and/or its DNA counterparts, microRNA and/or its DNA counterparts, peptides, proteins, and metabolites, identified according to the invention afford a description of a physiological state and can be used as a superior tool for diagnosing complex diseases.

The discrimination of pathological samples or tissues from healthy specimens requires a combination of data of at least two distinct types of biomolecules, a determination of their concentrations and a statistical processing and classifier generation according to the method depicted in Table 1 below.

As mentioned above a biological link between molecules combined in a biomarker by means of classification is entirely irrelevant to the outcome and selection of the issues and can not be necessarily explained by biological models.

The method according to the present invention comprises essentially the following steps:
**First,** a biological sample obtained from a subject or an organism is obtained.
**Second,** the amounts of biomolecules of the following types (RNA, microRNA, peptide or protein, metabolite) are measured from the biological sample and stored as raw data in a database.
**Third** the raw data from the database are preprocessed.
**Fourth,** the amount of RNA and/or its DNA counterparts, microRNA and/or its DNA counterparts, peptide or protein, metabolite detected in the sample is compared to either a standard amount of the respective biomolecule measured in a normal cell or tissue or a reference amount of the respective biomolecule stored in a database. If the amount of the biomolecules of interest in the sample is different to the amount of the biomolecules determined in the standard or control sample, the differential concentration data are processed and used for **step 5** classifier generation as described below.

The classifier is validated in **step 6** and used in **step 7:** according to the invention, the classifier utilizes data from at least two groups of biomolecules of the aforementioned types and afford a value or a score. This score is assigned to an altered physiological state of plasma, tissue or an organ with a computed probability and can indicate a diseased state, a state due to intervention (e.g. therapeutic intervention by treatment, surgery or pharmacotherapy) or an intoxication with some probability. This score can be used as a diagnostic tool to indicate that the subject or the organism is diagnosed as diseased, to indicate intoxication as having cancer.

The score and time-dependent changes of the score can be used to assess the success of a treatment or the success of a drug administered to the subject or the organism or assess the individual response of a subject or an organism to the treatment or to make a prognosis of the future course of the physiological state or the disease and the outcome. The prognoses are relative to a subject without the disease or the intoxication having normal levels or average values of the score or classifier composed of at least two biomolecules

### Table 1

**Table 1: Schematic diagram of proposed method. More details are given in text.**

| |
|---|
| **Step 1:** |
| **Biological sample obtained** |
| **Step 2:** |
| **Measurement of raw data (concentrations of biomolecules) and deposit in data base** |
| **Step 3:** |
| **Preprocessing of raw data from data base** |
| **Step 4:** |
| **Comparison to reference values and feature selection** |
| **Step 5:** |
| **Train classifier based on data of a composed biomarker composed of at least two types of biomolecules** |
| **Step 6:** |
| **Validate classifier** |
| **Step 7:** |
| **Use of the classifier to assess physiological state, as diagnostic tool to indicate a diseased state or as a prognostic tool** |

In case of mRNA and microRNA data the preprocessing of the data typically consists of background correction and normalization. The skilled person is aware of a number of suitable known background correction and normalization strategies; a comparative survey in case of Affymetrix data is given in L.M. Cope et al., A Benchmark for Affymetrix GeneChip Expression Measures, Bioinformatics 2004, 20(3), 323-331 or R.A. Irizarry et al., Comparison of Affymetrix GeneChip Expression Measures, Bioinformatics 2006, 22(7), 789-794, respectively.

Depending on the data at hand, it may also consist of some variance stabilizing transformation or transformation to normality as for instance taking the logarithm or using Box-Cox power transformations [Box, G. E. P. and Cox, D. R. An analysis of transformations (with discussion). Journal of the Royal Statistical Society B 1964, 26, 211-252].

Often also scaling e.g. by standard deviation or median absolute deviation (MAD) might be used to transform the raw data. However, this step is not necessary for all kind of data, respectively all kind of further statistical analyses and hence may also be omitted.

The feature (variable, measurement) selection step might also be optional. However, it is recommended if the number of features is larger than the number of samples. Feature selection methods try to find the subset of features with the highest discriminatory power.

Due to the high dimensionality of mRNA and microRNA data, most classification algorithms cannot be directly applied. One reason is the so-called curse of dimensionality: With increasing dimensionality the distances among the instances assimilate. Noisy and irrelevant features further contribute to this effect, making it difficult for the classification algorithm to establish decision boundaries. Further reasons why classification algorithms are not applicable on the full dimensional space are performance limitations. Ultimately, feature transformation techniques are applied before classification, e.g. in [J. S. Yu et al., Ovarian cancer identification based on dimensionality reduction for high-throughput mass spectrometry data, Bioinformatics, 21(10):2200-2209,2005]. Furthermore, also for the task of identifying unknown marker candidates, the use of traditional methods is limited due to the high dimensionality of the data.

To identify diseased subjects with the highest possible sensitivity and specificity is the main goal in diagnostic development. For this purpose, a large number of classification algorithms can be chosen e.g. logistic regression, (diagonal) linear or quadratic discriminant analysis (LDA, QDA, DLDA, DQDA), shrunken centroids regularized discriminant analysis (RDA), random forests (RF), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more can be applied to develop new marker candidates. These algorithms are trained on at least one training data set which contains instances labeled according to classes, e.g. healthy and diseased, and then tested on at least one test data set which includes novel instances not used for the training. In the training-test step one or more rounds of cross-validation, bootstrap or some split-sample approach can be used to estimate how accurately a predictive model will perform in practice. Finally, the classifier will be used to predict the class label of novel unlabeled instances [T. M. Mitchell. Machine Learning. McGraw-Hill, 1997].

Classifiers are typically deterministic functions that map a multi-dimensional vector of biological measurements to a binary (or n-ary) outcome variable that encodes the absence or existence of a clinically-relevant class, phenotype or distinct state of disease. The process of building or learning a classifier involves two steps: (1) selection of a family functions that can approximate the systems response, and using a finite sample of observations (training data) to select a function from the family of functions that best approximates the system's response by minimizing the discrepancy or expected loss between the system's response and the function predictions at any given point.

Depending on the chosen feature selection strategy, the combination of the different data (clinical data, mRNA, microRNA, metabolites, proteins) can take place before or after feature selection. The combined data is then used as input to train and validate the classifier. However, it is also possible to train several different classifiers for the different data separately and then combine the classifiers to the predictive signature. As the data types may be very different from qualitative/categorical to quantitative/numerical, not all classifiers may work for such multilevel data; e.g., some classifiers accept only quantitative data. Hence, depending on the data types one has to choose a class of functions for classification which has an appropriate domain.

Numerous feature selection strategies for classification have been proposed, for a comprehensive survey see e.g. [M. A. Hall and G. Holmes, Benchmarking Attribute Selection Techniques for Discrete Class Data Mining. IEEE Transactions on Knowledge and Data Engineering, 15(6): 1437-1447, 2003.]. Following a common characterization, it is distinguished between filter and wrapper approaches.

Filter approaches use an evaluation criterion to judge the discriminating power of the features. Among the filter approaches, it can further be distinguished between rankers and feature subset evaluation methods. Rankers evaluate each feature independently regarding its usefulness for classification. As a result, a ranked list is returned to the user. Rankers are very efficient, but interactions and correlations between the features are neglected. Feature subset evaluation methods judge the usefulness of subsets of the features. The information of interactions between the features is in principle preserved, but the search space expands to the size of O(2<d>). For high-dimensional data, only very simple and efficient search strategies, e.g. forward selection algorithms, can be applied because of the performance limitations.
The wrapper attribute selection method uses a classifier to evaluate attribute subsets. Cross-validation is used to estimate the accuracy of the classifier on novel unclassified objects. For each examined attribute subset, the classification accuracy is determined. Adapted to the special characteristics of the classifier, in most cases wrapper approaches identify attribute subsets with higher classification accuracies than filter approaches, cf. Pochet, N., De Smet, F., Suykens, J.A., and De Moor, B.L., Systematic benchmarking of microarray data classification: assessing the role of non-linearity and dimensionality reduction. Bioinformatics, 20(17):3185-95 (2004). As the attribute subset evaluation methods, wrapper approaches can be used with an arbitrary search strategy. Among all feature selection methods, wrappers are the most computational expensive ones, due to the use of a learning algorithm for each examined feature subset.

A preferred embodiment of the present invention is a method, wherein said complex disease is AML, said mammalian subject is a human being, said biological sample blood and/or blood cells and/or bone marrow;
wherein said different species of biomolecules are microRNA and proteins, in particular surface proteins from non-mature hematopoietic stem cells, preferably CD34;
wherein microRNA expression levels and CD34 presence are used as said parameters of step b);
wherein raw data of microRNA expression are preprocessed using a variance-stabilizing normalization and summarizing the normalized multiple probe signals (technical replicates) to a single expression value, using the median;
wherein a ranker, in particular a Mann-Whitney significance test combined with largest median of pairwise differences as filter for microRNA expression data is used for said feature selection;
wherein logistic regression is selected as suitable classifying algorithm, the training of the classifying algorithm including preprocessed and filtered microRNA expression data and CD34 information (positive or negative), is carried out with an n-fold cross-validation, in particular 5 to 10-fold, preferably 5-fold cross-validation;
applying said trained logistic regression classifier to said preprocessed microRNA expression data set and CD34 information to a subject under suspicion of having AML, and using the trained classifiers to diagnose a specific AML-type.

Another preferred embodiment of the present invention is a method, wherein said complex disease is colon cancer, said mammalian subject is a human being, said biological sample is colon tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for microRNA expression data is used for said feature selection;
wherein random forests are selected as suitable classifying algorithm, the training of the classifying algorithm including preprocessed and filtered mRNA and microRNA expression data, is carried out with a leave-one-out (LOO) cross-validation;
applying said trained random forests classifier to said preprocessed mRNA and microRNA expression data sets to a subject under suspicion of having colon cancer, and using the trained classifiers to diagnose colon cancer and/or a subtype thereof.

A further preferred embodiment of the present invention is a method, wherein said complex disease is kidney cancer, said mammalian subject is a human being, said biological sample is kidney tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Welch t-test (significance test) combined with largest mean of pairwise differences as filter for mRNA and microRNA expression data is used for said feature selection;
wherein single-hidden-layer neural networks are selected as suitable classifying algorithm, the training of the classifying algorithm including preprocessed and filtered mRNA and microRNA expression data, is carried out with a leave-one-out (LOO) cross-validation; applying said trained random forests classifier to said preprocessed mRNA and microRNA expression data sets to a subject under suspicion of having kidney cancer, and using the trained classifiers to diagnose kidney cancer and/or a subtype thereof.

Another preferred embodiment of the present invention is a method, wherein said complex disease is prostate cancer, said mammalian subject is a human being, said biological sample is urine and/or prostate tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization; wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for mRNA and microRNA expression data is used for said feature selection;
wherein linear discriminant analysis is selected as suitable classifying algorithm, the training of the classifying algorithm including preprocessed and filtered mRNA and microRNA expression data, is carried out with a leave-one-out (LOO) cross-validation;
applying said trained random forests classifier to said preprocessed mRNA and microRNA expression data sets to a subject under suspicion of having prostate cancer, and using the trained classifiers to diagnose prostate cancer and/or a subtype thereof.

Again another preferred embodiment of the present invention is a method, wherein said complex disease is transient ischemic attack (TIA) and/or ischemia and/or hypoxia, said mammalian subject is a human being, said biological sample blood and/or blood cells and/or cerebrospinal fluid and/or brain tissue;
wherein said different species of biomolecules are mRNA and/or its DNA counterparts and brain metabolites, in particular free prostaglandins, lipooxygenase derived fatty acid metabolites, glutamine, glutamic acid, leucin, alanine, serine, decosahexaenoic acid (DHA), 12(S)-hydroxyeicosatetraenoic acid (12S-HETE);
wherein mRNA expression levels and quantitative and/or qualitative molecular metabolite patterns (metabolomics data) are used as said parameters of step b);
wherein raw data of mRNA expression are preprocessed using action-β as reference genes and metabolomics data of said brain metabolites are preprocessed by a variance stabilizing transformation via the binary logarithm (i.e. to base 2);
wherein a ranker, in particular a Welch t-test (significance test) combined with largest mean of pairwise differences as filter for metabolomics data is used for said feature selection;
wherein support vector machines are selected as suitable classifying algorithm, the training of the classifying algorithm including preprocessed and filtered mRNA and microRNA expression data, is carried out with a leave-one-out (LOO) cross-validation;
applying said trained support vector machines classifier to said preprocessed mRNA expression data and said metabolomics data sets to a subject under suspicion of having ischemia and/or hypoxia, and using the trained classifiers to diagnose ischemia and/or hypoxia and/or the grades thereof.

### Examples

### Example 1: Method utilizing microRNA and protein data

As a first example, we use the microRNA and clinical data of Garzon R, Garofalo M, Martelli MP, Briesewitz R, Wang L, Fernandez-Cymering C, Volinia S, Liu CG, Schnittger S, Haferlach T, Liso A, Diverio D, Mancini M, Meloni G, Foa R, Martelli MF, Mecucci C, Croce CM, Falini B. Distinctive microRNA signature of acute myeloid leukemia bearing cytoplasmic mutated nucleophosmin. PNAS 2008, 105(10):3945-50.

These data are available in the ArrayExpress online database http://www.ebi.ac.uk/arrayexpress under accession number E-TABM-429. Overall the microRNA data of 85 adult de novo AML patients characterized for subcellular localization/mutation status of NPM1 and FLT3 mutations are available. The hybridizations' were done using the OSU-CCC human & mouse microRNA 11K v2 Microarray Shared Resource, Comprehensive Cancer Center, The Ohio State University (OSU-CCC).

Acute myeloid leukemia (AML) carrying NPM1 mutations and cytoplasmic nucleophosmin (NPM1+ AML) accounts for about one-third of adult AML and shows distinct features including a unique gene expression profile. The authors used microRNA expression values to distinguish NPMc+ mutated (n = 55) from the cytoplasmic-negative (NPMc-, i.e., NPM1 unmutated) cases (n = 30).

### Analysis:

For developing and validating a classifier based on these data we used logistic regression in combination with 5-fold cross-validation where each analysis step - including low level analysis - was repeated in each cross-validation step. Moreover, we repeated 5-fold cross-validation 20 times. This is one possibility. Of course, we could also have used a split-sample, a bootstrap or a different k-fold (k not equal to 5) cross-validation approach. Moreover, we could have used a different class of functions for classification e.g. (diagonal) linear or quadratic discriminant analysis (LDA, QDA, DLDA, DQDA), shrunken centroids regularized discriminant analysis (RDA), random forests (RF), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.
The low level analysis consisted of the variance stabilizing transformation of Huber et al. (2002) [Huber W, von Heydebreck A, Sueltmann H, Poustka A, Vingron M. Variance Stabilization Applied to Microarray Data Calibration and to the Quantification of Differential Expression. Bioinformatics 2002, 18: 96-104] (often called normalization) and the averaging of the normalized replicates using the median. Again there is a large number of alternative methods which could be used. Several examples are given in L.M. Cope et al., Bioinformatics 2004, 20(3), 323-331 or R.A. Irizarry et al., Bioinformatics 2006, 22(7), 789-794. In each cross validation step we selected those five normalized and averaged microRNA probes for classification which had the largest median of pairwise differences (in absolute value) beyond those microRNA probes with p value equal or smaller than 0.01 by the Mann-Whitney test. This is, we used a so called ranker for feature selection. Again there are numerous other feature selection strategies we could have used, some examples are given in [M. A. Hall and G. Holmes. IEEE Transactions on Knowledge and Data Engineering, 15(6): 1437-1447, 2003.]. Overall a microRNA probe may have been chosen up to 100 times due to the 20 replications of the 5-fold cross-validation. We obtain the estimated errors given in Table 2.

### Table 2

**Table 2: microRNA data, classification error via 5-fold cross-validation**

| **classifier vs. true** | **NPMc-** | **NPMc+** |
|---|---|---|
| **NPMc-** | 57.0% | 7.6% |
| **NPMc+** | 43.0% | 92.4% |

The estimated overall accuracy using 5-fold cross-validation is 79.9 %. In a second step we now use only those microRNA arrays where there additionally is information about CD34 (i.e., CD34 negative or CD34 positive); selecting these samples 54 NPMc+ and 29 NPMC-samples remain. Using only CD34 for classification we obtain the results given in Table 3. which corresponds to an overall accuracy of 85.5 %.

### Table 3

**Table 3: CD34 data, classification error**

| **classifier vs. true** | **NPMc-** | **NPMc+** |
|---|---|---|
| **NPMc-** | 75.9% | 9.3% |
| **NPMc+** | 24.1% | 90.7% |

Now, if we combine the information of the top five microRNA probes with the CD34 information, we obtain the results given in Table 4. That is, the estimated overall accuracy using cross-validation is 88.1 %. Hence, this combination increases the overall accuracy from 79.9 % respectively, 85.5 % to 88.1 %.

### Table 4

**Table 4: combinations of microRNA and CD34, classification error via 5-fold cross validation**

| **classifier vs. true** | **NPMc-** | **NPMc+** |
|---|---|---|
| **NPMc-** | 80.7% | 8.0% |
| **NPMc+** | 19.3% | 92.0% |

The probes which were selected during cross-validation are given in Table 5.

### Table 5

**Table 5: microRNA probes selected during 5-fold cross validation**

| **Seq-ID** | **Probe ID** | **Times selected** | **Probe sequence** |
|---|---|---|---|
| 1 | uc.124+ | 100 | TGCTCATCTGTGCACTTCTGTTCAACCTATCACACTGAGT |
| 2 | mmu-mir-335No2 | 97 | AAACCGTTTTTCATTATTGCTCCTGACCCCCTCTCATGGG |
| 3 | uC.368+A | 96 | TGCACAGGGGACCTTAACCAGATCATTAGTTTATATGCCT |
| 4 | uC.324+A | 93 | CACACACTCCAGAACAGATGGTATCCAGATGCCTTATGGG |
| 5 | uc. 156+ | 74 | GCGAACCATTTCTAATGTTCTGATTTTTCAGAGCCAGCCA |
| 6 | hsa-mir-340Nol | 12 | TGTGGGATCCGTCTCAGTTACTTTATAGCCATACCTGGTA |
| 7 | uc. 106+ | 6 | AGCTGAATGGTGATGGTGTGAAGTATAGGTTAAATTGGGT |
| 8 | hsa-mir-033b-prec | 4 | GTGCATTGCTGTTGCATTGCACGTGTGTGAGGCGGGTGCA |
| 9 | uc.54+A | 4 | AAAGCTGTAGGGCCTCCAGGTTCTCAAGCTGTGAGTGGAA |
| 10 | uc.85+ | 4 | TGGTTGACATATGGCTGCTAATGCCCTCCTTTCTAGTGGG |
| 11 | uc.78+A | 4 | GTGTGCGTAACGGCTGGTGTGTTTCTCTAGCTGAGCTAAT |
| 12 | mmu-mir-31No2 | 3 | ACCTGCTATGCCAACATATTGCCATCTTTCCTGTCTGACA |
| 13 | uc.195+A | 2 | ACAGTGAGTGCGAGTATTATTTCTTGCCAGCGGGTGGAAG |
| 14 | uc.7+A | 1 | ACACTGCTCGCTCTATGTTAATTTTAGCTCTTCCCCTGGA |

The results of the Sanger sequence search in accordance with Griffiths-Jones S, Saini HK, van Dongen S, Enright AJ. miRBase: tools for microRNA genomics, NAR 2008 36(Database Issue):D154-D158 for known human microRNAs are given in Table 6

### Table 6

**Table 6: Results of the Sanger sequence search for known human microRNAs for the microRNA probes selected during 5-fold cross validation**

| **Seq-ID** | **Probe** | **microRNA ID** | **Target sequence** |
|---|---|---|---|
| 15 | uc.124+ | hsa-mir-134 | CAGGGUGUGUGACUGGUUGACCAGAGGGGCAUGCAC UGUGUUCACCCUGUGGGCCACCUAGUCACCAACCCUC |
| 16 | mmu-mir-335No2 | hsa-mir-335 | UGUUUUGAGCGGGGUCAAGAGCAAUAACGAAAAAUG UUUGUCAUAAACCGUUUUUCAUUAUUGCUCCUGACCU CCUCUCAUUUGCUAUAUUCA |
| 18 | hsa-mir-340No1 | hsa-mir-340 | UUGUACCUGGUGUGAUUAUAAAGCAAUGAGACUGAUU GUCAUAUGUCGUUUGUGGAUCCGUCUCAGUUACUUU AUAGCCAUACCUGGUAUCUUA |
| 19 | uc.106+ | hsa-mir-138-1 | CCCUGGCAUGGUGUGGUGGGGCAGCUGGUGUUGUGA AUCAGGCCGUUGCCAAUCAGAGAACGGCUACUUCACA ACACCAGGGCCACACCACACUACAGG |
| 20 | hsa-mir-033b-prec | hsa-mir-33b | GCGGGCGGCCCCGCGGUGCAUUGCUGUUGCAUUGCA CGUGUGUGAGGCGGGUGCAGUGCCUCGGCAGUGCAG CCCGGAGCCGGCCCCUGGCACCAC |
| 21 | uc.54+A | hsa-mir-339 | CGGGGCGGCCGCUCUCCCUGUCCUCCAGGAGCUCAC GUGUGCCUGCCUGUGAGCGCCUCGACGACAGAGCCG GCGCCUGCCCCAGUGUCUGCGC |
| 22 | uc.85+ | hsa-mir-1976 | GCAGCAAGGAAGGCAGGGGUCCUAAGGUGUGUCCUCC UGCCCUCCUUGCUGU |
| 23 | uc.78+A | hsa-mir-223 | CCUGGCCUCCUGCAGUGCCACGCUCCGUGUAUUUGAC AAGCUGAGUUGGACACUCCAUGUGGUAGAGUGUCAGU UUGUCAAAUACCCCAAGUGCGGCACAUGCUUACCAG |
| 24 | mmu-mir-31No2 | hsa-mir-3 | GGAGAGGAGGCAAGAUGCUGGCAUAGCUGUUGAACUG GGAACCUGCUAUGCCAACAUAUUGCCAUCUUUCC |
| 25 | uc.195+A | hsa-mir-548a-3 | CCUAGAAUGUUAUUAGGUCGGUGCAAAAGUAAUUGCG AGUUUUACCAUUACUUUCAAUGGCAAAACUGGCAAUUA CUUUUGCACCAACGUAAUACUU |
| 26 | uc.7+A | hsa-mir-1912 | CUCUAGGAUGUGCUCAUUGCAUGGGCUGUGUAUAGUA UUAUUCAAUACCCAGAGCAUGCAGUGUGAACAUAAUAG AGAUU |

### Example 2.1.: mRNA and microRNA: Colon Cancer

We use the colon cancer data of Ramaswamy et al. (2001) [Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR. Multiclass cancer diagnosis using tumor gene expression signatures. Proc Natl Acad Sci USA. 2001;98(26):15149-54] and Lu et al. (2005) [Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature. 2005;435(7043):834-8] to develop a multilevel classifier using mRNA and microRNA data. The data are available from the home page of the Broad Institute [http://www.broad.mit.edu/pubjications/broad900 and http://www.broad.mit.edu/publications/broad993s].

Overall the mRNA and microRNA data of four normal tissues and seven tumor tissues are available. The hybridisations were done with a bead-based array containing microRNA probes as well as with the Affymetrix HU6800 and HU35KsubA array for measuring the mRNA. We used only the mRNA data of the HU6800 arrays.

### Analysis:

For developing and validating a classifier based on these data we used random forests [Breiman, L. Random Forests, Machine Learning 2001, 45(1), 5-32] in combination with leave-one-out (LOO) cross-validation where each analysis step - including low level analysis -was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naive Bayes and many more.

The preprocessing (also called low level analysis) consisted of the variance stabilizing transformation of Huber et al (2002) (often called normalization) in case of the microRNA as well as of the mRNA data. Again there is a large number of alternative methods which could be used. Several examples are given in Cope et al. (2004) or Irizarry et al. (2006). In each cross validation step we selected those six normalized microRNA probes, respectively those six normalized mRNA probes for classification which had the largest median of pairwise differences (in absolute value) beyond those probes with p value equal or smaller than 0.1 by the Mann-Whitney test. This is, we used a so called ranker for feature selection. Again there are numerous other feature selection strategies we could have used some examples are given in [M. A. Hall and G. Holmes. IEEE Transactions on Knowledge and Data Engineering, 15(6): 1437-1447, 2003.]. Overall a microRNA, respectively mRNA probe may have been chosen up to eleven times due to LOO cross-validation.
Using only microRNA data we obtain the estimated errors given in Table 7

### Table 7

**Table 7: microRNA data, classification error via leave-one-out cross validation**

| **classifier vs. true** | **colon cancer** | **normal** |
|---|---|---|
| **colon cancer** | 85.7% | 0.0% |
| **normal** | 14.3% | 100.0% |

That is, we observe a sensitivity of 85.7% and a specificity of 100.0%. The positive predictive value is equal to 100.0%, the negative predictive value is equal to 80%. The estimated overall accuracy using LOO cross-validation is 90.9 %. In a second step we used the mRNA data of the HU6800 array. The results can be read off from Table 8. We get an estimated overall accuracy of 72.7 % again using LOO cross-validation. The estimated sensitivity is equal to 85.7%, the estimated specificity is equal to 50%, the estimated positive predictive value is equal to 75.0%, the estimated negative predictive value is equal to 66.7%.

### Table 8

**Table 8: mRNA data, classification error via leave-one-out cross validation**

| **classifier vs. true** | **colon cancer** | **normal** |
|---|---|---|
| **colon cancer** | 85.7% | 50.0% |
| **normal** | 14.3% | 50.0% |

In the last step we combined microRNA and mRNA data and obtained the results given in Table 9. That is, the estimated overall accuracy using cross-validation is 100.0 %. Hence, this combination increases the overall accuracy from 90.9 % respectively, 72.7 % to 100.0 % Likewise sensitivity, specificity, positive predictive value and negative predictive value increase to 100%.

### Table 9

**Table 9: microRNAs and mRNA data, classification error via leave-one-out cross validation**

| **classifier vs. true** | **colon cancer** | **normal** |
|---|---|---|
| **colon cancer** | 100.0% | 0.0% |
| **normal** | 0.0% | 100.0% |

The microRNA probes which were selected during cross-validation are given in Table 10.

### Table 10

**Table 10: microRNA probes selected during leave-one-out cross validation**

| S**eq-ID** | **Probe ID** | **Times selected** | **Probe sequence** |
|---|---|---|---|
| 27 | hsa-miR-1 | 11 | ATACATACTTCTTTACATTCCA |
| 28 | mmu-miR-10b | 11 | ACACAAATTCGGTTCTACAGGG |
| 29 | hsa-miR-195 | 11 | GCCAATATTTCTGTGCTGCTA |
| 30 | hsa-miR-133a | 11 | ACAGCTGGTTGAAGGGGACCAA |
| 31 | hsa-miR-135b | 10 | CACATAGGAATGAAAAGCCATA |
| 32 | hsa-miR-182 | 7 | TGTGAGTTCTACCATTGCCAAA |
| 33 | hsa-miR-30e | 4 | TCCAGTCAAGGATGTTTACA |
| 34 | hsa-miR-99a | 1 | CACAAGATCGGATCTACGGGT |

The results of the Sanger sequence search (see Griffiths-Jones S, Saini HK, van Dongen S, Enright AJ. miRBase: tools for microRNA genomics. NAR 2008 36(Database Issue)-Dl54-D158) for known human microRNAs are given in Table 11

### Table 11.

**Table 11: Results of the Sanger sequence search for known human microRNAs or the microRNA probes selected during 5-fold cross validation**

| **Seq-ID** | **Probe ID** | **microRNA ID** | Target sequence |
|---|---|---|---|
| 35 | hsa-miR-1 | hsa-mir-1 | ACCUACUCAGAGUACAUACUUCUUUAUGUACCCAUAUGAA CAUACAAUGCUAUGGAAUGUAAAGAAGUAUGUAUUUUGG UAGGC |
| 36 | mmu-miR-10b | hsa-mir-10b | CCAGAGGUUGUAACGUUGUCUAUAUAUACCCUGUAGAACC GAAUUUGUGUGGUAUCCGUAUAGUCACAGAUUCGAUUCUA GGGGAAUAUAUGGUCGAUGCAAAAACUUCA |
| 37 | hsa-miR-195 | hsa-mir-195 | AGCUUCCCUGGCUCUAGCAGCACAGAAAUAUUGGCACAGG GAAGCGAGUCUGCCAAUAUUGGCUGUGCUGCUCCAGGCA GGGUGGUG |
| 38 | hsa-miR-133a | hsa-mir-133a | ACAAUGCUUUGCUAGAGCUGGUAAAAUGGAACCAAAUCGC CUCUUCAAUGGAUUUGGUCCCCUUCAACCAGCUGUAGCUA UGCAUUGA |
| 39 | hsa-miR-135b | hsa-mir-135b | CACUCUGCUGUGGCCUAUGGCUUUUCAUUCCUAUGUGAU UGCUGUCCCAAACUCAUGUAGGGCUAAAAGCCAUGGGCUA CAGUGAGGGGCGAGCUCC |
| 40 | hsa-miR-182 | hsa-mir-182 | GAGCUGCUUGCCUCCCCCGUUUUUGGCAAUGGUAGAAC UCACACUGGUGAGGUAACAGGAUCCGGUGGUUCUAGACU UGCCAACUAUGGGGCGAGGACUCAGCCGGCAC |
| 41 | hsa-miR-30e | hsa-mir-30e | GGGCAGUCUUUGCUACUGUAAACAUCCUUGACUGGAAGCU GUAAGGUGUUCAGAGGAGCUUUCAGUCGGAUGUUUACAG CGGCAGGCUGCCA |
| 42 | hsa-miR-99a | hsa-mir-99a | CCCAUUGGCAUAAACCCGUAGAUCCGAUCUUGUGGUGAAG UGGACCGCACAAGCUCGCUUCUAUGGGUCUGUGUCAGUG UG |

The mRNA probes which were selected during cross-validation are given in Table 12. The probe sequences were obtained from Bioconductor package hu6800probe [The Bioconductor Project, www.bioconductor.org (2008). huE800probe: Probe sequence data for microarrays of type hu6800. R package version 2.2.0.].

### Table 12

Miss match (MM) probes are obtained by altering the medium amino acid, more precise A becomes T, T becomes A, G becomes C and C becomes G. The probe sequences each have a lenght of 25, i.e. the respective 13. amino acids are replaced.

**Table 12: mRNA probes selected during leave-one-out cross validation**

| **Seq-ID** | **Affymetrix ID** | **Times selected** | **Probe Sequences (Perfect Match)** |
|---|---|---|---|
| 43-62 | AFFX-HSAC07/X00351_M_at | 11 | [1] AAGATCATTGCTCCTCCTGAGCGCA |
| | | | [2] CCTCCTGAGCGCAAGTACTCCGTGT |
| | | | [3]TGCGTGTGGATCGGCGGCTCCATCC |
| | | | [4]CAGATGTGGATCAGCAAGCAGGAGT |
| | | | [5] GTCCACCGCAAATGCTTCTAGGCGG |
| | | | [6] ACCACGGCCGAGCGGGAAATCGTGC |
| | | | [7] CTGTGCTACGTCGCCCTGGACTTCG |
| | | | [8] GAGCAAGAGATGGCCACGGCTGCTT |
| | | | [9] TCCTCCCTGGAGAAGAGCTACGAGC |
| | | | [10] CTGCCTGACGGCCAGGTCATCAGCA |
| | | | [11] CAGGTCATCACCATTGGCAATGAGC |
| | | | [12] CGGTTCCGCTGCCCTGAGGCACTCT |
| | | | [13] CCTGAGGCACTCTTCCAGCCTTCCT |
| | | | [14] GAGTCCTGTGGCATCCACGAAACTA |
| | | | [15] ATCCAGGAAACTACCTTCAACTCCA |
| | | | [16] AACTCCATCATGAAGTGTGACGTGG |
| | | | [17] GACATGCGCAAAGACCTGTACGCCA |
| | | | [18] AACACAGTGCTGTCTGGCGGCACCA |
| | | | [19] ACCATGTACCCTGGCATTGCCGACA |
| | | | [20] CAGAAGGAGATCACTGCCCTGGCAC |
| 63-81 | X03689_s_at | 10 | [1] AGATTCGGGCAAGTCGACCACTACT |
| | | | [2] TTCGGGCAAGTCCACCACTACTGGC |
| | | | [3] CACCACTACTGGCCATCTGATCTAT |
| | | | [4] CCATCTGATCTATAAATGCGGTGGC |
| | | | [5] TCTGATCTATAAATGCGGTGGCATC |
| | | | [6] TGCCTGGGTCTTGGATAAACTGAAA |
| | | | [7] TGAAAGCTGAGCGTGAACGTGGTAT |
| | | | [8] CGTGAACGTGGTATCAGCATTGATA |
| | | | [9] GAACGTGGTATCACCATTGATATCT |
| | | | [10] GTGGTATCACGATTGATATCTCCTT |
| | | | [11] TATCACCATTGATATCTCCTTGTGG |
| | | | [12] CCATTGATATCTCCTTGTGGAAATT |
| | | | [13]GTACTATGTGACTATCATTGATGCC |
| | | | [14] CTATGTGACTATCATTGATGCCCCA |
| | | | [15] GTCATATCAACATTGTCGTCATTGG |
| | | | [16] TATCAACATTGTCGTCATTGGACAC |
| | | | [17] CATTGTCGTCATTGGACACGTAGAT |
| | | | [18] TGTCGTCATTGGACAGGTAGATTCG |
| | | | [19] CGTCATTGGACACGTAGATTCGGGC |
| 82-101 | AFFX-HSAC071X00351_5_at | 9 | [1] GGGTCAGAAGGATTCCTATGTGGGC |
| | | | [2] GAAGGATTCCTATGTGGGCGACGAG |
| | | | [3] CCCCATCGAGCACGGCATCGTCACG |
| | | | [4] CGTCACCAACTGGGACGACATGGAG |
| | | | [5] CACCTTCTACAATGAGCTGCGTGTG |
| | | | [6] TCCCGAGGAGCACCCCGTGCTGCTG |
| | | | [7] GGCCAACCGCGAGAAGATGACCCAG |
| | | | [8] CCAGATCATGTTTGAGACCTTCAAG |
| | | | [9] CCCAGCCATGTACGTTGCTATCCAG |
| | | | [10] CGTTGCTATCCAGGCTGTGCTATCC |
| | | | [11] GGCTGTGCTATGCCTGTACGCCTCT |
| | | | [12]CGCCTCTGGCCGTACCACTGGCATC |
| | | | [13] TACCACTGGCATCGTGATGGACTCC |
| | | | [14] CGGTGACGGGGTCACCCACACTGTG |
| | | | [15] CCACACTGTGCCCATCTACGAGGGG |
| | | | [15] GCCCATCTACGAGGGGTATGCCCTC |
| | | | [17] TGCCATCCTGCGTCTGGACCTGGCT |
| | | | [18] TGATATCGCCGCGCTCGTCGTCGAC |
| | | | [19] GGTCGTCGACAACGGCTCGGGCATG |
| | | | [20] CGGCTCCGGCATGTGCAAGGCCGGC |
| 102-121 | M 18728 at | 8 | [1] ACCCTCCTAATAGTCATACTAGTAG |
| | | | [2] CTAATAGTCATACTAGTAGTCATAC |
| | | | [3] GTCATACTAGTAGTCATACTCCCTG |
| | | | [4] CTAGTAGTCATACTCCCTGGTGTAG |
| | | | [5] ATGCAGCCAGCCATCAAATAGTGAA |
| | | | [6] TAGTGAATGGTCTCTCTTTGGCTGG |
| | | | [7] TAACCCATGAAGGATAAAAGCCCCA |
| | | | [8] ATAGCACTAATGCTTTAAGATTTGG |
| | | | [9] CTTTAAGATTTGGTCACACTCTCAC |
| | | | [10] GATTGGTCACACTCTCACCTAGGT |
| | | | [11] CATTGAGCCAGTGGTGCTAAATGCT |
| | | | [12] GGTGCTAAATGCTACATACTCCAAC |
| | | | [13] TACATACTCCAACTGAAATGTTAAG |
| | | | [14] CTCCAACTGAAATGTTAAGGAAGAA |
| | | | [15] AACACAGGAGATTCCAGTCTACTTG |
| | | | [16] GCATAATACAGAAGTCCCCTCTACT |
| | | | [17] GTAACCTGAACTAATCTGATGTTAA |
| | | | [18] AATCTGATGTTAACCAATGTATTTA |
| | | | [19]CTGTTTCCTTGTTCCAATTTGACAA |
| | | | [20] GCTATCACTGTACTTGTAGAGTGGT |
| 122-141 | AFFX-HUMGAPDH/M33197_5_at | 7 | [1] GCGCCTGGTCACCAGGGCTGCTTTT |
| | | | [2] GGTCACCAGGGCTGCTTT"fAACTCT |
| | | | [3] TGCTTTTAACTCTGGTAAAGTGGAT |
| | | | [4] GGATATTGTTGCCATCAATGACCCC |
| | | | [5] CATCAATGACCCCTTCATTGACCTC |
| | | | [6] CTTCATTGACCTCAACTACATGGTT |
| | | | [7] CAACTACATGGTTTACATGTTCCAA |
| | | | [8]GGTTTACATGTTCCAATATGATTCC |
| | | | [9] CCAATATGATTCCACCCATGGCAAA |
| | | | [10] TGATTCCACGCATGGCAAATTCGAT |
| | | | [11] ATTCCATGGCACCGTCAAGGGTGAG |
| | | | [12]TGGCACCGTCAAGGCTGAGAACGGG |
| | | | [13] CATCAATGGAAATCCCATCACCATC |
| | | | [14] TCCCATCACCATCTTCCAGGAGCGA |
| | | | [15] CTTCCAGGAGCGAGATCCCTCCAAA |
| | | | [16] GCGAGATCCCTCCAAAATCAAGTGG |
| | | | [17] GGATGCTGGCGGTGAGTACGTCGTG |
| | | | [18] CGTGGAGTCCACTGGC GTCTTCACC |
| | | | [19] CTTCACCACCATGGAGAAGGCTGGG |
| | | | [20] GGGATTTGGTCGTATTGGGCGCCTG |
| 142-161 | X00351_f_at | 6 | [1] TCCTCCTGAGCGCAAGTACTCCGTG |
| | | | [2] TGAGCGGAAGTACTCCGTGTGGATC |
| | | | [3] CTTCCAGCAGATGTGGATCAGCAAG |
| | | | [4] GTGGATCAGCAAGCAGGAGTATGAC |
| | | | [5] CCGCAAATGCTTCTAGGCGGACTAT |
| | | | [6] ATGCTTCTAGGCGGACTATGACTTA |
| | | | [7] T AACTTGCGCAGAAAACAAGA TGAG |
| | | | [8] CAGCAGTCGGTTGGAGCGAGCATCC |
| | | | [9] CAATGTGGCCGAGGACTTTGATTGC |
| | | | [10] GGCCGAGGACTTTGATTGCACATTG |
| | | | [11]TGACGTGGACATCCGCAAAGACCTG |
| | | | [12] GTACGCCAACACAGTGCTGTCTGGC |
| | | | [13] CAACACAGTGGTGTCTGGCGGCAGG |
| | | | [14] GTCTGGCGGCACCACCATGTACCCT |
| | | | [15] CACCATGTACCCTGGGATTGCCGAC |
| | | | [16] GTACCCTGGCATTGCCGACAGGATG |
| | | | [17] TGCCGACAGGATGCAGAAGGAGATC |
| | | | [18] GGAGATCACTGCCCTGGCACCCAGC |
| | | | [19] CCTGGCACCCAGCACAATGAAGATC |
| | | | [20] ACCCAGCACAATGAAGATCAAGATC |
| 162-181 | M77349 at | 5 | [1] TGAAGCACTACAGGAGGAATGCACC |
| | | | [2] AGCTCTCCGCCAATTTGTCTCAGAT |
| | | | [3] AATGTACATGGGGCGCACCATAATG |
| | | | [4] CATGGGCCGCACCATAATGAGATGT |
| | | | [5] CGGCACCATAATGAGATGTGAGCCT |
| | | | [6] TGGCTGTTAACCCACTGCATGCAGA |
| | | | [7] TTAACCCACTGCATGCAGAAACTTG |
| | | | [8] CACTGCATGCAGAAACTfGGATGTC |
| | | | [9] TGGAATTGACTGCCTATGCCAAGTC |
| | | | [10] TGACTGCCTATGCCAAGTCCCTGGA |
| | | | [11] CTCATAAAACATGAATCAAGCAATC |
| | | | [12] GAATCAAGCAATCCAGCCTCATGGG |
| | | | [13] TTGTAAAGCCCTTGCACAGCTGGAG |
| | | | [14] TGCACAGCTGGAGAAATGGCATCAT |
| | | | [15]GCATCATTATAAGCTATGAGTTGAA |
| | | | [16] AATGTTCTGTCAAATGTGTCTCACA |
| | | | [17] AATGTGTCTCACATCTACACGTGGC |
| | | | [18] TCTCACATCTACACGTGGCTTGGAG |
| | | | [19] TTCCCTATTGTGACAGAGCCATGGT |
| | | | [20] ATTGTGACAGAGCCATGGTGTGTTT |
| 182-192 | M34516_r_at | 3 | [1] TTCTCCCTGCACTCATGAAACCCCA |
| | | | [2] TCTCCCTGCACTCATGAAACCCCAA |
| | | | [3] GCACTCATGAAAGCCCAATAAATAT |
| | | | [4] CACTCATGAAACCCCAATAAATATC |
| | | | [5] ACTCATGAAACCCCAATAAATATCC |
| | | | [6] CTCATGAAACCCCAATAAATATCCT |
| | | | [7] TCATGAAACCCCAATAAATATCCTC |
| | | | [8] CATGAAACCCCAATAAATATCCTCA |
| | | | [9] ATGAAACCCCAATAAATATCCTCAT |
| | | | [10] AAACCCCAATAAATATCCTCATTGA |
| | | | [11] AACCCCAATAAATATCCTCATTGAC |
| 193-199 | D49824_s_at | 2 | [1] GGCTGTCCTAGCAGTTGTGGTCATC |
| | | | [2] CTGTCCTAGCAGTTGTGGTCATCGG |
| | | | [3] TGTCCTAGCAGTIGTGGTCATCGGA |
| | | | [4]GTCCTAGCAGTTGTGGTCATCGGAG |
| | | | [5] TCCTAGCAGTTGTGGTCATCGGAGC |
| | | | [6] CTAGCAGTTGTGGTGATCGGAGCTG |
| | | | [7] TAGCAGTTGTGGTCATCGGAGCTGT |
| 220-239 | J03040_at | 1 | [1] GGTTTGCCTGAGGCTGTAACTGAGA |
| | | | [2] CCTGAGGCTGTAACTGAGAGAAAGA |
| | | | [3] ATTCTGGGGGTGTCTTATGAAAATA |
| | | | [4] ATAGACATTCTCACATAAGCCCAGT |
| | | | [5] ACATAAGCCCAGTTCATCACCATTT |
| | | | [6] TCACATTAGGCTGTTGGTTCAAACT |
| | | | [7] GAGCACGGACTGTCAGTTCTCTGGG |
| | | | [8] GGACTGTCAGTTCTCTGGGAAGTGG |
| | | | [9] GAAGTGGTCAGCGCATCCTGCAGGG |
| | | | [10] GTCAGCGCATCCTGCAGGGCTTCTC |
| | | | [11] TTTGGAGAACCAGGGCTCTTCTCAG |
| | | | [12] GAACCAGGGCTCTTCTCAGGGGCTC |
| | | | [13] TTCTCAGGGGCTCTAGGGACTGCCA |
| | | | [14] CTAGGGACTGCCAGGCTGTTTCAGC |
| | | | [15] TTTCAGCCAGGAAGGCCAAAATCAA |
| | | | [16] GGGATGGTCGGATCTCACAGGCTGA |
| | | | [17] GTCGGATCTCACAGGCTGAGAACTC |
| | | | [18] TCTCACAGGCTGAGAACTCGTTCAC |
| | | | [19] CCTCCAAGCATTTCATGAAAAAGCT |
| | | | [20] AGCATTTCATGAAAAAGCTGCTTCT |
| 240-259 | M13560_s_at | 1 | [1] CAGGATCTGGGCCCAGTCCCCATGT |
| | | | [2]GGCCCAGTCCCCATGTGAGAGCAGC |
| | | | [3] CCCATGTGAGAGCAGCAGAGGCGGT |
| | | | [4] AGAGCAGCAGAGGCGGTCTTCAACA |
| | | | [5] ACACAGCTACAGCTTTCTTGCTCCC |
| | | | [6] CAAGACAAACCAAGTCGGAACAGCA |
| | | | [7] CAAGTCGGAACAGCAGATAACAATG |
| | | | [8] TGCCCAATCTCCATCTGTCAACAGG |
| | | | [9] TGAGGTCCCAGGAAGTGGCCAAAAG |
| | | | [10] AGCTAGACAGATCCCCGTTCCTGAC |
| | | | [11] GACATCACAGCAGCCTCCAACACAA |
| | | | [12] CAACACAAGGCTCCAAGACCTAGGC |
| | | | [13] AAGACCTAGGCTCATGGACGAGATG |
| | | | [14] CCAGACCCCAGGCTGGACATGCTGA |
| | | | [15] CCTTTGGCCTTGGCTTTTCTAGCCT |
| | | | [16] TTGGCTTTTCTAGCCTATTTACCTG |
| | | | [17] AGCCTATTTACCTGCAGGCTGAGCC |
| | | | [18] GCTCAGCCAAGCTTGTTATCAGCTT |
| | | | [19] AAGCTTGTTATCAGCTTTCAGGGCC |
| | | | [20] ATCAGCTTTCAGGGCCATGGTTCAC |
| 260-264 | M34516_at | 1 | [1] TCCCTGCACTCATGAAACCCCAATA |
| | | | [2]CCCTGCACTCATGAAACCCCAATAA |
| | | | [3] CCTGCACTCATGAAACCCCAATAAA |
| | | | [4] CTGCACTCATGAAACCCCAATAAAT |
| | | | [5] TGCACTCATGAAACCCCAATAAATA |

The annotations of the selected mRNA probes are given in Table 13. The annotations were obtained from Bioconductor package hu6800.db [Marc Carlson, Seth Falcon, Herve Pages and Nianhua Li (2008). hu6800.db: Affymetrix HuGeneFL Genome Array annotation data (chip hu6800). R package version 2.2.3.] in combination with the information available via PubMed [http://www.ncbi.nlm.nih.gov/pubmed/].

**Table 13: Annotation of mRNA probes selected during LOO cross validation**

| **Seq-ID** | **Affymetrix ID** | **Accession number** | **RefSeq ID** | **Unigene ID** |
|---|---|---|---|---|
| 265 | AFFX-HSAC07/X00351_M_at | X00351 | NM_001101.2 | Hs.520640 Hs.708120 |
| 266 | X03689_s_at | X03689 | NM_001402.2 | Hs.520703 |
| | | | | Hs.586423 |
| | | | | Hs.644639 |
| | | | | Hs.703481 |
| | | | | Hs.708256 |
| 265 | AFFX-HSAC07/X00351_5_at | X00351 | NM_001101.2 | Hs.520640 |
| | | | | Hs.708120 |
| 267 | M18728_at | M 18728 | NM_002483.3 | Hs.466814 |
| 268 | AFFX-HUMGAPDHIM33197_5_at | M33197 | NM_002046.3 | Hs.544577 |
| | | | | Hs.592355 |
| | | | | Hs.711936 |
| 265 | X00351_f_at | X00351 | NM_001101.2 | Hs.520640 |
| | | | | Hs.708120 |
| 269 | M77349_at | M77349 | NM_000358.1 | Hs.369397 |
| | | | | Hs.645734 |
| 270 | M34516_r_at | M34516 | NM_001013618.1 | Hs.449585 |
| 271 | D49824_s_at | D49824 | NM_005514.5 | Hs.77961 |
| | | | | Hs.703277 |
| | | | | Hs.707171 |
| 272 | D00654_at | D00654 | NM_001615.3 | Hs.516105 |
| 273 | HG3044-HT3742_s_at | HG3044-HT3742 | NM_212482.1 | Hs.203717 |
| 274 | J03040_at | J03040 | NM_003118.2 | Hs.111779 |
| | | | | Hs.708558 |
| 275 | M13560_s_at | M13560 | NM_001025159.1 | Hs.436568 |
| 276 | M34516_at | M34516 | NM_020070.2 | Hs.348935 |

### Example 2.2: mRNA and microRNA: Kidney Cancer

We use the kidney cancer data of Ramaswamy et al. (2001) [Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR. Multiclass cancer diagnosis using tumor gene expression signatures. Proc Natl Acad Sci USA. 2001;98(26):15149-54] and Lu et al. (2005) [Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature. 2005;435(7043):834-8] to develop a multilevel classifier using RNA and microRNA data. The data are available from the home page of the Braoad Institute [see http://www.broad.mit.edulpublications/broad900 and http://www.broad.mit.edu/pubiications/broad993s]. Overall the mRNA and microRNA data of three normal tissues and four tumor tissues are available. The hybridisations were done with a bead-based array containing microRNA probes as well as with the Affymetrix HU6800 and HU35KsubA array for measuring the mRNA. We used only the mRNA data of the HU35KsubA arrays.

### Analysis:

For developing and validating a classifier based on these data we used single-hidden-layer neural networks [Ripley, B. D. (1996) Pattern Recognition and Neural Networks. Cambridge] in combination with leave-one-out (LOO) cross-validation where each analysis step - including low level analysis - was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), random forests (RF), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.
The low level analysis (preprocessing) consisted of the variance stabilizing transformation of Huber et al (2002) (often called normalization) in case of the microRNA as well as of the mRNA data. Again there is a large number of alternative methods which could be used. Several examples are given in Cope et al. (2004) or Irizarry et al. (2006). In each cross validation step we selected those six normalized microRNA probes, respectively those six normalized mRNA probes for classification which had the largest differences (in absolute value) of the mean values beyond those probes with p value equal or smaller than 0.1 by the Welch t-test. This is, we used a so called ranker for feature selection. Again there are numerous other feature selection strategies we could have used, some examples are given in Hall et al. (2003). Overall a microRNA, respectively mRNA probe may have been chosen up to seven times due to LOO cross-validation.
Using only microRNA data we obtain the estimated errors given in Table 14

### Table 14

**Table 14: microRNA data, classification error via LOO cross validation**

| **classifier vs. true** | **kidney cancer** | **Normal** |
|---|---|---|
| **kidney cancer** | 50.0% | 66.7% |
| **normal** | 50.0% | 33.3% |

The estimated overall accuracy using LOO cross-validation is 42.9 %, sensitivity is 50%, specificity is 33.3%, positive predictive value is 50% and negative predictive value is 33.3%. In a second step we used the mRNA data of the HU35KsubA array. The results can be read off from Table 15. We get an estimated overall accuracy of 42.9 % again using LOO cross-validation. The estimated values for sensitivity, specificity, positive and negative predictive value are 50%, 33.3%, 50% and 33.3%, respectively.

### Table 15

**Table 15: mRNA data, classification error via LOO cross validation**

| **classifier vs. true** | **kidney cancer** | **Normal** |
|---|---|---|
| **kidney cancer** | 50.0% | 66.7% |
| **normal** | 50.0% | 33.3% |

In the last step we combine microRNA and mRNA data and obtain the results given in Table 16. That is, the estimated overall accuracy using cross-validation is 71.4 %. Hence, this combination increases the overall accuracy from 42.9 % to 71.4 %. Sensitivity, specificity, positive and negative predictive value are increased to 75.0%, 66.7%, 75.0% and 66.7%, respectively.

### Table 16

**Table 16: microRNA and mRNA data, classification error via LOO cross validation**

| **classifier vs. true** | **kidney cancer** | **Normal** |
|---|---|---|
| **kidney cancer** | 75.0% | 33.3% |
| **normal** | 25.0% | 66.7% |

The microRNA probes which were selected during cross-validation are given in Table 17.

### Table 17

**Table 17: microRNA probes selected during LOO cross validation (1^{st} column is SEQ-ID-No)**

| **Seq-ID** | **Probe ID** | **Times selected** | **Probe sequence** |
|---|---|---|---|
| 277 | pre-control 3 | 5 + 5 + 5* | CTGACTGACTGACTGACTGACTG |
| 278 | pre-control 4 | 5 + 1 | TTGTACGTTTAGATGGAGGTC |
| 279 | hsa-let-7b | 4 | AACCACACAACCTACTACCTCA |
| 280 | FVR506 | 4 + 1 | TGTATTCCTCGCCTGTCCAG |
| 281 | hsa-miR-320 | 2 | TCGCCCTCTCAACCCAGCTTTT |
| 282 | hsa-let-7a | 2 | AACTATACAACCTACTACCTCA |
| 283 | hsa-let-7c | 1 | AACCATACAACCTACTACCTCA |
| 284 | hsa-miR-30b | 1 | GCTGAGTGTAGGATGTTTACA |
| 285 | has-miR-10a | 1 | ACACAAATTCGGTTCTACAGGG |
| 286 | PTG20210 | 1 + 1 | CATTGAGGCTGGCTGAGAGT |
| 33 | hsa-miR-30e | 1 | TCCAGTCAAGGATGTTTACA |
| 287 | hsa-miR-339 | 1 | TGAGCTCCTGGAGGACAGGGA |
| 288 | pre-control 5 | 1 | CTTGTACCAGTTATCTGCAA |

| | | | |
|---|---|---|---|
| ** Some probes occur in replicates* | | | |

The results of the Sanger sequence search in accordance with Griffiths-Jones et al. 2008 for known human microRNAs are given in Table 18

### Table 18

**Table 18: Results of the Sanger sequence search for known human microRNAs for microRNA probes selected during LOO cross validation (1^{st} column is SEQ-ID-No)**

| | pre-control 3 | | |
|---|---|---|---|
| 289 | pre-control 4 | hsa-mir-302d | CCUCUACUUUAACAUGGAGGCACUUGCUGUGACAU GACAAAAUAAGUGCUUCCAUGUUUGAGUGUGG |
| 290 | hsa-let-7b | hsa-let-7b | CGGGGUGAGGUAGUAGGUUGUGUGGUUUCAGGGCA GUGAUGUUGCCCCUCGGAAGAUAACUAUACAACCUA CUGCCUUCCCUG |
| 291 | FVR506 | hsa-mir-1238 | GUGAGUGGGAGCCCCAGUGUGUGGUUGGGGCCAU GGCGGGUGGGCAGCCCAGCCUCUGAGCCUUCCUCG UCUGUCUGCCCCAG |
| 292 | hsa-miR-320 | hsa-mir-320a | GCUUCGCUCCCCUCCGCCUUCUCUUCCCGGUUCUU CCCGGAGUCGGGAAAAGCUGGGUUGAGAGGGCGAA AAAGGAUGAGGU |
| 293 | hsa-let-7a | hsa-let-7a | UGGGAUGAGGUAGUAGGUUGUAUAGUUUUAGGGUC ACACCCACCACUGGGAGAUAACUAUACAAUCUACUG UCUUUCCUA |
| 294 | hsa-let-7c | hsa-let-7c | GCAUCCGGGUUGAGGUAGUAGGUUGUAUGGUUUAG AGUUACACCCUGGGAGUUAACUGUACAACCUUCUAG CUUUCCUUGGAGC |
| 295 | hsa-miR-30b | hsa-mir-30b | ACCAAGUUUCAGUUCAUGUAAACAUCCUACACUCAG CUGUAAUACAUGGAUUGGCUGGGAGGUGGAUGUUU ACUUCAGCUGACUUGGA |
| | PTG20210 | | |
| 41 | hsa-miR-30e | hsa-mir-30e | GGGCAGUCUUUGCUACUGUAAACAUCCUUGACUGG AAGCUGUAAGGUGUUCAGAGGAGCUUUCAGUCGGA UGUUUACAGCGGCAGGCUGCCA |
| 21 | hsa-miR-339 | hsa-mir-339 | CGGGGCGGCCGCUCUCCCUGUCCUCCAGGAGCUCA CGUGUGCCUGCCUGUGAGCGCCUCGACGACAGAGC CGGCGCCUGCCCCAGUGUCUGCGC |
| 297 | pre-control 5 | hsa-mir-150 | CUCCCCAUGGCCCUGUCUCCCAACCCUUGUACCAG UGCUGGGCUCAGACCCUGGUACAGGCCUGGGGGAC AGGGACCUGGGGAC |

The mRNA probes which were selected during cross-validation are given in Table 19. The probe sequences were obtained from Bioconductor package hu35ksubaprobe (see The Bioconductor Project, www.bioconductor.org (2008). hu35ksubaprobe: Probe sequence data for microarrays of type hu35ksuba. R package version 2.2.0.).

### Table 19

**Table 19: mRNA probes selected during LOO cross validation**

| **Seq-ID** | **Affymetrix ID** | **Times selected** | **Probe sequence** |
|---|---|---|---|
| 298-313 | AA285290 at | 5 | [1] GGAAAGCGCCGAGATGACGGGCTTT |
| | | | [2] GATGACGGGCTTTCTGCTGCCGCCC |
| | | | [3] CCCAAGTAGCTTTGTGGCTTCGTGT |
| | | | [4] TAGCTTTGTGGCTTCGTGTCCAACC |
| | | | [5] TGTGGCTTCGTGTCCAACCCTCTTG |
| | | | [6] CGCCTGTGTGCCTGGAGCCAGTCCC |
| | | | [7] GCTCGCGTTTCCTCCTGTAGTGCTC |
| | | | [8] GTTTCCTCCTGTAGTGCTGACAGGT |
| | | | [9] AGTGCTCACAGGTCCCAGCACCGAT |
| | | | [10] TCCCAGCACCGATGGCATTCCCTTT |
| | | | [11] TCCCTTTGCCCTGAGTCTGCAGCGG |
| | | | [12] TGCCCTGAGTCTGCAGCGGGTCCCT |
| | | | [13] TCAGGTAGCCTCTCTTCCCCTTGGG |
| | | | [14] ACCCGCGGTAACCAGCGTGAGCTCG |
| | | | [15] GCCCGCCAGAAGAATATGAAAAAGC |
| | | | [16] GACTCGGTTAAGGGAAAGCGGGGAG |
| 314-328 | AA464334_s_at | 4 | [1] TTATGAATGTCCAAATCTGTGTTTC |
| | | | [2] ATGAATGTCCAAATCTGTGTTTCCC |
| | | | [3] GAATGTCCRAATCTGTGTTTCCCCC |
| | | | [4] ATGTCCAAATCTGTGTTTCCCCCTG |
| | | | [5] CTCCCAGACTGTGTGGCCAGTTGAA |
| | | | [6] AGACTGTGTGGCCAGTTGAAAGTGT |
| | | | [7] ACTGTGTGGCCAGTTGAAAGTGTCT |
| | | | [8] TGGCCAGTTGAAAGTGTCTGGTTTG |
| | | | [9] TTGAAAGTGTCTGGTTTGTGTTCAT |
| | | | [10] AGTGTCTGGTTTGTGTTCATCTCTC |
| | | | [11] TGTCTGGTTTGTGTTCATCTCTCCC |
| | | | [12] GTGTTCATCTCTCCCTCATTTCTGG |
| | | | [13] TGCATCCACGCCTCTTTTGGACATT |
| | | | [14] CATCCACGCCTCTTTTGGACATTAA |
| | | | [15]TCCACGCCTCTTTTGGACATTAAAG |
| 329-343 | AA397610_at | 3 | [1] GGTGGCCTTCTTGCAGGTCCCCGTA |
| | | | [2]TGGCCTTCTTGCAGGTCCCCGTAGC |
| | | | [3] GGCCTTCTTGCAGGTCCCCGTAGCA |
| | | | [4] GCCTTCTTGCAGGTCCCCGTAGCAC |
| | | | [5] TCTTGCAGGTCCCCGTAGCACCCTG |
| | | | [6] TGCAGGTCCCCGTAGCACCCTGAGC |
| | | | [7] AGGTCCCCGTAGCACCCTGAGCCTG |
| | | | [8] GGTCCCCGTAGCACCCTGAGCCTGT |
| | | | [9] CCGTAGCACCCTGAGCCTGTACCTT |
| | | | [10]TAGCACCCTGAGCCTGTACCTTGGG |
| | | | [11] CACCCTGAGGCTGTACCTTGGGTGG |
| | | | [12] ACCGTGAGCGTGTACCTTGGGTGGC |
| | | | [13] CCCTGAGCCTGTACCTTGGGTGGCA |
| | | | [14] GAGCCTGTACCTTGGGTGGCACTTG |
| | | | [15] GCCTGTACCTTGGGTGGCACTTGTT |
| 344-359 | RC AA292427 _s_at | 3 | [1] TGCTGCCTCTGGGGACATGCGGAGT |
| | | | [2] GGGGAAGCCTTCCTCTCAATTTGTT |
| | | | [3] GGGAAGCCTTCCTCTCAATTTGTTG |
| | | | [4] GGAAGCCTTCCTCTCAATTTGTTGT |
| | | | [5] GAAGCCTTCCTCTCAATTTGTTGTC |
| | | | [6] AAGCCTTCCTCTCAATTTGTTGTCA |
| | | | [7] AGCCTTCCTCTCAATTTGTTGTCAG |
| | | | [8] CCTTCCTCTCAATTTGTTGTCAGTG |
| | | | [9] CTTCCTCTCAATTTGTTGTCAGTGA |
| | | | [10] TTCCTCTCAATTTGTTGTCAGTGAA |
| | | | [11] TCCTCTCAATTTGTTGTCAGTGAAA |
| | | | [12] CCTCTCAATTTGTTGTCAGTGAAAT |
| | | | [13] CTCTCAATTTGTTGTCAGTGAAATT |
| | | | [14] AATTCCAATAAATGGGATTTGCTCT |
| | | | [15] TGAGGGTGCACGTCTTCCCTCCTGT |
| | | | [16] TGGAGTGCTGCCTCTGGGGACATGC |
| 360-374 | RC_AA465694_r_at | 3 | [1] GGTTAATCCGCAAGCCCCAGCCCCG |
| | | | [2] TTAATCCGCAAGCCCCAGCCCCGAG |
| | | | [3] GGCGTCCCCCAGAGCCTGAGAAAGC |
| | | | [4] CCCCAGAGCCTGAGAAAGCGCCTCC |
| | | | [5] CCAGAGCCTGAGAAAGCGCCTCCCG |
| | | | [6] GAGCCTGAGAAAGCGCCTCCCGCTG |
| | | | [7] GCCTGAGAAAGCGCCTCCCGCTGCC |
| | | | [8] CTGAGAAAGCGCCTCCCGCTGCCCC |
| | | | [9] TGCCCCGACGCGGCCCTCGGCCCTG |
| | | | [10] CTCGGCCCTGGAGCTGAAGGTGGAG |
| | | | [11] CGGCCCTGGAGCTGAAGGTGGAGGA |
| | | | [12] GCCCTGGAGCTGAAGGTGGAGGAGC |
| | | | [13] CCTGGAGCTGAAGGTGGAGGAGCTG |
| | | | [14] GCTGAAGGTGGAGGAGCTGGAGGAG |
| | | | [15] AAGGTGGAGGAGCTGGAGGAGAAGG |
| 375-390 | AA422123_f_at | 2 | [1] GACTGCTTGAAACCAGGAGTTTGAG |
| | | | [2] GCTTGAAACCAGGAGTTTGAGACCA |
| | | | [3] AACCAGGAGTTTGAGACCAGCCTGA |
| | | | [4] TTGAGAGCAGCCTGAGCAACAAAGC |
| | | | [5] AGACCAGCCTGAGCAACAAAGCAAG |
| | | | [6] GAGCAACAAAGCAAGACCCCATCTC |
| | | | [7] CAACAAAGCAAGACCCCATCTCTAT |
| | | | [8] AAGCAAGACCCCATCTCTATAAAAA |
| | | | [9] AAGACAGGGTCTTGCTCATGTTGTA |
| | | | [10] ATTAGTTGGGCATGGTGGCACATGC |
| | | | [11] AGTTGGGCATGGTGGCACATGCCTG |
| | | | [12]ATCATCTGAGCCTCAGGAGGTTGAG |
| | | | [13] ATCTGAGCCTCAGGAGGTTGAGGCT |
| | | | [14] TGAGGCTGCAGTGAGCTGTGACTGC |
| | | | [15] CTTGCTCATGTTGTACATTCATCAT |
| | | | [16] AAGAGGCTGGGTGCAGTGGCTCACA |
| 391-410 | AFFX-HUMGAPDH/M33197_3 _at | 2 | [1] TCATTTCCTGGTATGACAACGAATT |
| | | | [2] ACAACGAATTTGGCTACAGCAACAG |
| | | | [3] GGGTGGTGGACCTCATGGCCCACAT |
| | | | [4] TCATGGCCCACATGGCCTCCAAGGA |
| | | | [5] ACATGGCCTCCAAGGAGTAAGACCC |
| | | | [6] AGGAGTAAGACCCCTGGACCACCAG |
| | | | [7] GCCCCAGCAAGAGCACAAGAGGAAG |
| | | | [8] GAGAGAGACCCTCACTGCTGGGGAG |
| | | | [9] CCTCACTGCTGGGGAGTCCCTGCCA |
| | | | [10]CCTCCTCACAGTTGCCATGTAGACC |
| | | | [11] AGTTGCCATGTAGACCCCTTGAAGA |
| | | | [12] CATGTAGACCCCTTGAAGAGGGGAG |
| | | | [13] TAGGGAGCCGCACGTTGTCATGTAC |
| | | | [14] GCCGCACCTTGTCATGTACCATCAA |
| | | | [15] TGTCATGTACCATCAATAAAGTACC |
| | | | [16] CCTCTGACTTCAACAGCGACACCCA |
| | | | [17] GGGCTGGCATTGCCCTCAACGACCA |
| | | | [18] CCCTCAACGACCACTTTGTCAAGCT |
| | | | [19] ACCACTTTGTCAAGCTCATTTCCTG |
| | | | [20] TTGTCAAGCTCATTTCCTGGTATGA |
| 411-426 | RC_AA130645_s_at | 2 | [1] GAATTCTGGTACCGTCAGCATCCAC |
| | | | [2] GAGAGAGACCTCATCTTTCATGCTT |
| | | | [3]TGACTCTCCTGGGGGCACCTCCTAT |
| | | | [4] ACTCTCCTGGGGGCACCTCCTATGA |
| | | | [5] TCCTGGGGGCACCTCCTATGAGAGA |
| | | | [6] CCTGGGGGCACCTCCTATGAGAGAT |
| | | | [7] CTGGGGGCACCTCCTATGAGAGATA |
| | | | [8] TGGGGGCACCTCCTATGAGAGATAC |
| | | | [9] GGGGGCACCTCCTATGAGAGATACG |
| | | | [10] GGGGCACCTCCTATGAGAGATACGA |
| | | | [11] GGGCACCTCCTATGAGAGATACGAT |
| | | | [12] GGCACCTCCTATGAGAGATACGATT |
| | | | [13] GCACCTCCTATGAGAGATACGATTG |
| | | | [14] CACCTCCTATGAGAGATACGATTGC |
| | | | [15] ACCTCCTATGAGAGATACGATTGCT |
| | | | [16]CCTCCTATGAGAGATACGATTGCTA |
| 427-442 | RC_AA236365_s_at | 2 | [1] CTCCTATTCCGGACTCAGACCTCTG |
| | | | [2] TCCTATTCCGGACTCAGACCTCTGA |
| | | | [3] CCTATTCCGGACTCAGACCTCTGAC |
| | | | [4] CTATTCCGGACTCAGACCTCTGACC |
| | | | [5] ATTCCGGACTCAGACCTCTGACCCT |
| | | | [6] TTCCGGACTCAGACCTCTGACCCTG |
| | | | [7]CGGACTCAGACCTCTGACCCTGCAA |
| | | | [8] GGACTCAGACCTCTGACCCTGCAAT |
| | | | [9] ACTCAGACCTCTGACCCTGCAATGC |
| | | | [10] CAGACCTCTGACCCTGCAATGCTGC |
| | | | [11]ACCTCTGACCCTGCAATGCTGCCTA |
| | | | [12] TCTGACCCTGCAATGCTGCCTACCA |
| | | | [13] CTGACCCTGCAATGCTGCCTACCAT |
| | | | [14] TGACCCTGCAATGCTGCCTACCATG |
| | | | [15] ACCCTGCAATGCTGCCTACCATGAT |
| | | | [16] CCTGCAATGCTGCCTACCATGATTG |
| 443-458 | RC_AA304344_f_at | 2 | [1] AGGCACGTACCACCATGCCCAGATA |
| | | | [2] TTTTTTGAGACAAAGTCCTCACTCT |
| | | | [3] GGGGTTTCACCATGTTGGCTAGGAT |
| | | | [4] CCATGTTGGCTAGGATGGTCTCCAT |
| | | | [5] GTTGGCTAGGATGGTCTCCATCGCC |
| | | | [6] CTAGGATGGTCTCCATCGCCTGACC |
| | | | [7]TGAGACAAAGTCCTCACTCTGTCAC |
| | | | [8] CTTGGCCTCCCAAAGTGCTGGGATT |
| | | | [9] CCTCCCAAAGTGCTGGGATTACAGG |
| | | | [10] GGATTACAGGCATGAGCCACCACAG |
| | | | [11] CAAAGTCCTCACTCTGTCACCAAGT |
| | | | [12] GCATGAGCCACCACAGCTGGCCGTA |
| | | | [13] GAGCCACCACAGCTGGCCGTAAATA |
| | | | [14] GTGCAGTGGCAGCAATCTCAGCTCA |
| | | | [15] GTGGCAGCAATCTCAGCTCACTGCA |
| | | | [16] AGCAATCTCAGCTCACTGCAAACCT |
| 459-473 | T89571_f at | 2 | [1] CACCGCGCCTGGCCCTAAATAGATT |
| | | | [2] GGGATTCATCATGTTGACCAGGCTG |
| | | | [3] TTCATCATGTTGACCAGGCTGGCCT |
| | | | [4] TGTTTGTCTTTCTGATAGGTTGAAA |
| | | | [5] TGTCTTTCTGATAGGTTGAAAATTG |
| | | | [6] GTTGACCAGGCTGGCCTCAAACTCC |
| | | | [7] ACCAGGCTGGCCTCAAACTCCTGAC |
| | | | [8] AGGCTGGCCTCAAACTCCTGACTTC |
| | | | [9] TGGCCTCAAACTCCTGACTTCAAGC |
| | | | [10] CTCAAACTCCTGACTTCAAGCGATC |
| | | | [11] AAACTCCTGACTTCAAGCGATCTCC |
| | | | [12] TTGGCCTCCCAAAGTGCTGGGATTG |
| | | | [13] CCTCCCAAAGTGCTGGGATTGCAGG |
| | | | [14] GCTGGGATTGCAGGTGTGAGCCACC |
| | | | [15] ATTGCAGGTGTGAGCCACCGCGCCT |
| 474-493 | AFFX-HSAC07/x00351_3_at | 1 | [1] TCTTGACAAAACCTAACTTGCGCAG |
| | | | [2] ATGAGATTGGCATGGCTTTATTTGT |
| | | | [3] GCAGTCGGTTGGAGCGAGCATCCCC |
| | | | [4] CCAAAGTTCACAATGTGGCCGAGGA |
| | | | [5] AAGTTCACAATGTGGCCGAGGACTT |
| | | | [6] ATGTGGCCGAGGACTTTGATTGCAC |
| | | | [7] CCGAGGACTTTGATTGCACATTGTT |
| | | | [8] TTTAATAGTCATTCCAAATATGAGA |
| | | | [9] AGTCATTCCAAATATGAGATGCATT |
| | | | [10] TGTTACAGGAAGTCCCTTGCCATCC |
| | | | [11] TACAGGAAGTCCCTTGCCATCCTAA |
| | | | [12]TCCCTTGCCATCCTAAAAGCCACCC |
| | | | [13] CTTCTCTCTAAGGAGAATGGCCCAG |
| | | | [14] GAGGTGATAGCATTGCTTTCGTGTA |
| | | | [15] TATTTTGAATGATGAGCCTTCGTGC |
| | | | [16] TTTGAATGATGAGCCTTCGTGCCCC |
| | | | [17] GTATGAAGGCTTTTGGTCTCCCTGG |
| | | | [18] GGTGGAGGCAGCCAGGGCTTACCTG |
| | | | [19] CAGGGCTTACCTGTACACTGACTTG |
| | | | [20] TTACCTGTACACTGACTTGAGACCA |
| 494-562 | hum_alu_at | 1 | [1] GCCTGGCCAACATGGTGAAACCCCG |
| | | | [2] GCGCGCGCCTGTAATCCCAGCTACT |
| | | | [3] GCGCGCCTGTAATCCCAGCTACTCG |
| | | | [4] CGCGCCTGTAATCCCAGCTACTCGG |
| | | | [5] GCGCCTGTAATCCCAGCTACTCGGG |
| | | | [6] CGCCTGTAATCCCAGCTACTCGGGA |
| | | | [7] GCCTGTAATCCCAGCTACTCGGGAG |
| | | | [8] CCTGTAATCCCAGCTACTCGGGAGG |
| | | | [9] CTGTAATCCCAGCTACTCGGGAGGC |
| | | | [10]TGTAATCCCAGCTACTCGGGAGGCT |
| | | | [11] GTAATCCCAGCTACTCGGGAGGCTG |
| | | | [12] TAATCCCAGCTACTCGGGAGGCTGA |
| | | | [13] AATCCCAGCTACTCGGGAGGCTGAG |
| | | | [14] ATCCCAGCTACTCGGGAGGCTGAGG |
| | | | [15] TCCCAGCTACTCGGGAGGCTGAGGC |
| | | | [16] CCCAGCTACTCGGGAGGCTGAGGCA |
| | | | [17] CCAGCTACTCGGGAGGCTGAGGCAG |
| | | | [18] TGGTGGCTCACGCCTGTAATCCCAG |
| | | | [19] GAGCCGAGATCGCGCCACTGCACTC |
| | | | [20] GTGGCTCACGCCTGTAATCCCAGCA |
| | | | [21] CACTGCACTCCAGCCTGGGCGACAG |
| | | | [22] ACTGCACTCCAGCCTGGGCGACAGA |
| | | | [23] CTGCACTCCAGCCTGGGCGACAGAG |
| | | | [24] TGCACTCCAGCCTGGGCGACAGAGC |
| | | | [25] GCACTCCAGCCTGGGCGACAGAGCG |
| | | | [26] CACTCCAGCCTGGGCGACAGAGCGA |
| | | | [27] TGGCTCACGCCTGTAATCCCAGCAC |
| | | | [28] ACTCCAGCCTGGGCGACAGAGCGAG |
| | | | [29] CTCCAGCCTGGGCGACAGAGCGAGA |
| | | | [30] TCCAGCCTGGGCGACAGAGCGAGAC |
| | | | [31] CCAGCCTGGGCGACAGAGCGAGACT |
| | | | [32] CAGCCTGGGCGACAGAGCGAGACTC |
| | | | [33] AGCCTGGGCGACAGAGCGAGACTCC |
| | | | [34] GGCTCACGCCTGTAATCCCAGCACT |
| | | | [35] GCTCACGCCTGTAATCCCAGCACTT |
| | | | [36] CTCACGCCTGTAATCCCAGCACTTT |
| | | | [37]TCACGCCTGTAATCCCAGCACTTTG |
| | | | [38] CACGCCTGTAATCCCAGCACTTTGG |
| | | | [39] ACGCCTGTAATCCCAGCACTTTGGG |
| | | | [40] CGCCTGTAATCCCAGCACTTTGGGA |
| | | | [41] GCCTGTAATCCCAGCACTTTGGGAG |
| | | | [42] CCTGTAATCCCAGCACTTTGGGAGG |
| | | | [43] CTGTAATCCCAGCACTTTGGGAGGG |
| | | | [44] TGTAATCCCAGCACTTTGGGAGGCC |
| | | | [45] GTAATCCCAGCACTTTGGGAGGCCG |
| | | | [46] TAATCCCAGCACTTTGGGAGGCCGA |
| | | | [47] AATCCCAGCACTTTGGGAGGCCGAG |
| | | | [48] ATCCCAGCACTTTGGGAGGCCGAGG |
| | | | [49] TCCCAGCACTTTGGGAGGCCGAGGT |
| | | | [50] CCCAGCACTTTGGGAGGCCGAGGTG |
| | | | [51] GTGGATCACCTGAGGTCAGGAGTTC |
| | | | [52] GGATCACCTGAGGTCAGGAGTTCAA |
| | | | [53] GATCACCTGAGGTCAGGAGTTCAAG |
| | | | [54] ATCACCTGAGGTCAGGAGTTCAAGA |
| | | | [55] TCACCTGAGGTCAGGAGTTCAAGAC |
| | | | [56] AGGAGTTCAAGACCAGCCTGGCCAA |
| | | | [57] GGAGTTCAAGACCAGCCTGGCCAAC |
| | | | [58] GAGTTCAAGACCAGCCTGGCCAACA |
| | | | [59] AGTTCAAGACCAGCCTGGCCAACAT |
| | | | [60] GTTCAAGACCAGCCTGGCCAACATG |
| | | | [61] TTCAAGACCAGCCTGGCCAACATGG |
| | | | [62] TCAAGACCAGCCTGGCCAACATGGT |
| | | | [63] CAAGACCAGCCTGGCCAACATGGTG |
| | | | [64] AAGACCAGCCTGGCCAACATGGTGA |
| | | | [65] AGACCAGCCTGGCCAACATGGTGAA |
| | | | [66] GACCAGCCTGGCCAACATGGTGAAA |
| | | | [67] ACCAGCCTGGCCAACATGGTGAAAC |
| | | | [68] CCAGCCTGGCCAACATGGTGAAACC |
| | | | [69] CAGCCTGGCCAACATGGTGAAACCC |
| 563-578 | R69648_at | 1 | [1] TAGAATTCTGTGCAGATGTCCTGAC |
| | | | [2] AATTCTGTGCAGATGTCCTGACTTG |
| | | | [3] TGACTTGGCAATTTTGTGTCCCTGC |
| | | | [4] GGCAATTTTGTGTCCCTGCCTCACT |
| | | | [5] GTCCTAGTGTTGTTCTGCCTCCTGT |
| | | | [6]TTGTTCTGCCTCCTGTCCTCTCTTG |
| | | | [7]CTGTCCTCTCTTGCTCTCTTGTCAG |
| | | | [8]GCTCTCTTGTCAGTCTCTGGCTTCC |
| | | | [9] GTCTCTGGCTTCCTCGGCCCCATTT |
| | | | [10] GGCCCCATTTCACTTCACTGAGTCC |
| | | | [11] CCCATTTCACTTCACTGAGTCCTGA |
| | | | [12] TCACTTCACTGAGTCCTGACACCCA |
| | | | [13] AAGGGTCTGTTCTGCTCAGCTCCAT |
| | | | [14] TGCTCAGCTCCATGTCCCCCATTTT |
| | | | [15] TTTACAGCATCCTGCACTCCAGCCT |
| | | | [16]TCCTCCACAATAAAACTGGGGACTG |
| 579-593 | RC AA232686 _s_at | 1 | [1] GCTGAGGCTCCCTTGCCTGACTGTG |
| | | | [2] GAGGCTCCCTTGCCTGACTGTGACT |
| | | | [3] GGCTCCCTTGCCTGACTGTGACTTG |
| | | | [4] GCTCCCTTGCCTGACTGTGACTTGT |
| | | | [5] CTCCCTTGCCTGACTGTGACTTGTG |
| | | | [6] TCCCTTGCCTGACTGTGACTTGTGC |
| | | | [7]CCCTTGCCTGACTGTGACTTGTGCC |
| | | | [8] CCTTGCCTGACTGTGACTTGTGCCT |
| | | | [9] CTTGCCTGACTGTGACTTGTGCCTC |
| | | | [10] CTGACTGTGACTTGTGCCTCTCTCC |
| | | | [11] TGACTGTGACTTGTGCCTCTCTCCT |
| | | | [12]GACTGTGACTTGTGCCTCTCTCCTG |
| | | | [13] CTGTGACTTGTGCCTCTCTCCTGCC |
| | | | [14] GGTGGGCAGGTGACCCAAGGAACCT |
| | | | [15] CAGGTGACCCAAGGAACCTTTCTGG |
| 594-609 | RC_AA417588_at | 1 | [1] TGAAGGTACTGAACGCCACCTCACT |
| | | | [2] AGGTACTGAACGCCACCTCACTGTA |
| | | | [3] GTACTGAACGCCACCTCACTGTAAG |
| | | | [4] TGAACGCCACCTCACTGTAAGACGG |
| | | | [5] AACGCCACCTCACTGTAAGACGGTA |
| | | | [6] ACGCCACCTCACTGTAAGACGGTAG |
| | | | [7] GCCACCTCACTGTAAGACGGTAGAT |
| | | | [8] CCACCTCACTGTAAGACGGTAGATT |
| | | | [9] ACCTCACTGTAAGACGGTAGATTTT |
| | | | [10] CCTCACTGTAAGACGGTAGATTTTG |
| | | | [11] TCACTGTAAGACGGTAGATTTTGTA |
| | | | [12] GACAGGGCTGCCTTCTGGGTGATGA |
| | | | [13] ACAGGGCTGCCTTCTGGGTGATGAG |
| | | | [14] AGGGCTGCCTTCTGGGTGATGAGAA |
| | | | [15] AATCAGATGGGATGGCTGCACGGCG |
| | | | [16] CTGCACGGCGTGGTGAAGGTACTGA |
| 610-624 | RC_AA459310_r_at | 1 | [1] CTGCAGTTCATGTCCCCCGCCAGGC |
| | | | [2] CCCCGCCAGGCCTCGAGGCTCAGGG |
| | | | [3] CGCCAGGCCTCGAGGCTCAGGGTGG |
| | | | [4] GCCTCGAGGCTCAGGGTGGGAGAGG |
| | | | [5] GAGGCTCAGGGTGGGAGAGGGCCCC |
| | | | [6]GCTCAGGGTGGGAGAGGGCCCCGGG |
| | | | [7] CCCCGGGCTGCCCTGTCACTCCTCT |
| | | | [8] CGGGCTGCCCTGTCACTCCTCTAAC |
| | | | [9] GCTGCCCTGTCACTCCTCTAACACT |
| | | | [10] CCTGTCACTCCTCTAACACTTCCCT |
| | | | [11] TCACTCCTCTAACACTTCCCTCCCG |
| | | | [12] CTCCTCTAACACTTCCCTCCCGTGT |
| | | | [13] CCCCAACATGCCCTGTAATAAAATT |
| | | | [14] CAACATGCCCTGTAATAAAATTAGA |
| | | | [15] CATGCCCTGTAATAAAATTAGAGAA |
| 625-639 | RC_AA496904_at | 1 | [1] TAGAATGACCCTTGGGAACAGTGAA |
| | | | [2] GACCCTTGGGAACAGTGAACGTAGA |
| | | | [3] TTTAGCAGAGTTTGTGACCAAAGTC |
| | | | [4] GCTCTGGCTGCCTTCTGCATTTATT |
| | | | [5] GCTGCCTTCTGCATTTATTTGCCTT |
| | | | [6] GCCTTGGCCTGTTGTCTTCCCCTAT |
| | | | [7] GCCTGTTGTCTTCCCCTATTTTCTG |
| | | | [8] TGTCTTCCCCTATTTTCTGTCCCAG |
| | | | [9] CTATTTTCTGTCCCAGCTCATCCGT |
| | | | [10] TTTTCTGTCCCAGCTCATCCGTGTC |
| | | | [11]TCTGTCCCAGCTCATCCGTGTCTCT |
| | | | [12] GTCCCAGCTCATCCGTGTCTCTGAA |
| | | | [13]CCAGCTCATCCGTGTCTCTGAAGAA |
| | | | [14] GCTCATCCGTGTCTCTGAAGAACAA |
| | | | [15] CCGTGTCTCTGAAGAACAAATATGC |
| 640-654 | RC_D59847_at | 1 | [1] TTGCCACCCTGAGCACTGCCCGGAT |
| | | | [2] GGATCCCGTGCACCCTGGGACCCAG |
| | | | [3] TCCCGTGCACCCTGGGACCCAGAAG |
| | | | [4] CGTGCACCCTGGGACCCAGAAGTGC |
| | | | [5] CCGCCAGCACGTCCAGAGCAACTTA |
| | | | [6] GCCAGCACGTCCAGAGCAACTTACC |
| | | | [7] AGCACGTCCAGAGCAACTTACCCCG |
| | | | [8]GCACGTCCAGAGCAACTTACCCCGG |
| | | | [9]CCGTGCCGCCGACCACGATGTGGGC |
| | | | [10] CGTGCCGCCGACCACGATGTGGGCT |
| | | | [11]TGCCGCCGACCACGATGTGGGCTCT |
| | | | [12] CGCCGACCACGATGTGGGCTCTGAG |
| | | | [13] GACCACGATGTGGGCTCTGAGCTGC |
| | | | [14] CACGATGTGGGCTCTGAGCTGCCCC |
| | | | [15] TGTGAAACGCCTAGAGACCCCGGCG |
| 655-669 | RC _D60607_at | 1 | [1] TCACAGCCCCGTTCAGCTGGTGGCT |
| | | | [2] CCCCGTTCAGCTGGTGGCTTTTAGA |
| | | | [3] TTTTAGAGGCTTCCAGAGTGTGCTT |
| | | | [4] CCAGAGTGTGCTTGGCCCCTTTACC |
| | | | [5] TGGCCCCTTTACCTCTATGCCATTG |
| | | | [6] CTCTATGCCATTGGGCCCAGGGGGA |
| | | | [7] CCTTTCTGTGTCTTGCTTGCCCCGT |
| | | | [8] TGTGTCTTGCTTGCCCCGTGTCTCC |
| | | | [9] TTGCTTGCCCCGTGTCTCCCAGTGA |
| | | | [10] GCCCCGTGTCTCCCAGTGAGTGGCC |
| | | | [11] TGTCTCCCAGTGAGTGGCCGCCCTG |
| | | | [12] CGGACAAGTCGCAGCCTCAGGGGGA |
| | | | [13] AGTCGCAGCCTCAGGGGGACCTCCC |
| | | | [14] CTGGCACTGCATCTTTCTGGGCCTG |
| | | | [15] CTTTCTGGGCCTGGCTCTGCTGCCT |
| 670-684 | T30851_i_at | 1 | [1] CAGAGTTATAAGCCCCAAACAGGTC |
| | | | [2] AGAGTTATAAGCCCCAAACAGGTCA |
| | | | [3] GAGTTATAAGCCCCAAACAGGTCAT |
| | | | [4] AGTTATAAGCCCCAAACAGGTCATG |
| | | | [5] GTTATAAGCCCCAAACAGGTCATGC |
| | | | [6] TTATAAGCCCCAAACAGGTCATGCT |
| | | | [7] TATAAGCCCCAAACAGGTCATGCTC |
| | | | [8] ATAAGCCCCAAACAGGTCATGCTCC |
| | | | [9] TAAGCCCCAAACAGGTCATGCTCCA |
| | | | [10] AAGCCCCAAACAGGTCATGCTCCAA |
| | | | [11] AGCCCCAAACAGGTCATGCTCCAAT |
| | | | [12] GCCCCAAACAGGTCATGCTCCAATA |
| | | | [13] CCCCAAACAGGTCATGCTCCAATAA |
| | | | [14] CCCAAACAGGTCATGCTCCAATAAA |
| | | | [15] CCAAACAGGTCATGCTCCAATAAAA |
| 685-700 | T80746_s_at | 1 | [1] CTTGCAACCTCCGGGACCATCTTCT |
| | | | [2] GCAACCTCCGGGACCATCTTCTCGG |
| | | | [3] GCTTCTGGGACCTGCCAGCACCGTT |
| | | | [4] GGGACCTGCCAGCACCGTTTTTGTG |
| | | | [5] TGCCAGCACCGTTTTTGTGGTTAGC |
| | | | [6] CAGCACCGTTTTTGTGGTTAGCTCC |
| | | | [7] TTGCCAACCAACCATGAGCTCCCAG |
| | | | [8] GCCAACCAACCATGAGCTCCCAGAT |
| | | | [9] AACCAACCATGAGCTCCCAGATTCG |
| | | | [10] CCATGAGCTCCCAGATTCGTCAGAA |
| | | | [11] TGAGCTCCCAGATTCGTCAGAATTA |
| | | | [12] GCTCCCAGATTCGTCAGAATTATTC |
| | | | [13] CCCAGATTCGTCAGAATTATTCCAC |
| | | | [14] GATTCGTCAGAATTATTCCACCGAC |
| | | | [15] TCGTCAGAATTATTCCACCGACGTG |
| | | | [16] TCAGAATTATTCCACCGACGTGGAG |
| 701-716 | X01677_s_at | 1 | [1] ACTGGCATGGCCTTCCGTGTCCCCA |
| | | | [2] CCACTGCCAACGTGTCAGTGGTGGA |
| | | | [3] ACTGCCAACGTGTCAGTGGTGGACC |
| | | | [4] TGCCAACGTGTCAGTGGTGGACCTG |
| | | | [5] CCAACGTGTCAGTGGTGGACCTGAC |
| | | | [6] CGTGTCAGTGGTGGACCTGACCTGC |
| | | | [7] GTCAGTGGTGGACCTGACCTGCCGT |
| | | | [8] CAGTGGTGGACCTGACCTGCCGTCT |
| | | | [9] GTGGTGGACCTGACCTGCCGTCTAG |
| | | | [10] GGTGGACCTGACCTGCCGTCTAGAA |
| | | | [11] GACCTGACCTGCCGTCTAGAAAAAC |
| | | | [12]CTGACCTGCCGTCTAGAAAAACCTG |
| | | | [13] GACCTGCCGTCTAGAAAAACCTGCC |
| | | | [14] TGCCGTCTAGAAAAACCTGCCAAAT |
| | | | [15] CCGTCTAGAAAAACCTGCCAAATAT |
| | | | [16] GTCTAGAAAAACCTGCCAAATATGA |

The annotations of the selected mRNA probes are given in Table 20. The annotations were obtained from Bioconductor package hu35ksuba.db (Marc Carlson, Seth Falcon, Herve Pages and Nianhua Li (2008). hu35ksuba.db: Affymetrix Human Genome HU35K Set annotation data (chip hu35ksuba). R package version 22.3.) in combination with the information available via PubMed [http://www.ncbi.nlm.nih.gov/pubmedl].

### Table 20

**Table 20: Annotation of mRNA probes selected during LOO cross validation (1^{st} column is SEQ-ID-No)**

| | | | | |
|---|---|---|---|---|
| 268 | X01677_s_at | X01677 | NM_002046.3 | Hs.544577 |

### Example2.3: mRNA and microRNA, prostate cancer

We use the prostate cancer data of Ramaswamy et al. (2001) [Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR. Multiclass cancer diagnosis using tumor gene expression signatures. Proc Natl Acad Sci USA. 2001;98(26):15149-54] and Lu et al. (2005) [Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature. 2005;435(1043):834-8] to develop a multilevel classifier using mRNA and microRNA data. The data are available from the home page of the Braoad Institute[see http://www.broad.mit.edu/publications/broad900 and http://www.broad.mit.edu/publications/broad993s]. Overall the mRNA and microRNA data of six normal tissues and six tumor tissues are available. The hybridisations were done with a bead-based array containing microRNA probes as well as with the Affymetrix HU6800 and HU35KsubA array for measuring the mRNA. We used only the mRNA data of the HU6800 arrays.

### Analysis:

For developing and validating a classifier based on these data we used linear discriminant analysis in combination with leave-one-out (LOO) cross-validation where each analysis step - including low level analysis - was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), random forests (RF), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.
The low level analysis consisted of the variance stabilizing transformation of Huber et al (2002) (often called normalization) in case of the microRNA as well as of the mRNA data. Again there is a large number of alternative methods which could be used. Several examples are given in Cope et al. (2004) or Irizarry et al. (2006) In each cross validation step we selected those two normalized microRNA probes, respectively those four normalized mRNA probes for classification which had the largest median of pairwise differences (in absolute value) beyond those microRNA probes with p value equal or smaller than 0.01 by the Mann-Whitney test. This is, we used a so called ranker for feature selection. Again there are numerous other feature selection strategies we could have used, some examples are given in Hall et al. 2003. Overall a microRNA, respectively RNA probe may have been chosen up to twelve times due to LOO cross-validation.
Using only microRNA data we obtain the estimated errors given in Table 21

### Table 21

**Table 21: microRNA data, classification error via LOO cross validation**

| **classifier vs. true** | **prostate cancer** | **Normal** |
|---|---|---|
| **prostate cancer** | 83.3% | 0.0% |
| **normal** | 16.7% | 100.0% |

The estimated overall accuracy using LOO cross-validation is 91.7 %. Sensitivity, specificity, positive and negative predictive value are 83.3%, 100%, 100% and 85.7%, respectively. In a second step we used the mRNA data of the HU6800 array. The results can be read off from Table 22. We get an estimated overall accuracy of 75.0 % again using LOO cross-validation.

Sensitivity, specificity, positive and negative predictive value are 83.3%, 66.7%, 71.4% and 80.0%, respectively.

### Table 22

**Table 22: mRNA data, classification error via LOO cross validation**

| **classifier vs. true** | **prostate cancer** | **Normal** |
|---|---|---|
| **prostate cancer** | 83.3% | 33.3% |
| **normal** | 16.7% | 66.7% |

In the last step we combine microRNA and mRNA data and obtain the results given in Table 23. That is, the estimated overall accuracy using cross-validation is 91.7 %. Sensitivity, specificity, positive and negative predictive value are 100.0%, 83.3%, 85.7% and 100.0%, respectively. Hence, this combination increases the sensitivity (correct classification of cancer samples) from 83.3 % to 100.0 % and negative predictive value form 85.7%, respectively 80.0% to 100.0%.

### Table 23

**Table 23: microRNA and mRNA data, classification error via LOO cross validation**

| **classifier vs. true** | **prostate cancer** | **Normal** |
|---|---|---|
| **prostate cancer** | 100.0% | 16.7% |
| **normal** | 0.0% | 83.3% |

The microRNA probes which were selected during cross-validation are given in Table 24.

### Table 24

**Table 24: microRNA probes selected during LOO cross validation (1^{st} column is SEQ-ID-No)**

| | | | |
|---|---|---|---|
| 735 | hsa-miR-206 | 2 | CCACACACTTCCTTACATTCCA |

The results of the Sanger sequence search according to Griffiths-Jones et al. (2008) for known human microRNAs are given in Table 25

### Table 25

**Table 25: Results of the Sanger sequence search for known human microRNAs for microRNA probes selected during LOO cross validation (1^{st} column is SEQ-ID-No)**

| | | | |
|---|---|---|---|
| 738 | hsa-miR-206 | hsa-mir-206 | UGCUUCCCGAGGCCACAUGCUUCUUUAUAUCCCCAUA UGGAUUACUUUGCUAUGGAAUGUAAGGAAGUGUGUG GUUUCGGCAAGUG |

The mRNA probes which were selected during cross-validation are given in Table 26. The probe sequences were obtained from Bioconductor package hu6800probe [The Bioconductor Project, www.bioconductor.org (2008). hu6800probe: Probe sequence data for microarrays of type hu6800. R package version 2.2.0].

### Table 26

**Table 26: mRNA probes selected during LOO cross validation**

| | | | |
|---|---|---|---|
| 833-852 | S82297 at | 2 | [1] GCTATCCAGCATTCAGGTTTACTCA |
| | | | [2]ATCCTGAAGCTGACAGCATTCGGGC |
| | | | [3] CCTGAAGCTGACAGCATTCGGGCCG |
| | | | [4] AAGCTGACAGCATTCGGGCCGAGAT |
| | | | [5] GCTGACAGCATTCGGGCCGAGATGT |
| | | | [6] TGACAGCATTCGGGCCGAGATGTCT |
| | | | [7] CATTCGGGCCGAGATGTCTCGCTCC |
| | | | [8] GGCCGAGATGTCTCGCTCCGTGGCC |
| | | | [9] GGAGGTTTGAAGATGCCGCAGGATC |
| | | | [10] GAGATGTCTCGCTCCGTGGCCTTAG |
| | | | [11] GATGTCTCGCTCCGTGGCCTTAGCT |
| | | | [12] TGTCTCGCTCCGTGGCCTTAGCTGT |
| | | | [13] CGTGGCCTTAGCTGTGCTCGCGCTA |
| | | | [14] CTTAGCTGTGCTCGCGCTACTCTCT |
| | | | [15] TAGCTGTGCTCGCGCTACTCTCTCT |
| | | | [16] GCTGTGCTCGCGCTACTCTCTCTTT |
| | | | [17] TGTGCTCGCGCTACTCTCTCTTTCT |
| | | | [18] GCCTGGAGGCTATCCAGCATTCAGG |
| | | | [19] CTGGAGGCTATCCAGCATTCAGGTT |
| | | | [20] GGAGGCTATCCAGCATTCAGGTTTA |
| 873-892 | J02611_at | 1 | [1] TGAGAAGATCCCAACAACCTTTGAG |
| | | | [2] GATCCCAACAACCTTTGAGAATGGA |
| | | | [3] CTTTGAGAATGGACGCTGCATCCAG |
| | | | [4] ACGCTGCATCCAGGCCAACTACTCA |
| | | | [5] CATCCAGGCCAACTACTCACTAATG |
| | | | [6] TTCCTGGTTTATGCCATCGGCACCG |
| | | | [7] GTTTATGCCATCGGCACCGTACTGG |
| | | | [8] GATCCTGGCCACCGACTATGAGAAC |
| | | | [9] GGCCACCGACTATGAGAACTATGCC |
| | | | [10] TGAGAACTATGCCCTCGTGTATTCC |
| | | | [11] CCTCGTGTATTCCTGTACCTGCATC |
| | | | [12] GTATTCCTGTACCTGCATCATCCAA |
| | | | [13] CTGTACCTGCATCATCCAACTTTTT |
| | | | [14] CTGCATCATCCAACTTTTTCACGTG |
| | | | [15] TGCTTGGATCTTGGCAAGAAACCCT |
| | | | [16] CACAGACCAGGTGAACTGCCCCAAG |
| | | | [17] CCAGGTGAACTGCCCCAAGCTCTCG |
| | | | [18] AGGTTCTACAGGGAGGCTGCACCCA |
| | | | [19] ACTCCATGTTACTTCTGCTTCGCTT |
| | | | [20] CCTGTTACCTTGCTAGCTGCAAAAT |

The annotations of the selected mRNA probes are given in Table 27. The annotations were obtained from Bioconductor package hu6800.db [Marc Carlson, Seth Falcon, Herve Pages and Nianhua Li (2008). hu6800.db: Affymetrix HuGeneFL Genome Array annotation data (chip hu6800). R package version 2.2.3.] in combination with the information available via PubMed [http://www.ncbi.nlm.nih.gov/pubmed/].

### Table 27

**Table 27: Annotation of mRNA probes selected during LOO cross validation (1^{st} column is SEQ-ID-No)**

| | | | | |
|---|---|---|---|---|
| 900 | J02611_at | J02611 | NM_001647.2 | Hs.522555 |

### Example 3: Metabolizes and mRNA: Ischemia/Hypoxia

### Ischemia and hypoxia

Early diagnosis will buy critical time for timely intervention and selection of the appropriate therapy and thus to prevent fatal permanent brain damage
As for infants, in industrial countries the percentage of preterm subjects has increased during the last decades and now risen up to 12% of all live births [Martin JA, Hamilton BE, Sutton PD et al. Births: final data for 2004. Natl Vital Stat Rep. 2006; 55:1-101; Martin JA, Hamilton BE, Sutton PD et al. Births: final data for 2005. Natl Vital Stat Rep. 2007; 56:1-103].

However, developmental brain injury and the subsequent neurological sequelae are still a major personal burden for affected individuals and their families and constitutes a considerable socioeconomic problem.
Early detection of a status of ischemia/hypoxia or stroke in man or of perinatal brain lesions in adult patients and preterm infants will enable and the application of successful therapeutic regimens and allow to control the consequences of these measures.

We use the ischemia data obtained from a rat hypoxia model to develop a multi-level classifier using metabolite data from brain samples and qPCR data from plasma.

### Animal model

A model of HI brain injury based on Rice-Vanucci's procedure was performed at postnatal day 7 (P7) [Rice JE, III, Vannucci RC, Brierley JB. The influence of immaturity on hypoxic-ischemic brain damage in the rat. Ann Neurol. 1981; 9:131-141] Sprague-Dawley rat pups (from Charles River, Wilmington, MA, U.S.A.) of either sex were randomly assigned a) the experimental groups and b) the time. For operation animals were anesthetized with inhaled isoflurane 3% in 02, the right carotid artery was accessed through a midline incision and surgical ligation was performed with a double suture and a permanent incision. The procedure was performed at room temperature (23-25°C). After closure of the neck wound, pups were returned to their dams for 2 h. The entire surgical procedure lasted no longer than 10 min. The pups were then exposed to hypoxia at 8% oxygen for 100 minutes. Adequate measures were taken to minimize pain and discomfort, complying with the European Community guidelines for the use of experimental animals. The study protocol was approved by the Austrian committee for animal experiments.
Sham-operated animals underwent anesthesia, neck incision and vessel manipulation without ligation or hypoxia. Control animals were kept without any damage. Animals were euthanized i) immediately after hypoxia (P7), ii) after 24hrs (P8), iii) after 5 days (P12), brains were collected, rinsed with PBS and immediately frozen in liquid nitrogen and stored at -70°C until further preparation.

### Sample preparation

Brain samples were thawed on ice for 1 hour and homogenates were prepared by adding PBS-buffer (phosphate buffered saline, 0.1 µmol/L; Sigma Aldrich, Vienna, Austria) to tissue sample, ratio 3:1 (w/v), and homogenized with a Potter S homogenizer (Sartorius, Goettingen, Germany) at 9 g on ice for 1 minute. To enable analysis of all samples in one batch, samples were frozen again (-70°C), thawed on ice (1 h) on the day of analysis and centrifuged at 18000 g at 2°C for 5 min. All tubes were prepared with 0.001% BHT (butylated hydroxytoluene; Sigma-Aldrich, Vienna, Austria) to prevent autooxidation [Morrow, J. D. and L. J. Roberts. Mass spectrometry of prostanoids: F2-isoprostanes produced by non-cyclooxygenase free radical-catalyzed mechanism. Methods Enzymol. 233 (1994): 163-74] .

Overall the data obtained from nine control and seven ischemic animal samples were processed. The metabolite concentrations were measured using a commercial Kit (Marker *IDQ*^{™}, Biocrates AG, Innsbruck, Austria) as well as other mass-spectroscopy based methods described below.

Extracted samples were analyzed by a new developed online solid phase extraction liquid chromatography tandem mass spectrometry method (online SPE-LC-MS/MS). All procedures (sample handling, analytics) were performed by co-workers blinded to the groups. For simultaneous quantitation of free prostaglandins and lipoxygenase derived fatty acid metabolites in brain homogenates we used a LC-MS/MS based method as described by Unterwurzacher et al. [Unterwurzacher I, Koal T, Bonn GK et al. Rapid sample preparation and simultaneous quantitation of prostaglandins and lipoxygenase derived fatty acid metabolites by liquid chromatography-mass spectrometry from small sample volumes. Clin Chem Lab Med. 2008; 46:1589-1597] for brain tissue. Due to matrix effects observed during analysis of brain samples, an online solid phase extraction (SPE) step was implemented prior to chromatographic separation using a C18 Oasis HLB column (2.1 x 20 mm, 25 µm particle size; Waters, Vienna, Austria) as online SPE column. The quantification of the metabolites in the extracted biological sample is achieved by reference to appropriate internal standards and by use of the most sensitive and selective electrospray ionization (ESI) multiple reaction monitoring (MRM) MS/MS detection mode. The method was validated for tissue samples homogenates according the "Guidance for Industry - Bioanalytical Method Validation", U.S. Department of Health and Human Services, Food and Drug Administration, 2001. For the online SPE-LC-MS/MS analysis 20 µL of the extracted homogenate was injected.

### RNA extraction and cDNA synthesis:

The two divided brain hemispheres of newborn RNU rats were collected in 1 ml TRIzol Reagent (Invitrogen Life Technologies, Austria), frozen in liquid nitrogen and stored at -80°C until further processing. The RNA extraction was done according to manufacturer's instructions. Briefly, the brain hemispheres were homogenized in TRIzol on ice using a micropistill. After complete homogenization a chloroform extraction step resulting in an RNA containing aqueous phase, followed by precipitation with isopropyl alcohol was affiliated. After two washing steps with 75% ethanol the briefly air dried RNA was resuspended in DEPC-treated water, the RNA concentration was determined using an UV-spectrophotometer (Ultrospec 3300 pro, Amersham, USA) and stored at -80°C until processing for cDNA synthesis. Prior to reverse trancription (RT) an amount of 1µg of total RNA was treated with DNase I, RNase-free (Deoxyribonuclease I, Fermentas, Germany) according to manufacturer's instructions to remove potential contaminating DNA. After DNase I treatment the samples were processed for cDNA synthesis using the RevertAid M-MuLV reverse transcriptase (Fermentas, Germany). Each reaction consisted of 5x RT-reaction buffer, 10 mM deoxyribonucleotide triphosphate mixture (dNTPs), 0,2 µg/µl random hexamer primer, an RNase inhibitor and the RevertAid M-MuLV-RT (all from Fermentas, Germany). Samples were incubated at 25°C for 10 minutes followed by 60 minutes at 42°C in a waterbath. The reaction was terminated by heating to 70°C for 10 minutes followed by chilling on ice. The cDNA samples were stored at -20°C until processing for quantitative real-time PCR using the BioRad iCycler iQ. The cDNA samples were prediluted 1:10 before used as template for quantitative real-time PCR.

### Quantitative real-time PCR (q-RT-PCR):

The quantitative real-time PCR was carried out in 96-well 0,2 ml thin-wall PCR plates coverd with optically clear adhesive seals (BioRad Laboratories, Austria) in a total volume of 25 µl. The real-time PCR reaction mixture consisted of 1x iQ SYBR Green Supermix (BioRad Laboratories, Austria), 0,4 µM of each gene specific primer and 5 µl of prediluted cDNA. Initially the mixture was heated to 95°C for 3 minutes to activate the iTaq DNA polymerase, followed by 45 cycles consisting of denaturation at 95°C for 20 seconds and annealing at 60°C for 45 seconds. After the amplification a melting curve analysis was added to confirm PCR product specificity. No signals were detected in the no-template controls.
The results were analysed using the iCycler iQ5 Optical System Software Version 2.0 (BioRad Laboratories, Austria). The baseline was manually set and the threshold automatically by the software.

The crossing point of the amplification curve with the threshold line represents the cycle threshold (ct). All samples were run in triplicates and the mean value was used for further calculations.
During the optimization process all gene specific primer pairs were run in a gradient PCR to determine the optimal annealing temperature, the PCR products were loaded on a 2% agarose gel containing ethidium bromide to confirm specificity of the amplification product and the absence of primer dimer formation.
The sequence of gene specific primer pairs used are given in Table 28 (1^{st} column is SEQ-ID-No).

### Table 28

**Table 28: Metabolite data, classification error via LOO cross validation**

| | | | |
|---|---|---|---|
| 901 | rSDF1a-LC1 | 181 bp | 5'-AGTGACGGTAAGCCAGTCAG-3' |
| 902 | rSDF1a-LC2 | | 5'-TCCACTTTAATTTCGGGTCA-3' |
| 903 | rVEGF-LC1 | 195 bp | 5'-GAAAGGGAAAGGGTCAAAAA-3' |
| 904 | rVEGF-LC2 | | 5'-CACATCTGCAAGTACGTTCG-3' |
| 905 | rACTB-LC1 | 160 bp | 5'-AAGAGCTATGAGCTGCCTGA-3' |
| 906 | rACTB-LC2 | | 5'-TACGGATGTCAACGTCACAC-3' |

### Analysis of qPCR and Metabolomics data:

For developing and validating a classifier based on these data we used support vector machines [Schöllkopf, B. and Smola, A. (2001) Learning with Kernels: Support Vector Machines, Regularization, Optimization, and Beyond. MIT Press, Cambridge] in combination with leave-one-out (LOO) cross-validation where each analysis step - including low level analysis was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), random forests (RF), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more. The low level analysis consisted of a variance stabilizing transformation via the binary logarithm (i.e., log to base 2) for the metabolite data In each cross validation step we selected those four normalized metabolites, which had the largest differences (in absolute value) of the mean values beyond those probes with p value equal or smaller than 0.1 by the Welch t-test. This is, we used a so called ranker for feature selection. Again there are numerous other feature selection strategies we could have used, some examples are given in Hall et al. 2003 Overall a metabolite may have been chosen up to 16 times due to LOO cross-vaiidation. Using only metabolomics data we obtain the estimated errors given in Table 29.

### Table 29.

**Table 29: Metabolite data, classification error via LOO cross validation**

| **classifier vs. true** | **ischemia** | **control** |
|---|---|---|
| **Ischemia** | 57.1 % | 33.3% |
| **Control** | 42.9% | 66.7% |

The estimated overall accuracy using LOO cross-validation is 62.5 %, sensitivity is 57.1%, specificity is 66.7%, positive predictive value is 57.1% and negative predictive value is 66.7%. In a second step we used qPCR data obtained for SDF1 and VEGF. The PCR data was normalized via the reference gene Actin-beta. The classification results can be read off from Table 30. We get an estimated overall accuracy of 68.9 % again using LOO cross-validation. The estimated values for sensitivity, specificity, positive and negative predictive value are 57.1%, 77.8%, 66.7% and 70.0%, respectively.

### Table 30

**Table 30: qPCR data, classification error via LOO cross validation**

| **classifiers vs. true** | **ischemia,** | **normal** |
|---|---|---|
| **Ischemia** | 57.1% | 22.2% |
| **Normal** | 42.9% | 77.8% |

In the last step we combine metabolite and qPCR data and obtain the results given in Table 31. That is, the estimated overall accuracy using cross-validation is 75.0 %. Hence, this combination increases the overall accuracy from 62.5 % resp. 68.9% to 75.0 %. Sensitivity, specificity, positive and negative predictive value are 71.4%, 77.8%, 71.4% and 77.8%, respectively. Hence, beside overall accuracy, sensitivity as well as positive and negative predictive value are enhanced.

### Table 31

**Table 31: Metabolite and qPCR data, classification error via LOO cross validation**

| **classifier vs. true** | **ischemia** | **normal** |
|---|---|---|
| **Ischemia** | 71.4% | 22.2% |
| **Normal** | 18.6% | 77.8% |

The metabolites which were selected during cross-validation are given in Table 32.

### Table 32

**Table 32: Metabolites selected during LOO cross validation**

| **Nr.** | **Metabolite** | **Times selected** | **Comments** |
|---|---|---|---|
| 1 | Gln-PTC | 16 | PTC = Phenylthiocarbamoyl |
| 2 | xLeu-PTC | 15 | |
| 3 | Ala-PTC | 11 | |
| 4 | 12S-HETE | 8 | = 12(S)-Hydroxyeicosatetraenoic acid |
| 5 | Alanine | 4 | |
| 6 | xLeucine | 3 | |
| 7 | DHA | 3 | = Decosahexaenoic acid |
| 8 | Ser-PTC | 2 | |
| 9 | Glu | 1 | |
| 10 | Glutamic Acid | 1 | |

In Table 32, the total of times selected must be 64, wherein each individual metabolite might be selected a maximum of 16 times.

### Table 33

**Table 33: Metabolite data, classification error via LOO cross validation (1^{st} column is SEQ-ID-No)**

| | | | |
|---|---|---|---|
| 265 | ACTB | NM_001101.2 | Hs.520640 |
| | | | Hs.708120 |

### Embodiments of the invention

In one embodiment, first, a biological sample from a subject in need of diagnosis, or response or survival prognostication is obtained. Second, an amount of a RNA, microRNA, peptide or protein, metabolite is selected and is measured from the biological sample. Third, the amount of RNA, microRNA, peptide or protein, metabolite, is detected in the sample and is compared to either a standard amount of the respective biomolecule present in a normal cell or a non-cancerous cell or tissue or plasma, or an amount of the RNA, microRNA, peptide or protein, metabolite is present in the control sample. If the amount of RNA, microRNA, peptide or protein, metabolite in the sample is different to the amount of RNA, microRNA, peptide or protein, metabolite in the standard or control sample, the processing and classification of concentration data and classifier generation as described before (Table 1) from at least two groups/species of biomolecules comprising RNA, microRNA, peptide or protein, metabolites affords a value or score assigned to a diseased state with some probability then the subject is diagnosed as having cancer, the prognosis is a low expected response to the cancer treatment, or the prognosis is a low expected survival of the subject. The prognoses are relative to a subject with cancer having normal levels of the RNA, microRNA, peptide or protein, metabolite or relative to the average expected response or survival of a patient having a complex disease. It is clear that these complex diseased states can also be due to intoxication and drug abuse.

Another embodiment of the method of detecting or diagnosing a complex disease, prognosticating an expected response to a, or prognosticating an expected survival comprises the following steps. First, a biological sample containing RNA, microRNA, peptide or protein, metabolite is obtained from the subject. The biological sample is reacted with a reagent capable of binding to an RNA, microRNA, peptide or protein, metabolite. The reaction between the reagent and the microRNA forms a measurable RNA, microRNA, peptide or protein, metabolite product or complex. The measurable RNA, microRNA, peptide or protein, metabolite product or complex is measured, the data processed to afford a score applying the steps as specified under Fig. 1 and then compared to either the standard or the control score value.

The examples indicate that the method according to the invention includes the analysis and classifier generation from quantitative data of the aforementioned types of biomolecules obtained from different, distinct tissues from one individual and show that this is advantageous in recognizing distinct states related to complex diseases as data from different sites of an affected organism contribute to biomarker/dassifier description.

The invention can be practiced on any mammalian subject including humans, that has any risk of developing a complex disease in the sense of the present invention.

Samples to be used in the invention can be obtained in any manner known to a skilled artisan. The sample optimally can include tissue believed to be cancerous, such as a portion of a surgically removed tumor but also blood containing cancer cells. However, the invention is not limited to just tissue believed to be altered (with regard to concentrations of biomolecules such as RNA, micro RNA, protein, peptide, metabolites) due to a complex disease. Instead, samples can be derived from any part of the subject containing at least some tissue or cells believed to be affected by the complex disease, in particular, cancer and/or having being exposed or in contact to cancer tissue or cells or by contact to body liquids such as blood distributing certain biomolecules within the body.

Another example of a method of quantifying RNA or microRNA is as follows: hybridizing at least a portion of the RNA or microRNA with a fluorescent nucleic acid, and reacting the hybridized RNA or microRNA with a fluorescent reagent, wherein the hybridized RNA or microRNA emits a fluorescent light. Another method of quantifying the amount of RNA or microRNA in a sample is by hybridizing at least a portion the RNA or microRNA to a radiolabeled complementary nucleic acid. In instances when a nucleic acid capable of hybridizing to the RNA or microRNA is used in the measuring step, in case of the microRNA the nucleic acid is at least 5 nucleotides, at least 10 nucleotides, at least 15 nucleotides, at least 20 nucleotides, at least 25 nucleotides, at least 30 nucleotides or at least 40 nucleotides; and may be no longer than 25 nucleotides, no longer than 35 nucleotides; no longer than 50 nucleotides; no longer than 75 nucleotides, no longer than 100 nucleotides or no longer than 125 nucleotides in length. The nucleic acid is any nucleic acid having at least 80% homology, 85% homology, 90% homology, 95% homology or 100% homology with any of the complementary sequences for the microRNAs. A suitable RNA parameter, e.g. is the amount of RNA or microRNA which is compared to either a standard amount of the RNA or microRNA present in a normal cell or a non-cancerous cell, or to the amount of RNA or microRNA in a control sample. The comparison can be done by any method known to a skilled artisan. An example of comparing the amount of the RNA or microRNA in a sample to a standard amount is comparing the ratio between 5S rRNA and the RNA or microRNA in a sample to a published or known ratio between 5S rRNA and the RNA or microRNA in a normal cell or a non-cancerous cell. An example of comparing the amount of microRNA in a sample to a control is by comparing the ratios between 5 S rRNA and the RNA or microRNA found in the sample and in the control sample. In instances when the amount of RNA or microRNA is compared to a control, the control sample may be obtained from any source known to have normal cells or non-cancerous cells. Preferably, the control sample is tissue or body fluid from the subject believed to be unaffected by the respective complex disease contain only normal cells or non-cancerous cells.

Measuring the amount of RNA, microRNA, peptide or protein, metabolite can be performed in any manner known by one skilled in the art of measuring the quantity of RNA, microRNA, peptide or protein within a sample. An example of a method for quantifying RNA or microRNA is quantitative reverse transcriptase polymerase chain reaction, PCR or quantitation and relative quantitation applying sequencing or second generation sequencing.

Protein measurement, absolute and relative protein quantitation of individual protein species as well as quantitation of metabolites within a tissue or in a preparation of cells can be performed applying Western blotting, Enzyme Linked Immunoassay (ELISA) Radioimmunoassay or other assays utilizing antibodies or other protein binding molecules, mass spectrometry for protein or peptide identification, quantitation or relative quantitation using MALDI, Electrospray or other types of ionisation, protein and antibody arrays employing antibodies or other molecules binding proteins such as aptamers. The compound capable of binding to RNA, microRNA, peptide or protein and metabolite can be any compound known to a skilled artisan as being able to bind to the RNA, microRNA, peptide or protein in a manner that enables one to detect the presence and the amount of the molecule. An example of a compound capable of binding RNA, microRNA, peptides or proteins as well as low molecular weight compounds and metabolites is a nucleic acid capable of hybridizing or an aptamer capable of binding to nucleic acids, RNA, microRNA, proteins and peptides. The nucleic acid preferably has at least 5 nucleotides, at least 10 nucleotides, at least 15 nucleotides, at least 20 nucleotides, at least 25 nucleotides, at least 30 nucleotides, at least 40 nucleotides or at least 50 nucleotides. The nucleic acid is any nucleic acid having preferably at least 80% homology, 85% homology, 90% homology, 95% homology or 100% homology with a sequence complementary to an RNA or microRNA , which also might be derived from corresponding DNA data, or an aptamer capable of binding RNA, microRNA, peptide or protein or metabolite. One specific example of a nucleic acid capable of binding to RNA or microRNA is a nucleic acid primer for use in a reverse transcriptase polymerase chain reaction.

The binding of the compound to at least a portion of the RNA, microRNA, peptide or protein and metabolite forms a measurable complex. The measurable complex is measured according to methods known to a skilled artisan. Examples of such methods include the methods used to measure the amount of the RNA, microRNA, peptide or protein, metabolite employed in the inventive method discussed above.

If there is an increased or decreased level of measurable complex relative to a standard amount of RNA, microRNA, peptide or protein found in a normal or a non-cancerous cell, or in a control sample, then the sample either contains a pre-cancerous cell or cancer cell, thereby being diagnostic of a cancer; prognosticates an expected response to a cancer treatment; or prognosticates an expected survival of the subject.

The inventive composition of the different types of biomolecules can be used in the inventive method (embodiments of which are described above). One embodiment of the inventive composition comprises a compound capable of binding to at least a portion of RNA, microRNA, peptide, protein or metabolite selected from the group consisting of RNA, microRNA, peptide or protein, metabolite. The composition comprises a compound capable of binding to at least a portion of a RNA, microRNA, peptide or protein selected from the group consisting of molecules summarized in the described examples and the lists of molecules and binding probes binding these endogenous biomolecules but is not limited to that. The various examples described above demonstrate that the method generally functions with a composition of 2-4 types of the defined biolmolecules, proteins or peptides, RNA, microRNA (i.e. RNA plus microRNA, RNA plus protein, protein plus microRNA, RNA plus protein plus microRNA, and a combination of these biomolecules and combinations of biomolecules with metabolites, selected and combined from various experiments investigating tissue from a subject having a complex disease with a performance which is superior than that of a test or diagnostic or prognostic tool comprising a set of preselected biomolecules composed of just one type such as RNA, protein, metabolite or microRNA solely.

Another embodiment of the inventive composition is a composition comprising a second compound capable of binding to a RNA, microRNA, peptide or protein and metabolite that is different from the RNA, microRNA, peptide or protein, metabolite that the first compound is capable of binding. Another embodiment of the inventive composition is a composition comprising a third compound capable of binding to a RNA, microRNA, peptide or protein, metabolite that is different from the RNA, microRNA, peptide or protein, metabolite that the first and second compounds are capable of binding.

The present invention further provides a method for evaluating candidate therapeutic agents. The method can be applied to identify molecules that modulate the concentrations of one to several of the mentioned biomolecules assigned to at least two or more of the stated molecule classes; RNA, microRNA, peptide/proteins, metabolites. Alternatively, assays may be conducted to identify molecules that modulate the activity of a protein encoded by a gene.

Another aspect of the invention is a kit for diagnosing, or prognosticating a complex disease. In one embodiment of this aspect, the kit is for diagnosing a subject with a complex disease. Another embodiment of this aspect is a kit for prognosticating a a complex disease, wherein the prognosis is an expected response by a subject to a treatment of the a complex disease. In another embodiment of this aspect, the kit is for prognosticating a a complex disease, wherein the prognosis is an expected survival of a subject with a complex disease. The kit comprises a composition capable of binding to at least a portion of a RNA, microRNA, peptide or protein, metabolite with increased or decreased concentration, over- or under-expressed in a cancer cell, wherein the RNA, microRNA, peptide or protein, metabolite is selected from-but not limited to the group consisting of the molecules listed in the examples outlined above or binding to the binding probes or determined quantitatively by methods described in the examples above and and wherein the differential expression (over-expression or under-expression or the concentration changes of several molecules out of RNA, microRNA, peptide or protein, metabolites in a combination of at least molecules from 2 different biomolecule classes (RNA plus microRNA, RNA plus proteins or peptides, microRNA plus protein or peptides, RNA plus microRNA plus proteins or peptides and combinations of all these with metabolites), comprising, but not limited, to the classes of compounds, the described binding probes, the agents and sequences specified in the described examples is diagnostic for a complex disease, or prognosticates the expected response or survival of the subject. The binding of the nucleic acid or aptamer or antibody to the target RNA, microRNA, peptide or protein, and or metabolite is diagnostic for a complex disease, prognosticates an expected response to a treatment, or prognosticates an expected survival of a subject having a complex disease.

The isolated RNA, microRNA, peptide or protein, metabolite can be associated with known diagnostic tools, such as protein chips, antibody chips, aptamer chips, DNA or RNA chips with various modes of detection of binding including but not limited to detection by use of fluorophores, electrochemical detection or transfer of an chemical signal to a change of electrical current, resistance or charge, RNA probes, or RNA primers.

One aspect of the invention is a method of detecting for early diagnosing a complex disease, prognosticating an expected response to a treatment, or prognosticating an expected survival. The present invention finds use with complex diseases, cancer, in a special embodiment with Leukemia (AML), prostate and kidney cancer as well as transient ischemic attack, hypoxia/ischemia. However, as evident already from these distinct and unrelated diseases and diverse types of cancer, diseases with completely different molecular etiology, phenotypes, genotypes and genetic dispositions, the method is applicable to complex diseases in general.

In a specific embodiment, data obtained from different types of biomolecules from different compartments (tissues) of the organism (subject, patient) are used and processed together according to the method thus providing improved classification and diagnosis of complex diseases.

The above descriptions are illustrative and not restrictive. It is to be understood that this invention is not limited to particular methods, and experimental conditions described, as such methods and conditions may vary.

The sequence listing accompanying the present application comprising sequences with SEQ-IDs No 1 to is SEQ-IDs No 908 is part of the disclosure of the present invention.

## Claims

1. A method for in vitro diagnosing a complex disease or subtypes thereof, selected from the group consisting of:
cancer, in particular, acute myeloid leukemia (AML), colon cancer, kidney cancer, prostate cancer; transient ischemic attack (TIA), ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis, Alzheimer and Parkinson's disease;
in at least one biological sample of at least one tissue of a mammalian subject comprising the steps of:
a) selecting at least two different species of biomolecules, wherein said species of biomolecules are selected from the group consisting of: RNA and/or its DNA counterparts, microRNA and/or its DNA counterparts, peptides, proteins, and metabolites;
b) measuring at least one parameter selected from the group consisting of presence (positive or negative), qualitative and/or quantitative molecular pattern and/or molecular signature, level, amount, concentration and expression level of a plurality of biomolecules of each species in said sample using at least two sets of different species of biomolecules and storing the obtained set of values as raw data in a database;
c) mathematically preprocessing said raw data in order to reduce technical errors being inherent to the measuring procedures used in step b);
d) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), self organizing maps (SOM), recursive partitioning and regression trees, K-nearest neighbor classifiers (K-NN), fuzzy classifiers, bagging, boosting, and naive Bayes; and applying said selected classifier algorithm to said preprocessed data of step c);
e) said classifier algorithms of step d) being trained on at least one training data set containing preprocessed data from subjects being divided into classes according to their pathophysiological, physiological, prognostic, or responder conditions, in order to select a classifier function to map said preprocessed data to said conditions;
f) applying said trained classifier algorithms of step e) to a preprocessed data set of a subject with unknown pathophysiological, physiological, prognostic, or responder condition, and using the trained classifier algorithms to predict the class label of said data set in order to diagnose the condition of the subject.

2. Method according to claim 1, **characterized in that** said tissue is selected from the group consisting of blood and other body fluids, cerebrospinal fluids, bone tissue, bone marrow tissue, muscular tissue, glandular tissue, brain tissue, nerve tissue, mucous tissue, connective tissue, and skin tissue and/or said sample is a biopsy sample and/or said mammalian subject includes humans; and/or
in step a) DNA is selected; and/or
further **characterized in that** standard lab parameters commonly used in clinical chemistry, such as serum and/or plasma levels of low molecular weight biochemical compounds, enzymes, enzymatic activities, cell surface receptors and/or cell counts, in particular red and/or white cell counts, platelet counts, are additionally selected as species of biomolecules.

3. Method according to claim 1 or 2, **characterized in that** said step of mathematically preprocessing of said raw data obtained in step b) is carried out by a statistical method selected from the group consisting of:
in case of raw data obtained by optical spectroscopy (UV, visible, IR, Fluorescence): background correction and/or normalization;
in case of raw data obtained from metabolomics and/or proteomics obtained by mass spectroscopy coupled to liquid or gas chromatography or capillary electrophoresis or by 2D gel electrophoresis, quantitative determination with ELISA or RIA or determination of concentrations/amounts by quantitation of immunoblots or quantitation of amounts of biomolecules bound to aptamers: smoothing, baseline correction, peak picking, optionally, additional further data transformation such as taking the logarithm in order to carry out a stabilization of the variances;
in case of raw data obtained from transcriptomics: Summarizing single pixel to a single intensity signal; background correction; summarizing of multiple probe signals to a single expression value, in particular perfect match/mismatch probes; normalization;

4. Method according to anyone of claims 1 through 3, **characterized in that** after preprocessing step c) a further step of feature selection is inserted, in order to find a lower dimensional subset of features with the highest discriminatory power between classes;
and/or
said feature selection is carried out by a filter and/or a wrapper approach;
and/or
wherein
said filter approach includes rankers and/or feature subset evaluation methods; and/or
wherein
said wrapper approach is applied, where a classifier is used to evaluate attribute subsets.

5. Method according to anyone of claims 1 through 4, **characterized in that** said pathophysiological condition corresponds to the label "diseased" and said physiological condition corresponds to the label "healthy" or said pathophysiological condition corresponds to different labels of "grades of a disease", "subtypes of a disease", different values of a "score for a defined disease"; said prognostic condition corresponds to a label "good", "medium", "poor", or "therapeutically responding" or "therapeutically non-responding" or "therapeutically poor responding".

6. Method according to anyone of claims 1 through 5, **characterized in that** said metabolic data is high- throughput mass spectrometry data.

7. Method according to anyone of claims 1 through 6, **characterized in that** said complex disease is AML, said mammalian subject is a human being, said biological sample blood and/or blood cells and/or bone marrow;
wherein said different species of biomolecules are microRNA and proteins, in particular surface proteins from non-mature hematopoietic stem cells, preferably CD34;
wherein microRNA expression levels and CD34 presence are used as said parameters of step b);
wherein raw data of microRNA expression are preprocessed using a variance-stabilizing normalization and summarizing the normalized multiple probe signals (technical replicates) to a single expression value, using the median;
wherein a ranker, in particular a Mann-Whitney significance test combined with largest median of pairwise differences as filter for microRNA expression data is used for said feature selection;
wherein logistic regression is selected as suitable classifying algorithm, the training of the classifying algorithm including preprocessed and filtered microRNA expression data and CD34 information (positive or negative), is carried out with an n-fold cross-validation, in particular 5 to 10-fold, preferably 5-fold cross-validation;
applying said trained logistic regression classifier to said preprocessed microRNA expression data set and CD34 information to a subject under suspicion of having AML, and using the trained classifiers to diagnose a specific AML-type.

8. Method according to claim 7, **characterized in that** the following DNA probes for targeting said microRNA are used: Seq-ID No. 1 to Seq-ID No. 14;
and/or
the following microRNA-target sequences are used: Seq-ID Nos. 15 to 26.

9. Method according to anyone of claims 1 through 8, **characterized in that** said complex disease is colon cancer, said mammalian subject is a human being, said biological sample is colon tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for microRNA expression data is used for said feature selection;
wherein random forests are selected as suitable classifying algorithm, the training of the classifying algorithm including preprocessed and filtered mRNA and microRNA expression data, is carried out with a leave-one-out (LOO) cross-validation;
applying said trained random forests classifier to said preprocessed mRNA and microRNA expression data sets to a subject under suspicion of having colon cancer, and using the trained classifiers to diagnose colon cancer and/or a subtype thereof.

10. Method according to claim 9, **characterized in that** the following DNA probes for targeting said microRNA are used: Seq-ID No. 27 to Seq-ID No. 34;
and/or
the following microRNA-target sequences are used: Seq-ID No. 35 to Seq-ID No. 42;
and/or
the following DNA probes for targeting said mRNA are used: Seq-ID No. 43 to Seq-ID No. 264;
and/or
the following target DNA sequences are used: Seq-ID No. 265 to 276.

11. Method according to anyone of claims 1 through 6, **characterized in that** said complex disease is kidney cancer, said mammalian subject is a human being, said biological sample is kidney tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Welch t-test (significance test) combined with largest mean of pairwise differences as filter for mRNA and microRNA expression data is used for said feature selection;
wherein single-hidden-layer neural networks are selected as suitable classifying algorithm, the training of the classifying algorithm including preprocessed and filtered mRNA and microRNA expression data, is carried out with a leave-one-out (LOO) cross-validation; applying said trained random forests classifier to said preprocessed mRNA and microRNA expression data sets to a subject under suspicion of having kidney cancer, and using the trained classifiers to diagnose kidney cancer and/or a subtype thereof.

12. Method according to claim 11, **characterized in that** the following DNA probes for targeting said microRNA are used: Seq-ID Nos. 33, and 277 to 288;
and/or
the following microRNA-target sequences are used: Seq-ID Nos. 21, 41, 289 to 297;
and/or
the following DNA probes for targeting said mRNA are used: Seq-ID Nos. 298 to 716;
and/or
the following DNA target sequences are used: Seq-ID Nos. 265, 268, 717 to 732.

13. Method according to anyone of claims 1 through 6, **characterized in that** said complex disease is prostate cancer, said mammalian subject is a human being, said biological sample is urine and/or prostate tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for mRNA and microRNA expression data is used for said feature selection;
wherein linear discriminant analysis is selected as suitable classifying algorithm, the training of the classifying algorithm including preprocessed and filtered mRNA and microRNA expression data, is carried out with a leave-one-out (LOO) cross-validation;
applying said trained random forests classifier to said preprocessed mRNA and microRNA expression data sets to a subject under suspicion of having prostate cancer, and using the trained classifiers to diagnose prostate cancer and/or a subtype thereof.

14. Method according to claim 13, **characterized in that** the following DNA probes for targeting said microRNA are used: Seq-ID Nos 733 to 735;
and/or
the following microRNA-target sequences are used: Seq-ID Nos 736-738;
and/or
the following DNA probes for targeting said mRNA are used: Seq-ID No. 739 to Seq-ID No. 892;
and/or
the following DNA target sequences are used: Seq-ID Nos. 893 to 900.

15. Method according to anyone of claims 1 through 6, **characterized in that** said complex disease is transient ischemic attack (TIA) and/or ischemia and/or hypoxia, said mammalian subject is a human being, said biological sample blood and/or blood cells and/or cerebrospinal fluid and/or brain tissue;
wherein said different species of biomolecules are mRNA and/or its DNA counterparts and brain metabolites, in particular free prostaglandins, lipoxygenase derived fatty acid metabolites, glutamine, glutamic acid, leucin, alanine, serine, decosahexaenoic acid (DHA), 12(S)-hydroxyeicosatetraenoic acid (12S-HETE);
wherein mRNA expression levels and quantitative and/or qualitative molecular metabolite patterns (metabolomics data) are used as said parameters of step b);
wherein raw data of mRNA expression are preprocessed using actin-ß as reference genes and metabolomics data of said brain metabolites are preprocessed by a variance stabilizing transformation via the binary logarithm (i.e. to base 2);
wherein a ranker, in particular a Welch t-test (significance test) combined with largest mean of pairwise differences as filter for metabolomics data is used for said feature selection;
wherein support vector machines are selected as suitable classifying algorithm, the training of the classifying algorithm including preprocessed and filtered mRNA and microRNA expression data, is carried out with a leave-one-out (LOO) cross-validation;
applying said trained support vector machines classifier to said preprocessed mRNA expression data and said metabolomics data sets to a subject under suspicion of having ischemia and/or hypoxia, and using the trained classifiers to diagnose ischemia and/or hypoxia and/or the grades thereof.

16. Method according to claim 15, **characterized in that** the samples are analyzed by solid phase extraction liquid chromatography tandem mass spectrometry (online SPE-LC-MS/MS), wherein preferably a C18 column is used as solid phase extraction column; and wherein the quantification of the measured metabolite concentrations in said biological tissue sample preferably is calibrated by reference to internal standards and by using an electrospray ionization multiple reaction monitoring tandem mass spectrometry detection mode.

17. Method according to anyone of claims 15 through 17, **characterized in that** the mRNA expression data are obtained by quantitative real time PCR (q-RT-PCR);
and/or
the following primer pairs are used: Seq-ID Nos. 901 to 906; and/or the following DNA target sequences are used: Seq-ID Nos. 265, 907 and 908.

18. Kit for carrying out a method in accordance with anyone of claims 1 to 17, in a biological sample, comprising:
a) detection agents for the detection of at least two different species of biomolecules, wherein said species of biomolecules are selected from the group consisting of: RNA and/or its DNA counterparts, microRNA and/or its DNA counterparts, peptides, proteins, and metabolites;
b) positive and/or negative controls; and
c) classification software for classification of the results achieved with said detection agents.

## Amended claims

### Amended claims in accordance with Rule 137(2) EPC.

**1.** A method for in vitro diagnosing a complex disease or subtypes thereof, selected from the group consisting of:
cancer, in particular, acute myeloid leukemia (AML), colon cancer, kidney cancer, prostate cancer; transient ischemic attack (TIA), ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis, Alzheimer and Parkinson's disease;
in at least one biological sample of at least one tissue of a mammalian subject comprising the steps of:
a) selecting at least two different species of biomolecules, wherein said species of biomolecules are selected from the group consisting of: RNA and/or its DNA counterparts, microRNA and/or its DNA counterparts, peptides, proteins, and metabolites;
b) measuring at least one parameter selected from the group consisting of presence or absence, qualitative and/or quantitative molecular pattern and/or molecular signature, level, amount, concentration and expression level of a plurality of biomolecules of each species in said sample using at least two sets of different species of biomolecules and storing the obtained set of values as raw data in a database;
c) mathematically preprocessing said raw data in order to reduce technical errors being inherent to the measuring procedures used in step b);
d) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), self organizing maps (SOM), recursive partitioning and regression trees, K-nearest neighbor classifiers (K-NN), fuzzy classifiers, bagging, boosting, and naïve Bayes; and applying said selected classifier algorithm to said preprocessed data of step c);
e) said classifier algorithms of step d) being trained on at least one training data set containing preprocessed data from subjects being divided into classes according to their pathophysiological, physiological, prognostic, or responder conditions, in order to select a classifier function to map said preprocessed data to said conditions;
f) applying said trained classifier algorithms of step e) to a preprocessed data set of a subject with unknown pathophysiological, physiological, prognostic, or responder condition, and using the trained classifier algorithms to predict the class label of said data set in order to diagnose the condition of the subject.

**2.** Method according to claim 1, **characterized in that** said tissue is selected from the group consisting of blood and other body fluids, cerebrospinal fluids, bone tissue, bone marrow tissue, muscular tissue, glandular tissue, brain tissue, nerve tissue, mucous tissue, connective tissue, and skin tissue and/or said sample is a biopsy sample and/or said mammalian subject includes humans; and/or
further **characterized in that** standard lab parameters commonly used in clinical chemistry, such as serum and/or plasma levels of low molecular weight biochemical compounds, enzymes, enzymatic activities, cell surface receptors and/or cell counts, in particular red and/or white cell counts, platelet counts, are additionally selected.

**3.** Method according to claim 1 or 2, **characterized in that** said step of mathematically preprocessing of said raw data obtained in step b) is carried out by a statistical method selected from the group consisting of:
in case of raw data obtained by optical spectroscopy (UV, visible, IR, Fluorescence): background correction and/or normalization;
in case of raw data obtained from metabolomics and/or proteomics obtained by mass spectroscopy coupled to liquid or gas chromatography or capillary electrophoresis or by 2D gel electrophoresis, quantitative determination with ELISA or RIA or determination of concentrations/amounts by quantitation of immunoblots or quantitation of amounts of biomolecules bound to aptamers: smoothing, baseline correction, peak picking, optionally, additional further data transformation such as taking the logarithm in order to carry out a stabilization of the variances;
in case of raw data obtained from transcriptomics: Summarizing single pixel to a single intensity signal; background correction; summarizing of multiple probe signals to a single expression value, in particular perfect match/mismatch probes; normalization;

**4.** Method according to anyone of claims 1 through 3, **characterized in that** after preprocessing step c) a further step of feature selection is inserted, in order to find a lower dimensional subset of features with the highest discriminatory power between classes; and
said feature selection is carried out by a filter and/or a wrapper approach;
wherein said filter approach includes rankers and/or feature subset evaluation methods.

**5.** Method according to anyone of claims 1 through 4, **characterized in that** said pathophysiological condition corresponds to the label "diseased" and said physiological condition corresponds to the label "healthy" or said pathophysiological condition corresponds to different labels of "grades of a disease", "subtypes of a disease", different values of a "score for a defined disease"; said prognostic condition corresponds to a label "good", "medium", "poor", or "therapeutically responding" or "therapeutically non-responding" or "therapeutically poor responding".

**6.** Method according to anyone of claims 1 through 5, **characterized in that** said metabolic data is high-throughput mass spectrometry data.

**7.** Method according to anyone of claims 1 through 6, **characterized in that** said complex disease is AML, said mammalian subject is a human being, said biological sample blood and/or blood cells and/or bone marrow;
wherein said different species of biomolecules are microRNA and proteins, in particular surface proteins from non-mature hematopoietic stem cells, preferably CD34;
wherein microRNA expression levels and CD34 presence are used as said parameters of step b);
wherein raw data of microRNA expression are preprocessed using a variance-stabilizing normalization and summarizing the normalized multiple probe signals (technical replicates) to a single expression value, using the median;
wherein a ranker, in particular a Mann-Whitney significance test combined with largest median of pairwise differences as filter for microRNA expression data is used for said feature selection;
wherein logistic regression is selected as suitable classifying algorithm, the training of the classifying algorithm including preprocessed and filtered microRNA expression data and CD34 information (positive or negative), is carried out with an n-fold cross-validation, in particular 5 to 10-fold, preferably 5-fold cross-validation;
applying said trained logistic regression classifier to said preprocessed microRNA expression data set and CD34 information to a subject under suspicion of having AML, and using the trained classifiers to diagnose a specific AML-type.

**8.** Method according to claim 7, **characterized in that** the following DNA probes for targeting said microRNA are used: Seq-ID No. 1 to Seq-ID No. 14;
and/or
the following microRNA-target sequences are used: Seq-ID Nos. 15 to 26.

**9.** Method according to anyone of claims 1 through 8, **characterized in that** said complex disease is colon cancer, said mammalian subject is a human being, said biological sample is colon tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for microRNA expression data is used for said feature selection;
wherein random forests are selected as suitable classifying algorithm, the training of the classifying algorithm including preprocessed and filtered mRNA and microRNA expression data, is carried out with a leave-one-out (LOO) cross-validation;
applying said trained random forests classifier to said preprocessed mRNA and microRNA expression data sets to a subject under suspicion of having colon cancer, and using the trained classifiers to diagnose colon cancer and/or a subtype thereof.

**10.** Method according to claim 9, **characterized in that** the following DNA probes for targeting said microRNA are used: Seq-ID No. 27 to Seq-ID No. 34;
and/or
the following microRNA-target sequences are used: Seq-ID No. 35 to Seq-ID No. 42;
and/or
the following DNA probes for targeting said mRNA are used: Seq-ID No. 43 to Seq-ID No. 264;
and/or
the following target DNA sequences are used: Seq-ID No. 265 to 276.

**11.** Method according to anyone of claims 1 through 6, **characterized in that** said complex disease is kidney cancer, said mammalian subject is a human being, said biological sample is kidney tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Welch t-test (significance test) combined with largest mean of pairwise differences as filter for mRNA and microRNA expression data is used for said feature selection;
wherein single-hidden-layer neural networks are selected as suitable classifying algorithm, the training of the classifying algorithm including preprocessed and filtered mRNA and microRNA expression data, is carried out with a leave-one-out (LOO) cross-validation; applying said trained single-hidden-layer neural networks classifier to said preprocessed mRNA and microRNA expression data sets to a subject under suspicion of having kidney cancer, and using the trained classifiers to diagnose kidney cancer and/or a subtype thereof.

**12.** Method according to claim 11, **characterized in that** the following DNA probes for targeting said microRNA are used: Seq-ID Nos. 33, and 277 to 288;
and/or
the following microRNA-target sequences are used: Seq-ID Nos. 21, 41, 289 to 297;
and/or
the following DNA probes for targeting said mRNA are used: Seq-ID Nos. 298 to 716;
and/or
the following DNA target sequences are used: Seq-ID Nos. 265, 268, 717 to 732.

**13.** Method according to anyone of claims 1 through 6, **characterized in that** said complex disease is prostate cancer, said mammalian subject is a human being, said biological sample is urine and/or prostate tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for mRNA and microRNA expression data is used for said feature selection;
wherein linear discriminant analysis is selected as suitable classifying algorithm, the training of the classifying algorithm including preprocessed and filtered mRNA and microRNA expression data, is carried out with a leave-one-out (LOO) cross-validation;
applying said trained linear discriminant analysis classifier to said preprocessed mRNA and microRNA expression data sets to a subject under suspicion of having prostate cancer, and using the trained classifiers to diagnose prostate cancer and/or a subtype thereof.

**14.** Method according to claim 13, **characterized in that** the following DNA probes for targeting said microRNA are used: Seq-ID Nos 733 to 735;
and/or
the following microRNA-target sequences are used: Seq-ID Nos 736-738; and/or
the following DNA probes for targeting said mRNA are used: Seq-ID No. 739 to Seq-ID No. 892;
and/or
the following DNA target sequences are used: Seq-ID Nos. 893 to 900.

**15.** Method according to anyone of claims 1 through 6, **characterized in that** said complex disease is transient ischemic attack (TIA) and/or ischemia and/or hypoxia, said mammalian subject is a human being, said biological sample blood and/or blood cells and/or cerebrospinal fluid and/or brain tissue;
wherein said different species of biomolecules are mRNA and/or its DNA counterparts and brain metabolites, in particular free prostaglandins, lipoxygenase derived fatty acid metabolites, glutamine, glutamic acid, leucin, alanine, serine, decosahexaenoic acid (DHA), 12(S)-hydroxyeicosatetraenoic acid (12S-HETE);
wherein mRNA expression levels and quantitative and/or qualitative molecular metabolite patterns (metabolomics data) are used as said parameters of step b);
wherein raw data of mRNA expression are preprocessed using actin-ß as reference genes and metabolomics data of said brain metabolites are preprocessed by a variance stabilizing transformation via the binary logarithm (i.e. to base 2);
wherein a ranker, in particular a Welch t-test (significance test) combined with largest mean of pairwise differences as filter for metabolomics data is used for said feature selection;
wherein support vector machines are selected as suitable classifying algorithm, the training of the classifying algorithm including preprocessed and filtered mRNA and microRNA expression data, is carried out with a leave-one-out (LOO) cross-validation;
applying said trained support vector machines classifier to said preprocessed mRNA expression data and said metabolomics data sets to a subject under suspicion of having ischemia and/or hypoxia, and using the trained classifiers to diagnose ischemia and/or hypoxia and/or the grades thereof.

**16.** Method according to claim 15, **characterized in that** the samples are analyzed by solid phase extraction liquid chromatography tandem mass spectrometry (online SPE-LC-MS/MS), wherein preferably a C18 column is used as solid phase extraction column; and wherein the quantification of the measured metabolite concentrations in said biological tissue sample preferably is calibrated by reference to internal standards and by using an electrospray ionization multiple reaction monitoring tandem mass spectrometry detection mode.

**17.** Method according to anyone of claims 15 through 17, **characterized in that** the mRNA expression data are obtained by quantitative real time PCR (q-RT-PCR);
and/or
the following primer pairs are used: Seq-ID Nos. 901 to 906; and/or the following DNA target sequences are used: Seq-ID Nos. 265, 907 and 908.

**18.** Kit for carrying out a method in accordance with anyone of claims 1 to 17, in a biological sample, comprising:
a) detection agents for the detection of at least two different species of biomolecules, wherein said species of biomolecules are selected from the group consisting of: RNA and/or its DNA counterparts, microRNA and/or its DNA counterparts, peptides, proteins, and metabolites;
b) positive and/or negative controls; and
c) classification software for classification of the results achieved with said detection agents.
